# FASCICULE DE BREVET EUROPEEN

(11) **EP 2 480 672 B1**
(45) Date de publication et mention de la délivrance du brevet: **28.12.2016**
(21) Numéro de dépôt: 10769025.7
(22) Date de dépôt: 23.09.2010
(51) Int. Cl.: C12N 15/62

(54) **NOUVEAUX POLYNUCLEOTIDES ET POLYPEPTIDES CHIMERIQUES PERMETTANT LA SECRETION D'UN POLYPEPTIDE D'INTERET EN ASSOCIATION AVEC DES EXOSOMES ET LEURS UTILISATIONS**
NEUE CHIMÄRE POLYNUKLEOTIDE UND POLYPEPTIDE ZUR ABSCHEIDUNG EINES POLYPEPTIDS VON INTERESSE IN KOMBINATION MIT EXOSOMEN UND VERWENDUNGEN DAVON
NOVEL CHIMERIC POLYNUCLEOTIDES AND POLYPEPTIDES ENABLING THE SECRETION OF A POLYPEPTIDE OF INTEREST IN COMBINATION WITH EXOSOMES AND USES THEREOF

(30) Priorité: 24.09.2009 FR 0904576
(43) Date de publication de la demande: 01.08.2012
(73) Titulaire: Centre National de la Recherche Scientifique, 75794 Paris Cedex 16 (FR); Université Montpellier 2 Sciences et Techniques, 34095 Montpellier Cedex 5 (FR)
(72) Inventeur: MAMOUN, Robert, Zaine El Abiddine, F-34725 St André de Sangonis (FR)
(74) Mandataire: Desaix, Anne
(86) Numéro de dépôt international: PCT/FR2010/052006
(87) Numéro de publication internationale: WO 2011/036416

(56) Documents cités:
- WO-A1-2009/115561
- NOVAKOVIC S ET AL: "Dileucine and YXXL motifs in the cytoplasmic tail of the bovine leukemia virus transmembrane envelope protein affect protein expression on the cell surface", JOURNAL OF VIROLOGY, THE AMERICAN SOCIETY FOR MICROBIOLOGY, US LNKD- DOI:10.1128/JVI.78.15.8301-8311.2004, vol. 78, no. 15, 1 août 2004 (2004-08-01), pages 8301-8311, XP002508133, ISSN: 0022-538X
- DE GASSART A ET AL: "Exosomal sorting of the cytoplasmic domain of bovine leukemia virus TM Env protein", CELL BIOLOGY INTERNATIONAL, ACADEMIC PRESS, GB LNKD- DOI:10.1016/J.CELLBI.2008.10.001, vol. xx, 1 janvier 2008 (2008-01-01), pages 1-13, XP002508299, ISSN: 1065-6995 [extrait le 2008-10-01]
- REUTHER G W ET AL: "Analysis of function and regulation of proteins that mediate signal transduction by use of lipid-modified plasma membrane-targeting sequences", METHODS IN ENZYMOLOGY 2000 US LNKD- DOI:10.1016/S0076-6879(00)27288-1, vol. 327, 2000, pages 331-350, XP009132699, ISSN: 0076-6879
- SILVERMAN LAUREN ET AL: "Lysine residues form an integral component of a novel amino-terminal membrane targeting motif for myristylated pp60-v-src", JOURNAL OF CELL BIOLOGY, vol. 119, no. 2, 1992, pages 415-425, XP002579784, ISSN: 0021-9525

## Description

La demande et l'invention concernent des polynucléotides et polypeptides chimériques permettant la sécrétion d'un polypeptide d'intérêt en association avec des exosomes, et leur utilisation notamment pour la production de compositions immunogènes à base de polypeptides chimériques, d'ADN ou d'exosomes, pour le criblage d'interactions protéiques, ou pour le transport de protéines ou d'acides nucléiques.

La demande et l'invention concernent un polypeptide chimérique capable d'être sécrété en association avec des exosomes lorsqu'il est exprimé dans des cellules eucaryotes appropriées, ledit polypeptide chimérique comprenant plusieurs domaines polypeptidiques.

La demande et l'invention concernent également une vésicule membranaire, en particulier un exosome, qui comporte un polypeptide de la demande ou l'invention, une composition immunogène à base de tels exosomes et un procédé de production de tels exosomes.

La demande et l'invention concernent également un polynucléotide codant pour un polypeptide de la demande ou l'invention et une composition immunogène le comprenant, en particulier un vaccin à ADN le comprenant.

La demande et l'invention concernent également la mise en oeuvre des propriétés des vésicules membranaires et des compositions immunogènes de la demande ou l'invention pour la prophylaxie et/ou le traitement d'une infection par un agent pathogène, un organisme pathogène, un antigène tumoral ou un antigène cytoplasmique, en particulier pour éliciter ou favoriser *in vivo,* chez un hôte (humain ou non humain), une réponse humorale et/ou cellulaire contre un virus, une bactérie, un parasite ou une tumeur.

La demande et l'invention concernent en outre l'utilisation d'exosomes comportant un polypeptide de la demande ou l'invention comme outil de diagnostic.

La demande et l'invention concernent également la mise en oeuvre des propriétés des vésicules membranaires et des compositions protéiques de la demande ou l'invention pour la prophylaxie et/ou le traitement d'une maladie due à un déficit fonctionnel ou métabolique, en particulier pour transporter une protéine ou un acide nucléique, en particulier pour compenser ou suppléer un déficit enzymatique, ou en particulier pour induire une modification transcriptionnelle ou traductionnelle dans les cellules ou les organes visées, ou pour modifier le métabolisme cellulaire.

La demande et l'invention concernent également l'utilisation de vésicules membranaires de la demande ou l'invention pour produire des anticorps dirigés contre le peptide ou le polypeptide d'intérêt.

La demande et l'invention concernent également l'utilisation de vésicules membranaires de la demande ou l'invention pour étudier des interactions entre protéines et pour étudier des molécules interagissant avec des protéines d'intérêt.

Les exosomes se présentent sous la forme de petites sphères limitées par une bicouche lipidique. Ces vésicules membranaires sont naturellement sécrétées par différents types de cellules, en particulier par les cellules épithéliales, des cellules tumorales et certaines cellules du système immunitaire, (mastocytes, lymphocytes T et B, cellules dendritiques, notamment cellules de Langerhans). Les exosomes se distinguent d'autres vésicules membranaires sécrétées par les cellules notamment par leur petite taille (de 50 à 100 nm de diamètre) et par leur composition en protéines membranaires (des molécules d'adhésion, de transport, de transduction de signal et des molécules du complexe majeur d'histocompatibilité entre autres).

Les exosomes pourraient notamment correspondre à des vésicules internes des endosomes multivésiculaires (notamment des endosomes tardifs) sécrétées par la cellule lors de la fusion de ces endosomes avec la membrane plasmique ; les endosomes multivésiculaires sont généralement impliqués dans le transport de molécules dans les compartiments lysosomiques (voie de dégradation des protéines) mais, dans certaines cellules telles que les réticulocytes et certaines cellules présentatrices d'antigènes, ils pourraient être dirigés vers la membrane plasmique, avec laquelle ils fusionneraient pour libérer des exosomes dans le milieu extracellulaire.

Des études antérieures ont démontré que les exosomes sont capables d'induire des réponses immunes humorales et/ou cellulaires (Delcayre *et al.,* 2002). Les exosomes sont considérés comme des vecteurs d'antigènes capables de stimuler directement *in vitro* des lymphocytes T de manière antigène spécifique. En effet, les exosomes sécrétés par les cellules dendritiques expriment des molécules du complexe majeur d'histocompatibilité (CMH) de classe I et II. Les complexes peptides/CMH fonctionnels portés par les exosomes seraient transférés d'une cellule dendritique à une autre qui n'a jamais vu l'antigène dont dérivent les peptides associés aux molécules du CMH. En stimulant de proche en proche les cellules dendritiques naïves, les exosomes sécrétés et présentant à leur surface un peptide antigénique contribueraient à l'amplification de la réponse T CD4 et T CD8 spécifique (Delcayre et Le Pecq, 2006). Par exemple, chargés avec des peptides tumoraux et injectés à des souris, ces exosomes sont capables de promouvoir une forte réponse immunitaire et de faire régresser des tumeurs solides pré-établies (Zitvogel et al., 1998).

Les exosomes sont capables de présenter des antigènes exogènes soit sous la forme de protéines entières natives, soit sous la forme de peptides associés aux molécules du CMH I et II (Colino et Snapper 2006). La présentation d'antigène à la surface d'exosomes est similaire à une présentation à la membrane d'une cellule ou d'un virus enveloppé. Toutefois, les exosomes n'étant ni vivants ni infectieux, ils présentent l'avantage de pouvoir être manipulés comme un produit ordinaire et sans précautions de confinement, contrairement à un virus. Les exosomes peuvent donc être utilisés comme présentateurs de peptides ou de polypeptides antigéniques à des fins d'immunisation. Cette technique dite « exosome display » ne requiert pas nécessairement la présentation directe de l'antigène par le CMH (Chaput et al., 2004). Néanmoins le développement de cette technique de vaccination inédite suppose de disposer d'un « outil » moléculaire efficace permettant le ciblage des protéines antigéniques avecles exosomes. Or, à ce jour, un tel « outil » n'a pas été décrit.

L'étude des rétrovirus, et plus particulièrement du virus d'immunodéficience humaine (VIH), a révélé leur capacité à détourner la machinerie cellulaire de biogenèse des endosomes multivésiculaires afin de bourgeonner à la membrane plasmique (Pornillos *et al.,* 2002). Ces virus peuvent également utiliser cette machinerie au niveau de la membrane endosomale, son site normal de fonctionnement (Raposo et al., 2002). Ainsi, selon l'hypothèse des « exosomes chevaux de Troie » (Trojan Exosomes ; Gould et al., 2003), ces virus utiliseraient la voie préexistante de biogenèse des exosomes, pour la formation de particules virales.

L'enveloppe des rétrovirus est constituée de la glycoprotéine d'enveloppe externe (SU) et de la glycoprotéine trans-membranaire (TM). Ces glycoprotéines d'enveloppe sont issues du clivage de la protéine précurseur nommée ENV. L'expression du gène *env* aboutit à la synthèse des glycoprotéines d'enveloppe des rétrovirus, sous forme d'un précurseur protéique qui transite par le Golgi avant d'atteindre la portion de membrane (endosomale ou plasmique), qui servira d'enveloppe virale lors du bourgeonnement des virions. Lors de ce trajet, ce précurseur oligomérique est glycosylé et clivé en glycoprotéines de surface (SU) et transmembranaire (TM). Les protéines SU et TM demeurent associées et sont ancrées dans la membrane vésiculaire ou cellulaire par l'intermédiaire d'une hélice hydrophobe transmembranaire de la protéine TM.

La glycoprotéine TM des rétrovirus présente de multiples facettes dues à l'association de ses domaines externe, transmembranaire et cytoplasmique (CD™), qui en font la seule protéine des rétrovirus permettant une communication de part et d'autre des membranes du virus et de la cellule infectée (Cann et al., 1992; Delamarre et al., 1996). Elle est notamment impliquée dans le phénomène de pénétration du virus dans la cellule cible, en provoquant la fusion entre les membranes virales et cellulaires. De plus, lors de son cheminement intracellulaire, la protéine TM est susceptible d'influer sur le tri, le devenir, le ciblage et le bourgeonnement des particules virales, par interactions avec le cytosquelette, ainsi qu'avec les machineries d'ubiquitination et de bourgeonnement (Cann et al., 1992 ; Delamarre et al.,1996 ; Straub et Levy, 1999).

Une étude antérieure suggère que le domaine cytoplasmique de la protéine TM de deux rétrovirus, le virus de la leucémie bovine (BLV, pour « Bovine Leukemia Virus ») et le VIH, permet d'induire l'adressage et la sécrétion d'une protéine recombinante dans les exosomes (De Gassart et al., 2004 ; 2009). Des cellules de la lignée K562 (une lignée cellulaire érythroleucémique d'origine humaine), qui sécrètent des exosomes de façon constitutive, ont été transfectées avec des vecteurs rétroviraux permettant d'exprimer, en système eucaryote, deux types de protéine chimère : (i) une chimère comportant le domaine extracellulaire de la protéine CD8 murine et les domaines transmembranaire et cytoplasmique de la glycoprotéine TM du BLV (chimère TM-BLV/CD8 ; De Gassart et al., 2004 ; 2009), et (ii) une chimère comportant les domaines extracellulaire et transmembranaire de la protéine CD8 murine et le domaine cytoplasmique de la protéine TM du VIH (chimère TM-HIV/CD8 ; De Gassart et al.,2004). Les deux chimères sont exprimées à la fois dans les cellules K562 transfectées et dans les exosomes sécrétés par ces cellules. En particulier, l'expression de la protéine chimère TM-BLV/CD8 dans les cellules de la lignée K562 disparaît rapidement après transit dans le réseau trans-golgien et les compartiments endosomaux tardifs pour se retrouver dans les exosomes sécrétés par ces cellules. II semble que la chimère comportant le domaine cytoplasmique de la protéine TM du BLV soit plus fortement adressée vers les exosomes que la chimère comportant le domaine cytoplasmique de la protéine TM du VIH.

Cependant, comme le prouvent les résultats présentés dans la partie exemple de la demande, des exosomes portant la construction chimère TM-BLV/CD8 décrite par De Gassart *et al.* ne sont produits efficacement que dans les cellules de la lignée K562, et sont peu ou pas produits efficacement dans d'autres types cellulaires tels que les cellules de la lignée HEK293.

La demande et l'invention concernent de nouveaux polypeptides chimériques ayant des propriétés d'adressage dans les exosomes. Les polypeptides chimériques de la demande ou l'invention comportent uniquement des domaines cytosoliques et/ou des domaines nucléaires et s'ancrent dans la membrane des vésicules membranaires, en particulier des exosomes, sans s'insérer dans la bicouche lipidique d'une membrane vésiculaire ou cellulaire, ni traverser cette bicouche lipidique. La demande décrit également d'autres polypeptides chimériques capables de s'ancrer dans la membrane des vésicules membranaires, en particulier des exosomes. Ces polypeptides chimériques additionnels comprennent un ou plusieurs domaine(s) membranaire(s), en particulier au moins un domaine transmembranaire. La fonction d'ancrage dans les membranes vésiculaires ou cellulaires de ces polypeptides chimériques additionnels est assurée par leur domaine(s) membranaire(s), qui leur permettent de s'insérer dans la bicouche lipidique d'une membrane vésiculaire ou cellulaire, et le cas échéant de la traverser en partie.

Selon un aspect particulier, ces polypeptides chimériques additionnels sont utilisés conjointement avec les polypeptides chimériques de la demande ou l'invention.

Les polypeptides chimériques décrits dans cette demande et en particulier les polypeptides chimériques de l'invention se distinguent notamment de ceux décrits dans l'art antérieur et, en particulier de ceux décrits dans De Gassart et al, en ce qu'ils permettent d'adresser de façon beaucoup plus efficace un peptide ou un polypeptide d'intérêt vers des exosomes produits par les cellules de la lignée HEK293 et ainsi, d'amplifier très fortement (de 10 à 100 fois) la production d'exosomes comportant ledit peptide ou polypeptide d'intérêt. Par ailleurs, contrairement aux constructions décrites dans De Gassart et al, qui ne peuvent être utilisées efficacement que dans des cellules de la lignée K562, ces polypeptides peuvent être adressés à la membrane d'exosomes et sécrétés en association avec des vésicules membranaires, en particulier des exosomes produit(e)s par différents types cellulaires, en particulier des cellules HEK293 ou des lymphocytes T ou des lymphocytes B, et pas uniquement par des cellules de la lignée K562. La demande ou invention permet notamment de produire des quantités importantes de vésicules membranaires, en particulier d'exosomes, utilisables comme outils de recherche mais aussi en diagnostic, dans des applications médicales et en particulier en immunisation et notamment en vaccination.

De telles vésicules membranaires (notamment exosomes) pourraient également être produites *in vivo* par un hôte humain ou non humain (en particulier un mammifère humain ou non humain ou un oiseau), que l'on souhaite par exemple immuniser, et en particulier vacciner, en administrant, à cet hôte, une composition et en particulier une composition immunogène dont le principe actif est un polynucléotide codant pour un polypeptide de la demande ou l'invention, en particulier une composition immunogène à base d'ADN, et plus particulièrement un vaccin à ADN, ou une composition immunogène dont le principe actif consiste en des vésicules membranaires (en particulier des exosomes) comportant un polypeptide de la demande ou l'invention.

La demande décrit un polypeptide chimérique caractérisé en ce qu'il comprend ou consiste en les domaines suivants:
- (i) un peptide ou polypeptide d'intérêt ;
- (ii) un domaine de ciblage sous-membranaire; et
- (iii) un domaine cytoplasmique (CD) d'une protéine membranaire, permettant, dans des cellules eucaryotes, l'adressage dudit polypeptide chimérique vers les vésicules membranaires, en particulier vers les vésicules formant des exosomes, et/ou vers le(s) compartiment(s) cellulaire(s) impliqué(s) dans la formation des vésicules membranaires, et en particulier des vésicules formant les exosomes, ou un dérivé muté de ce domaine CD, ce dérivé muté étant défini par la substitution, la délétion et/ou l'insertion d'un ou plusieurs résidu(s) dans la séquence du domaine CD de référence et ce dérivé muté conservant la susdite capacité d'adressage du domaine CD, le domaine CD ou son dérivé muté comprenant au moins un motif YxxL, et un motif PxxP, dans x représente un résidu quelconque,
étant entendu que les domaines présents dans le polypeptide chimérique, en particulier les domaines (i), (ii) et (iii), sont des domaines cytosoliques ou nucléaires (par exemple, les domaines (i) et (iii) sont cytosoliques) et que ledit polypeptide chimérique est dépourvu de peptide signal d'importation dans le réticulum endoplasmique. Le terme « résidu » tel qu'utilisé dans la présente demande ou invention fait référence à un résidu d'acide aminé. Ces résidus sont indiqués en utilisant le code abrégé à une lettre, par exemple, Y pour un résidu tyrosine, L pour un résidu leucine, P pour un résidu proline et x pour un résidu quelconque.

Un objet de l'invention est un polypeptide chimérique capable d'être sécrété en association avec des exosomes, caractérisé en ce qu'il comprend ou consiste en les domaines suivants:
(i) un peptide ou polypeptide d'intérêt ;
(ii) un domaine de ciblage sous-membranaire, permettant audit polypeptide chimérique d'être ancré à la membrane d'un exosome par l'intermédiaire d'une ou plusieurs molécule(s) d'ancrage et/ou d'interactions entre ledit domaine de ciblage sous-membranaire et ladite membrane, mais sans s'insérer ni traverser la bicouche lipidique de la membrane de cet exosome, la séquence dudit domaine de ciblage sous-membranaire étant la séquence d'un fragment d'une protéine de la famille Src et comprenant ou consistant en la séquence M-G-S-S-K-S-K-P-K-D-P-S-Q-R-R-R ou en la séquence M-G-S-S-K-S-K-P-K-D-P-S-Q-R-R-R-K-S-R-G-P-G-G; et
(iii) un dérivé muté du domaine cytoplasmique (CD) d'une protéine membranaire, ce dérivé muté étant défini par la substitution, la délétion et/ou l'insertion d'un ou plusieurs résidu(s) dans la séquence dudit domaine CD, ledit domaine CD étant le domaine CD de SEQ ID NO : 6, qui est le domaine CD de la protéine transmembranaire (TM) du virus de la leucémie bovine (BLV), ledit dérivé muté ayant conservé la capacité dudit domaine CD à adresser ledit polypeptide chimérique aux exosomes et/ou à un(des) compartiment(s) cellulaire(s) impliqué(s) dans la formation des exosomes, ledit dérivé muté de domaine CD comprenant au moins un motif YxxL ou DYxxL, et un motif PxxP, dans lequel x représente un résidu quelconque et Y, L, D et P représentent respectivement un résidu tyrosine, leucine, acide aspartique et proline, la séquence dudit dérivé muté étant identique à au moins 80% à la séquence de SEQ ID NO : 8, et étant différente de la séquence de SEQ ID NO : 6 par au moins l'une des caractéristiques (a)-(b) suivantes :
   (a) ledit dérivé muté est dépourvu de la séquence KCLTSRLLKLLRQ qui est contenue dans la séquence de SEQ ID NO : 6,
   (b) dans ledit dérivé muté, le résidu cystéine de la séquence PCP qui est contenue dans SEQ ID NO : 6 est délété ou est remplacé par le résidu alanine ;
   étant entendu que lesdits domaines (i) et (ii) et (iii) sont des domaines cytosoliques ou nucléaires et que ledit polypeptide chimérique est dépourvu de peptide signal d'importation dans le réticulum endoplasmique, et dans lequel les domaines (i) à (iii) sont positionnés successivement dans l'ordre suivant, de l'extrémité N-terminale vers l'extrémité C-terminale dans le polypeptide : (ii) - (i)-(iii), ou (ii) - (iii) - (i).

Le motif PXXP dans le domaine CD ou son dérivé muté est de préférence le motif PSAP (SEQ ID NO. 88) ou le motif PTAP (SEQ ID NO. 89).

Le polypeptide chimérique de la demande ou l'invention est en particulier capable d'être sécrété en association avec des vésicules membranaires, en particulier avec des exosomes, lorsqu'il est exprimé dans des cellules eucaryotes appropriées. Lorsque des exosomes comportant le polypeptide de la demande ou l'invention sont sécrétés, le peptide ou le polypeptide d'intérêt se trouve inclus (en totalité) dans la fraction cytosolique de ces exosomes. Ceci permet d'attester notamment que le polypeptide chimérique de la demande ou l'invention est dépourvu de peptide signal qui permettrait l'importation dans le réticulum endoplasmique.

Selon un mode de réalisation particulier de la demande, les domaines (i) à (iii) sont positionnés successivement dans l'ordre suivant, de l'extrémité N-terminale vers l'extrémité C-terminale dans le polypeptide chimérique de la demande : domaine de ciblage sous-membranaire (ii) - peptide ou polypeptide d'intérêt (i) - domaine CD ou son dérivé muté (iii).

Alternativement, les domaines (i) à (iii) peuvent être positionnés dans un ordre différent, par exemple dans l'ordre suivant : domaine de ciblage sous-membranaire (ii) - domaine CD ou son dérivé muté (iii) - peptide ou polypeptide d'intérêt (i). L'un ou l'autre des domaines (ii) ou (iii) ou les deux domaines (ii) et (iii) peuvent aussi être positionnés entre deux domaines différents ou identiques de type (i), ils peuvent être aussi insérés à l'intérieur d'un domaine (i), par exemple dans l'ordre N-terminal de (i) - (ii) [ou (iii)] - (iii) [ou (ii)] - fragment C-terminal restant de (i).

Le terme « chimérique » désigne ici un polypeptide qui associe plusieurs domaines, d'au moins deux types différents par leur fonction et/ou par leur localisation cellulaire, au moins deux de ces domaines provenant de molécules distinctes, en particulier provenant soit de différentes protéines d'une même espèce ou d'espèces différentes soit d'une même protéine de différentes espèces.

Un « polypeptide chimérique » tel que défini dans la demande ou invention peut être le produit d'expression d'un polynucléotide recombinant et peut être exprimé par voie recombinante dans une cellule hôte. Ledit polypeptide chimérique est donc par exemple un polypeptide de fusion.

Par « domaine » d'une protéine ou d'un polypeptide on entend une région ayant une propriété fonctionnelle et/ou une localisation, cellulaire pour ladite protéine ou ledit polypeptide.

Le terme « domaine de ciblage sous-membranaire » ou « domaine d'adressage à la membrane » ou « domaine de recrutement à la membrane » (ou d'association aux membranes cellulaires et vésiculaires) désigne, dans la demande ou invention, un domaine capable, dans une cellule et en particulier dans une cellule eucaryote (par exemple une cellule productrice d'exosome), de s'ancrer à une membrane cellulaire et/ou une membrane vésiculaire sans être inséré dans ladite ou lesdites membrane(s), l'ancrage à la membrane ou aux membranes pouvant être assuré au moyen d'une ou plusieurs molécule(s) d'ancrage (ledit domaine étant capable de lier ladite ou lesdites molécule(s) d'ancrage) et/ou par des interactions (en particulier des interactions électrostatiques) entre ledit domaine de ciblage sous-membranaire et ladite ou lesdites membrane(s). Selon un mode de réalisation particulier, ledit domaine est capable, dans une cellule et en en particulier dans une cellule eucaryote (par exemple une cellule productrice d'exosome), de se fixer à ou d'interagir avec la face interne (c'est-à-dire cytoplasmique) de la membrane plasmique et/ou de vésicules membranaires, par l'intermédiaire d'une ou plusieurs molécule(s) d'ancrage et/ou par des interactions (en particulier des interactions électrostatiques).

Le terme « ciblage sous-membranaire », « adressage à la membrane » ou « recrutement à la membrane », lorsqu'il est appliqué au domaine (ii) présent dans le polypeptide chimérique de la demande ou l'invention, implique une capacité à interagir (par l'intermédiaire d'une ou plusieurs molécule(s) d'ancrage et/ou par des interactions, en particulier des interactions électrostatiques) avec une membrane cellulaire (en particulier la membrane plasmique) et/ou une membrane vésiculaire. Le cas échéant (mais pas nécessairement), ce terme implique également une prise en charge cellulaire active permettant d'amener ledit domaine (ii) et, par conséquent, un polypeptide chimérique comprenant ledit domaine (ii), à proximité d'une membrane cellulaire (en particulier la membrane plasmique) ou vésiculaire, de telle sorte que des interactions avec ladite membrane deviennent possibles.

Le domaine de ciblage sous-membranaire est suffisant pour permettre l'ancrage du polypeptide chimérique de la demande ou l'invention à la bicouche lipidique de membranes cellulaires ou vésiculaires (par l'intermédiaire d'une ou plusieurs molécule(s) d'ancrage et/ou par des interactions). Ainsi, de part sa présence dans le polypeptide chimérique de la demande ou l'invention, le domaine de ciblage sous-membranaire permet au polypeptide chimérique de la demande ou l'invention exprimé dans une cellule et en particulier une cellule eucaryote, d'être ancré à (parfois indiqué par l'expression « ancré dans ») une membrane cellulaire ou vésiculaire, sans que ledit polypeptide soit inséré dans ladite membrane. Selon un mode de réalisation préféré de la demande ou l'invention, le domaine de ciblage sous-membranaire confère au polypeptide chimérique de la demande ou l'invention la propriété de se fixer (par l'intermédiaire d'une ou plusieurs molécule(s) d'ancrage et/ou par des interactions, en particulier des interactions électrostatiques) à la membrane vésiculaire et cellulaire et en particulier à la face interne de la membrane plasmique et de vésicules membranaires.

Selon un mode de réalisation particulier de la demande ou l'invention, le domaine de ciblage sous-membranaire comporte 5 à 40 résidus, de préférence 8 à 25 ou 12 à 25 résidus et plus préférablement 14 à 25 ou 16 à 23 résidus, par exemple 8, 9, 10, 11, 12, 13, 14, 15, 17, 18, 19, 20 , 21, 22, 23, 24, ou 25 résidus.

Par « molécule d'ancrage », on entend, dans la demande ou invention, toute molécule susceptible de s'insérer dans la bicouche lipidique (en particulier dans au moins un feuillet de la bicouche lipidique) d'une membrane cellulaire ou vésiculaire et en particulier un lipide ou une molécule lipidique (c'est-à-dire une molécule comportant un ou plusieurs lipide(s)) ; le domaine de ciblage sous-membranaire et le polypeptide chimérique de la demande ou l'invention sont alors dits « à ancrage lipidique ».

Selon un mode de réalisation particulier de la demande ou l'invention, la molécule d'ancrage au sens de la demande ou l'invention comprend ou consiste en un ou plusieurs lipide(s), ledit ou lesdits lipides comportant une chaîne carbonée hydrophobe lui (leur) permettant de s'enchâsser dans la bicouche lipidique d'une membrane cellulaire ou vésiculaire.

Selon un mode de réalisation particulier de la demande ou l'invention, le(s) lipide(s) présent(s) dans la molécule d'ancrage est (sont) choisi(s) parmi les acides gras, par exemple parmi les acides myristiques, les acides palmitiques, et les isoprénoides, en particulier les géranyl-géranyl et les farnésyl.

Selon un mode de réalisation particulier de la demande ou l'invention, la ou les molécule(s) d'ancrage est (sont) liée(s) au domaine de ciblage sous-membranaire présent dans le polypeptide chimérique de la demande ou l'invention par une liaison covalente.

Selon un mode de réalisation particulier de la demande ou l'invention le terme domaine de ciblage sous-membranaire » permet au polypeptide chimérique de la demande ou l'invention d'être ancré à la bicouche lipidique par l'intermédiaire d'un ou plusieurs acide(s) gras, en particulier un ou plusieurs acide(s) gras choisi(s) parmi les acides myristiques, les acides palmitiques et les géranyl-géranyl, et/ou par l'intermédiaire d'un peptide ou d'une structure peptidique capable d'interagir avec un ou plusieurs lipide(s) ou motif(s) lipidique(s) présent(s) dans une bicouche lipidique, en particulier avec un phospholipide. Ainsi, le polypeptide chimérique de la demande ou l'invention est ancré aux membranes cellulaires et vésiculaires sans être inséré dans lesdites membranes.

Selon un mode de réalisation préféré, ce domaine est suffisant pour lier un acide gras et en particulier un acide myristique, un acide palmitique ou un géranyl-géranyl. Cette liaison peut se faire notamment sur un résidu G (par exemple dans le cas d'un acide myristique), C ou S du domaine de ciblage sous-membranaire. Il peut notamment s'agir d'une liaison amide ou thioester. L'acide gras, en particulier l'acide myristique peut se lier au domaine de ciblage sous-membranaire par une liaison covalente.

Selon un mode de réalisation particulier de la demande, le domaine de ciblage sous-membranaire comprend ou consiste en un peptide ou une structure peptidique (c'est à dire une structure comprenant un ou plusieurs résidu(s) d'acides aminés et de préférence un ou plusieurs enchaînement(s) d'au moins deux résidu(s) d'acides aminés consécutifs) capable d'interagir avec un ou plusieurs lipide(s) (en particulier un ou plusieurs acide(s) gras) ou avec un ou plusieurs motif(s) lipidique(s) (en particulier un ou plusieurs motif(s) lipidique(s) comprenant ou consistant en un ou plusieurs acide(s) gras)) présent(s) dans une bicouche lipidique, en particulier avec un phospholipide.

Le domaine de ciblage sous-membranaire et le peptide ou le polypeptide d'intérêt du polypeptide chimérique de la demande ou l'invention peuvent être dérivés ou non d'une même entité, en particulier d'une même protéine.

Selon un mode de réalisation préféré, le domaine de ciblage sous-membranaire est celui d'une protéine membranaire extrinsèque ou est un dérivé muté du domaine de ciblage sous-membranaire d'une protéine membranaire extrinsèque.

Selon un mode de réalisation préféré, le « domaine de ciblage sous-membranaire » comporte ou consiste une séquence consensus permettant l'attachement, par exemple par acylation ou par prénylation, d'un acide gras et en particulier acide myristique, d'un acide palmitique ou d'un géranyl-géranyl.

Ladite séquence consensus peut comprendre ou consister en la séquence suivante : M-G-X₁-X₂-X₃-S/C (dans laquelle X₁ X₂, et X₃ désignent indépendamment un résidu quelconque), en particulier dans le cas où elle permet l'attachement d'un acide myristique. Lorsqu'un acide gras se lie à cette séquence consensus, c'est généralement sur le résidu G, par exemple en position 2.

Selon un mode de réalisatrion particulier,
- X₁ est choisi parmi C, S et L ; et/ou
- X₂ est choisi parmi S , I, V, M et L ; et/ou
- X₃ est choisi parmi K, Q, H, F, C et S.

Lorsque ladite séquence consensus permet l'attachement d'un acide myristique, elle est de préférence localisée en position N-terminale (par exemple en position 2) dans le domaine de ciblage sous-membranaire, et donc dans le polypeptide de la demande.

Selon un mode de réalisation préféré, le « domaine de ciblage sous-membranaire » comporte plusieurs résidus d'acides aminés basiques, en particulier plusieurs résidus choisis parmi K, R et H. « Plusieurs » signifie ici au moins deux, et de préférence au moins trois, par exemple, deux, trois quatre, cinq, ou plus de cinq.

Ces acides aminés peuvent être notamment impliqués dans des interactions avec des lipides des membranes cellulaires ou vésiculaires, notamment avec la choline (par exemple avec la phosphatidyl choline) et permettent ainsi d'augmenter l'affinité du ciblage sous-membranaire pour ces membranes.

Les acides aminés basiques du domaine de ciblage sous-membranaire peuvent être localisés dans la séquence consensus décrite ci-dessus et/ou en dehors de cette séquence consensus.

Ainsi, selon un mode de réalisation particulier de la demande, le domaine de ciblage sous-membranaire de la demande :
- comprend ou consiste en une séquence choisie parmi les séquences suivantes :M-G-x-x-K-S/C-K-x-K et M-G-x-x-K-S/C-K-x-K-x-x-x-x-R-R-R dans lesquelles x désigne un résidu quelconque) ; une telle séquence peut par exemple permettre l'attachement d'un acide myristique (par exemple en position 2), ou
- consiste en un dérivé muté de cette séquence, ledit dérivé muté conservant la capacité de ce domaine à à s'y ancrer dans la bicouche lipidique d'une membrane cellulaire.

La séquence du domaine de ciblage sous-membranaire du polypeptide chimérique de l'invention est la séquence d'un fragment d'une protéine de la famille Src et comprend ou consiste en la séquence M-G-S-S-K-S-K-P-K-D-P-S-Q-R-R-R ou en la séquence M-G-S-S-K-S-K-P-K-D-P-S-Q-R-R-R-K-S-R-G-P-G-G.

Le domaine de ciblage sous-membranaire du polypeptide de la demande ou l'invention peut être notamment issu d'une protéine de la famille des protéines Src, en particulier d'une protéine choisie parmi les protéines Src, Yes, Lyn, Fyn, Lck, Blk, Fgr, Hck et Yrk (Resh, 1994), et plus particulièrement de la partie N-terminale d'une de ces protéines. Par exemple ce domaine peut être issu de la protéines c-Src ou v-Src (de préférence c-Src). Ce domaine peut par exemple comprendre ou consister en les 15, 16, 17, 18, 19, 20, 21, 22, 23, 24 ou 25 acides aminés N-terminaux d'une de ces protéines, et en particulier de la protéine Src.

Alternativement, le domaine de ciblage sous-membranaire du polypeptide de la demande peut être issu d'autres protéines acylées, en particulier de protéines de capsides virales, exemple protéine MA du virus de l'immunodéficience humaine (VIH), ou de protéines de filovirus.

Ainsi, le domaine de ciblage sous-membranaire du polypeptide de la demande peut être issu d'une protéine Src, et en particulier comporter ou consister en la séquence suivante : M-G-S-S-K-S-K-P-K-D-P-S-Q-R-R-R (SEQ ID NO.:104) ou M-G-S-S-K-S-K-P-K-D-P-S-Q-R-R-R-K-S-R-G-P-G-G (SEQ ID NO.:105) ou consister en un dérivé muté du domaine de séquence M-G-S-S-K-S-K-P-K-D-P-S-Q-R-R-R ou M-G-S-S-K-S-K-P-K-D-P-S-Q-R-R-R-K-S-R-G-P-G-G.

Le terme « issu d'une protéine particulière » indique, dans la demande ou invention que le peptide ou polypeptide visé comprend ou consiste en cette protéine particulière, ou en un fragment de ladite protéine particulière, ou un dérivé muté de ladite protéine ou du fragment par substitution ou délétion d'acides aminés.

Parmi d'autres peptides ou structures peptidiques capables d'interagir avec un ou plusieurs lipide(s) ou un motif lipidique particulier, qui permettent de cibler la face interne de membranes cellulaires ou vésiculaires, on peut citer les domaines FYVE (Hurley, JH ; 2006). Ainsi, selon un mode de réalisation particulier de la demande, le domaine de ciblage sous-membranaire comprend ou consiste en un ou plusieurs domaine(s) FYVE.

L'expression « vésicule membranaire » désigne au sens de la demande toute vésicule composée d'une bicouche lipidique renfermant une fraction cytosolique telle que produite par des cellules eucaryotes. Cette expression inclut notamment les vésicules sécrétées dans l'espace extracellulaire, c'est-à-dire les exosomes.

Par « exosomes », on entend, dans la demande ou invention, des nanovésicules des membranes cellulaires telles que définies ci-dessus. Ces exosomes peuvent être purifiés à partir des surnageants de culture de cellules par centrifugation différentielle, par ultra-filtration ou par adsorption sur un support ou par tout autre procédé, comme illustré dans les exemples.

L'expression « sécrété en association avec des vésicules membranaires, en particulier avec des exosomes » signifie, dans la demande ou invention, qu'un polypeptide chimérique de la demande ou l'invention et/ou au moins un de ses produits de dégradation est sécrété dans l'espace extracellulaire, non pas sous forme soluble dans cet espace extracellulaire, mais sous une forme ancrée à la face interne de la membrane des vésicules membranaires, en particulier des exosomes.

Dans la mesure où le polypeptide chimérique de la demande ou l'invention ne comporte pas de peptide signal d'importation dans le réticulum endoplasmique, c'est un résident permanent du cytosol ou du noyau (il reste localisé dans le cytosol ou dans le noyau) et va donc se trouver non pas intégré dans la membrane ni à la surface d'une vésicule membranaire et en particulier d'un exosome sécrété par une cellule productrice d'exosome, mais à l'intérieur (c'est-à-dire dans la lumière) de cette vésicule membranaire. De plus, ce polypeptide est adressé à la membrane et ancré à la membrane des vésicules membranaires et en particulier des exosomes par l'intermédiaire d'un peptide capable d'interagir avec un ou plusieurs lipide(s) ou un motif lipidique présent(s) dans la couche interne d'une bicouche lipidique, en particulier un phospholipide, ou, de préférence, par l'intermédiaire d'un ou plusieurs acide(s) gras (en particulier un ou plusieurs acide(s) gras choisi(s) parmi les acides myristiques, les acides palmitiques et les géranyl-géranyl), qui interagissent avec le domaine de ciblage sous-membranaire.

Ce(s) acide(s) gras peuvent être attachés de façon co-traductionnelle et permettent au polypeptide chimérique produit dans le cytoplasme d'une cellule de se s'ancrer de façon post-traductionnelle dans des membranes cellulaires ou vésiculaires.

Selon un mode de réalisation particulier, le polypeptide chimérique de la demande ou l'invention est produit avec et est intégré dans les exosomes avant leur sortie de la cellule.

La sécrétion d'un peptide ou d'un polypeptide en association avec des vésicules membranaires (en particulier des exosomes) requiert notamment (1) l'adressage dudit peptide ou d'un polypeptide au(x) lieu(x) de formation des vésicules membranaires et en particulier des exosomes, et (2) le bourgeonnement vésiculaire à partir de la membrane dans laquelle ledit peptide ou polypeptide est ancré.

Le terme « adressage » encore appelé « tri », « aiguillage » ou « routage intracellulaire » fait référence, dans la demande ou invention, au processus qui permet à un polypeptide, dont la synthèse débute dans le cytosol, d'être dirigé vers et d'atteindre les compartiments impliqués dans le bourgeonnement de vésicules membranaires, en particulier des vésicules formant les exosomes et/ou d'atteindre les vésicules membranaires, en particulier les vésicules formant les exosomes.

Le terme « sécrétion » désigne, dans le cadre de la demande ou l'invention, le processus par lequel des vésicules membranaires, alors appelées « exosomes », sont sécrétées, c'est-à-dire relarguées dans l'espace extracellulaire à partir d'une ou plusieurs cellule(s). Ce processus peut notamment se produire lorsque des endosomes multivésiculaires fusionnent avec la membrane plasmique d'une cellule, relarguant ainsi les vésicules membranaires qu'ils contiennent à l'extérieur de la cellule.

Comme illustré dans les exemples ci-après, un test permettant de mettre en évidence les propriétés d'adressage et de sécrétion d'un polypeptide chimérique tel que défini dans la demande ou invention consiste à vérifier que ledit polypeptide chimérique et/ou ses produits de dégradation se retrouvent bien associés avec des vésicules membranaires (en particulier des exosomes) lorsque ledit polypeptide est exprimé dans des cellules eucaryotes appropriées.

Une cellule eucaryote « appropriée» est avantageusement une cellule eucaryote comportant des vésicules internes de sécrétion, cultivable, capable d'exocytose, modifiable génétiquement et, préférentiellement, dont les vésicules internes peuvent être sécrétées sous l'effet d'une stimulation externe. Il s'agit en particulier d'une cellule de mammifère et plus particulièrement d'une cellule d'origine humaine ou une cellule ayant pour origine un mammifère non humain. Il peut également s'agir de cultures primaires ou de lignées immortalisées. De telles cellules eucaryotes « appropriées » incluent notamment les cellules eucaryotes naturellement capables de produire des exosomes, en particulier les mastocytes, lymphocytes T et B et les cellules dendritiques (par exemple les cellules de Langerhans) ou des cellules dérivées de ces types cellulaires, ainsi que les cellules eucaryotes ou les lignées cellulaires eucaryotes modifiées par génie génétique de façon à les rendre capables de sécréter des exosomes.

Le terme « bicouche lipidique », désigne la structure de base de la membrane plasmique et de toute membrane biologique, c'est-à-dire tout assemblage de lipides amphiphile en un double feuillet (ou double couche) séparant une cellule ou une vésicule de son environnement et délimitant le cytoplasme d'une cellulaire ou d'une vésicule, ou délimitant les organites à l'intérieur du cytoplasme. Ce terme englobe donc toute membrane de la cellule, c'est-à-dire à la fois la membrane plasmique et les membranes des différents compartiments intracellulaires, en particulier celles du réticulum endoplasmique, celles de l'appareil de Golgi, ou encore celle des vésicules membranaires, par exemple celle des exosomes ou des endosomes.

Par « domaine cytoplasmique » (CD), on entend, dans la demande ou invention, un domaine cytoplasmique particulier capable d'être adressé vers les vésicules membranaires, en particulier vers les vésicules formant des exosomes, ou vers le(s) compartiment(s) cellulaire(s) impliqué(s) dans la formation des vésicules membranaires, et en particulier des vésicules formant les exosomes dans des cellules eucaryotes; ce domaine peut ainsi être sécrété dans l'espace extracellulaire en association avec des exosomes, lorsqu'il est exprimé dans des cellules eucaryotes appropriées. Ainsi, ce domaine permet, lorsqu'il est intégré à un polypeptide chimérique comprenant un peptide ou un polypeptide d'intérêt, d'adresser ledit polypeptide chimérique vers les vésicules membranaires et/ou vers leurs(s) lieu(x) de formation et en particulier d'adresser ledit polypeptide chimérique à la membrane de vésicules membranaires, de façon à ce que ce ledit polypeptide puisse être sécrété en association avec des vésicules membranaires (en particulier des exosomes) par une cellule, en particulier une cellule eucaryote appropriée.

Un « dérivé muté » d'un domaine au sens de la demande ou invention fait référence à tout polypeptide ou peptide modifié par rapport au domaine original ou de référence, sous réserve que ce dérivé muté conserve la fonction impliquant normalement ledit domaine : cette fonction est par exemple la capacité d'adressage vers les vésicules membranaire (et en particulier vers les exosomes) normalement attachée au domaine CD de référence, la fonction d'ancrage à une bicouche lipidique normalement attachée au domaine de ciblage sous-membranaire, et, pour les peptides chimériques additionnels la fonction d'insertion et d'ancrage dans une bicouche lipidique impliquant normalement un domaine membranaire et en particulier la capacité à traverser entièrement et s'ancrer dans une bicouche lipidique impliquant normalement un domaine transmembranaire. Un tel dérivé muté peut donc correspondre par exemple à un fragment composé de résidus contigus dudit domaine original ou de référence (ce dérivé étant obtenu notamment par délétion et éventuellement substitution d'un ou plusieurs résidu(s) dans la séquence originale) ou, au contraire à un polypeptide de taille plus importante que le domaine original ou de référence, en particulier un polypeptide comprenant le domaine original ou de référence (ce dérivé étant obtenu notamment par insertion d'un ou plusieurs résidu(s) dans la séquence originale).

Selon un mode de réalisation particulier, le « dérivé muté» diffère de la séquence originale ou de référence par la substitution d'au moins un résidu, de préférence un, deux, trois, quatre, cinq, voire plus de cinq résidu(s), consécutifs ou non, dans la séquence du domaine original, ces substitutions pouvant être conservatives, semi-conservatives ou non conservatives, et/ou par la délétion d'au moins un résidu, de préférence un, deux, trois, quatre, cinq, voire plus de cinq résidus, consécutifs ou non, et/ou par l'insertion d'au moins un résidu, de préférence un, deux, trois, quatre, cinq, voire plus de cinq résidus, consécutifs ou non, dans la séquence du domaine original.

La séquence de ce « dérivé muté » peut présenter au moins 60 ou 70%, en particulier au moins 80%, 90% ou 95%, de similarité ou d'identité avec le domaine original dont elle dérive, par référence à la séquence complète du domaine original. Par « pourcentage d'identité », on entend, dans la demande ou invention, le nombre de résidus identiques rapporté au nombre total de résidus du peptide ou polypeptide étudié. Le « pourcentage de similarité », définit le nombre de résidus identiques ou chimiquement similaires rapporté au nombre total de résidus du peptide ou polypeptide étudié. Le pourcentage d'identité ou de similarité est déterminé en alignant les deux séquences à comparer et en utilisant l'algorithme de Needleman et Wunsch, qui permet un alignement global entre deux séquences. Le pourcentage de similarité ou d'identité est alors calculé sur la totalité de la longueur de ces deux séquences.

Selon un mode de réalisation particulier, le domaine CD ou son dérivé muté comprend deux ou trois motifs YxxL, dans lesquels x représente un résidu quelconque. Le motif ou un des motifs YxxL présent(s) dans le domaine CD peut (peuvent) être, par exemple, le motif YINL (SEQ ID NO. 91) ou YSHL (SEQ ID NO. 97).

Selon un mode de réalisation particulier, le domaine CD ou son dérivé muté comprend un motif DYxxL, dans lequel x représente un résidu quelconque. A titre d'exemple illustré dans la partie expérimentale, on citera le motif DYINL (SEQ ID NO. 93).

Alternativement ou de façon complémentaire, le domaine CD comprend au moins un motif équivalent à un motif YxxL ou à un motif DYxxL, par exemple un motif YxxF ou DYxxF respectivement, dans lequel x représente un résidu quelconque. Par exemple, le récepteur de la transférine, qui est une protéine cellulaire, comporte un domaine YxxF.

La description de la demande ou invention, faite par référence au domaine YxxL en général ou tel qu'illustré par des séquences particulières, vaut également pour les domaines DYxxL ou DYxxF, définis en termes généraux ou ayant des séquences spécifiques dérivées des exemples donnés de motifs.

Selon un mode de réalisation préféré,le domaine CD ou son dérivé muté comprend en outre deux, trois ou quatre motif(s) PxxP, dans lequel x représente un résidu quelconque, au moins un de ces motifs PxxP étant le motif PSAP (SEQ ID NO. 88) ou PTAP (SEQ ID NO. 89). Dans un mode de réalisation particulier de la demande ou l'invention, le motif PxxP du domaine CD ou de son dérivé muté est PSAP ou PTAP (plus préférablement PSAP) et le motif YxxL est YINL ou YSHL.

Dans un mode de réalisation particulier, le motif YxxL du domaine CD (par exemple le motif YINL ou YSHL), ou un des motifs est YxxL, est localisé en position C-terminale par rapport au motif PxxP (par exemple le motif PSAP).

Dans un mode de réalisation particulier, le motif YxxL du domaine CD (par exemple le motif YINL ou YSHL) est localisé en position N-terminale par rapport au motif PxxP (par exemple le motif PSAP).

Parmi les protéines ayant un domaine CD comportant au moins un motif YxxL, on trouve notamment des protéines cellulaires et des protéines virales. Ces protéines virales sont notamment des protéines de virus enveloppés, en particulier les glycoprotéines TM de virus enveloppés et notamment de rétrovirus.

Selon un mode de réalisation particulier de la demande, le domaine CD est celui d'une protéine TM du virus de la leucémie bovine (BLV).Ce rétrovirus, qui fait partie des Oncovirinae (c'est un Oncovirus), induit, chez les bovins, une prolifération des cellules B pouvant engendrer une leucémie.

Le domaine CD de la protéine TM du BLV est composé de 58 résidus, et a pour séquence SEQ ID NO.6. Le domaine CD du polypeptide chimérique de l'invention ou son dérivé muté correspond soit au domaine de séquence SEQ ID NO.6, soit à un dérivé muté du domaine de séquence SEQ ID NO.6.

Dans un mode de réalisation particulier de la demande ou l'invention, le dérivé muté du domaine CD est un fragment de domaine CD natif constitué essentiellement par une séquence d'acides aminés s'étendant du motif PSAP (ou PTAP) jusqu'au motif YxxL (par exemple YINL ou YSHL) qui, dans un mode de réalisation particulier, le suit en partie C-terminale. Le cas échéant, un ou plusieurs résidus d'acide aminé naturellement présent(s) dans le domaine CD, entre ces deux motifs, est (sont substitué(s) ou délété(s).

Selon un mode de réalisation particulier, le domaine CD du polypeptide chimérique de la demande ou l'invention ou son dérivé muté, en particulier le dérivé muté du domaine CD, est dépourvu de la séquence KCLTSRLLKLLRQ. Ainsi, le dérivé muté du domaine CD peut différer du domaine CD original en ce que sa séquence est dépourvue de la séquence : KCLTSRLLKLLRQ.

En outre, ou alternativement, la séquence du dérivé muté du domaine CD peut présenter au moins 60 ou 70%, en particulier au moins 80%, 90% ou 95%, de similarité ou d'identité avec la séquence du domaine CD original dépourvue de l'enchaînement KCLTSRLLKLLRQ.

Il peut également être préférable que le domaine CD ou son dérivé muté, et en particulier son dérivé muté soit dépourvu de la séquence PC et/ou de la séquence CP. Selon un mode de réalisation particulier, ledit domaine CD ou son dérivé muté, en particulier son dérivé muté est notamment dépourvu de la séquence PCP. Ainsi, si le domaine CD original contient une séquence PCP, son dérivé muté peut être obtenu notamment en délétant, dans la séquence du domaine CD original, le résidu cystéine de ladite séquence PCP, ou en le substituant par un autre résidu, de préférence par un résidu non palmitoylable, par exemple un résidu alanine.

Selon un mode de réalisation particulier, le domaine CD ou son dérivé est dépourvu à la fois de la séquence PCP et de l'enchaînement KCLTSRLLKLLRQ.

A titre d'exemple, le dérivé muté du domaine CD de la protéine TM du BLV peut comprendre ou consister en la séquence SEQ ID NO.8, qui correspond à la séquence SEQ ID NO.6 dans laquelle les 13 résidus N-terminaux ont été délétés.

A titre d'exemple également, la séquence du dérivé muté de la protéine TM du BLV peut différer de la séquence du domaine CD la protéine TM du BLV par la substitution d'au moins un résidu, de préférence un, deux, trois, quatre, cinq, voire plus de cinq résidu(s), consécutifs ou non, et/ou par la délétion et/ou l'insertion d'au moins un résidu, de préférence un, deux, trois, quatre, cinq, voire plus de cinq résidu(s), consécutifs ou non dans la séquence dudit domaine correspondant à la séquence SEQ ID NO.8.

Encore à titre d'exemple, la séquence du dérivé muté du domaine CD de la protéine TM du BLV (domaine CD de séquence SEQ ID NO.6) peut présenter au moins 60 ou 70%, en particulier au moins 80%, 90% ou 95%, de similarité ou d'identité avec la séquence SEQ ID NO.8. De préférence, la séquence dudit dérivé muté conserve notamment le motif YINL, YSHL (ou, le cas échant, DYINL) et le motif PSAP de la séquence SEQ ID NO.8.

La séquence du dérivé muté du domaine CD de séquence SEQ ID NO.6 est, de préférence, dépourvue de motif PC (proline - cystéine) et CP (cystéine - proline) et, plus préférablement dépourvue de motif PCP (proline - cystéine - proline). Par exemple, il peut comprendre ou consister en la séquence SEQ ID NO. 10 ou SEQ ID NO.12.

Selon un mode de réalisation particulier, le domaine CD ou son dérivé muté, en particulier le dérivé muté du domaine CD de séquence SEQ ID NO.6, comprend ou consiste en une séquence choisie parmi les séquences suivantes:
PxxPxxxxPxxPxSxYxxLxPxxPExYxxLxPxxPDYxxL ;
PxxPxₙPxxPxₙSxYxxLxₙPxxPExₙYxxLxₙPxxPDYxxL ;
PxxPxxxxPxxPxSxYxxLxPxxPExYxxLxPxxPDYxxLxxxx ; et
PxxPxₙPxxPxₙSxYxxLxₙPxxPExₙYxxLxₙPxxPDYxxLxxxx ;
dans lesquelles x et xₙ représentent respectivement un résidu quelconque et un ou plusieurs résidu(s) quelconque(s), et dans lesquelles au moins un des motifs PxxP est le motif PSAP ou PTAP .

Selon un mode de réalisation particulier, le domaine CD ou son dérivé muté, en particulier le dérivé muté du domaine CD de séquence SEQ ID NO.6, comprend ou consiste en une séquence choisie parmi les séquences suivantes:
PxxPxxxxxxxxxxxxYxxL ;
PxxPxxxxxxxxxxxDYxxL ;
PxxPxxYxxxxxxxxxYxxL ;
PxxPxxYxxxxxxxxDYxxL ;
PxxPExYxxLxPxxPDYxxL ;
PxxPxₙYxxL ;
PxxPxₙDYxxL ;
PxxPxₙYxₙYxxL ;
PxxPxₙYxₙDYxxL ;
PxxPExₙYxxLxₙPxxPDYxxL ;
PxxPxxxxPxxPxxxYxxLxPxxPExYxxLxPxxPDYxxL ;
PxxPxₙPxxPxₙYxxLxₙPxxPEXₙYxxLxₙPxxPDYxxL ;
PxxPxxxxPxxPxxxYxxLxPxxPExYxxLxPxxPDYxxLxxxx ; et
PxxPxₙPxxPxₙYxxLxₙPxxPEXₙYxxLxₙPxxPDYxxLxxxx.
dans lesquelles x et xₙ représentent respectivement un résidu quelconque et un ou plusieurs résidu(s) quelconque(s), au moins un des motifs PxxP étant le motif PSAP ou PTAP.

En particulier n est supérieur ou égal à 1 et inférieur à 50. n peut avoir en particulier toute valeur entre 1 et 20.

A titre d'exemple, le motif PxxP qui, dans un mode de réalisation particulier, se trouve en position N-terminale dans les séquences indiquées ci-dessus, peut être le motif PSAP ou PTAP.

Alternativement ou de façon complémentaire, le motif YxxL qui, dans un mode de réalisation particulier, se trouve en position C-terminale dans les séquences indiquées ci-dessus, peut être par exemple le motif YINL ou YSHL.

Dans un mode de réalisation particulier de la demande ou l'invention, lorsque le domaine CD ou son dérivé muté comprend l'une des séquences ci-dessus, les résidus d'acides aminés consécutifs ajoutés en amont ou en aval de cette séquence ne forment pas un motif PxxP ni un motif YxxL, YxxF, DYxxL ou DYxxF.

Selon un mode de réalisation particulier, ledit dérivé muté du domaine CD de séquence SEQ ID NO.6 comporte 6 à 100 résidus, en particulier 20 à 80, 30 à 70 ou 40 à 60, par exemple 40, 41, 42, 43, 44, 45, 46, 47, 48, 49 ou 50 résidus.

A titre d'exemple, la séquence du dérivé muté du domaine CD peut comprendre ou consister en la séquence SEQ ID NO. 30, SEQ ID NO. 42, SEQ ID NO. 44 ou SEQ ID NO. 95 ou présenter au moins 60 ou 70%, en particulier au moins 80%, 90% ou 95%, de similarité ou d'identité avec la séquence SEQ ID NO.30, SEQ ID NO.42, SEQ ID NO.44 ou SEQ ID NO.95 par référence respectivement à la séquence SEQ ID NO. 30, SEQ ID NO. 42, SEQ ID NO. 44 ou SEQ ID NO. 95 complète.

Par « peptide ou polypeptide d'intérêt » on entend, dans la demande ou invention, tout un enchaînement de plusieurs (au moins deux) résidus successifs, formant la structure d'un peptide ou d'un polypeptide. Un peptide désigne une chaîne de 2 à 20 résidus successifs (en particulier 2, 3, 4, 5, 6, 7, 8, 9, 10, 11, 12, 13, 14, 15, 16, 17, 18, 19 ou 20 résidus), en particulier une chaîne de 5 à 10, de 10 à 15 ou de 15 à 20 résidus successifs. Un polypeptide, qui désigne également une protéine ou un fragment d'une protéine, est un enchaînement de plus de 20 (au moins 21) résidus successifs, en particulier une chaîne de 21 à 1000 résidus successifs, de préférence de 21 à 500, 21 à 250 ou 21 à 150 résidus successifs, par exemple 21 à 50, 50 à 100, 100 à 150 résidus successifs. Ledit peptide ou polypeptide d'intérêt peut être en particulier comprendre ou consister en un ou plusieurs domaines d'une protéine soluble, membranaire, transmembranaire et/ou multimérique.

Selon un mode de réalisation particulier, le « peptide ou polypeptide d'intérêt », en particulier le peptide ou polypeptide d'intérêt présent dans le polypeptide chimérique de la demande ou l'invention comprend ou consiste en un ou plusieurs domaine(s) d'une protéine cytosolique ou d'une protéine nucléaire, ou un ou des fragment(s) de ce(s) domaine(s).

Par « fragment » d'un domaine on entend, dans la demande ou invention, une portion composée d'au moins 6 résidus contigus dudit domaine, et en particulier une portion présentant au moins 50%, de préférence au moins 60%, 70%, 80%, ou au moins 90%, voire 100% d'identité avec la séquence complète dudit domaine. Quoi qu'il en soit, un fragment est de taille inférieure à la taille de la protéine dont il dérive.

Selon un mode de réalisation particulier, ledit peptide ou polypeptide d'intérêt est antigénique, c'est-à-dire qu'il est capable d'éliciter une réponse immunitaire dirigée contre ledit peptide ou polypeptide d'intérêt. Ledit peptide ou polypeptide d'intérêt peut en particulier comprendre ou consister en un ou plusieurs épitope(s) d'une protéine.

Selon un mode de réalisation particulier, ledit peptide ou polypeptide d'intérêt provient d'un organisme pathogène, par exemple un virus, une bactérie ou un parasite, ou d'un agent pathogène, par exemple une cellule tumorale, une toxine.... Tout composant peptidique cytoplasmique ou nucléaire d'un tel organisme ou agent pathogène peut être utilisé, qu'il s'agisse ou non d'une protéine de structure. Ce peut être notamment un antigène de pathogène, et en particulier un antigène viral, bactérien ou un antigène provenant d'un parasite.

Ledit peptide ou polypeptide d'intérêt peut également provenir d'un antigène cytoplasmique, et en particulier d'un antigène tumoral cytoplasmique, ou de la partie cytosolique d'une protéine transmembranaire. Ce peut être alternativement une enzyme ou une partie d'enzyme ou un dérivé muté d'une enzyme, ou une toxine protéique, ou une partie de toxine protéique, ou un composé choisi pour sa capacité d'agir spécifiquement sur une cellule (ledit composé pouvant avoir un effet délétère ou au contraire bénéfique pour une cellule cible). Ce peut être également une protéine (par exemple une protéine G) ou un fragment de protéine, capable de fixer spécifiquement un acide nucléique, par exemple la protéine de capside du phage MS2. Ce peut être également un peptide ou polypeptide capable de fixer un acide nucléique particulier.

En fonction du type de peptide ou de polypeptide d'intérêt choisi, la demande ou l'invention pourra être utilisée *in vivo,* notamment pour une application médicale, notamment en immunisation, en particulier en vaccination.

A titre d'exemple, dans deux polypeptides chimériques de l'invention décrits dans les exemples, le polypeptide d'intérêt est un dérivé muté de la protéine hAGT. Ce dérivé muté est la protéine SNAP, qui est disponible dans le commerce (Covalys, NEB). Selon un mode de réalisation particulier, un polypeptide chimérique tel que défini dans la demande ou invention comprend en outre au moins une molécule de liaison (ou linker). Le terme « linker » désigne tout élément permettant de lier deux domaines successifs. Celui-ci peut-être de longueur et de nature variable.

Selon un mode de réalisation particulier, au moins deux domaines successifs du polypeptide de la demande ou l'invention sont liés de façon covalente, par exemple par l'intermédiaire de liaisons peptidiques. Ainsi, selon un mode de réalisation particulier, ledit linker est un polypeptide ou un peptide. Ce linker polypeptidique peut consister en une séquence de 2 à 50 résidus, de préférence de 2 à 30 résidus, par exemple 2 à 5, 5 à 10, 10 à 20 ou 20 à 30 résidus successifs.

Selon un mode de réalisation particulier, la séquence nucléotidique codant pour ledit linker comprend ou consiste en un site de restriction. Par « site de restriction », on entend une séquence nucléotidique particulière reconnue par une enzyme de restriction de type II comme un site de coupure dans la molécule d'ADN. Ainsi, ce linker peut être, par exemple, le linker de séquence SR, ou AS, ou PAGSGAP (SEQ ID NO.:106), codés respectivement par les séquences nucléotidiques TCTAGA, ou GCTAGC, ou CCTGCAGGAAGCGGCGCGCCC (SEQ ID NO.:107) qui comprennent respectivement les sites des enzymes de restriction Xbal, Nhe I, et Sbf I-Asc I.

Selon un mode de réalisation préféré, le peptide ou polypeptide d'intérêt se trouve dans sa conformation native dans les polypeptides chimériques décrits dans la demande et en particulier dans le polypeptide chimérique de l'invention.

Selon un mode de réalisation particulier, un polypeptide chimérique tel que défini dans la demande ou invention est multimérique et en particulier est sous la forme d'un dimère ou d'un trimère. Ceci peut être le cas, en particulier, lorsque le peptide ou le polypeptide d'intérêt provient d'une protéine qui, sous sa forme native, se dimérise ou se trimérise.

Selon un mode de réalisation particulier, un polypeptide chimérique tel que défini dans la demande ou invention comprend en outre une séquence tag qui permet de le purifier. Par exemple, ladite séquence tag peut comprendre ou consister en une pluralité de résidus histidine liés consécutivement, en particulier une séquence de 6 résidus histidine consécutifs.

Selon un mode de réalisation particulier, un polypeptide chimérique tel que défini dans la demande ou invention comprend une protéine tag ou rapporteur, qui peut être par exemple une enzyme, et qui est destinée à marquer le polypeptide, par exemple en lui associant un fluorophore, ou tout autre marqueur ou substrat permettant de fixer des marqueurs (par exemple la biotine). Une telle protéine peut être par exemple la protéine SNAP ou un dérivé muté de la protéine SNAP, qui conserve les propriétés de marquage normalement attachées à la protéine SNAP.

Selon un mode de réalisation particulier, un polypeptide chimérique tel que défini dans la demande ou invention comporte la protéine hAGT, dont le numéro d'accession dans la base de données GenBank est M29971.1, ou un dérivé issu ou muté de la protéine hAGT (Keppler et al.,2002) contenu dans le plasmide pSNAPm (NEB, USA).

Selon un mode de réalisation particulier, le polypeptide chimérique comprend en outre un marqueur permettant de détecter le polypeptide chimérique par ELISA ou en Western Blot. De préférence, ledit marqueur est un épitope reconnu par un anticorps monoclonal spécifique, par exemple un épitope myc.

Dans un mode de réalisation particulier de la demande ou l'invention, dans les polypeptides chimériques décrits dans la demande et en particulier dans le polypeptide chimérique de l'invention, le peptide ou polypeptide d'intérêt a avantageusement sa conformation native (en particulier est sous forme de multimère, par exemple sous forme de dimère ou de trimère) lorsqu'il est associé aux exosomes.

La demande et l'invention concernent également l'utilisation d'un polypeptide chimérique de la demande ou l'invention, pour adresser (*in vivo* ou *in vitro*) un peptide ou polypeptide d'intérêt qu'il comprend, vers les vésicules membranaires, en particulier vers les vésicules formant des exosomes, et/ou vers le(s) compartiment(s) cellulaire(s) impliqué(s) dans la formation des vésicules membranaires, et en particulier des vésicules formant les exosomes, et pour permettre ainsi la sécrétion, par des cellules eucaryotes appropriées, dudit peptide ou polypeptide d'intérêt en association avec lesdites vésicules membranaires.

Suivant un aspect particulier de la demande ou l'invention, le polypeptide chimérique de la demande ou l'invention peut être utilisé en association avec un polypeptide chimérique, dénommé ci-après « polypeptide chimérique additionnel ».

Selon un mode de réalisation particulier de la demande, le polypeptide chimérique additionnel comprend ou consiste en les domaines suivants:
- un peptide ou un polypeptide d'intérêt ;
- un domaine transmembranaire; et
- un domaine cytoplasmique (CD) ou un dérivé muté de ce domaine CD, ledit domaine CD et son dérivé muté étant tels que définis dans la demande, ces domaines étant positionnés successivement dans l'ordre suivant, de l'extrémité N-terminale vers l'extrémité C-terminale : peptide ou polypeptide d'intérêt - domaine transmembranaire - domaine CD ou son dérivé muté ; ou domaine CD ou son dérivé muté - domaine transmembranaire - peptide ou polypeptide d'intérêt.

Conformément à l'invention, le polypeptide chimérique additionnel est capable d'être sécrété en association avec des exosomes, et est caractérisé en ce qu'il comprend ou consiste en les domaines suivants:
- un peptide ou polypeptide d'intérêt;
- un domaine transmembranaire; et
- le domaine cytoplasmique (CD) de la protéine transmembranaire (TM) du virus de la leucémie bovine (BLV), qui est de SEQ ID NO : 6, ou un dérivé muté de ce domaine CD de SEQ ID NO : 6, ledit dérivé muté étant défini par la substitution, la délétion et/ou l'insertion d'un ou plusieurs résidu(s) dans la séquence dudit domaine CD et ledit dérivé muté ayant conservé la capacité à adresser ledit polypeptide chimérique additionnel à des exosomes et/ou à un(des) compartiment(s) cellulaire(s) impliqué(s) dans la formation des exosomes, ledit dérivé muté comprenant au moins un motif YxxL ou DYxxL et un motif PxxP, dans lequel x représente un résidu quelconque et Y, L, D et P représentent respectivement un résidu tyrosine, leucine, acide aspartique et proline ;
le domaine CD ou le dérivé muté du polypeptide chimérique additionnel étant identique à ou différent du dérivé muté du domaine CD du polypeptide chimérique selon l'invention.

Le polypeptide chimérique additionnel comprend nécessairement au moins un domaine membranaire (le domaine transmembranaire) mais il peut en comprendre plusieurs ; le peptide ou polypeptide d'intérêt pouvant comporter zéro, un ou plusieurs domaine(s) membranaire(s), en particulier transmembranaire(s) (voir ci-dessous), le polypeptide chimérique additionnel comporte un ou plusieurs domaines membranaires (en particulier transmembranaire(s)). Lorsque ledit polypeptide chimérique ne comporte qu'un seul domaine membranaire (le domaine transmembranaire), celui-ci peut être ou non dérivé de la même entité, en particulier de la même protéine que le peptide ou polypeptide d'intérêt dudit polypeptide.

Le domaine CD ou le dérivé muté du polypeptide chimérique additionnel peut être identique à ou différent du domaine CD ou du dérivé muté du domaine CD du polypeptide chimérique de la demande ou l'invention.

Un « domaine membranaire », désigne, dans la demande ou invention, tout domaine capable d'interagir avec une bicouche lipidique et en particulier capable de s'ancrer - et ainsi ancrer un polypeptide le comprenant - dans une bicouche lipidique et en particulier dans une membrane vésiculaire ou cellulaire et dans la membrane d'un exosome. Selon un mode de réalisation particulier, ce domaine membranaire est capable de se lier d'une part à un premier domaine (par exemple un peptide ou un polypeptide d'intérêt) et d'autre part à un deuxième domaine (par exemple un domaine cytoplasmique). Il peut par exemple comporter 10 à 50 résidus, de préférence 15 à 40 résidus et plus préférablement 20 à 30 résidus. Selon un mode de réalisation particulier, ledit domaine membranaire est un domaine transmembranaire, c'est-à-dire un domaine membranaire traversant entièrement la bicouche lipidique d'une membrane vésiculaire ou cellulaire. Un domaine transmembranaire est généralement agencé en hélice α hydrophobes, les protéines transmembranaires à traversées multiples pouvant contenir plusieurs, en particulier 2, 3, 4, 5, 6, 7 8, 9 ou 10, voire 20 ou plus de 20 hélices α hydrophobes. Il peut également être agencé en feuillet β. Un ou plusieurs domaines transmembranaires peuvent également adopter une structure en tonneau β transmembranaire, généralement composée de 8 à 22 brins β.

Par « protéine membranaire » on entend toute chaîne polypeptidique comportant un ou plusieurs domaine(s) membranaire(s) tel(s) que défini(s) ci-dessus.

Comme le polypeptide chimérique de la demande ou l'invention, le polypeptide chimérique additionnel est capable d'être sécrété en association avec des vésicules membranaires, en particulier avec des exosomes, lorsqu'il est exprimé dans des cellules eucaryotes appropriées.

De par son ou ses domaine(s) membranaire(s), en particulier de par son ou ses domaine(s) transmembranaire(s), le polypeptide chimérique additionnel ou un produit de dégradation dudit polypeptide peut s'ancrer dans la membrane d'une vésicule membranaire (en particulier d'un exosome) en s'insérant dans la bicouche lipidique de cette membrane. Un produit de dégradation dudit polypeptide chimérique peut également être ancré dans la membrane d'une vésicule membranaire par l'intermédiaire du domaine membranaire (en particulier transmembranaire) d'une molécule du CMH (de classe I ou II) à laquelle ledit produit de dégradation dudit polypeptide chimérique est associé.

Dans un polypeptide chimérique additionnel ainsi ancré dans la membrane d'une vésicule membranaire (en particulier d'un exosome), le peptide ou un polypeptide d'intérêt peut se trouver exposé (en totalité ou en partie) à l'extérieur de ladite vésicule membranaire et/ou inclus (en totalité ou en partie) dans la membrane de ladite vésicule membranaire (c'est le cas lorsque ledit polypeptide ou peptide d'intérêt comporte un ou plusieurs domaines membranaires) et/ou inclus (en totalité ou en partie) dans la fraction cytosolique de ladite vésicule membranaire.

Selon un mode de réalisation particulier de la demande ou l'invention, lorsque le domaine CD et un domaine transmembranaire dudit polypeptide chimérique additionnel sont dérivés d'une même protéine, alors au moins le domaine CD, son dérivé muté ou le domaine transmembranaire dérivé de la même protéine que le domaine CD est un dérivé muté par substitution et/ou délétion d'un ou plusieurs résidu(s) dans la séquence du domaine original.

Le domaine transmembranaire peut être dérivé d'une ou plusieurs protéine(s) transmembranaire(s), qui traverse(nt) une fois ou plusieurs fois, en particulier 2, 3, 4, 5, 6, 7 8, 9 ou 10 fois, voire 20 fois, ou plus, une membrane vésiculaire ou cellulaire. Lesdites protéines membranaires ou transmembranaires peuvent être notamment choisies parmi : des protéines humaines, des protéines d'un animal non humain, des protéines d'un organisme pathogène ou d'un agent pathogène, en particulier des protéines virales, bactériennes, ou des protéines exprimées par un parasite ou une cellule tumorale.

Le ou au moins un des domaine(s) transmembranaire(s) du polypeptide chimérique additionnel peut être en particulier celui d'une seule et même protéine transmembranaire ou être un dérivé muté du domaine transmembranaire d'une protéine membranaire. Ledit dérivé muté peut par exemple être obtenu en remplaçant une partie de la séquence du domaine de référence par une séquence dérivée du domaine transmembranaire d'une autre protéine transmembranaire.

Selon un mode de réalisation particulier de la demande ou l'invention, lorsque le domaine CD du polypeptide chimérique additionnel ou son dérivé muté comprend ou consiste en le domaine CD la protéine TM du BLV, de séquence SEQ ID NO.6, le(s) domaine(s) membranaire(s) dudit polypeptide chimérique additionnel est(sont) dépourvu(s) du domaine transmembranaire de la protéine TM du BLV, qui a pour séquence SEQ ID NO.4. Un domaine transmembranaire du polypeptide chimérique additionnel peut en revanche correspondre à un dérivé muté du domaine de séquence SEQ ID NO.4, ledit dérivé muté ne comprenant pas la séquence SEQ ID NO.4. Un tel dérivé muté peut être obtenu, par délétion et éventuellement substitution d'un ou plusieurs résidu(s) dans la séquence SEQ ID NO.4, comme illustré dans les exemples.

Selon un mode de réalisation particulier, lorsque un des domaine(s) transmembranaire(s) et le domaine CD dérivent d'une même protéine, notamment lorsqu'ils dérivent de la protéine TM du BLV, on utilise, en tant que domaine (iii), un dérivé muté du domaine CD dépourvu de la séquence PCP (le résidu C de la séquence PCP étant par exemple remplacé par un résidu A) et/ou dépourvu de la séquence KCLTSRLLKLLRQ.

Selon un mode de réalisation particulier, lorsque un des domaines membranaires du polypeptide chimérique additionnel comprend ou consiste en le domaine transmembranaire de la protéine TM du BLV, de séquence SEQ ID NO.4, le domaine CD ou son dérivé muté est dépourvu du domaine CD de la protéine TM du BLV, de séquence SEQ ID NO.6. Le domaine CD ou son dérivé muté peut en revanche correspondre à, ou comprendre un dérivé muté du domaine de séquence SEQ ID NO.6. Comme illustré dans les exemples, un tel dérivé muté peut être obtenu, par exemple, en délétant des résidus situés dans la partie N-terminale de la séquence SEQ ID NO.6, en particulier en délétant la séquence KCLTSRLLKLLRQ et/ou en délétant la séquence PCP dans la séquence SEQ ID NO.6, ou en substituant par exemple le résidu cystéine de la séquence PCP un autre résidu, de préférence par un résidu non palmitoylable, par exemple un résidu alanine.

Le peptide ou le polypeptide d'intérêt présent dans ce polypeptide chimérique additionnel peut être tel que défini dans la demande ou l'invention pour le polypeptide chimérique de la demande ou l'invention. Alternativement ou cumulativement, ledit peptide ou polypeptide d'intérêt du polypeptide chimérique additionnel peut également comprendre ou consister en un ou plusieurs domaine(s) d'une protéine extracellulaire ou d'une protéine de surface ou un ou des fragment(s) d'un ou plusieurs de ce(s) domaine(s).

Selon un mode de réalisation particulier, le peptide ou le polypeptide d'intérêt présent dans ce polypeptide chimérique additionnel comprend ou consiste en un ou plusieurs domaine(s) d'une protéine extracellulaire ou de surface ou un ou des fragment(s) d'un ou plusieurs de ce(s) domaine(s) et, le cas échéant en :
- un ou plusieurs domaine(s) membranaire d'une protéine membranaire, en particulier d'une protéine transmembranaire ou un ou plusieurs fragment(s) de ce(s) domaine(s), et/ou
- un ou plusieurs domaine(s) cytoplasmique(s) d'une protéine membranaire, en particulier d'une protéine transmembranaire, ou un ou plusieurs fragment(s) de ce(s) domaine(s).

Selon un mode de réalisation particulier de la demande ou l'invention, le peptide ou polypeptide d'intérêt du polypeptide chimérique additionnel est un récepteur, par exemple un récepteur à multiples domaines membranaires et en particulier un récepteur CXCR4 ou GPR, ou tout autre peptide ou polypeptide qui permet de cibler une cellule cible ou un type particulier de cellules cibles (par exemple des cellules tumorales ou des cellules présentant un trouble métabolique ou fonctionnel).

Selon un mode de réalisation particulier de la demande ou l'invention, le peptide ou polypeptide d'intérêt de ce polypeptide chimérique additionnel comprend ou consiste en un ou plusieurs ectodomaine(s) et/ou un ou plusieurs domaine(s) membranaire(s) et/ou un ou plusieurs domaines(s) cytoplasmique(s) d'une protéine membranaire, en particulier d'une protéine transmembranaire, ou un ou plusieurs fragment(s) d'un ou plusieurs de ce(s) domaine(s).

Selon un mode de réalisation particulier de la demande ou l'invention, ledit peptide ou polypeptide d'intérêt de ce polypeptide chimérique additionnel provient d'un organisme pathogène, par exemple un virus, une bactérie ou un parasite, ou d'un agent pathogène, par exemple une cellule tumorale, une toxine..., ou d'un antigène tumoral, d'un antigène cytoplasmique, d'un récepteur de ligand, notamment d'un récepteur à multiples domaines membranaires, par exemple un récepteur à sept domaines transmembranaires, d'un récepteur de cytokines ou d'un ligand de récepteur, notamment d'une cytokine ou d'un fragment de ceux-ci. Tout composant peptidique d'un organisme ou d'agent pathogène peut être utilisé, qu'il s'agisse ou non d'une protéine de structure. Ce peut être notamment un antigène de pathogène, et en particulier un antigène viral, bactérien ou un antigène provenant d'un parasite.

Ledit peptide ou polypeptide d'intérêt de ce polypeptide chimérique additionnel peut également provenir d'un antigène tumoral, d'un antigène cytoplasmique, d'une protéine transmembranaire, notamment d'une intégrine ou d'un co-récepteur ou d'une protéine intervenant dans des interactions (en particulier les protéines ICAM, CD4, CD8), d'un récepteur de ligand, notamment d'un récepteur à un seul domaine membranaire, par exemple un récepteur de cytokine (en particulier les récepteurs de la famille HER répondant à l'EGF, par exemple le récepteur EGF-R1), notamment d'un récepteur à multiples domaines membranaires, par exemple un récepteur à sept domaines transmembranaires (en particulier le récepteur CXCR4 du VIH ou un récepteur de l'acide gamma amino butyrique (GABA)), ou d'un ligand de récepteur, notamment d'une cytokine ou d'un fragment de ceux-ci.

En fonction du type de peptide ou de polypeptide d'intérêt présent dans ce polypeptide chimérique additionnel, la demande ou l'invention pourra être utilisée *in vitro,* par exemple pour cribler des molécules interagissant avec ledit peptide ou polypeptide d'intérêt.

Selon un mode de réalisation particulier, ledit peptide ou polypeptide d'intérêt provient d'une protéine présente à la surface d'un virus, par exemple une protéine responsable de la fixation d'une particule virale à un récepteur situé sur une cellule cible et/ou responsable de la fusion de l'enveloppe virale ou de la membrane plasmique d'une cellule infectée avec la membrane plasmique d'une cellule cible, ou un fragment d'une telle protéine.

A titre d'exemple, on peut utiliser, en tant que domaine (i), une protéine de l'enveloppe d'un virus enveloppé ou un fragment de cette protéine. Ce virus enveloppé peut être notamment choisi parmi les familles suivantes :
- les Poxviridae, en particulier ceux du genre Orthopoxvirus, qui comprend notamment le virus de la variole et le virus de la vaccine ;
- les Herpesviridae, en particulier ceux du genre Herpesvirus, qui comprend notamment les Herpesvirus de types 1 et 2, le virus de la Varicelle, le virus d'Epstein Barr, le Cytomégalovirus, et les Herpesvirus de types 6, 7, 8 ;
- les Hepadnaviridae, qui comprennent notamment le virus de l'hépatite B ;
- les Orthomyxoviridae, en particulier ceux du genre virus influenza A, B ou C, qui comprend notamment le virus de la grippe aviaire H5N1 ;
- les Paramyxoviridae, en particulier ceux du genre Paramyxovirus, qui comprend notamment les virus parainfluenzae et le virus des oreillons, ceux du genre Morbillivirus, qui comprend notamment les virus de la rougeole, et ceux du genre Pneumovirus, qui comprend notamment le virus respiratoire syncytial ;
- les Rhabdoviridae, en particulier ceux du genre Lyssavirus, qui comprend notamment le virus de la rage ;
- les Filoviridae, qui comprennent notamment le virus de Marburg et le virus Ebola ;
- les Togaviridae, en particulier ceux du genre Flavivirus, qui comprend notamment le virus de la fièvre jaune et le virus de l'hépatite C (HCV), ceux du genre Alphavirus et ceux du genre Rubivirus, qui comprend notamment le virus de la rubéole ;
- les Coronaviridae, en particulier ceux du genre Coronavirus, qui comprend notamment des virus responsables d'infections respiratoires et digestives telles que le syndrome respiratoire aigu sévère (SRAS);
- les Arenaviridae, en particulier ceux du genre Arenavirus, qui comprend notamment le virus de Lassa ;
- les Bunyaviridae, en particulier ceux du genre Bunyavirus, Hantavirus, Phlebovirus ; et
- les Retroviridae, en particulier ceux du genre Lentivirus et notamment les virus d'immunodéficience humaine (VIH).

Encore à titre d'exemple, le peptide ou polypeptide d'intérêt peut être issu d'une protéine de l'enveloppe d'un virus de la grippe, en particulier de l'hémagglutinine (HA) d'un virus de la grippe, et plus particulièrement de la protéine HA du virus de la grippe aviaire H5N1. Ce peut être l'ectodomaine de cette protéine ou un fragment de cet ectodomaine ou un fragment comprenant ou consistant en un ou plusieurs épitope(s) de cet ectodomaine.

D'autres polypeptides antigéniques de ces virus peuvent naturellement être utilisés, tels que la protéine ou un fragment de la polyprotéine GAG du VIH, la protéine ou un fragment de capside du poliovirus ou d'un papillomavirus.

A titre d'exemple également, le peptide ou polypeptide d'intérêt peut être issu d'une protéine de l'enveloppe externe de l'un de ces virus. Il peut être issu d'une protéine l'enveloppe d'un coronavirus, en particulier de la protéine Spike (S) d'un coronavirus, et plus particulièrement la protéine Spike d'un coronavirus du syndrome respiratoire aigu sévère (coronavirus du SRAS ou SARS-CoV en anglais). Ce peptide ou polypeptide d'intérêt peut en particulier comprendre ou consister en l'ectodomaine de cette protéine ou un fragment comprenant ou consistant en un ou plusieurs épitope(s) de cet ectodomaine.

Dans un mode de réalisation particulier, le peptide ou polypeptide d'intérêt du polypeptide chimérique additionnel a la capacité de reconnaître une protéine présente à la surface de cellules cibles, par exemple de cellules dendritiques, de lymphocytes, de cellules tumorales...

L'adressage du polypeptide chimérique additionnel au(x) lieu(x) de formation des vésicules membranaires et en particulier au(x) lieu(x) de formation des exosomes peut nécessiter notamment que ledit polypeptide chimérique additionnel comprenne un peptide signal d'importation dans le réticulum endoplasmique, afin que ledit polypeptide chimérique additionnel puisse être inséré dans une membrane vésiculaire ou cellulaire (la fonction d'ancrage dans la membrane étant assurée par le domaine transmembranaire). Ainsi, selon un mode de réalisation particulier, contrairement au polypeptide chimérique de la demande ou l'invention, le polypeptide chimérique additionnel peut comporter un (ou plusieurs) peptide(s) signal d'importation dans le réticulum endoplasmique.

Par « peptide signal d'importation dans le réticulum endoplasmique», on entend une petite séquence polypeptidique continue d'environ 5 à environ 60 résidus, en particulier de 15 à 60 résidus et plus particulièrement de 15 à 30 résidus, qui permet le passage d'une protéine le comportant à travers la membrane du réticulum endoplasmique, le passage de la protéine pouvant être complet ou être partiel, l'arrêt du passage de la protéine dépendant de la présence d'un autre signal (ou d'autres signaux) additionnel(s). Les peptides signaux pour une même destination étant interchangeables d'une protéine à une autre, tout peptide signal permettant l'adressage d'une protéine au réticulum endoplasmique peut-être utilisé dans le cadre de la demande ou invention.

A titre d'exemple, on peut utiliser en tant que peptide signal d'importation dans le réticulum endoplasmique un peptide de 27 résidus de séquence SEQ ID NO.:2. A titre d'exemple également, on pourrait également utiliser le peptide signal d'une protéine membranaire telle que les protéines CD4, CD8 et hémagglutinine (HA), le peptide signal d'un récepteur de cytokine tel que IL1R1, EGFR1 (HER1), HER2, HER3 ou HER4, ou encore, le peptide signal d'une protéine sécrétée, par exemple, celui d'une cytokine. Ainsi, on peut utiliser un peptide signal choisi parmi : celui de la protéine CD4 humaine (peptide de séquence SEQ ID NO.:49) ou CD4 de souris (peptide de séquence SEQ ID NO.:50), celui de la protéine CD8 alpha de souris (peptide de séquence SEQ ID NO.:51), CD8 alpha bovine (peptide de séquence SEQ ID NO.:52), CD8 alpha humaine (peptide de séquence SEQ ID NO.:53), ou CD8 alpha de rat (peptide de séquence SEQ ID NO.:54), celui des récepteurs IL1R1 humain (peptide de séquence SEQ ID NO.:55), EGFR1 (HER1) humain (peptide de séquence SEQ ID NO.:56), HER2 humain (peptide de séquence SEQ ID NO.:57), HER3 humain (peptide de séquence SEQ ID NO.:58) ou HER4 humain (peptide de séquence SEQ ID NO.:59), ou celui des cytokines IL-2 de souris (peptide de séquence SEQ ID NO.:60), IL-6 de souris (peptide de séquence SEQ ID NO.:61), IL-7 humaine (peptide de séquence SEQ ID NO.:62), IL-10 de souris (peptide de séquence SEQ ID NO.:63), ou MIP-1-alpha chimiokine humaine (peptide de séquence SEQ ID NO.:64), celui de l' hémagglutinine du virus Influenza B (peptide de séquence SEQ ID NO.:65), celui de l' hémagglutinine des virus Influenza A H1N1 (peptide de séquence SEQ ID NO.:66), Influenza A H2N2 (peptide de séquence SEQ ID NO.:67), Influenza A H3N2 (peptide de séquence SEQ ID NO.:68), Influenza A H4N6 (peptide de séquence SEQ ID NO.:69), Influenza A H5N1 (peptide de séquence SEQ ID NO.:70), Influenza A H6N5 (peptide de séquence SEQ ID NO.:71), Influenza A H7N7 (peptide de séquence SEQ ID NO.:72), Influenza A H8N4 (peptide de séquence SEQ ID NO.:73), Influenza A H9N2 (peptide de séquence SEQ ID NO.:74), Influenza A H10N7 (peptide de séquence SEQ ID NO.:75), Influenza A H11 N6 (peptide de séquence SEQ ID NO.:76), Influenza A H12N5 (peptide de séquence SEQ ID NO.:77), ou Influenza A H13N6 (peptide de séquence SEQ ID NO.:78).

Selon un autre mode de réalisation particulier, ledit peptide signal d'importation dans le réticulum endoplasmique peut faire partie du peptide ou polypeptide d'intérêt du polypeptide chimérique additionnel. C'est par exemple le cas lorsque ledit peptide ou polypeptide d'intérêt est une protéine membranaire ou une cytokine.

Indépendamment ou en combinaison avec le mode de réalisation ci-dessus, ledit peptide signal d'importation dans le réticulum endoplasmique peut faire partie d'un des domaines membranaires (en particulier un domaine transmembranaire) du polypeptide chimérique additionnel. Cela peut être par exemple le cas lorsque le domaine transmembranaire ou un des domaines membranaires dudit polypeptide est dérivé d'un récepteur à sept domaines transmembranaires.

Alternativement, selon un autre mode de réalisation, ledit peptide signal d'importation ne fait partie d'aucun des trois domaines principaux (à savoir les domaines : peptide ou polypeptide d'intérét ; domaine transmembranaire ; domaine CD ou son dérivé muté), et est, par conséquent, ajouté en plus de ces trois domaines dans polypeptide chimérique additionnel. Il peut alors être placé à différentes localisations dans la séquence linéaire dudit polypeptide, mais se trouve généralement à une extrémité dudit polypeptide et est de préférence en position N-terminale dans ledit polypeptide.

Si un deuxième peptide signal d'importation dans le réticulum endoplasmique est ajouté dans le polypeptide chimérique additionnel, il permet le cas échéant d'augmenter le ciblage membranaire.

Sous une forme mature, en particulier lorsqu'il est ancré dans la membrane d'une vésicule membranaire, par exemple d'un exosome, le polypeptide chimérique additionnel ne comporte généralement pas ou plus de peptide signal N- ou C- terminal d'importation dans le réticulum endoplasmique; après qu'il a rempli sa fonction, le peptide signal N- ou C- terminal peut être est séparé du polypeptide par un clivage protéolytique, par exemple dans le réticulum endoplasmique ou dans le Golgi.

La demande et invention concernent également une vésicule membranaire, et plus précisément un exosome, qui comporte un polypeptide chimérique de la demande ou l'invention, et/ou un ou plusieurs produit(s) de dégradation dudit polypeptide chimérique de la demande ou l'invention, ce(s) produit(s) de dégradation étant le cas échéant associé(s) à une molécule du complexe majeur d'histocompatibilité (CMH) de type I et/ou de type II.

L'invention est relative à un exosome qui comporte un polypeptide chimérique de l'invention.

Le polypeptide chimérique de la demande ou l'invention et/ou se(s) produit(s) de dégradation est(sont) ancré(s) dans la membrane de ladite vésicule membranaire par l'intermédiaire de leur domaine de ciblage sous-membranaire ou par l'intermédiaire du domaine membranaire de la molécule du CMH (de classe I ou II) à laquelle il est(sont) associé(s). Selon un mode de réalisation particulier, un produit de dégradation comprend ou consiste en un fragment du peptide ou polypeptide d'intérêt, en particulier en un fragment comprenant ou consistant en un ou plusieurs épitope(s) dudit peptide ou polypeptide d'intérêt. Dans un mode de réalisation particulier, la vésicule membranaire de la demande, et plus précisément l'exosome de la demande ou l'invention, comporte, outre le polypeptide chimérique de la demande ou l'invention, et/ou ses éventuels produit(s) de dégradation, un ou plusieurs polypeptide(s) chimérique(s) additionnel(s) décrits dans la demande ou l'invention, et/ou un ou plusieurs produit(s) de dégradation de ce(s) polyeptide(s) chimérique(s) additionnel(s), le(s) produit(s) de dégradation du (des) polypeptide(s) chimérique(s) additionnel(s) étant le cas échéant associé(s) à une molécule du complexe majeur d'histocompatibilité (CMH) de type I et/ou de type II.

Alternativement, la vésicule de la demande, ou l'exosome de la demande ou invention, qui comporte un polypeptide chimérique de la demande ou invention, peut être utilisée en combinaison avec une vésicule membranaire (en particulier un exosome), dénommée ci-après « vésicule membranaire additionnelle » (et en particulier « exosome additionnel »), qui comporte un ou plusieurs polypeptide(s) chimérique(s) additionnel(s), et/ou un ou plusieurs produit(s) de dégradation de ce(s) polypeptide(s) chimérique(s) additionnel(s) décrits dans la demande ou l'invention, ce(s) produit(s) de dégradation étant le cas échéant associé(s) à une molécule du complexe majeur d'histocompatibilité (CMH) de type I et/ou de type II.

A titre d'exemple, la vésicule membranaire de la demande, et plus précisément l'exosome de la demande ou l'invention, peut comporter :
- d'une part, un polypeptide chimérique de la demande ou l'invention (et/ou ses éventuels produit(s) de dégradation) dans lequel le peptide ou polypeptide d'intérêt est une protéine cytosolique, par exemple un antigène, une protéine G, une enzyme (par exemple une enzyme cytosolique qui est défectueuse dans de(s) cellules cibles), une toxine ou tout autre peptide ou polypeptide cytosolique pouvant avoir un effet délétère ou au contraire bénéfique pour une cellule cible, ou encore un peptide ou polypeptide capable de fixer un acide nucléique particulier, ledit polypeptide chimérique de la demande ou l'invention étant éventuellement marqué, par exemple par un premier fluorophore ;
- d'autre part, un polypeptide chimérique additionnel, (et/ou ses éventuels produit(s) de dégradation) dans lequel le peptide ou polypeptide d'intérêt est par exemple un récepteur (par exemple un récepteur à multiples domaines membranaires et en particulier un récepteur CXCR4 ou GPR) ou tout autre peptide ou polypeptide qui permet de cibler une cellule cible ou un type particulier de cellules cibles (par exemple des cellules tumorales ou des cellules présentant un trouble métabolique ou fonctionnel), ledit polypeptide chimérique additionnel étant de préférence marqué, par exemple par un deuxième fluorophore (distinct du premier fluorophore).

Une telle vésicule de la demande, ou un tel exosome de la demande ou invention, peut avoir de nombreuses applications et peut être notamment utilisée pour modifier le contenu de cellules cibles. Lorsqu'elle/il est administré à une cellule, à une population de cellules ou un hôte humain ou non humain, cette vésicule ou cet exosome peut être internalisé par certaines cellules (par exemple des cellules dendritiques ou des cellules tumorales) ciblées via le peptide ou polypeptide d'intérêt présent dans le polypeptide chimérique additionnel (ou via ses éventuels produit(s) de dégradation) de cette vésicule ou de cet exosome. En internalisant ladite vésicule ou ledit exosome, les cellules cibles internalisent le peptide ou polypeptide d'intérêt du polypeptide chimérique de la demande ou l'invention présent dans cette vésicule, ce qui, en fonction de la nature dudit peptide ou polypeptide d'intérêt, peut avoir diverses applications et notamment permettre de:
- traiter les cellules cibles (par l'apport d'une enzyme par exemple);
- au contraire détruire les cellules cibles (par exemple des cellules tumorales) ; ou
- faire du diagnostic, par exemple en détectant un acide nucléique lié au peptide ou polypeptide d'intérêt du polypeptide chimérique de la demande ou l'invention ;
- d'induire une réponse immunitaire dirigée par exemple contre un antigène cytosolique présent dans le polypeptide chimérique de la demande ou l'invention ;
- faire du criblage de protéines et/ou analyser d'éventuelles interactions entre protéines (par exemple en détectant d'éventuels transferts d'énergie entre les deux fluorophores utilisés).

La vésicule membranaire de l'invention, ou l'exosome de la demande ou invention, qu'elle/il comprenne ou non aussi un polypeptide chimérique additionnel, peut être utilisé notamment pour produire *in vivo* ou *in vitro* des anticorps monoclonaux ou polyclonaux, vaccinants ou non vaccinants, dirigés contre ledit peptide ou polypeptide d'intérêt ou leur fragment. De tels anticorps peuvent être utilisés notamment en diagnostic ou pour étudier des interactions protéiques, en particulier pour réaliser des criblages à haut débit de molécules telles que des drogues ou des cytokines capables d'interagir avec le peptide ou le polypeptide d'intérêt ou leur fragment.

En outre, la vésicule membranaire de l'invention, ou l'exosome de la demande ou invention, qu'elle/il comprenne ou non aussi un polypeptide chimérique additionnel, peut être utilisé *in vivo,* en immunisation, pour éliciter ou favoriser, chez un hôte (humain ou non humain), une réponse humorale et/ou cellulaire contre une tumeur, ou contre le virus, la bactérie ou le parasite dont le peptide ou le polypeptide d'intérêt dérive. La réponse immune élicitée ou favorisée pour la vésicule membranaire de la demande, en particulier par un exosome de la demande ou l'invention, peut être, selon la nature des polypeptides associés aux exosomes, une réponse tolérogène ou de défense. Une réponse tolérogène peut permettre, par exemple, à l'hôte de lutter contre l'asthme ou de supporter une greffe.

Dans une vésicule membranaire comprenant un polypeptide chimérique additionnel, (et/ou ses éventuels produit(s) de dégradation), le peptide ou le polypeptide d'intérêt se trouvant dans ledit polypeptide(s) chimérique(s) additionnel(s), ou un fragment dudit peptide ou le polypeptide d'intérêt, est exposé, en partie ou en totalité, à la surface, à l'extérieur de la vésicule membranaire. Par conséquent, une vésicule de l'invention, ou un exosome de la demande ou invention, comprenant aussi un polypeptide chimérique additionnel peut être utilisé notamment pour produire ou sélectionner ou cibler *in vivo* ou *in vitro* des cellules procaryotes ou eucaryotes ou des virus (par exemple des phages) ou des ribosomes interagissant directement ou indirectement avec ledit peptide ou polypeptide d'intérêt ou leur fragment. Selon un mode de réalisation préféré, ledit peptide ou polypeptide d'intérêt du polypeptide chimérique additionnel, ou le fragment dudit peptide ou polypeptide d'intérêt, est exposé (en partie ou en totalité) dans sa conformation native à la surface de la vésicule membranaire.

Selon un mode de réalisation particulier, le peptide ou le polypeptide d'intérêt du polypeptide chimérique additionnel ou le fragment dudit peptide ou polypeptide d'intérêt, est inclus en partie ou en totalité dans la membrane de la vésicule membranaire de la demande ou de l'exosome de la demande ou de l'invention, et/ou inclus en partie ou en totalité dans la fraction cytosolique de ladite vésicule membranaire ou dudit exosome.

Une composition, en particulier une composition thérapeutique (par exemple pharmaceutique) ou une composition immunogène, dont le principe actif comprend un ou plusieurs polypeptide(s) chimérique(s) de la demande ou l'invention ou une ou plusieurs vésicule(s) membranaire(s) de la demande, en particulier un ou plusieurs exosome(s) de la demande ou l'invention fait également partie de la demande ou l'invention.

Selon un mode de réalisation particulier, ladite composition comprend en outre :
- un ou plusieurs polypeptide(s) chimérique(s) additionnel(s) ; ou
- une ou plusieurs vésicule(s) membranaire(s) additionnelle(s) (en particulier un ou plusieurs exosome(s) additionnel(s)).

Alternativement, ladite composition peut être utilisée (en particulier administrée à un hôte humain ou non humain) en conjonction avec une autre composition, en particulier une autre composition thérapeutique ou immunogène, qui comprend un ou plusieurs polypeptide(s) chimérique(s) additionnel(s) et une ou plusieurs vésicule(s) membranaire(s) additionnelle(s).

Selon un mode de réalisation particulier, ladite ou lesdites composition(s) comprend (comprennent) en outre un ou plusieurs véhicule(s), diluant(s), et/ou adjuvant(s) ou une de leurs combinaisons. Dans le cas d'une administration injectable, on peut notamment choisir une formulation dans une solution aqueuse, non aqueuse ou isotonique.

Le terme « véhicule » désigne, dans la demande ou invention, tout support (c'est-à-dire tout ce qui peut transporter au moins un principe actif) qui n'altère pas l'efficacité de l'activité biologique des substances actives de la demande ou l'invention. De nombreux véhicules sont connus dans l'état de la technique. Les véhicules utilisés peuvent être, par exemple, de l'eau, une solution saline, de l'albumine sérique, une solution Ringer, le polyéthylène glycol, des solvants miscibles dans l'eau, des sucres, des lieurs, des excipients, des pigments, des huiles végétales ou minérales, des polymères solubles dans l'eau, des agents tensioactifs, des agents épaississants ou gélifiants, des agents cosmétiques, des agents de solubilisation, des agents de stabilisation, des agents conservateurs, des agents alcalinisants ou acidifiants ou une de leurs combinaisons.

Le terme « diluant » signifie, dans la demande ou invention, un agent de dilution, et inclut les diluants solubles et les diluants insolubles. On utilise généralement un diluant insoluble quand le principe actif est soluble et un diluant soluble quand le principe actif est insoluble. Un principe actif « insoluble » peut être totalement insoluble en milieu aqueux ou avoir une solubilité limitée (c'est à dire une solubilité inférieure à 10mg/ml dans 250 ml d'eau à un pH de 1.0 à 7.5) en milieu aqueux. Des exemples de diluants insolubles incluent la cellulose microcristalline, la cellulose microcristalline silicifiée, l'hydroxyméthylcellulose, le phosphate de dicalcium, le carbonate de calcium, le sulfate de calcium, le carbonate de magnésium, le phosphate de tricalcium, etc. Des exemples de diluants solubles incluent le mannitol, le glucose, le sorbitol, le maltose, les dextrates, les dextrines, le dextrose, etc.

Le terme « adjuvant » désigne, dans la demande ou invention, un produit qui, ajouté au contenu à une composition immunogène, en particulier à un vaccin, accroît l'intensité de la réaction immunitaire induite chez l'hôte (humain ou non humain) auquel ladite composition est administrée. Un adjuvant peut notamment accroître la quantité d'anticorps spécifiques que ledit mammifère est capable de produire après l'administration de ladite composition et accroît donc l'efficacité de la l'immunisation. Les adjuvants sont notamment utiles quand l'antigène utilisé seul ne provoque qu'une réaction immunitaire trop faible pour procurer une bonne protection, pour réduire la quantité d'antigène à administrer à un hôte, ou encore pour faciliter certains modes d'administration de ladite composition, par exemple dans le cas d'une administration au niveau des muqueuses. Les adjuvants susceptibles d'être utilisés dans le cadre de la demande ou l'invention sont en particulier des saponines, du phosphate d'aluminium (alum), des peptidoglycanes, des carbohydrates, des peptides, par exemple le muramyl dipeptide (N-acétylmuramyl-L-alanyl-D-isoglutamine, MDP), des émulsions huile/eau, des polysaccharides, des cytokines, des hormones, l'hémocyanine de patelle, des adjuvants de la famille des dinucléotides CpG non méthylés, des adjuvants de la famille des poly IC, des adjuvants de la famille du monophosphoryl lipide A et des acides nucléiques, en particulier des ADN bactériens ou des ADN codant pour une protéine ayant un effet adjuvant, par exemple un facteur de croissance ou une cytokine, plus particulièrement le GM-CSF ou l'IL4.

Selon un mode de réalisation préféré, ledit ou lesdits véhicule(s), diluant(s), et/ou adjuvant(s) ou leur combinaison sont des substances ou une combinaison de substances pharmaceutiquement acceptables, c'est à dire appropriée(s) pour une administration à un hôte (par exemple un humain, un mammifère non humain ou un oiseau) à des fins thérapeutiques ou prophylactiques. Une telle substance ou combinaison de substance est donc préférentiellement non toxique pour l'hôte auquel elle est administrée.

La demande et l'invention concernent également un polynucléotide caractérisé en ce qu'il code pour un polypeptide chimérique, selon le code génétique universel, et en tenant compte de la dégénérescence de ce code. Le terme « polynucléotide » couvre toute molécule d'ADN ou d'ARN (mono ou bicaténaire). Ce polynucléotide peut être nu ou, alternativement, il peut être inséré dans un vecteur de clonage ou d'expression, de préférence un vecteur approprié pour l'expression dans des cellules eucaryotes. Ce vecteur est, de préférence un plasmide. Ledit polynucléotide peut être notamment assemblé par PCR. Il comporte de préférence 2000 à 50000 nucléotides.

Le terme « code » ne signifie pas nécessairement que ledit polynucléotide ne comprend que la partie codante. Ledit polynucléotide peut en effet comprendre en outre des séquences de régulation de l'expression et notamment comprendre un promoteur, par exemple un promoteur eucaryote

A titre d'exemple, ledit polynucléotide comprend, en tant que séquence codant pour le domaine CD du polypeptide chimérique de la demande ou l'invention ou pour le dérivé muté de ce domaine CD, une séquence comprenant ou consistant en une séquence choisie parmi les séquences SEQ ID NO.5, SEQ ID NO.7, SEQ ID NO. 9, SEQ ID NO. 11 SEQ ID NO. 47 et SEQ ID NO. 94.

Selon un mode de réalisation particulier, le polynucléotide de la demande ou l'invention code en outre pour un polypeptide chimérique additionnel tel que défini dans la demande ou invention. Alternativement, le polynucléotide de la demande ou l'invention peut être utilisé en conjonction avec un polynucléotide distinct, dénommé ci-après « polynucléotide additionnel », qui code pour un polypeptide chimérique additionnel tel que défini dans la demande ou invention.

Selon un mode de réalisation particulier, un polynucléotide codant pour un polypeptide chimérique additionnel comprend une séquence codant pour un signal d'importation dans le réticulum endoplasmique. Par exemple, cette séquence peut être la séquence SEQ ID NO.:1 ou une séquence codant pour un peptide de séquence SEQ ID NO.:49 à SEQ ID NO.:78. Comme indiqué précédemment, le peptide signal codé par cette séquence, lorsqu'il se trouve en position N- ou C-terminale dans le polypeptide codé par ledit polypeptide, peut être clivé lors d'une étape de maturation du polypeptide chimérique, qui a généralement lieu dans le réticulum endoplasmique ou dans le Golgi.

Le polynucléotide de la demande ou l'invention, de même que le polynucléotide additionnel, peut être placé sous le contrôle d'éléments de régulation, de clonage ou d'expression.

Ainsi, selon un mode de réalisation particulier, ledit (lesdits) polynucléotide(s) est (sont) inséré dans un vecteur de clonage ou d'expression, de préférence un vecteur d'expression et plus préférablement un vecteur approprié pour l'expression dans des cellules eucaryotes. Ledit vecteur est de préférence un plasmide. Le polynucléotide chimérique de la demande ou l'invention et le polynucléotide chimérique additionnel peuvent être insérés dans le même vecteur ou dans deux vecteurs distincts.

Lesdits polynucléotides sont en général placés sous le contrôle d'un promoteur eucaryote, de préférence un promoteur eucaryote fort tel qu'un promoteur viral, par exemple le promoteur d'un virus choisi parmi : le virus SV40, le virus du sarcome de Rous, le virus des leucémies murines (MuLV), le virus des leucémies à cellules T de l'homme adulte (HTLV-I), le virus de la leucémie bovine (BLV), le cytomégalovirus, un promoteur hybride dérivé de ces promoteurs viraux ou un promoteur viral contenant des séquences modifiées.

Lesdits polynucléotides peuvent en outre comporter une séquence kozak, en particulier la séquence nucléotidique ACCATGG ou une séquence dérivée ACCATG, dans laquelle la séquence ATG correspond au codon d'initiation de la séquence codante.

Par ailleurs, lesdits polynucléotides peuvent en outre comporter un intron.

Selon un mode de réalisation particulier, lesdits polynucléotides comprennent au moins un linker nucléotidique codant pour un linker polypeptidique tel que défini ci-dessus. Ce linker nucléotidique peut en particulier comprendre ou consister en un site de restriction. Par « site de restriction », on entend une séquence nucléotidique particulière reconnue par une enzyme de restriction de type II comme un site de coupure dans le polynucléotide. A titre d'exemple, ces linkers nucléotidiques peuvent consister en les séquences nucléotidiques TCTAGA, ou GCTAGC, ou CCTGCAGGAAGCGGCGCGCCC qui comprennent respectivement les sites des enzymes de restriction Xbal, Nhe I, et Sbf I-Asc I.

Selon un mode de réalisation particulier, la séquence dudit polynucléotide de la demande ou l'invention et/ou la séquence du polynucléotide additionnel sont optimisées pour une utilisation chez un hôte (par exemple un hôte eucaryote), en particulier un être humain, un mammifère non humain et/ou un oiseau.

La demande concerne également un vecteur de clonage ou d'expression, qui est, de préférence un plasmide, caractérisé en ce qu'il comprend un insert polynucléotidique constitué par un polynucléotide de la demande, sous le contrôle d'éléments de régulation, de clonage ou d'expression.

Ce vecteur de clonage ou d'expression peut en outre comprendre un insert polynucléotidique constitué par polynucléotide additionnel, sous le contrôle d'éléments de régulation, de clonage ou d'expression. Alternativement, ce vecteur de clonage ou d'expression peut être utlisé en conjonction avec un autre vecteur de clonage dénommé ci-après vecteur de clonage ou d'expression additionnel, qui comprend un insert polynucléotidique constitué par polynucléotide additionnel, sous le contrôle d'éléments de régulation, de clonage ou d'expression.

La demande concerne également une culture cellulaire sélectionnée parmi le groupe comprenant les cultures cellulaires de bactéries, les cultures cellulaires primaires de cellules eucaryotes animales et les lignées cellulaires, ladite culture cellulaire contenant un polynucléotide de la demande ou l'invention, un vecteur de clonage ou d'expression de la demande ou l'invention, ou un polypeptide chimérique de la demande ou l'invention. Le cas échéant, ladite culture cellulaire peut en outre comprendre un polynucléotide additionnel, un vecteur de clonage ou d'expression additionnel, ou un polypeptide chimérique additionnel. La demande concerne également une composition, en particulier une composition thérapeutique ou immunogène, comprenant un acide nucléique caractérisé en ce qu'il comprend ou consiste en un polynucléotide de la demande ou l'invention, et un support, un diluant ou un véhicule pharmaceutiquement acceptable.

L'invention est relative à une composition thérapeutique comprenant :
- un acide nucléique, caractérisé en ce qu'il comprend ou consiste en un polynucléotide de l'invention ; et
- un support, un diluant ou un véhicule pharmaceutiquement acceptable.

Ladite composition de la demande ou l'invention peut en outre comprendre un acide nucléique comprenant ou consistant en un polynucléotide chimérique additionnel. Alternativement, ladite composition de la demande ou l'invention peut être utilisée (en particulier administrée à un hôte humain ou non humain) en association avec une composition (dite additionnelle) comprenant un acide nucléique qui comprend ou consiste en un polynucléotide additionnel, et un support, un diluant ou un véhicule pharmaceutiquement acceptable.

Selon un mode de réalisation particulier, ladite ou lesdites composition(s) comprend (comprennent) en outre un adjuvant tel que défini dans la demande.

Selon un mode de réalisation particulier, ledit (lesdits) acide(s) nucléique(s) est(sont) un ADN. Les compositions immunogènes à base d'ADN ciblent *in vivo* des cellules de l'hôte à immuniser, en particulier les cellules dendritiques (notamment les cellules de Langerhans), qui sont d'excellents producteurs d'exosomes vaccinants. Ainsi, cette composition immunogène peut permettre d'induire la production, par les propres cellules d'un hôte immunisé avec ladite composition immunogène, d'exosomes porteurs du peptide ou du polypeptide d'intérêt ou d'un fragment de celui-ci.

Selon un mode de réalisation particulier, ladite ou lesdites composition(s) immunogène(s) est (sont) un vaccin, en particulier un vaccin à ADN.

La demande et l'invention concernent également une cellule recombinante productrice d'exosomes, en particulier une cellule du système immunitaire et plus particulièrement une cellule du système immunitaire choisie parmi les mastocytes, les lymphocytes, en particulier les lymphocytes B et T, et les cellules dendritiques, en particulier les cellules de Langerhans, caractérisée en ce qu'elle est recombinée avec un ou plusieurs polynucléotide(s) de la demande ou l'invention ou un vecteur de clonage ou d'expression de la demande et, le cas échéant, un ou plusieurs polynucléotide(s) additionels, ou un vecteur de clonage ou d'expression additionnel, ou qu'elle a absorbé une vésicule membranaire de la demande, ou un exosome de la demande ou l'invention, et/ou une vésicule membranaire additionnelle. En outre, la demande concerne également un élément choisi parmi un polypeptide chimérique de la demande ou l'invention, une ou plusieurs vésicule(s) membranaire(s) de la demande (en particulier un ou plusieurs exosome(s) de la demande ou l'invention), un polynucléotide de la demande ou l'invention et une composition de la demande ou l'invention, pour une utilisation comme médicament.

Lesdits éléments peuvent être utilisés en particulier pour la prophylaxie et/ou le traitement d'une infection bactérienne, virale, parasitaire, ou d'une tumeur, ou d'un déficit fonctionnel ou métabolique, en particulier un déficit enzymatique. Ils sont notamment destinés à une utilisation en immunisation, en particulier pour éliciter ou favoriser (c'est-à-dire notamment amplifier) *in vivo,* chez un hôte (humain ou non humain) une réponse humorale et/ou cellulaire contre la tumeur, le virus, la bactérie ou le parasite dont le peptide ou polypeptide d'intérêt dérive. Ils peuvent être notamment utilisés *in vivo* pour éliciter ou amplifier une réponse T CD4 et/ou T CD8 spécifique dirigée contre le peptide ou le polypeptide d'intérêt et/ou pour produire des anticorps polyclonaux et/ou monoclonaux dirigés contre le peptide ou le polypeptide d'intérêt, en particulier des anticorps dirigés contre un peptide ou un polypeptide comprenant ou consistant en un ou plusieurs épitopes conformationnels.

Dans un mode de réalisation particulier, ces éléments sont utilisés pour cibler des cellules déterminées *in vivo.*

Lesdits éléments peuvent être utilisés en particulier pour traiter des cellules cibles chez un hôte humain ou non humain (par l'apport d'une enzyme par exemple), et/ou pour détruire des cellules cibles chez un hôte humain ou non humain et/ou pour induire une réponse immunitaire dirigée par exemple contre un antigène cytosolique présent dans le polypeptide chimérique de la demande ou l'invention chez un hôte humain ou non humain (par exemple des cellules tumorales) et/ou pour faire du diagnostic (par exemple en détectant un acide nucléique lié au peptide ou polypeptide d'intérêt du polypeptide chimérique de la demande ou l'invention).

En outre, lesdits éléments peuvent être utilisés *in vitro,* pour faire du criblage de protéines et/ou analyser d'éventuelles interactions entre protéines (par exemple en détectant d'éventuels transferts d'énergie entre deux fluorophores utilisés).

La demande et l'invention concernent également l'utilisation d'un de ces éléments pour la fabrication d'un médicament destiné à la prophylaxie et/ou au traitement d'un déficit fonctionnel ou métabolique, en particulier un déficit enzymatique ou d'une tumeur ou d'une infection par un organisme pathogène ou par un agent pathogène, notamment une infection bactérienne, virale ou parasitaire, et/ou pour éliciter ou amplifier une réponse immunitaire par exemple telle que décrite ci-dessus. Suite à l'administration d'une composition de la demande ou l'invention, dont le principe actif est un polynucléotide de la demande ou l'invention (et, le cas échéant, un polynucléotide additionnel) ou une ou plusieurs vésicule(s) membranaire(s) de la demande ou un ou plusieurs exosomes de la demande ou invention (et, le cas échéant, une ou plusieurs vésicule(s) membranaire(s) additionnelle(s)), à un hôte (humain ou non humain), les cellules productrices d'exosomes de cet hôte, en particulier les cellules dendritiques, vont produire des exosomes, comportant un polypeptide chimérique et/ou un produit de dégradation de ce dernier, ce produit de dégradation pouvant s'associer naturellement à une molécule du complexe majeur d'histocompatibilité. Ces vésicules membranairesvont permettre d'éliciter ou favoriser une réponse immune dirigée contre le peptide ou le polypeptide d'intérêt.

Un « hôte » au sens de la présente demande désigne un humain ou un animal non humain.

Le terme « animal non humain » tel qu'utilisé dans la demande ou invention inclut tout mammifère non humain, notamment un rongeur (en particulier une souris, un rat, un hamster ou un lapin), un singe, un camélidé, un chat, un chien, un cheval, un mulet, un bovin, un ovin, un porcin, et inclut également un oiseau, en particulier un poulet.

L'expression « infection » telle qu'utilisée dans la demande ou invention signifie que ledit hôte (humain ou non humain) a été exposé à un organisme pathogène ou un agent pathogène en particulier à un virus enveloppé tel que défini dans la demande ou invention. En particulier une telle infection est capable d'évoluer vers des signes cliniques de pathologies induites ou accompagnant ladite infection. Le terme « infection » englobe donc également tout signe clinique, symptôme ou maladie apparaissant chez un hôte (humain ou non humain) suite à son exposition à un organisme pathogène ou un agent pathogène. Par exemple, une « infection virale » ou une « infection bactérienne » au sens de la demande ou invention inclut à la fois les phases les plus précoces de la contamination virale, bactérienne ou parasitaire, les phases intermédiaires, et les phases les plus tardives de la contamination, ainsi que les pathologies diverses qui sont la conséquence de la contamination d'un hôte par un virus, par des bactéries ou par un parasite, il inclut aussi la présence de tout ou partie de génome d'un organisme pathogène.

Le terme « prophylaxie » désigne tout degré de retardement dans le moment d'apparition de signes cliniques ou de symptômes de l'infection ou de l'apparition d'une tumeur ou de toutes autres pathologies, en particulier d'un déficit fonctionnel ou métabolique (par exemple un déficit enzymatique), ainsi que tout degré d'inhibition de la sévérité des signes cliniques ou symptômes de l'infection ou de la tumeur, y compris, mais sans limitation à, la prévention totale de l'infection ou d'un cancer. Ceci nécessite que les polypeptides chimériques décrits dans la demande ou l'invention (en particulier le polypeptide chimérique de la demande ou l'invention), les vésicules membranaires décrites dans la demande (en particulier vésicules membranaires de la demande ou exosomes de la demande ou l'invention) ou les compositions décrites dans la demande ou invention (en particulier les compositions de l'invention) soient administrés à l'hôte susceptible d'être exposé à un organisme pathogène ou un agent pathogène et/ou susceptible de développer une tumeur ou de toutes autres pathologies, en particulier d'un déficit fonctionnel ou métabolique, avant l'apparition de tout signe clinique ou symptôme de la maladie. L'administration prophylactique peut avoir lieu avant que ledit hôte ne soit exposé à l'organisme ou à l'agent pathogène responsable de l'infection, ou au moment de l'exposition. Une telle administration prophylactique sert à empêcher, et/ou réduire la sévérité de n'importe quelle infection subséquente. La prophylaxie au sens de la demande ou invention couvre également la prévention totale d'une infection ou d'un cancer ou de toutes autres pathologies.

Par « traitement », on entend l'effet thérapeutique que produisent sur un hôte les polypeptides chimériques décrits dans la demande ou l'invention (en particulier le polypeptide chimérique de la demande ou l'invention), les polynucléotides décrits dans la demande ou l'invention (en particulier le polynucléotide de la demande ou l'invention), les vésicules membranaires décrits dans la demande (en particulier les vésicules membranaires de la demande ou les exosomes de la demande ou l'invention), ou une des compositions décrites dans la demande ou invention (en particulier les compositions de l'invention), lorsqu'ils sont administrés audit hôte au moment de l'exposition à un organisme ou un agent pathogène, après l'exposition ou après l'apparition de signes cliniques ou de symptômes de l'infection ou après l'apparition d'une tumeur. Lorsque les substances actives de la demande ou l'invention sont administrées à un hôte après la contamination par un virus, ils peuvent être administrés pendant la phase de primo-infection, pendant la phase asymptomatique ou encore après l'apparition de signes cliniques ou de symptômes de la maladie.

Le terme « traitement » inclut tout effet curatif obtenu grâce à une substance active de la demande ou l'invention, ainsi que l'amélioration des signes cliniques ou des symptômes observés chez l'hôte (humain ou non humain), de même que l'amélioration de la condition de l'hôte. Ainsi, le terme « traitement » couvre notamment le ralentissement, la diminution, l'interruption, ainsi que l'arrêt d'une infection virale, bactérienne ou parasitaire ou de la croissance de tumeurs ou de toutes autres pathologies et/ou des conséquences néfastes de l'infection ou de l'apparition de la tumeur ou de toutes autres pathologies; un traitement n'exige pas nécessairement l'élimination complète de tous les signes cliniques de l'infection ou de la tumeur et/ou de tous les symptômes de la maladie, ni même l'élimination complète du virus, des bactéries, des parasites ou des cellules tumorales, ou des cellules fonctionnellement déficientes.

Les substances actives de la demande ou l'invention peuvent donc être administrées à un hôte qui présente des risques d'être exposé à un organisme ou un agent pathogène et de développer une infection ou une tumeur (prophylaxie) ou après l'exposition de l'hôte à un organisme ou un agent pathogène, en particulier après la manifestation des premiers signes cliniques ou symptômes de la maladie, par exemple après que des protéines ou anticorps spécifiques d'un virus, de bactéries, de parasites ou d'une tumeur ont été détectés dans le sang de l'hôte (traitement).

La demande et l'invention concernent a également une méthode pour prévenir et/ou traiter une infection virale, bactérienne ou parasitaire ou une tumeur ou de toutes autres pathologies, ladite méthode comprenant au moins une étape d'administration *in vivo,* à un hôte le nécessitant, du polypeptide chimérique, d'une ou plusieurs vésicule(s) membranaire(s) de la demande ou l'invention (en particulier un ou plusieurs exosome(s) de la demande ou l'invention), d'un polynucléotide de la demande ou l'invention ou d'une composition immunogène de la demande ou l'invention. Ladite méthode de traitement est, en particulier, appropriée pour et destinée à éliciter ou favoriser, *in vivo,* chez ledit hôte, une réponse humorale et/ou cellulaire contre la tumeur, le virus, la bactérie ou le parasite dont le peptide ou polypeptide d'intérêt dérive.

Les termes « administration » et « administrer » tels qu'utilisés dans la demande ou invention incluent toute administration, quelle que soit la voie d'administration choisie.

Les voies d'administration et les posologies varient en fonction d'une variété de paramètres, par exemple en fonction de l'état de l'hôte, du type d'infection et de la sévérité de l'infection à traiter ou de l'importance de la tumeur.

Le polypeptide chimérique, de même qu'une ou plusieurs vésicule(s) membranaire(s) de la demande (en particulier un ou plusieurs exosome(s) de la demande ou l'invention), le polynucléotide de la demande ou l'invention ou une composition de la demande ou l'invention sont susceptibles d'être administrés à un hôte humain ou non humain sous forme sèche, solide (en particulier cachet, poudre, gélule, pilule, granule, suppositoire, capsule polymère ou comprimé, et plus précisément comprimé à libération accélérée, comprimé gastrorésistant ou comprimé à libération prolongée), sous forme gélatineuse ou sous la forme d'une solution ou d'une suspension liquide (en particulier sirop, solution injectable, infusible ou buvable, nébulisats, microvésicules, liposomes) ou sous la forme de patch. Ces composés peuvent également se présenter sous la forme de doses sous forme sèche (poudre, lyophilisat, etc.) à reconstituer au moment de l'utilisation en utilisant un diluant approprié. Par ailleurs, ils peuvent être conditionnés pour une administration sous la forme d'une dose unique (monodose) ou multiple (multidose).

Les substances actives de la demande ou l'invention peuvent être formulées pour une administration par voie entérale, parentérale (intraveineuse, intramusculaire ou sous-cutanée), transcutanée (ou transdermique ou percutanée), cutanée, orale, mucosale, en particulier transmuqueuse-buccale, nasale, ophtalmique, otologique (dans l'oreille), vaginale, rectale, ou encore les voies intragastrique, intracardiaque, intrapéritonéale, intrapulmonaire ou intratrachéale.

Afin d'augmenter les effets bénéfiques des compositions de la demande ou l'invention (en particulier des compositions immunogènes de la demande ou l'invention), il est en effet envisageable de procéder à une administration sous la forme de plusieurs administrations successives, répétées à une ou plusieurs occasions, après un intervalle de temps particulier. Ils peuvent en outre être administrés avec un second agent thérapeutique, en particulier un agent antiviral, antibactérien, antiparasitaire ou antitumoral.

Dans le cadre d'une utilisation en vaccination, il peut également être préférable d'immuniser un hôte, dans un premier temps avec une composition immunogène à base d'un polynucléotide de la demande ou l'invention, en particulier avec un vaccin à ADN de la demande, puis, dans un deuxième temps, avec les vésicules membranaires de la demande, une composition immunogène à base desdites vésicules ou une composition immunogène dont le principe actif est un polypeptide chimérique de la demande ou l'invention ou un ou plusieurs fragment(s) de celui-ci (ledit polypeptide chimérique ou ses(s) fragment(s) pouvant être obtenus notamment par purification ou par synthèse chimique). Alternativement, il peut également être préférable d'immuniser un hôte, dans un premier temps avec les vésicules membranaires de la demande ou une composition immunogène à base desdites vésicules, puis, dans un deuxième temps, avec une composition immunogène à base d'un polynucléotide de la demande ou l'invention, en particulier avec un vaccin à ADN de la demande.

Les compositions immunogènes à base d'un polynucléotide de la demande ou l'invention et en particulier les vaccins à ADN de la demande sont de préférence administré(e)s à un hôte par voie intramusculaire ou sous-cutanée, en utilisant soit une aiguille et une seringue, soit un injecteur dépourvu d'aiguille, en particulier un pistolet à air comprimé capable de propulser, à l'intérieur de cellules d'un hôte, des microbilles d'or, de tungstène ou de platine chargées d'ADN (pistolet « biolistique » ou pistolet à gène), par exemple le pistolet « Helios® Gene Gun System» de la société BioRad.

La quantité de principe actif administrée à un hôte humain ou non humain est une quantité thérapeutiquement efficace, c'est-à-dire une quantité active, suffisante, à des posologies et pendant des périodes de temps nécessaires, pour obtenir un effet significatif et en particulier apporter un bénéfice significatif à l'hôte dans le cadre d'une administration pour la prophylaxie ou le traitement tel que défini dans la demande ou invention. Une quantité thérapeutiquement efficace est également une quantité pour laquelle les effets bénéfiques l'emportent sur un quelconque effet toxique ou néfaste du ou des principe(s) actif(s). Une telle quantité peut correspondre à une quantité suffisante pour inhiber la réplication virale, la prolifération bactérienne ou la croissance d'une tumeur de façon significative ou pour faire disparaître, réduire, ou améliorer toute infection existante provoquée l'agent ou l'organisme pathogène. La quantité thérapeutiquement efficace varie en fonction de facteurs tels que l'état d'infection, l'âge, le sexe ou le poids de l'hôte. Les régimes posologiques peuvent être ajustés de façon à obtenir un effet thérapeutique optimum. La demande et l'invention concernent également un procédé d'obtention *in vivo* de vésicules membranaires, en particulier d'exosomes, caractérisé en ce qu'il comprend une étape d'administration d'un polypeptide chimérique de la demande ou l'invention (et le cas échéant d'un polypeptide chimérique additionnel), d'une ou plusieurs vésicule(s) membranaire(s) de la demande, en particulier un ou plusieurs exosome(s) de la demande ou l'invention (et le cas échéant d'une ou plusieurs vésicule(s) membranaire(s) additionnelle(s)), d'un polynucléotide de la demande ou l'invention (et le cas échéant d'un polynucléotide additionnel), ou d'une composition de la demande ou l'invention (et le cas échéant d'une composition additionnelle), à un hôte (humain ou non humain). Ce procédé peut être notamment mis en oeuvre dans le cadre d'une méthode pour la prophylaxie et/ou le traitement d'une tumeur ou d'une infection par un organisme pathogène ou par un agent pathogène, notamment une infection bactérienne, virale ou parasitaire.

La demande et l'invention concernent en outre un procédé de production *in vitro* de vésicules membranaires et en particulier d'exosomes comportant un peptide ou un polypeptide d'intérêt et/ou un produit de dégradation de ce peptide ou un polypeptide d'intérêt, ce produit de dégradation pouvant le cas échéant s'associer à une molécule du complexe majeur d'histocompatibilité. Ledit procédé comprend les étapes suivantes :
a) l'introduction, dans une cellule productrice d'exosomes, d'un ou plusieurs polynucléotide(s) de la demande ou l'invention, qui code(nt) pour un polypeptide comprenant ledit peptide ou polypeptide d'intérêt, ou la mise en contact d'une cellule productrice d'exosomes avec une ou plusieurs vésicule(s) membranaire(s) de la demande ou avec un ou plusieurs exosome(s) de la demande ou l'invention, qui comporte(nt) un polypeptide comprenant ledit peptide ou polypeptide d'intérêt et/ou un produit de dégradation de ce polypeptide, ou avec une composition à base de telles vésicules;
b) la culture de ladite cellule productrice d'exosomes;
c) la récupération des vésicules membranaires et en particulier des exosomes produit(e)s par ladite cellule productrice d'exosomes.

Selon un mode de réalisation particulier, la cellule productrice d'exosomes est une cellule de la lignée HEK293, ou une lignée dérivée, ou une cellule du système immunitaire. En particulier, la cellule du système immunitaire peut être choisie parmi les mastocytes, les lymphocytes, en particulier les lymphocytes T et B, et les cellules dendritiques. La cellule du système immunitaire est de préférence une cellule dendritique, par exemple une cellule de Langerhans.

Selon un mode de réalisation particulier, l'étape a) consiste en la mise en contact d'une ou plusieurs vésicule(s) membranaire(s) de la demande, en particulier d'un ou plusieurs exosome(s) de la demande ou l'invention, avec une cellule dendritique.

Selon un mode de réalisation particulier, ledit procédé comprend en outre une étape intermédiaire entre les étapes a) et b), au cours de laquelle la cellule est sélectionnée et/ou stimulée pour induire et/ou augmenter la sécrétion des exosomes ou pour obtenir des exosomes présentant des qualités définies, en particulier pour induire une spécificité dans la composition des exosomes en certaines protéines cellulaires, par exemple la protéine ICAM.

Selon un mode de réalisation particulier, l'introduction, d'un ou plusieurs polynucléotide(s) de la demande ou l'invention dans une cellule productrice d'exosomes à l'étape a) est réalisée par transfection ou par transduction.

Selon un mode de réalisation particulier, l'étape c) est réalisée en purifiant les vésicules membranaires et en particulier les exosomes à partir du surnageant de culture de la cellule productrice d'exosomes par centrifugation différentielle, par ultrafiltration, par adsorption sur un support, ou par tout autre procédé.

Les vésicules membranaires et en particulier les exosomes obtenus par ce procédé font également partie de la demande ou l'invention.

La demande et l'invention concernent également l'utilisation d'une ou plusieurs vésicule(s) membranaire(s) de la demande (et, le cas échéant, d'une ou plusieurs vésicule(s) membranaire(s) additionnelle(s)), ou d'une composition à base de vésicule(s) membranaire(s) de la demande (et, le cas échéant, d'une composition additionnelle), pour produire des d'anticorps dirigés contre le peptide ou polypeptide d'intérêt, ces anticorps étant destinés à une utilisation en diagnostic ou en recherche. La demande et l'invention concernent également un procédé de préparation d'un sérum polyclonal dirigé contre un ou plusieurs peptide(s) ou polypeptide(s) antigénique(s) d'intérêt exprimé(s) à la surface de vésicules membranaires, en particulier d'exosomes, ledit procédé comprenant les étapes suivantes :
a) l'administration, le cas échéant répétée, à un animal non humain, de vésicules membranaires de la demande ou d'exosome de la demande ou l'invention, d'une composition immunogène de la demande ou l'invention ou d'un polynucléotide de la demande ou l'invention, associés ou non à un adjuvant; et
b) la récupération des anticorps formés, capables de reconnaître le ou les peptide(s) ou polypeptide(s) antigénique(s) d'intérêt.

Selon un mode de réalisation particulier, l'étape a) est suivie d'une étape de sacrifice de l'animal non humain.

La demande concerne également deux procédés de préparation d'anticorps monoclonaux dirigés contre un ou plusieurs peptide(s) ou polypeptide(s) antigénique(s) exprimé(s) à la surface de ou dans des vésicules membranaires, en particulier d'exosomes. Le premier procédé de la demande ou invention comprend les étapes suivantes:
a) la fusion, avec des cellules de myélome, de cellules spléniques préalablement obtenues chez un hôte (humain ou non humain), par exemple une souris Balb/c, auquel on a administré des vésicules membranaires de la demande ou des exosomes de la demande ou l'invention, une composition immunogène de la demande ou l'invention ou un polynucléotide de la demande ou l'invention, le cas échéant en association avec un adjuvant et le cas échéant par administration répétée;
b) la culture et la sélection des hybridomes dans des conditions permettant la production d'anticorps;
c) la récupération des anticorps monoclonaux dirigés contre les peptide(s) ou polypeptide(s) antigénique(s) d'intérêt.

Le deuxième procédé de préparation d'anticorps monoclonaux de la demande comprend les étapes suivantes:
a) l'immortalisation de cellules productrices d'anticorps par exemple des lymphocytes ou des lymphoblastes, à partir de cellules hématopoïétiques, plus particulièrement de cellules du sang, préalablement obtenues chez un hôte (humain ou non humain), par exemple une souris Balb/c, auquel on a administré des vésicules membranaires de la demande une composition immunogène de la demande ou un polynucléotide de la demande, le cas échéant en association avec un adjuvant et le cas échéant par administration répétée;
b) la culture et la sélection des cellules immortalisées dans des conditions permettant la production d'anticorps;
c) la récupération des anticorps monoclonaux dirigés contre les peptide(s) ou polypeptide(s) antigénique(s) d'intérêt.

La demande et l'invention permettent notamment de produire des anticorps cytosoliques.

L'immortalisation des cellules productrices d'anticorps à l'étape a) peut être réalisée notamment par infection de ces cellules avec un virus immortalisant. Ce virus immortalisant peut être un virus herpétique, par exemple le virus d'Epstein-Barr. Cette immortalisation peut également être effectuée par modification du génome des cellules productrices d'anticorps avec un composant immortalisant. Ce composant immortalisant peut être un composant viral, par exemple d'un virus herpétique, ou un gène cellulaire, par exemple le gène de la télomérase.

L'animal non humain auquel on a administré des vésicules membranaires, une composition immunogène ou un polynucléotide de la demande dans le cadre du procédé de préparation d'un sérum polyclonal ou des procédés de préparation d'anticorps monoclonaux peut être notamment un rongeur (en particulier une souris, par exemple une souris Balb/c, un rat, un hamster ou un lapin), un oiseau, en particulier un poulet, ou un mulet.

Selon un mode de réalisation particulier des deux procédés de préparation d'anticorps monoclonaux, les cellules spléniques ou les cellules productrices d'anticorps de l'étape a) ont été préalablement obtenues chez un hôte non humain après une étape de sacrifice dudit animal non humain.

Si nécessaire, les anticorps monoclonaux ou polyclonaux produits par les procédés de préparation d'anticorps de la demande ou l'invention peuvent être "humanisés", c'est-à-dire modifiés par génie génétique de façon remplacer au maximum les fragments constants Fc de l'espèce d'origine par des fragments humains.

La demande et l'invention concernent également l'utilisation d'un polypeptide chimérique de la demande ou l'invention (et, le cas échéant, d'un polypeptide chimérique additionnel), d'une ou plusieurs vésicule(s) membranaire(s) de la demande (et, le cas échéant, d'une ou plusieurs vésicule(s) membranaire(s) additionnelle(s)), ou d'une composition de la demande ou l'invention (et le cas échéant d'une composition additionnelle), pour la détection (en particulier *in vitro*) de partenaires spécifiques capables d'interagir avec ledit peptide ou un polypeptide d'intérêt ou avec un fragment dudit peptide ou un polypeptide d'intérêt. La demande et l'invention concernent également un procédé de criblage *in vitro* de molécules ou un procédé de sélection de cellules productrices de molécules interagissant avec un peptide ou un polypeptide d'intérêt ou avec un fragment dudit peptide ou un polypeptide d'intérêt, ledit procédé comprenant :
a) la mise en contact de vésicules membranaires de la demande ou d'exosomes de la demande ou l'invention avec une ou plusieurs molécules susceptibles d'interagir avec ledit peptide ou polypeptide d'intérêt présent dans le polypeptide chimérique additionnel;
b) la détection d'une éventuelle interaction entre ledit peptide ou polypeptide d'intérêt ou un fragment dudit peptide ou polypeptide d'intérêt et ladite ou lesdites molécules.

Les interactions entre ledit peptide ou polypeptide d'intérêt et un éventuel partenaire peuvent être mises en évidence *in vitro* par toute technique permettant de démontrer des interactions protéiques et en particulier entre une protéine et son ligand, par exemple, par des expériences de coimmunoprécipitation, par exemple par ELISA, par exemple par cytofluorimétrie en flux, par exemple par électrophorèse PAGE-SDS ou par transfert de type «Western» (Western Blot), ainsi que par toute technique de criblage à haut débit permettant de démontrer des interactions protéiques et en particulier entre une protéine et son ligand, par exemple des techniques mesurant des modifications en inositol phosphates, ou en cAMP, ou en calcium, ou des transferts d'énergie (par exemple technique FRET ou BRET) entre molécules ou entre deux domaines de molécules.

Selon un mode de réalisation particulier, les vésicules membranaires utilisées à l'étape a) du procédé de criblage de la demande ou l'invention sont telles que au moins un peptide ou polypeptide d'intérêt ou au moins un de leurs fragments est exposé, en partie ou en totalité, à l'extérieur de ladite vésicule membranaire. Ledit peptide ou polypeptide d'intérêt peut être en particulier un récepteur à multiples domaines transmembranaires, par exemple le récepteur CXCR4 ou un récepteur comportant un seul domaine transmembranaire, par exemple le récepteur CD4 ou EGF-R1.

Enfin, la demande concerne également un kit comprenant un polynucléotide de la demande et une notice d'utilisation, et, le cas échéant, un polynucléotide additionnel.

D'autres caractéristiques et avantages de la l'invention apparaissent dans les exemples et les figures qui suivent.

### DESCRIPTION DES DESSINS

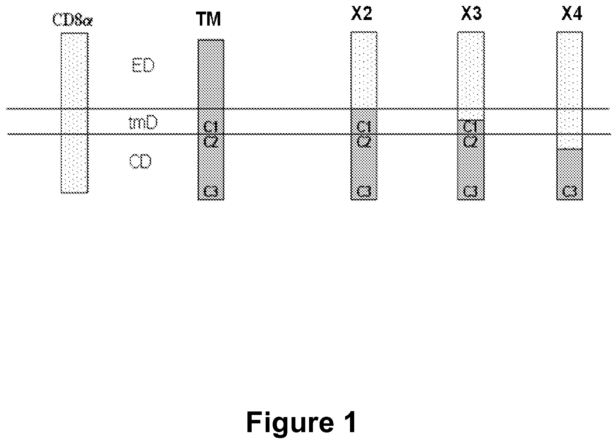

**Figure 1** **:** Représentation des différents types de constructions CD8-CD™ étudiées. **Construction X2** (séquence SEQ ID NO.79 et SEQ ID NO.80): l'ectodomaine (ED) de CD8 est fusionné avec les domaines transmembranaires (tmD) et cytoplasmique (CD) de la protéine TM du BLV. Cette construction conserve les deux sites de palmitoylation Cys 1 et Cys 2 ainsi que le résidu cystéine régulateur non palmitoylable (Cys 3) de la protéine TM du BLV. **Construction X3** (séquence SEQ ID NO.81 et SEQ ID NO.82): ED et une portion de tmD de CD8 sont fusionnés avec une portion tmD du BLV et la totalité de CD™ BLV. TmD du BLV est alors amputée de ses 15 premiers résidus. Cette construction conserve les deux sites de palmitoylation (les résidus cystéine en positions 153 et 158) ainsi que le résidu cystéine régulateur non palmitoylable (le résidu cystéine en position C-terminale ; Cys 3) de la protéine TM du BLV. **Construction X4** (séquence SEQ ID NO.83 à SEQ ID NO.86): La majeure partie de CD™ BLV est conservée. Dans cette construction, le domaine transmembranaire du CD8 alpha de souris est lié par un linker de séquence "RSR" au domaine CD de la protéine TM du BLV. Cette construction ne comporte que le résidu cystéine régulateur non palmitoylable (Cys 3) de la protéine TM du BLV.

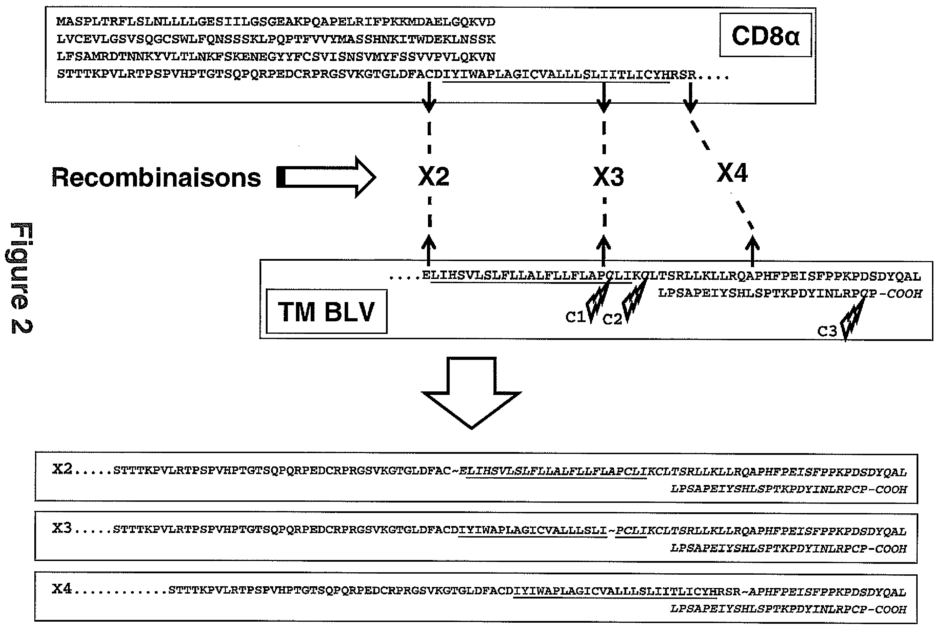

**Figure 2** **:** Récapitulatif des séquences des protéines chimères obtenues à partir de la protéine CD8 alpha de souris et de la protéine TM du BLV. Les résidus soulignés correspondent aux résidus des hélices transmembranaires des protéines CD8 alpha et TM. Les trois résidus cystéine de la protéine TM du BLV qui ont fait l'objet d'une mutation (substitution par un résidu alanine) sont indiqués par C1, C2 et C3. Les protéines chimères crées correspondent aux constructions X2 (séquence SEQ ID NO.79 et SEQ ID NO.80), X3 (séquence SEQ ID NO.81 et SEQ ID NO.82) et X4 (séquence SEQ ID NO.83 à SEQ ID NO.86).
**Figure 3** **:** Préparations des ADN par la méthode STET. Les chiffres 1 à 7 correspondent aux numéros des échantillons des produits STET. « M » représente le marqueur de taille. Les bandes correspondant à l'ADN plasmidique super enroulé sont encadrées.
**Figure 4** **:** Contrôle de la présence des ADN dans les produits de purification sur colonne. Les bandes correspondant à l'ADN plasmidique super enroulé sont encadrées. **A :** ADN obtenu par la méthode STET et déposé sur colonne. **E** et **E**' : Fractions retenues sur la colonne puis éluées. **FT :** Fraction non retenue. **M** : Marqueur de taille.
**Figure 5** **:** Dosages spectrométriques des aliquotes et ajustement des concentrations des ADN après digestion enzymatique. La double flèche indique que les concentrations ont été réajustées.
**Figure 6** **:** Contrôle de l'identité des ADN après digestion enzymatique.-Discriminations des mutants de CD8-CD™. A: discrimination des mutants pX2 AAC et pX2 CCA. B : discrimination des mutants X2 entre eux.
**Figure 7** **:** Analyse par Western Blot de l'expression des différentes chimères CD8-CD™. La chimère CD8-CD™ est indiquée par une flèche.
**Figure 8** **:** Analyse par Western Blot de l'expression des différentes chimères CD8-CD™ après immunoprécipitation. La chimère CD8-CD™ est indiquée par une flèche.
**Figure 9** **:** Analyse par Western Blot de l'expression de CD8-CD™ dans les exosomes isolés par ultracentrifugation. Le signal à 55 kDa correspond à la présence de CD8-CD™.
**Figure 10** **:** Analyse par Western Blot du contenu en CD8-CD™ des vésicules isolées après sédimentation sur gradient de densité de sucrose, pour les mutants pX4 --C et pX4 --A.
**Figure 11** **:** Analyse par Western Blot de l'expression de CD8-CD™ après lyse cellulaire, selon la présence ou non d'inhibiteurs de transport vésiculaire. La chimère CD8-CD™ est indiquée par une flèche.
**Figure 12** **:** Analyse par Western Blot de l'expression de CD8-CD™ au sein des exosomes, selon la présence ou non d'inhibiteurs de transport vésiculaire. La chimère CD8-CD™ est indiquée par une flèche.
**Figure 13** **:** Analyse par imagerie d'immunofluorescence confocale des phénotypes généraux - **Phénotype A -** Cellules HEK293 transfectées avec pX2 CAC. Ce phénotype est retrouvé chez les mutants pX2 conservant la Cys 3 : pX2 CCC, pX2 ACC, pX2 CAC et pX2 AAC. (Voir partie résultats, « localisation par immunofluorescence)
**Figure 14** **:** Analyse par imagerie d'immunofluorescence confocale des phénotypes généraux - **Phénotype B -** Cellules transfectées avec pX2 CAA. Ce phénotype est retrouvé chez les mutants pX2 ne conservant pas la Cys 3 : pX2 CCA, pX2 ACA, pX2 CAA et pX2 AAA. (Voir partie résultats, « localisation par immunofluorescence)
**Figure 15** **:** Analyse par imagerie d'immunofluorescence confocale des phénotypes généraux - **Phénotype C -** Cellules transfectées avec pX3 CCC. Ce phénotype est retrouvé chez les deux mutants pX3 étudiés : pX3 CCC et pX3 ACC. (Voir partie résultats, « localisation par immunofluorescence)
**Figure 16** **:** Analyse par imagerie d'immunofluorescence confocale des phénotypes généraux - **Phénotype D -** Cellules transfectées avec pX4 --C. Ce phénotype est retrouvé chez les deux mutants pX4 étudiés : pX4 --C et pX4 --A. (Voir partie résultats, « localisation par immunofluorescence)
**Figure 17** **:** Analyse par imagerie d'immunofluorescence confocale des phénotypes généraux - **Phénotype E -** Cellules transfectées avec pX4 stp. Ce phénotype est retrouvé uniquement chez pX4 stp. (Voir partie résultats, « localisation par immunofluorescence)
**Figure 18** **:** Analyse par imagerie d'immunofluorescence confocale des zones périnucléaires présentant un fort signal FITC. Cellules transfectées avec pX3 CCC.
**Figure 19** : Représentation du panel de mutants CD™.
**Figure 20** : Schéma du plasmide Topo^{®} (pCR-Blunt II-TOPO) utilisé pour cloner les différents produits PCR. Le vecteur Topo fournit dans le kit Topo-blunt cloning (Invitrogen) est linéarisé et possède, à chacune de ses extrémités 3'-phosphate, la Topoisomérase I du virus de la vaccine, qui permet de liguer les produits de PCR avec le vecteur Topo linéarisé.
**Figure 21** **:** Vecteur d'expression pBluescript II KS (+).
**Figure 22****:** Schéma du plasmide pKSII-CD8α.
**Figure 23** **:** Schéma du plasmide rétroviral pLPCX utilisé pour l'expression des gènes chimères. Ces gènes sont introduits au niveau du site multiple de clonage.
**Figure 24** **:** Schéma des constructions chimériques finales clonées dans le vecteur d'expression rétroviral pLPCX.
**Figure 25** **:** Visualisation de l'expression et ciblage exosomal des protéines chimères. Western blot anti-CD™ et anti-CD8α pour les lysats cellulaires et exosomaux de l'ensemble des protéines mutantes et sauvage CD8α-CD™ (taille variant de 31 kDa à 27kDa) ainsi que du témoin négatif (vecteur d'expression pLPCX contenant le CD8α seul). Les sérums de lapin anti-CD™ et anti-CD8α sont dilués au 1/200^{ème}. L'anticorps secondaire anti-IgG de lapin couplé à la peroxydase est dilué au 1/5000^{ème}.
**Figure 26** **:** Résultats comparés des expériences de détection de CD8α associé aux exosomes par cytofluorimétrie de flux et Western Blot. Tableau donnant les résultats de l'expression et le ciblage vers les exosomes des différentes protéines chimères analysées. Les mutants signalés par une étoile sont significatifs pour le ciblage exosomal. La présence de CD8α sur les exosomes est repérée par cytofluorimétrie de flux au moyen d'un anticorps monoclonal de souris fluorescent spécifique d'un épitope conformationnel de la protéine CD8α (anticorps 53-6.7 de chez Pharmingen).

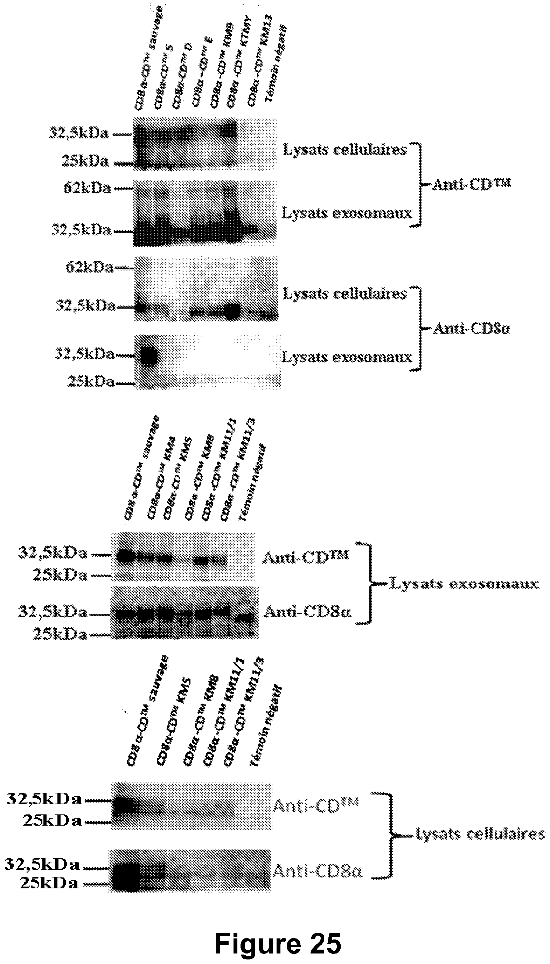

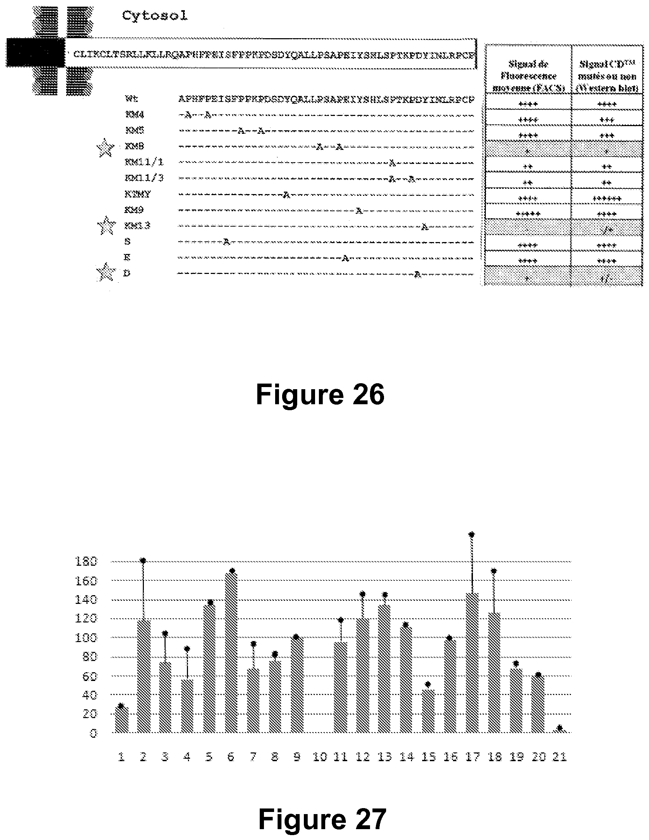

**Figure 27****: Expression du CD8 à la surface des exosomes.** Histogramme représentant la moyenne des mesures de l'exposition de chaque protéine chimère à la surface des exosomes. Ces mesures ont été effectuées par cytofluorimétrie de flux. L'exposition de chaque protéine chimère est exprimée en pourcentage par rapport à l'exposition de la protéine chimère CD8a-CDTM sauvage (construction n°9 sur l'histogramme = 100 %). L'écart type est indiqué. Les protéines chimères analysées sont : **1 :** témoin négatif (vecteur d'expression pLPCX contenant le CD8α seul) ; **2 :** KS5 ; **3 :** KS6 ; **4 :** KS8 ; **5 :** KS9 ; **6 :** KS10 ; **7 :** KS12 ; **8 :** KS14 ; **9 :** séquence sauvage ; 10 : aucune construction ; **11 :** KM4 ; **12 :** KM5 ; **13 :** S ; **14** : KTMY ; **15 :** KM8 ; **16** : E ; **17 :** KM9 ; **18 :** KM11/1 ; **19 :** KM11/3; **20 :** D et **21** : KM13. Les résultats observés confirment les résultats présentés en figures 25 et 26.
**Figure 28** **:** Représentation de différents plasmides d'expression pLPCX obtenus après clonage de gènes chimères codant pour des protéines à simple ou multiples domaines transmembranaires.
**Figure 29****:** A. Analyse par Western Blot anti-CD™-BLV réalisée sur les **extraits de protéines cellulaires** de cellules HEK293T non transfectées (N-T) ou transfectées avec les vecteurs d'expression pLPCX contenant les trois constructions codant pour les trois protéines chimères. L'anticorps primaire de lapin anti-CD™-BLV est utilisé dilué au 1/200. L'anticorps secondaire anti-IgG de lapin couplé à la peroxydase est utilisé au 1/2000. B. Les tailles des bandes observées correspondent aux tailles attendues et sont notées à droite.
**Figure 30** **:** A. Analyse Western Blot anti-CD™-BLV réalisée sur les **extraits de protéines exosomales** produites par des cellules HEK293T non transfectées (N-T) ou transfectées avec les vecteurs d'expression pLPCX contenant les trois constructions codant pour les trois protéines chimères. L'anticorps primaire de lapin anti-CD™-BLV est utilisé dilué au 1/200. L'anticorps secondaire anti-IgG de lapin couplé à la peroxydase est utilisé au 1/2000. B. Les tailles des bandes observées correspondent aux tailles attendues et sont notées à droite.
**Figure 31** **:** Révélation au Bleu de Coomassie des empreintes protéiques des différents extraits cellulaires (A) et exosomaux (B).

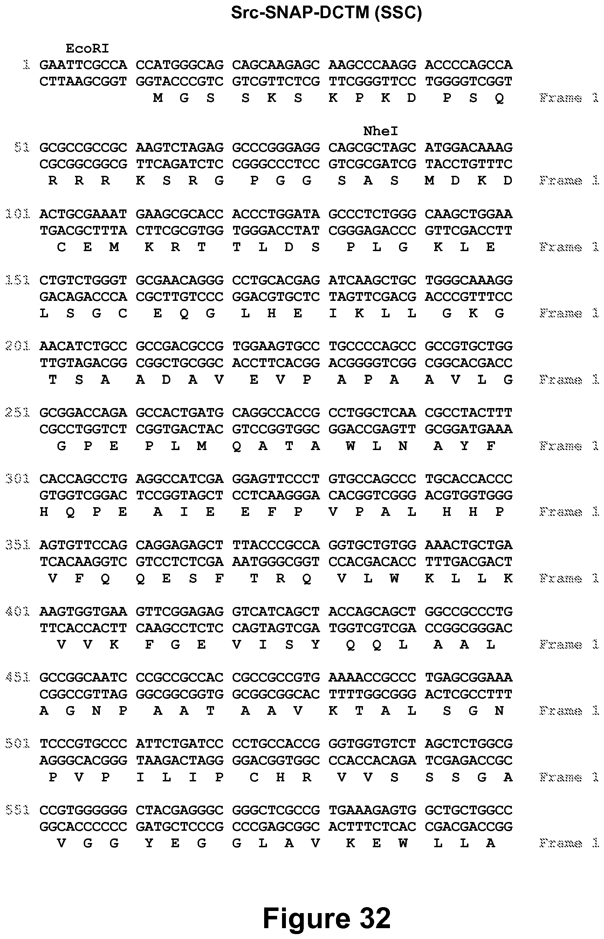

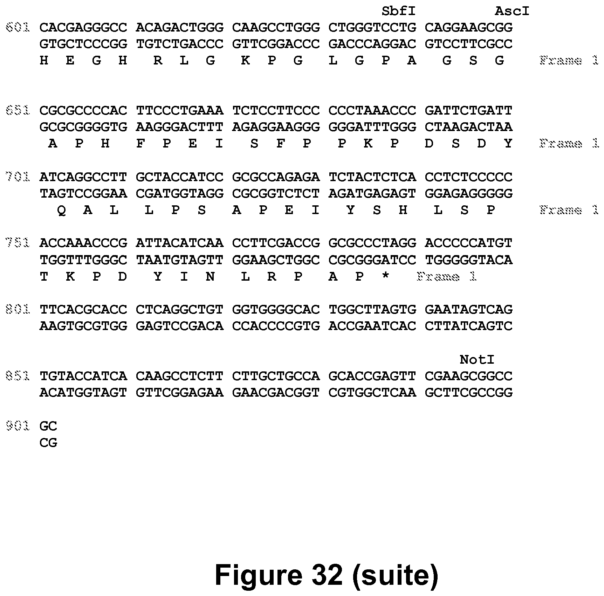

**Figure 32** **:** Séquence du gène Src-SNAP-DCTM annotée des sites de restriction bordant les 3 fragments d'ADN fusionnés et séquence de la protéine SSC codée par ce gène (SEQ ID NOs.:121 et 122).

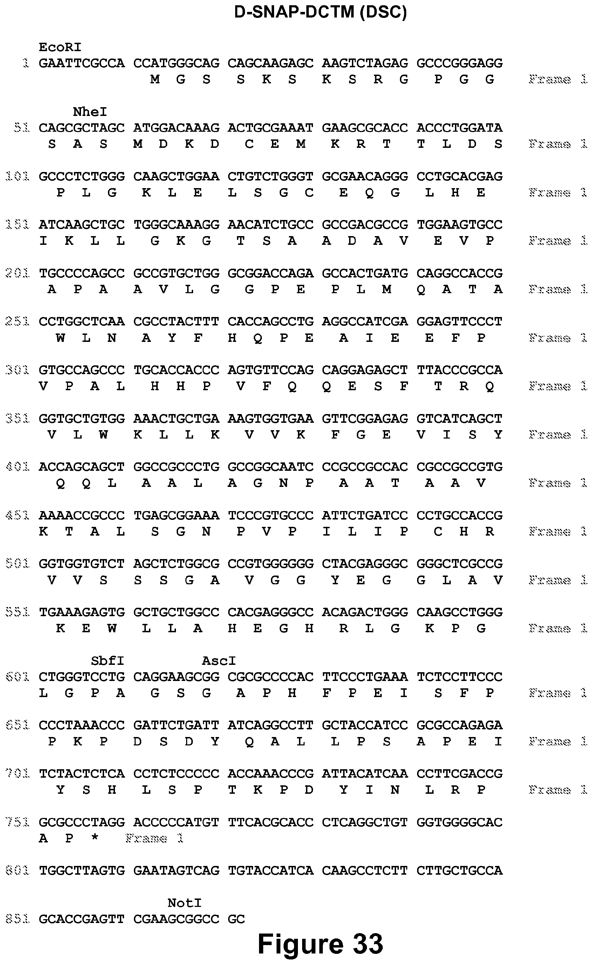

**Figure 33** **:** Séquence du gène D-SNAP-DCTM annotée des sites de restriction bordant les 3 fragments d'ADN fusionnés et séquence de la protéine DSC codée par ce gène (SEQ ID NOs.:123 et 124).

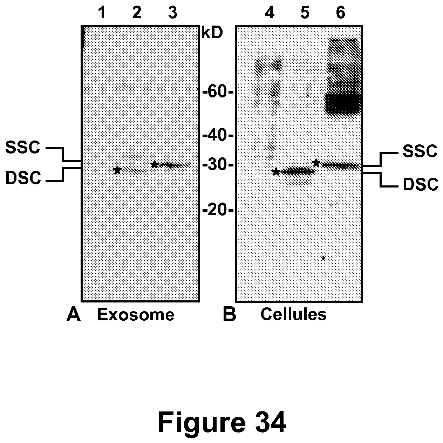

**Figure 34** **: Démonstration du ciblage dans les exosomes des protéines SSC et DSC.** Des cellules HEK 293 (5 x 10⁵ cellules) sont transfectées en présence de 2µl de JetPrime (PolyPlus transfection) avec 1µg d'ADN vecteur d'expression eucaryote (pCDNA 3.1) contenant les gènes chimères Src-SNAP-CDTM (pistes 3 et 6), ou D-SNAP-CDTM (pistes 2 et 5) ou dépourvu de ces gènes (piste 1 et 4). Les protéines des exosomes (A) sécrétés dans le milieu et des cellules sont séparés par électrophorése sur gel (PAGE-SDS) et transférées sur membrane Immobilon (Millipore). Les Western blots sont révélés au moyen d'un sérum de lapin anti-CDTM et d'un anticorps secondaire anti-IgG de lapin marqués à la peroxidase: A) analyse de 2 µg d'exosomes, B) analyse de 20 µg d'extrait cellulaire.

### EXEMPLES

### EXEMPLE 1

### MATERIEL ET METHODES

### I. Préparations des ADN plasmidiques :

### A. Transformation de bactéries :

Des bactéries compétentes DH5α (200µl) sont transformées par choc thermique avec 12,5 ng de chacun des 13 ADN étudiés codant pour les CD™ sauvages ou mutants du virus BLV, ainsi que par un plasmide dépourvu d'insert (pLPCX) faisant office de témoin négatif.

Les bactéries sont ensuite étalées sur un milieu LB/Agar contenant 50µg/ml d'ampicilline, à 37°C pendant 16h. Elles sont ensuite conservées à 4°C.

### B. Pré-culture et culture de bactéries :

Une colonie de chaque type de bactéries est ensuite mise en pré-culture dans 3 ml de milieu LB contenant 100 µg/ml d'ampicilline, à 37 °C sous agitation, pendant environ 8 h.

Chaque pré-culture servira à inoculer, au 1/200, deux flacons contenant chacun 250 ml de LB/Amp (100 µg/ml). L'incubation se fait à 37 °C, sous agitation, pendant 12 à 16 h.

### C. Maxipréparation STET :

Les cultures obtenues sont centrifugées (GR 412, Jouan) 20 min à une vitesse de 3600 rpm et à une température de 4 °C. Les culots sont repris dans 25 ml de tampon STET (*Sucrose 8* %, *Triton X100 5%, Tris HCl pH 8 50 mM, EDTA 50 mM)* auquel on ajoute 500µl de lysozyme (10 mg/ml ; Sigma) et 250 µl de RNase (2 mg/ml ; Sigma). Les tubes sont alors incubés 10 min à 100 °C puis centrifugés à 16000 rpm pendant 30 min. Les surnageants obtenus sont incubés 45 min à 65 °C en présence de 1 mg/ml de protéinase K (Amresco). Les surnageants sont prélevés et l'ADN est précipité avec 0,15 volume d'acétate de sodium (AcNa) 3 M pH 6 et 0,6 volume d'isopropanol. Les solutions sont centrifugées (Aventi 30, Beckman) à 4 °C pendant 15 min à 15000 rpm. Les culots obtenus sont lavés avec 10 ml d'Ethanol avant d'être de nouveau centrifugés selon les paramètres précédents. Les acides nucléiques obtenus sont ensuite séchés et repris dans 2 ml de TE 1X et conservés à 4 °C.

La présence d'ADN super-enroulé est vérifiée, pour chaque produit de Maxipréparation, par électrophorèse de 0,2 µl et 1 µl des produits par électrophorèse sur gel d'agarose 0,8 %.

### D. Purification sur colonne :

Les maxipréparations STET permettent l'obtention de quantités importantes de plasmides, mais dont le degré de pureté peut être amélioré. Pour cela nous avons purifié chacun des 14 plasmides en réalisant un double passage sur colonnes AX100 (Kit Nucleobond PC 100, Macherey Nagel). Après précipitation à l'isopropanol, les plasmides purifiés obtenus sont repris dans 500 µl de TE1X et conservé à 4 °C.

La présence d'ADN super-enroulé est ensuite vérifiée par électrophorèse sur gel d'agarose (0,8 %) pour chaque éluat obtenu ainsi que pour la fraction non-retenue sur les colonnes (FT=flow-trough).

La concentration en ADN des solutions obtenues est évaluée par spectrophotométrie, à une longueur d'onde de 260 nm.

### E. Aliquotage et précipitation à l'EtOH :

On aliquote chaque type d'ADN par tubes de 50 ou 100 µg, puis on réalise une précipitation avec de l'ethanol (EtOH) et NaCl, sous hotte a flux laminaire, afin de stériliser les plasmides. Les culots obtenus sont repris à raison de 200 µl de TE1X pour 100 µg de plasmides, soit une concentration de 500 ng/µl. La présence d'ADN dans les aliquotes est vérifiée par électrophorèse sur gel d'agarose 0,8 %.

### F. Dosages spectrophotométriques des aliquotes obtenus :

Les aliquotes sont dosés par spectrophotométrie à une longueur d'onde de 260 nm. Les échantillons sont dilués au 1/50 et sont dosés dans un volume final de 500 µl.

### G. Digestions enzymatiques :

L'identité de chaque plasmide est contrôlée par digestion de 20 ng de chacun d'eux par des enzymes de restriction (New England Biolabs): le couple Hind III/Not I, Xba I, Aat II, Pac I, Sfo I. Les plasmides sont discriminés selon le nombre de bandes obtenus et leur poids moléculaire, après électrophorèse sur gel d'agarose (0,8 %) de chaque produit de digestions.

### II. Analyse de l'expression des chimères

### A. Culture cellulaire :

Les cellules HEK293 (Human Embryonic Kidney cells, cellules embryonnaires de rein humain) sont cultivées dans un milieu DMEM (Dulbecco's modified Eagle's medium), complémenté par 10 % de sérum de veau foetal (SVF) et 20 µg/ml de gentamicine, à 37 °C sous 5 % de CO₂.

### B. Transfection :

En vue des transfections, on ensemence 5.10⁵ cellules dans 2 ml de milieu par puits de 9,6 cm² (plaques 6 puits). Après incubation une nuit à 37 °C sous 5% de CO₂,le milieu est remplacé par du DMEM complet sans antibiotiques.

Les cellules sont alors transfectées par un polyplexe formé par la complexation, dans un tampon NaCl (0,15 M), de 6 µl de Jet PEI (Qbiogen) et 3 µg de chaque acide nucléique à tester; Un plasmide exprimant LacZ fait office de témoin positif pour la transfection. Après 24 h d'incubation à 37 °C sous 5 % de CO₂, le milieu est éliminé et remplacé par du DMEM complet avec 20 µg/ml de gentamicine. L'expression optimum des plasmides est obtenue 48 h après le début de la transfection.

Dans certains cas, afin d'analyser l'importance du transport vésiculaire dans la dégradation et le ciblage de CD™, nous avons utilisé des inhibiteurs de transport vésiculaire qui sont la bafilomycine et Ly 294002. 32 h après transfection, chaque inhibiteur est ajouté au milieu de culture à une concentration de 0,5 µM pour la bafilomycine et 10 µM pour Ly 294002.

### C. Extraction protéique :

48 h après transfection les cellules sont lysées par un tampon TNE-NP40 0,5% auquel on ajoute 0,1 mM de PMSF. Après clarification par centrifugation (14000 rpm, 15 min, 4 °C ; Eppendorf 5417R), les lysats sont dosés par spectrophotométrie (à 595 nm) selon la technique de Bradford.

Pour chaque échantillon, on prélève 200 µg de protéines que l'on complète par du tampon de lyse pour un volume finale de 30 µl auquel on rajoute 10 µl de tampon d'échantillon 4X (CB 4X : *NaOH 200 mM, EDTA 20 mM, SDS* 2%, *Vert de bromocrésol 0,05%, Glycérol 10%*).

### D. Préparation des exosomes :

Avant lyse des cellules, les milieux de cultures sont récupérés et précentrifugés à 10000 rpm pendant 20 min à une température de 4 °C (Aventi 30, Beckman). Les surnageants obtenus sont ensuite ultracentrifugés (Optima LE-80K, rotor Ti 50, Beckman) à 100000 g pendant 2 h à une température de 4 °C. Les culots obtenus sont repris dans 100 µl de CB 1X.

Nous avons aussi analysé les exosomes par sédimentation sur gradient de densité de sucrose. Les culots obtenus après ultracentrifugation des milieux de culture sont alors repris dans 100 µl d'une solution de sucrose à 0,25 M.

Les vésicules sont ensuite déposées sur un gradient de sucrose préparé avec 8 couches (de 1,2 ml) de densités différentes (en molarité) : 0,5 / 0,75 / 1 / 1,25 / 1,5 / 1,75 / 2 / 2,5. Les tubes sont alors centrifugés (Optima LE-80K, rotor SW 41, Beckman) à 39 000 rpm pendant 16 h, à une température de 4 °C.

Après centrifugation, on prélève le gradient par fraction de 700 µl. Les protéines sont ensuite précipitées par addition d'un même volume de TCA 30 %. Les tubes sont conservés 2h à 4°C puis centrifugés (Eppendorf, 5417R) à une température de 4 °C pendant 20 min à 13000 rpm. Les culots sont repris dans 500 µl d'acétone puis de nouveaux centrifugés comme précédemment.

Les culots alors obtenus sont repris dans 80 µl de CB1X puis analysés par migration sur gel d'acrylamide-SDS et Western Blot.

### E. Western Blot et Immunoprécipitation :

Les échantillons protéiques obtenus sont analysées par western blot : après migration et séparation sur gel d'acrylamide (12,5 %), les protéines sont transférées sur membrane hydrophobe PVDF (Immobillon-P, Millipore).

La présence des mutants de la protéine TM est révélée par immunomarquage à l'aide des anti-corps suivant :
- *Anticorps primaire :* antisérum de lapin Anti-CD™ BLV (Dilution 1/200).
- *Anticorps secondaire :* Anti-IgG de lapin marqué à la péroxydase (Dilution 1/5000 ; Jackson Immuno Research, JIR)

La présence de récepteurs à la transferrine est révélée par immunomarquage à l'aide des anti-corps suivant :
- *Anticorps primaire :* IgG de souris anti-TFr humain (Dilution 1/200 ; Zymed)
- *Anticorps secondaire :* Anti-IgG de souris (Dilution 1/5000 ; JIR)

Afin d'accroître sélectivement la concentration de la protéine étudiée, nous avons aussi réalisé des immunoprécipitations sur les lysats protéiques, préalablement à la migration sur gel. Après normalisation des quantités de protéines, l'adsorption du lysat se fait sur de la sepharose 6B, afin d'éliminer les réactions non spécifiques et l'immunoprécipitation est réalisée avec de la protéine sepharose 6A, en présence de 2,5 µg des anticorps suivant :
• *Anticorps primaires :*
   IgG de souris Anti-CD8 de souris (19/178) (JIR)
   IgG de rat Anti-CD8 de souris (53/672) (JIR)
• *Anticorps secondaires :*
   Anti-IgG de souris (JIR)
   Anti-IgG de rat (JIR)

Les culots obtenus sont alors repris dans 70 µl de CB 1,5X.

### III. Localisation par immunofluorescence

### A. Préparation des lamelles.

Les lamelles type coverslips sont stérilisées à l'EtOH absolu, sous hotte à flux laminaire, puis placées dans des puits de 1,9/cm² (plaques 24 puits) avant d'être « coatées » à la poly-L-Lysine (25 µg/ml, Sigma) pendant 1 h à 37 °C. Après lavage au PBS, les lamelles sont conservées à 4 °C, dans 1 ml de PBS.

### B. Culture cellulaire et transfection.

Les cellules HEK293 sont cultivées et transfectées selon la méthode décrite dans la partie « Analyses de l'expression des chimères ». 24 h après transfection, les cellules sont reprises dans 35 ml de DMEM complet, puis distribuées dans des puits de 1,9 cm² (plaques 24 puits), contenant les lamelles préalablement stérilisées et « coatées », à raison de 1 ml de dilution cellulaire par puits. Les cellules ainsi mises en culture sont incubées 24 h à 37 °C, sous 5 % de CO₂.

### C. Fixation et perméabilisation.

Les cellules sont ensuite fixées pendant 30 min avec une solution de formaldéhyde (4 %) puis perméabilisées au Triton X-100 (0,2 % final). Après 3 rinçages de 10 min au PBS, les lamelles sont conservées avec 1 ml de PBS, à 4 °C.

### D. Marquages pour l'immunofluorescence.

Les différents anticorps à disposition nous ont permis de tester plusieurs marquages pour la localisation par immunofluorescence:

### Marquages CD8 :

N°1 : IgG de souris / anti-CD8 de souris (19/178) (JIR) + Anti-IgG de souris FITC (JIR)
N°2 : Ascites de souris / anti-CD8 de souris (19/178) (JIR) + Anti-IgG de souris FITC (JIR)
N°3 : IgG de rat / anti-CD8 de souris (53/672) (JIR) + Anti-IgG de rat FITC (JIR)
N°4 : IgG de rat (53/6.7) / anti-CD8 de souris FITC (Pharmingen)

### Marquages des compartiments intra-cellulaires :

N°5 : IgG de souris / anti-CD63 (Lamp3) humain (Zymed) + Anti-IgG de souris Cy3 (Sigma)
N°6 : IgG de souris / anti-Lamp1 humain + Anti-IgG de souris Cy3 (Sigma)
N°7 : IgG de souris / anti-Tf2 humain (Zymed) + Anti-IgG de souris Cy3 (Sigma)
N°8 : IgG de lapin / anti-caveolin (BD) + Anti-IgG de lapin TRITC (JIR)

### RESULTATS

Les connaissances sur le processus de formation des exosomes sont partielles. De même, les fonctions associées au domaine cytoplasmique de Env sont mal caractérisées. La présente étude repose sur l'hypothèse que le modèle du virus de la leucémie bovine (BLV) est un bon outil pour étudier les phénomènes de formations des exosomes et des particules virales et que le domaine cytoplasmique de la protéine TM (CD™) du BLV est un outil potentiellement intéressant pour le développement de la vaccination par « exosome display ».

Pour évaluer ces potentialités et afin de caractériser les fonctions responsables du ciblage de la protéine TM du BLV, et plus particulièrement le rôle des résidus cystéine palmitoylés ou non, nous avons développé plusieurs vecteurs permettant l'expression, au sein de cellules eucaryotes, de protéines transmembranaires chimères comprenant l'ectodomaine de la protéine CD8 de souris et le domaine CD™ (protéines chimères CD8-CD™). Des chimères CD8-CD™ sauvages ainsi que des chimères CD8-CD™ mutées ont été exprimées.

L'ectodomaine CD8 de souris est, dans une cellule humaine, un élément neutre n'interférant pas avec les récepteurs cellulaires. Les chimères utilisées nous assurent donc de l'absence d'interactions entre l'ectodomaine des protéines et les structures cellulaires.

En conséquences, ces chimères permettent d'étudier spécifiquement les domaines cytoplasmique et transmembranaire de la protéine TM, en évitant les phénomènes engendrés par l'ectodomaine de la protéine TM et par la protéine SU qui lui est associée.

Trois types de constructions ont été utilisés (voir figures 1 et 2). A partir de ces constructions, nous avons développé différents vecteurs d'expression pLPCX permettant l'expression de CD8-CD™ sauvages ou mutées au niveau des résidus cystéine 1, 2 et 3 (schématisé par le sigle : CCC). Les résidus cystéine sont alors remplacés, ou non, par des résidus alanine, qui ne peuvent pas être palmitoylés.

Les protéines CD8-CD™ sauvages ou mutés étudiées sont :
- 1 : pX2 CCC (phénotype sauvage)
- 2 : pX2 ACC
- 3 : pX2 CAC
- 4 : pX2 AAC
- 5 : pX2 CCA
- 6 : pX2 ACA
- 7 : pX2 CAA
- 8 : pX2 AAA
- 9 : pX3 CCC (phénotype sauvage)
- 10 : pX3 CAC
- 11 : pX4 - - C (phénotype sauvage)
- 12 : pX4 - - A
- 13 : pX4 stp (*pX4 stp est composé uniquement par ED, tmD et une petite partie du CD*™ *de CD8).*

Ces constructions nous ont permis d'étudier, au niveau du ciblage de la protéine TM : les conséquences de la mutation des résidus cystéine, l'importance de l'intégrité du domaine trans-membranaire de la protéine TM, et l'importance du domaine cytoplasmique de la protéine TM (CD™).

### I. Préparation des ADN plasmidiques :

Dans un premier temps, nous avons produit, en grande quantité, chacun de nos 13 ADN ainsi qu'un vecteur sans insert (pLPCX) faisant office de témoin négatif. Après culture de bactéries transformées par nos différents plasmides, les ADN obtenus ont été purifiés successivement par la méthode STET puis sur colonne. L'identité des plasmides obtenus a ensuite été contrôlée par digestion enzymatique.

### A. Préparations des ADN par la méthode STET :

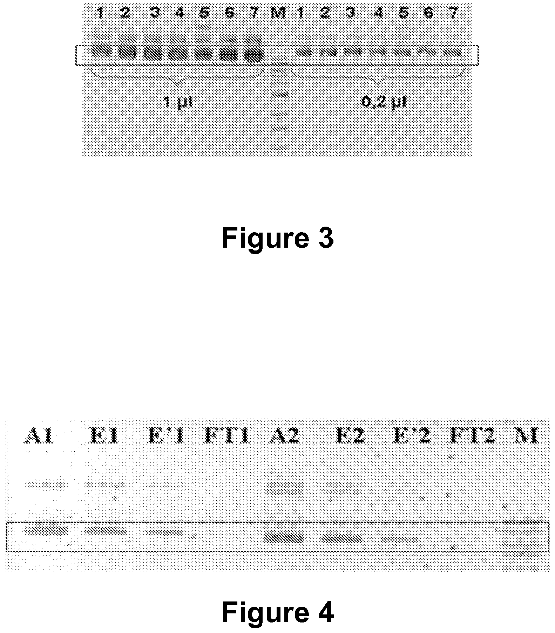

Afin de vérifier l'intégrité des ADN plasmidiques et le rendement des préparations STET, nous avons réalisé une migration de 1µl et 0,20µl de chacun d'eux, sur gel d'agarose (0,8% ; voir figure 3). La non-dégradation des ADN est mise en évidence par la présence de bandes distinctes d'ADN super enroulés de tailles homogènes.

### B. Contrôle de la présence des ADN dans les produits de purification sur colonne :

Les ADN obtenus par la méthode STET sont purifiés sur colonnes, les fractions retenues puis éluées ainsi que les fractions non retenues sont ensuite analysées par électrophorèse sur gel afin de vérifier l'intégrité des ADN purifiés obtenus. Le gel présenté en figure 4 nous indique que l'ADN super-enroulé est bien présent dans les fractions pures éluées des colonnes (E et E') mais indétectable dans les fractions non retenues (FT).

### C. Dosages spectrométriques des aliquotes et ajustement des concentrations des ADN après digestion enzymatique :

Les ADN purifiés sont ensuite dosés au spectrophotomètre. Les concentrations alors obtenues varient, en fonction des aliquotes, entre 149 µg/ml et 584 µg/ml. Après précipitation à l'EtOH, les ADN des aliquotes sont repris dans du TE1X et ajustés à une même concentration.

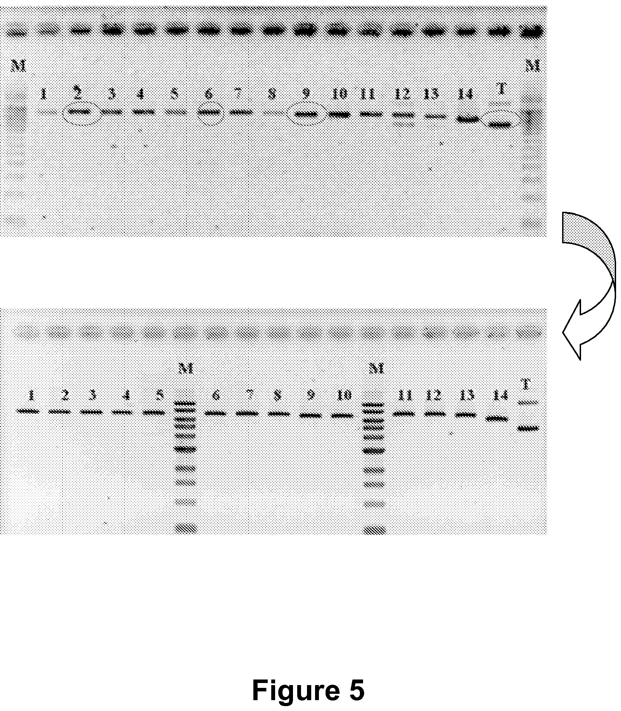

Afin de vérifier les concentrations d'ADN, nous avons linéarisé les plasmides par digestion enzymatique, à l'aide du couple Hind III/Not I, puis nous les avons analysé sur gel d'électrophorèse (voir figure 5, profil supérieur). Il est apparu que l'intensité des bandes obtenues était encore variable. Nous avons donc réajusté les concentrations en fonction des dosages spectrométriques et de l'intensité des bandes obtenues après la digestion (voir figure 5 - profil inférieur). L'obtention de bandes d'intensités égales, en plus d'assurer l'exactitude des concentrations de nos ADN avant leurs transfections dans des cellules, permettra une meilleure lisibilité des électrophorèses après digestions enzymatiques, lors du contrôle de l'identité des plasmides.

### D. Contrôle de l'identité des ADN après digestion enzymatique.

### 1) Principe général :

Afin de s'assurer de l'identité des 14 ADN préparés, tous sont contrôlés par digestion à l'aide des enzymes de restriction Hind III et Not I, Xba I, Aat II, Pac I ainsi que Sfo I qui doivent résulter en des combinaisons différentes de profils sur gels pour chacun des ADN. La migration sur gel d'agarose (0,8%) des produits de digestions permet la discrimination des ADN en fonction du nombre et de la taille des bandes obtenues, comme indiqué dans le tableau 1 (remarque : les bandes d'une taille inférieure à une centaine de paires de bases ne sont pas visibles sur nos gels d'électrophorèse).

La digestion des constructions étudiées permet de s'assurer de l'identité de chaque plasmide. La discrimination des grands types de construction CD8-CD™ se fait par l'analyse du nombre et de la taille des bandes obtenus après digestion avec les enzymes Hind III/Not I et Xba I. La discrimination entre les différents mutants des résidus cystéine se fait après digestion par les enzymes Aat II, Pac I et Sfo I. Le nombre de bandes alors obtenus est plus important qu'avec les digestions par Hind III/Not I et Xba I, et la discrimination est réalisée en se basant principalement sur la présence ou non de bandes spécifiques (en gras).

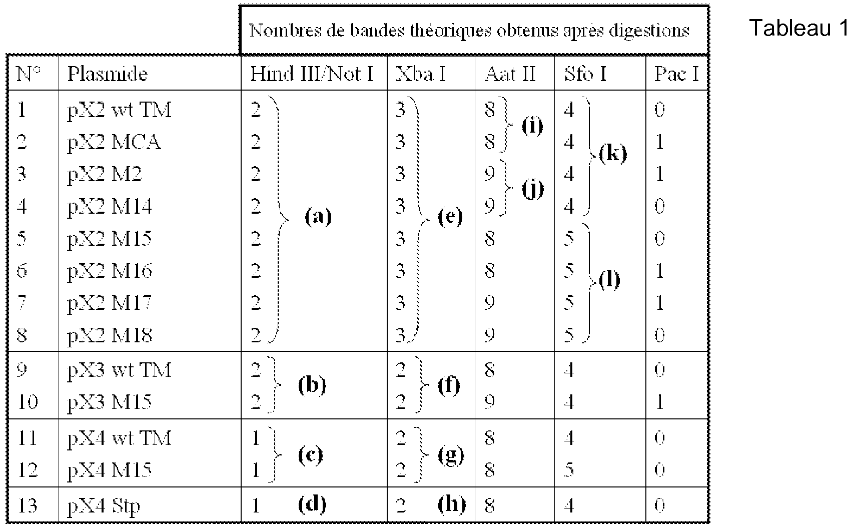

Tailles des bandes attendues (en bp) :

| | |
|---|---|
| **(a) :** 7206 / 49 | **(g) :** 6552 / 610 |
| **(b) :** 6868 / 311 | **(h) :** 6552 / 385 |
| **(c)** : 7162 | **(i) : 4285** / 1055 / 801 / 498 / 294 / 186 / 83 / 53 |
| **(d)** : 6937 | **(j) : 3423** / 1055 / **862** / 801 / 294 / 186 / 83 / 53 |
| **(e) :** 6822 / 367 / 66 | **(k) :** 4276 / **2538** / 310 / 131 |
| **(f) :** 6812 /367 | **(I)** :4276 / **1839** / **699** / 310 / 131 |

### 2) Exemple:

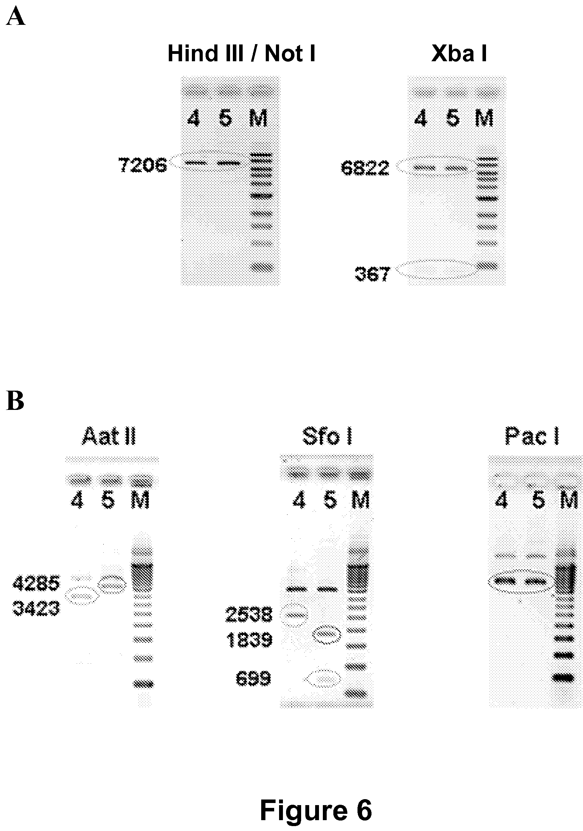

Pour la discrimination des mutants pX2 AAC et pX2 CCA, les gels obtenons sont représentés en figure 6A.

### Interprétation :

- Digestion par Hind III/ Not I : l'obtention d'une bande unique, à environ 7206 pb, correspond à la présence de constructions X2 ou X4 dans chacun des deux échantillons.
- Digestion par Xbal : l'obtention de deux bandes à environ 6822 (ou 6812) et 367 bp correspondent à la présence de constructions X2 ou X3 dans chacun des deux échantillons.

En recoupant ces deux résultats nous pouvons affirmer que nous sommes donc bien en présence de ***deux constructions de type X2.***

On cherche ensuite à discriminer les mutants X2 entre eux (voir figure 6B):

### Interprétation :

- Digestion par AatII :
   - **Echantillon 4** : l'obtention d'une 1 bande spécifique avoisinant 3423 pb (rouge) correspond à la présence d'un des plasmides X2 suivant : ***pX2 CAC, pX2 AAC**, **pX2 CAA*** ou ***pX2 AAA.***
   - **Echantillon 5** : l'obtention d'une 1 bande spécifique avoisinant 4285 pb (bleu) correspond à la présence d'un des plasmides X2 suivant : ***pX2 CCC, pX2 ACC, pX2 CCA*** ou ***pX2 ACA**.*
- Digestion par Sfo I :
   - **Echantillon 4 :** l'obtention d'une bande spécifique avoisinant 2538 pb (rouge) correspond à la présence d'un des plasmides X2 suivant : ***pX2 CCC, pX2 ACC**, **pX2 CAC, pX2 AAC**.* En recoupant ce résultat avec celui de la digestion par Aat II, nous pouvons alors en déduire que nous sommes en présence d'un des plasmides suivant : ***pX2 AAC*** ou ***pX2 CAC.***
   - **Echantillon 5** : l'obtention de 2 bandes spécifiques avoisinant 1839 pb (noir) et 699 (vert) pb correspond à la présence d'un des plasmides X2 suivant : ***pX2 CCA, pX2 ACA, pX2 CAA, pX2 AAA**.* En recoupant ce résultat avec celui de la digestion par Aat II, nous pouvons alors en déduire que nous sommes en présence d'un des plasmides suivant : ***pX2 CCA*** ou ***pX2 ACA**.*
- Digestion par Pac I :
   Nous obtenons pour chaque échantillon, 1 bande de faible intensité correspondant à de l'ADNp non super enroulé ainsi qu'une bande de forte intensité correspondant à nos ADNp super enroulé (bleu) mettant en évidence l'absence de digestion par Pac I, pour ces échantillons.

Les échantillons 4 et 5 correspondent respectivement aux mutants ***pX2 AAC*** et ***pX2 CCA**.*

Ce processus a été appliqué à l'identification de tous les plasmides.

### 3) Conclusion :

Lors de cette étape de préparation des ADN, nous avons obtenus plusieurs milligrammes de chacun des 14 plasmides purifiés et leurs identités ont toutes été confirmées après contrôle par digestions enzymatiques et analyses sur gels. De plus, tous les ADN ont été ajustés à une même concentration.

### II. Analyse de l'expression des chimères :

Afin d'évaluer l'expression des ADN, une même quantité de chacun des plasmides a été transfecté au sein de cellules HEK293. Les protéines ainsi exprimées sont analysées par migration sur gel suivie d'un transfert sur membrane PVDF. Les membranes sont ensuite révélées à l'aide d'un sérum de lapin Anti-CD™ et d'un anticorps secondaire Anti-Lapin marqué à la péroxydase.

### A. Analyse de la présence de CD8-CD™ au sein des lysats cellulaires :

### 1) Analyses des lysats bruts :

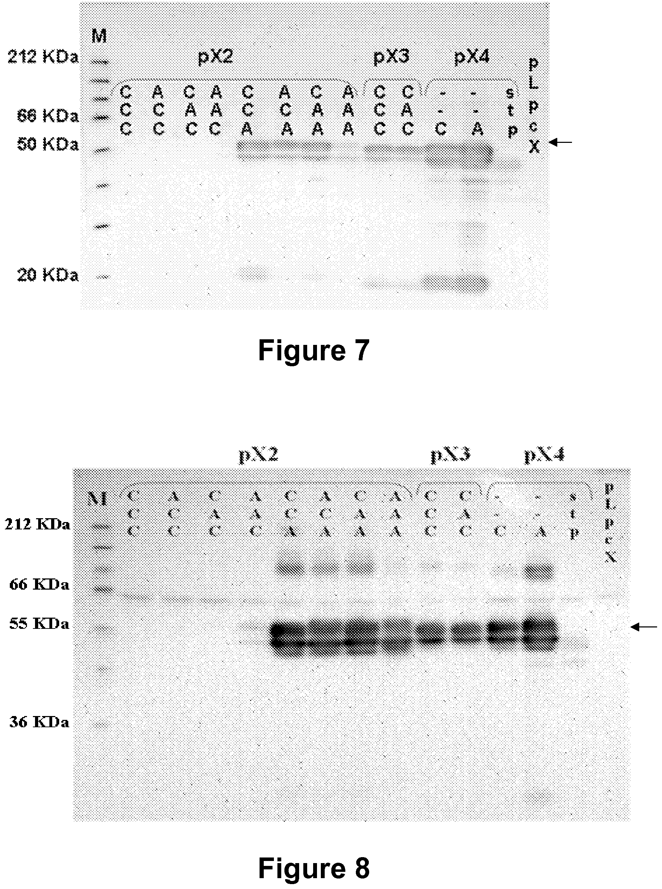

Après 48h de transfection, les cellules sont lysées et les extraits obtenus sont dosés selon la technique de Bradford. Nous avons analysé 200µg de protéines brutes, issues de ces lysats, par migration sur gel et western blot révélé avec un anticorps anti-CD™ (voir figure 7).

Différents signaux spécifiques sont alors observables selon les échantillons :
- Une double bande à 50 kDa (bleu) correspondant à la présence de CD8-CD™. Les deux bandes correspondent à deux niveaux de glycosylation de CD8.
- Un signal à environ 20 kDa (rouge) d'origine indéterminée.

Les échantillons pX2 CCC, pX2 ACC, pX2 CAC, pX2 AAC, pX4 stp et pLPCX ne présentent pas ces bandes.

### 2) Analyse des lysats après immunoprécipitation par des anticorps anti-CD8:

Afin d'abaisser le seuil de détection de CD8-CD™ au sein des lysats cellulaires, nous avons utilisé une grande quantité d'extrait et concentré les chimères par immunoprécipitation avec des anticorps monoclonaux spécifique d'un épitope conformationnel de l'ectodomaine du CD8.

Après 48h de transfection, les cellules sont lysées et les extraits obtenus sont dosés selon la technique de Bradford. Après normalisation des concentrations en protéines, les extrait sont immunoprécipités en présence des anticorps Anti-CD8 et de protéine A sepharose. Les produits obtenus sont analysés par migration sur gel et Western Blot révélés avec un anticorps anti-CD™ (voir figure 8). Différents signaux sont alors observables selon les échantillons :
- Une double bande à 55 kDa (bleu) correspondant à la présence de CD8-CD™.
- Un signal non identifié, retrouvé pour chaque échantillon, à environ 66 kDa.
- Un ou plusieurs signaux d'un poids moléculaire supérieur à 66 KDa correspondant probablement à la présence de protéines CD8-CD™ multimérisées.

Ici, l'échantillon pX2 AAC présente un signal détectable à 50KDa contrairement à l'analyse effectuée sans immunoprécipitation. En revanche aucun signal n'est détectable pour les échantillons pX2 CCC, pX2 ACC, pX2 CAC, pX4 stp et pLPCX.

### 3) Récapitulatifs de l'analyse de l'expression de CD8-CD™ au sein de lysats cellulaires :

En corrélant les données obtenues par western blot sur les lysats cellulaires avec les caractéristiques de chaque chimère (intégrité de tmD BLV, présence du troisième résidu de cystéine et nombre de résidus cystéine), nous pouvons établir le tableau 2 ci-dessous.

Il apparaît que, en plus du CD™ du BLV, deux facteurs puissent jouer sur la présence des chimères CD8-CD™. Ces facteurs sont : la présence ou non de tmD BLV, et la présence ou non du troisième résidu de cystéine (Cys 213).

### B. Analyse de l'association de CD8-CD™ avec les exosomes :

Les différents ADN utilisés ne varient que de quelques nucléotides. II est donc probable que les quantités de CD8-CD™ synthétisées soient équivalentes. Cependant il est apparu, lors de l'analyse de l'expression de CD™ au sein de lysats cellulaires, que certaines chimères ne sont pas détectables 48h après transfection. Cette absence de signal serait le reflet de la disparition de certaines de nos protéines chimériques. Cette disparition pourrait être due soit à une dégradation rapide de ces chimères, soit à leur sécrétion sous forme d'exosomes. Nous avons donc cherché à détecter la présence de CD8-CD™ au sein d'exosomes.

**Tableau 2**

| Construction | tmD BLV | Nbre de Cystéines | Signal CD™ BLV |
|---|---|---|---|
| pX2 CC**C** | Complet | 3 | Nul |
| pX2 AC**C** | Complet | 2 | Nul |
| pX2 CA**C** | Complet | 2 | Nul |
| pX2 AA**C** | Complet | 1 | + |
| pX2 CC**A** | Complot | 2 | + + + + |
| pX2 AC**A** | Complet | 1 | + + + + |
| PU2 CA**A** | Complet | 1 | + + + + |
| pX2 AA**A** | Complet | 0 | + + |
| pX3 CC**C** | - 15 aa | 3 | + + + |
| pX3 CA**C** | - 15 aa | 2 | + + + |
| pX4 - - **C** | Absent | 1 | + + + + |
| pX4 - - **A** | Absent | 0 | + + + + + |
| pX4 stp | Absent | 0 | Nul |
| pLpcX | Absent | 0 | Nul |

### 1) Contenu en CD8-CD™ des vésicules isolées par centrifugation :

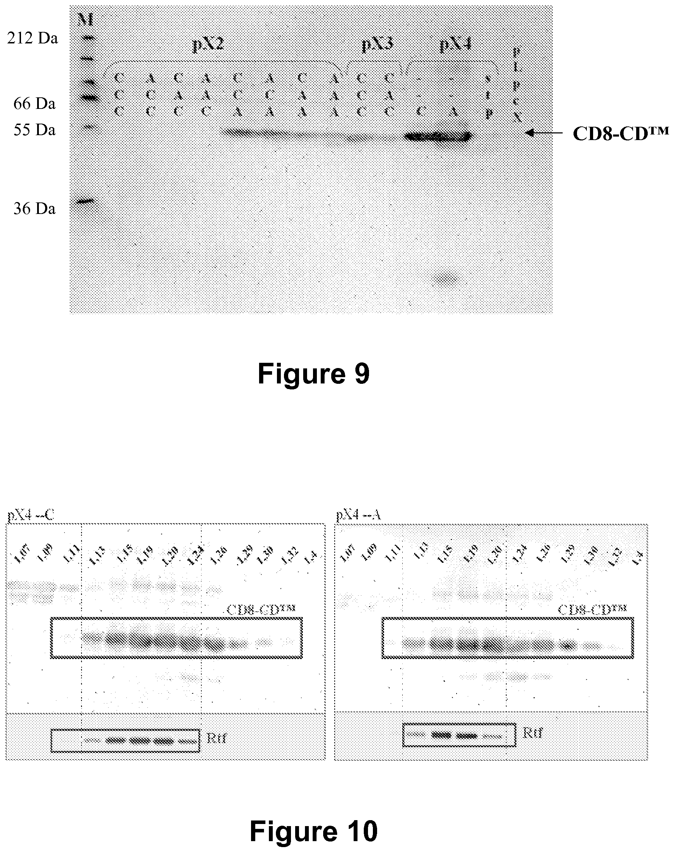

Après 48h de transfection, les milieux de cultures sont récupérés et centrifugés en vue d'isoler les exosomes. Les culots obtenus sont alors analysés par migration sur gel et Western Blot révélé avec un anticorps anti-CD™ (voir figure 9).

On peut visualiser, selon les échantillons, un signal à 55 kDa correspondant à la présence de CD8-CD™. Ce signal est non détectable pour les échantillons pX2 CCC, pX2 ACC, pX2 CAC, pX2 AAC, pX4 stp et pLPCX.

### 2) Contenu en CD8-CD™ après sédimentation des vésicules sur gradient de densité de sucrose :

Afin de nous assurer que le signal obtenu après ultracentrifugation des milieux de culture est bien du à la présence des chimères au sein d'exosomes et non pas à la présence de débris cellulaires contenant CD8-CD™, nous avons analysé les culots d'exosomes obtenus selon la méthode décrite précédemment par sédimentation sur un gradient de densité de sucrose. Les exosomes flottent alors à une densité allant de 1,13 à 1,20 g/ml en fonction des cellules utilisées et de la composition des vésicules. Après la sédimentation par ultracentrifugation, le gradient est prélevé par fractions de 700µl. Les protéines sont précipitées à l'aide de TCA et analysées par migration sur gel puis Western Blots révélés avec un anticorps anti-CD™ et un anticorps anti-récepteur de la transferrine (RTf) permettant de détecter la présence de vésicules cellulaires (endosomes ou exosomes). Cette analyse a été réalisée pour les mutants pX4 -C et pX4 --A.(voir figure 10).

Chez pX4 --C et pX4 --A, on détecte un signal correspondant à CD8-CD™ dans les fractions de densité allant de 1,13 à 1,25 g/ml. Pour ces mêmes densités, nous avons également pu détecter un signal correspondant à RTf. Ces résultats révèlent la présence de CD8-CD™ dans les fractions contenant les exosomes.

### 3) Récapitulatif de l'analyse de l'association des exosomes avec CD8-CD™ :

L'association de CD8-CD™ avec les exosomes a été détectée pour tous les mutants contenant CD™ exceptés pour les pX2 possédant Cys 213. Les mutants pX4 --C et pX4 --A apparaissent comme très efficacement ciblés dans les exosomes. Les mutants pX3 et pX2 ne possédant pas Cys 213 sont eux aussi retrouvés dans les exosomes mais dans des proportions moindre que pour les mutants pX4 --C et pX4--A.

Il apparaît donc que, en plus de la présence du CD™ viral, deux facteurs puissent jouer sur le ciblage des chimères CD8-CD™ dans les exosomes.

Ces facteurs sont :
- La présence ou non du domaine transmembranaire du BLV.
- La présence ou non de la Cys 3.

### C. Influence de l'inhibition du transport vésiculaire sur l'expression des chimères CD8-CD™:

L'absence de détection des chimères pX2 CCC, pX2 ACC, pX2 CAC et pX2 AAC n'étant pas due à une sécrétion exosomale accrue, nous avons cherché à savoir si elle pouvait être due à une dégradation par la voie de tri des protéines des MVBs vers les lysosomes.

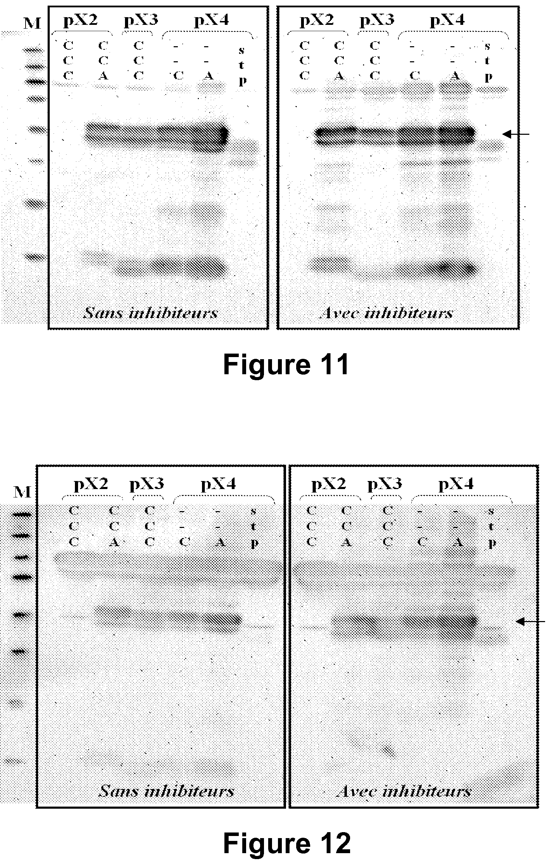

Pour cela, nous avons utilisé les inhibiteurs de transport vésiculaire que sont la bafilomycine et Ly294002. Ces inhibiteurs permettent de bloquer la voie de dégradation lysosomale, favorisant ainsi la sécrétion de protéines par les exosomes. Les inhibiteurs sont ajoutés aux milieux de cultures 32h après la transfection ; 16h plus tard, les cellules sont lysées et les milieux de cultures sont récupérés puis centrifugés en vue d'isoler les exosomes. Les échantillons obtenus sont alors analysés par migration sur gel et Western Blot révélé avec un anticorps anti-CD™. Nous avons ainsi analysé l'expression des chimères pX2 CCC, pX2 CCA, pX3 CCC, pX4 -- C, pX4 -- A et pX4 stp .(voir figures 11 et 12).

Il apparaît que, pour ces mutants, le profil des Western Blots des protéines obtenues en présence d'inhibiteurs est le même que lors de l'analyse sans inhibiteur, que cela soit pour les lysats cellulaires ou pour les exosomes.

L'absence des chimères pX2 CCC, pX2 ACC, pX2 CAC et pX2 AAC n'est donc apparemment due ni à une sortie exosomale accélérée, ni à une dégradation dans les lysosomes. Il est probable qu'elle soit la conséquence d'une dégradation très précoce au niveau du système de contrôle du repliement au sein du réticulum endoplasmique (RE) et du TGN.

### III. Localisation par immunofluorescence :

Afin d'apprécier le ciblage membranaire de CD8-CD™ ainsi que sa localisation dans les compartiments cellulaires, nous avons réalisé des observations en microscopie d'immunofluorescence confocale. 48h après transfection, les cellules ont été fixées puis marquées à l'aide de différents anticorps.

### A. Choix des anticorps :

Dans un premier temps, chaque type de marquage (voir Matériels et Méthodes) est testé, avec des dilutions d'anticorps variables, sur des cellules fixées exprimant pX4 -- C ou pLPCX. Après observation sur un microscope à immunofluorescence classique (ZEISS Axiovert 200 M), nous avons testé les différents anticorps à disposition (voir partie « Matériels et Méthodes ») et déterminé leur efficacité ainsi que leurs dilutions optimales. Il est apparu que plusieurs anticorps anti-compartiments intra-cellulaires présentaient un signal nul ou aspécifique.

Pour l'observation en imagerie confocale, nous n'avons donc pu utiliser que deux types de marquages :
- Marquage CD8-CD™ :
   IgG de rat (53/6.7) Anti-CD8 de souris FITC (Pharmingen, dilution 1/50)
- Marquage Lamp3 :
   IgG de souris Anti-CD63 (Lamp3) humain (Zymed, dilution 1/50) + Anti-IgG de souris Cy3 (Sigma, dilution 1/500)

### B. Observations de la répartition de CD8-CD™ :

Après fixation et marquage des cellules à l'aide de nos différents anticorps, nous avons observé la répartition du marquage issu de CD8-CD™ ainsi que sa colocalisation avec Lamp3 à l'aide d'un microscope confocal (ZEISS LSM 510).

Les cellules exprimant pLPCX (témoin négatif) ne présentent pas de signal FITC. Ce témoin nous permet donc de s'assurer que la fluorescence FITC visualisée pour les autres mutants est bien issue de la présence de CD8-CD™.

De façon surprenante, malgré une absence de détection par Western Blot, les constructions pX2 comprenant la Cys 3 sont visibles, bien que faiblement, en immunofluorescence.

### Analyse des phénotypes généraux :

Les cellules HEK293 sont transfectées pendant 48h puis fixées. L'observation est réalisée à l'aide d'un microscope confocale ZEISS LSM 510 (objectif X63 à immersion).
**CD8 :** Marquage FITC (vert) révélant la présence des chimères contenant le CD8. **Lamp3 :** Marquage Cy3 (rouge) révélant de la présence de la protéine Lamp3 caractéristique des endosomes tardifs.
**CD8-CD™ / Lamp3 :** Superposition des images FITC et Cy3. La nuance jaune obtenu met en évidence la colocalisation de CD8-CD™ avec Lamp3.

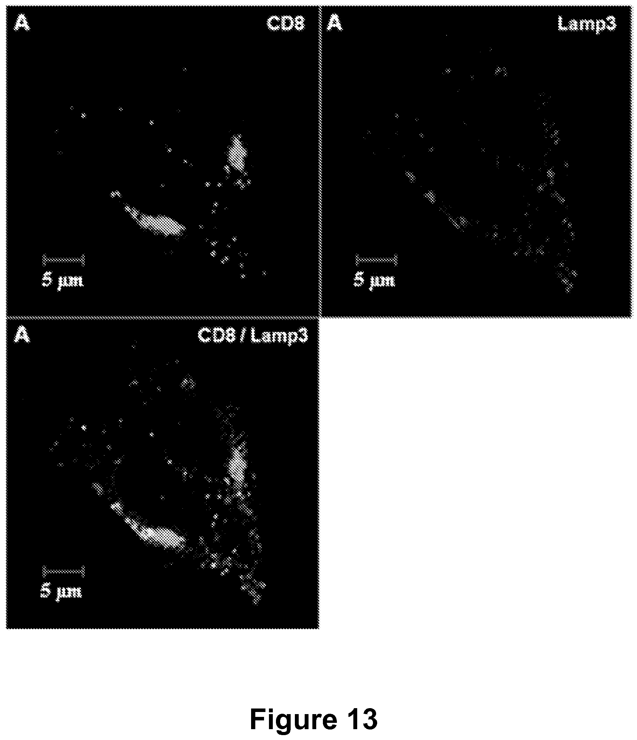

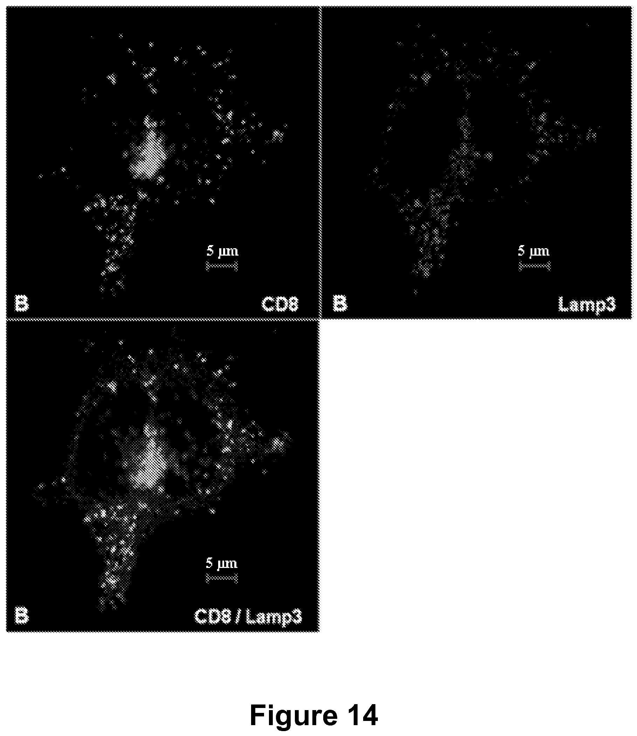

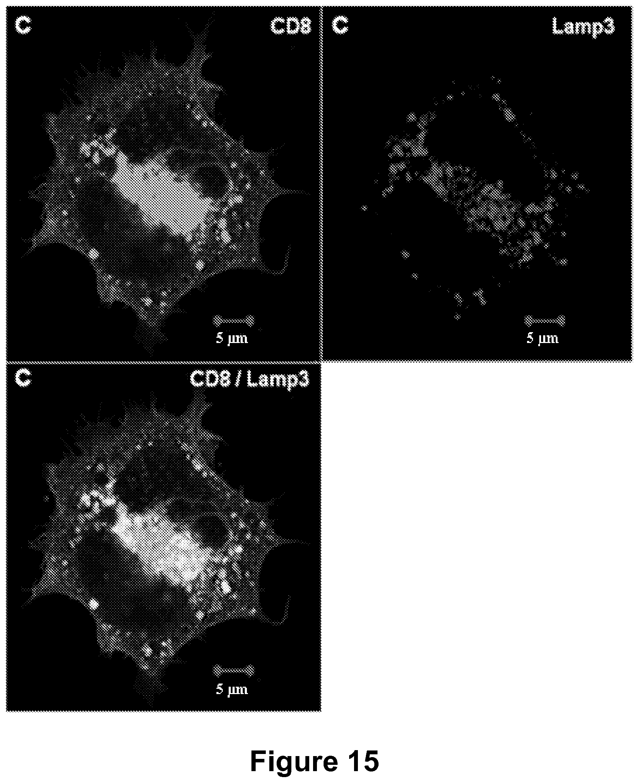

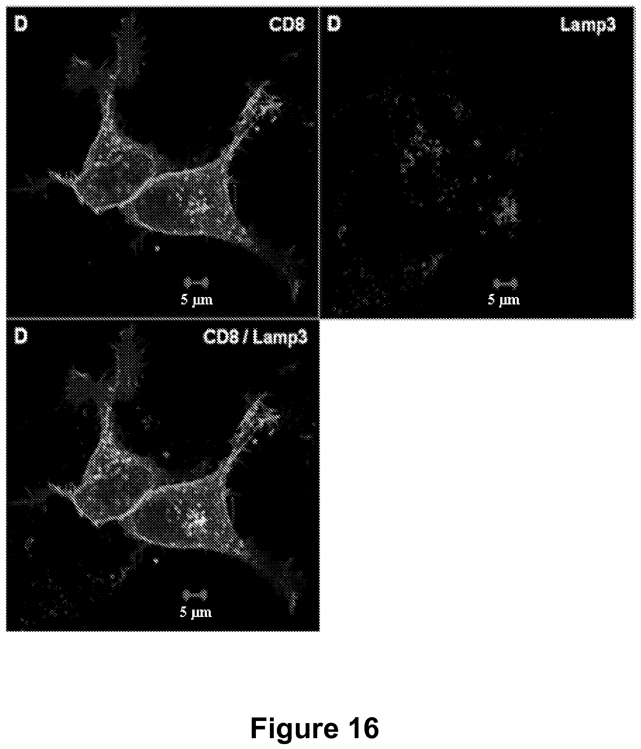

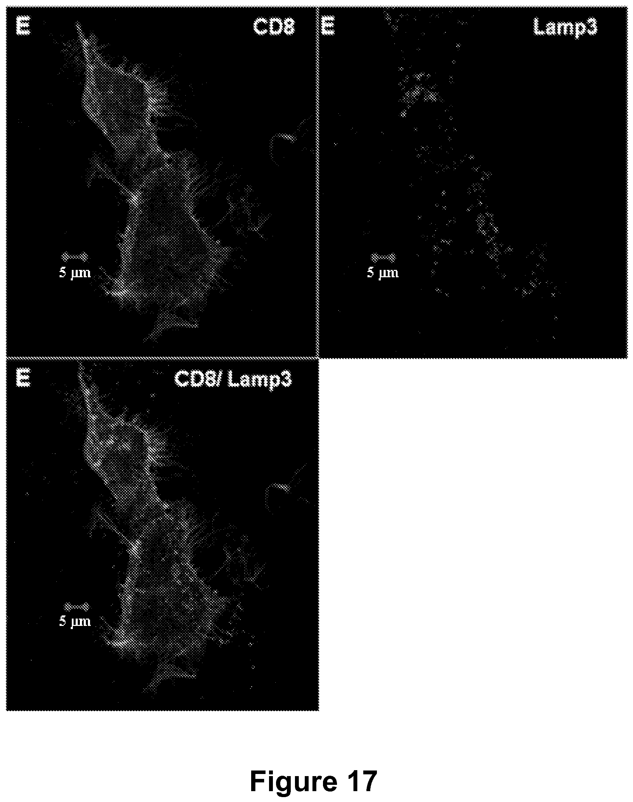

Après observations, nous avons pu distinguer 5 phénotypes généraux (voir figures 13 à 17) en se basant sur l'aspect (vésiculaire, membranaire ou périnucléaire) et l'intensité du marquage FITC. La colocalisation du marquage FITC d'aspect vésiculaire avec Lamp3 ne rentre pas en ligne de compte pour la détermination de ces phénotypes car elle est avérée chez tous les mutants. De plus cette co-localisation est toujours partielle.

### Analyse des zones périnucléaires présentant un fort signal FITC :

Pour tous les échantillons, excepté pX4 stp, on retrouve de manière plus ou moins constante un signal périnucléaire CD8 homogène important à la périphérie du noyau. Les paramètres d'intensité requis pour la visualisation de la majeure partie de la fluorescence FITC présente dans les cellules entraînent la saturation de cette zone. Un signal ainsi saturé étant peu exploitable, nous avons réalisé des acquisitions de paramètres d'intensité plus faible, en nous concentrant sur l'analyse de ces zones, notamment pour déterminer la pertinence des colocalisations qu'elles présentent avec Lamp3.
**CD8**-**CD**™ **/ Lamp3 [a] :** Colocalisation de CD8-CD™ (vert) avec Lamp3 (rouge). Les paramètres d'intensité pour l'acquisition, mêmes faibles, entraînent une saturation du signal périnucléaire et l'apparition de nuances jaunes à ce niveau, mettant en évidence une colocalisation partielle entre ces deux marquages. En raison de la saturation du signal, il est impossible de dire si la colocalisation mise en évidence est réelle ou artéfactuelle.
**CD8**-**CD**™ [d] : Signal FITC issu de CD8-CD™. Les paramètres d'intensité ont été abaissés sensiblement afin d'éliminer tout phénomène de saturation du signal. Le signal FITC apparaît diffus, homogène et non ponctué.
**CD8**-**CD**™ **/ Lamp3 [d] :** Colocalisation de CD8-CD™ (vert) avec Lamp3 (rouge). Les paramètres d'intensité ont été abaissés sensiblement afin d'éliminer tout phénomène de saturation du signal. Il en résulte une absence de nuances jaunes mettant en évidence l'absence de colocalisation entre FITC et Lamp3.

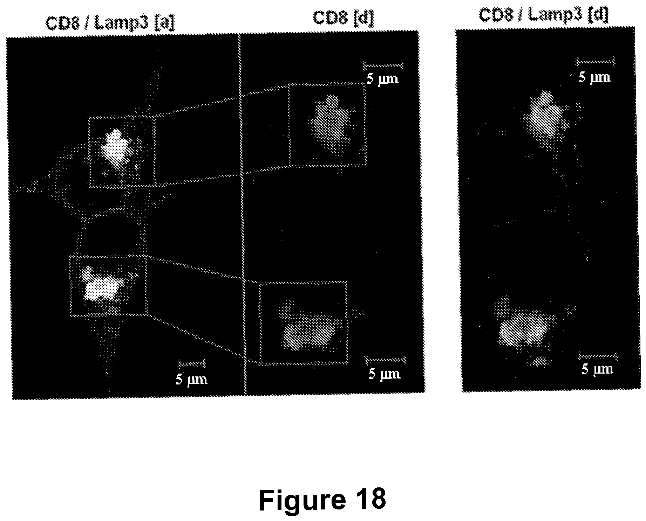

D'après ces acquisitions, le marquage issu de Lamp3 apparaît en fait comme étant situé à l'intérieur de cette zone périnucléaire mais n'est en tout cas jamais colocalisé avec le marquage FITC (voir figure 18).

De plus le marquage issu de CD8-CD™ de ces zones périnucléaires apparaît diffus, homogène et non ponctué, et semble mettre en évidence la présence de CD8-CD™ au sein d'une structure cellulaire. La localisation et l'aspect de ce marquage, ainsi que l'absence de colocalisation réelle avec Lamp3 suggèrent la présence de CD8-CD™ dans l'appareil de Golgi.

Ce phénotype est retrouvé, de façon plus ou moins intense, chez tous les mutants, hormis pX4 stp. Chez les mutants pX4 --C et pX4 --A, ce phénotype n'est visible que dans une minorité de cellules, contrairement aux mutants pX2 et pX3 chez qui il est présent de manière constante.

**Tableau 3**

| | | | Localisation du marquage FITC | | |
|---|---|---|---|---|---|
| Phénotype | Mutants | Intensité FITC | Vésiculaire | Membranaire | TGN |
| **A** | pX2 CC**C** | + | +++ | - | ++ |
| | pX2 AC**C** | + | +++ | - | ++ |
| | pX2 CA**C** | + | +++ | - | ++ |
| | pX2 AA**C** | + | +++ | - | ++ |
| **B** | pX2 CC**A** | +++ | ++++ | - | ++ |
| | pX2 AC**A** | +++ | ++++ | - | ++ |
| | pX2 CA**A** | +++ | ++++ | - | ++ |
| | pX2 AA**A** | +++ | ++++ | - | ++ |
| **C** | pX3 CC**C** | ++++++ | + | +++ | +++++ |
| | pX3 CA**C** | ++++++ | + | +++ | +++++ |
| **D** | pX4 - - **C** | ++++++ | ++ | ++++ | + |
| | pX4 - - **A** | ++++++ | ++ | ++++ | + |
| **E** | pX4 stp | ++++ | + | +++ | - |

Ainsi, l'observation spécifique des zones périnucléaires fortement marquées par FITC nous a permis de mettre en évidence la présence probable de CD8-CD™ au sein du TGN.

D'après les données obtenues, nous pouvons établir le tableau récapitulatif ci-dessous (voir tableau 3).

Ces observations confirment que, outre le CD™ viral, deux facteurs jouent un rôle sur la stabilité et le ciblage de nos chimères.

Ces facteurs sont :
- La présence ou non de tmD BLV.
- La présence ou non des résidus N-terminaux du CD™ du BLV
- La présence ou non de la Cys 3.

### CONCLUSION :

Au cours de nos analyses, il est apparu que la chimère pX4 stp, composée uniquement de l'ectodomaine et du tmD de CD8, s'accumule dans les cellules HEK293 en étant efficacement ciblée vers la membrane plasmique.

De la même façon, les chimères pX4 --C et pX4 --A s'accumulent et se retrouvent dans la membrane plasmique mais dans des proportions plus importantes que pX4 stp. Ces chimères sont très efficacement sécrétées au sein d'exosomes.

Les chimères pX3 quant à elles sont très présentes au sein de l'appareil de Golgi, contrairement aux chimères pX4. Leur ciblage au sein de la membrane plasmique et dans les exosomes parait moins efficace que chez les chimères pX4 mais demeure important.

Les chimères pX2 apparaissent peu stables et sont retrouvées au sein du TGN mais pas dans la membrane plasmique. Les constructions pX2 comportant Cys 3 sont très peu stables dans les cellules et ne sont pas détectées dans les exosomes. La substitution de la Cys terminale dans ce type de constructions semble contribuer à un gain de stabilité des protéines chimériques. En effet, les constructions pX2 sans Cys 3 sont détectables dans les cellules et dans les exosomes.

Toutes les chimères étudiées présentent, en immunofluorescence, un marquage vésiculaire qui est partiellement colocalisé avec un marqueur des endosomes tardifs.

Cette étude a permis de mettre en évidence l'importance de la présence, ou non, de Cys 3 dans la stabilité et dans le ciblage des chimères CD8-CD™. Il semble que l'absence de ce résidu cystéine favorise la stabilité et le ciblage membranaire des chimères étudiées ainsi que leur présence dans les exosomes. Ces phénomènes pourraient être indépendants de l'hyperpalmitoylation associée à l'absence de Cys 3. En effet, la construction pX2 AAC, qui est non palmitoylée, est plus stable que les trois mutants pX2 possédant Cys 3 ainsi que Cys 1 et/ou Cys 2, qui sont palmitoylables. Cependant, une implication différentielle de la palmitoylation dans la stabilité et le ciblage de nos chimères ne doit pas être exclue.

Cette étude a permis de découvrir l'importance fondamentale de tmD du BLV. En effet, la délétion de tout ou partie de tmD BLV semble accroître sensiblement la stabilité des chimères ainsi que leur ciblage au niveau de la membrane plasmique. Ainsi de façon étonnante, la présence de tmD BLV favoriserait la dégradation précoce des chimères. Cette dégradation pourrait intervenir au niveau du système du contrôle du repliement au sein des compartiments cellulaires que sont le RE et le TGN puisque la voie de dégradation lysosomale ne semble pas en cause. L'absence d'effets d'inhibiteurs de transport vésiculaire sur la stabilité et le ciblage exosomal des chimères CD8-CD™ ainsi que leur colocalisation très partielle avec les endosomes tardifs, même pour les chimères les moins stables, vient soutenir cette hypothèse.

L'attrait principal de nos travaux réside dans la découverte d'outils potentiellement efficaces pour le développement d'un mode de vaccination basé sur le concept « exosome display ». En effet, les chimères pX4 --C et surtout pX4 --A semblent disposer d'un « moteur » moléculaire permettant un ciblage très efficace d'antigène peptidique (ici le CD8) vers les exosomes. Ce « moteur » serait donc situé dans le domaine cytoplasmique de la protéine TM du BLV.

### Exemple 2 : Identification d'acides aminés impliqués dans le ciblage exosomal du peptide CD™

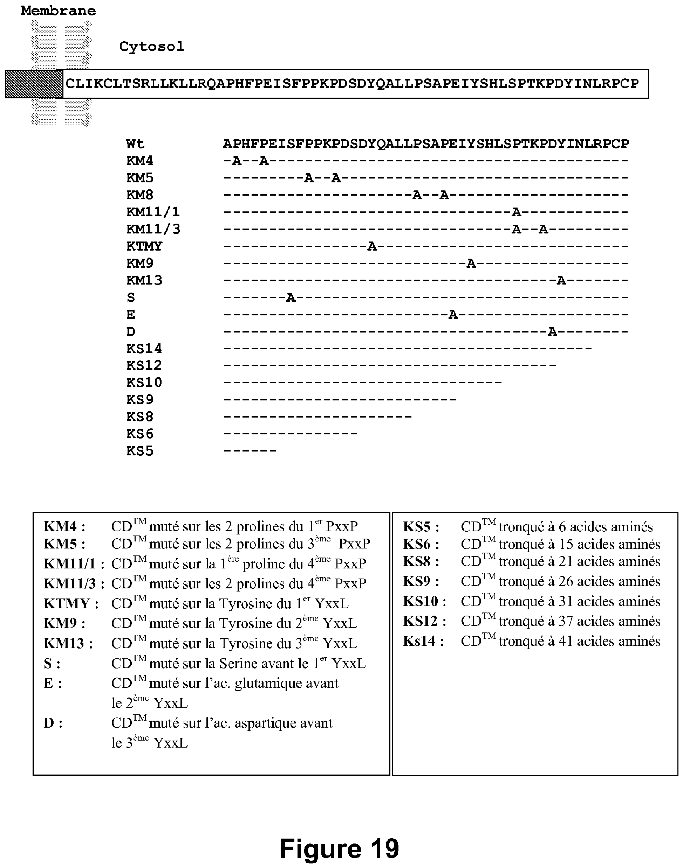

Afin de préciser la nature exacte du « moteur » situé dans le domaine cytoplasmique de la protéine TM du BLV avant de procéder à de premiers essais d'immunisation, nous avons étudié l'effet de 18 types de mutations dans le domaine cytoplasmique de la protéine TM du BLV (voir figure 19):
- délétion des 13 résidus N-terminaux et substitution des 2 résidus proline du premier motif PxxP (SEQ ID NO.:13 et SEQ ID NO. :14 ; mutation KM4) ;
- délétion des 13 résidus N-terminaux et substitution des 2 résidus proline du deuxième motif PxxP (SEQ ID NO.:15 et SEQ ID NO.:16; mutation KM5) ;
- délétion des 13 résidus N-terminaux et substitution des 2 résidus proline du troisième motif PxxP (SEQ ID NO.:17 et SEQ ID NO. :18; mutation KM8) ;
- délétion des 13 résidus N-terminaux et substitution du premier résidu proline du quatrième motif PxxP (SEQ ID NO.:19 et SEQ ID NO.:20; mutation KM11/1) ;
- substitution des 2 résidus proline du quatrième motif PxxP (SEQ ID NO.:21 et SEQ ID NO.:22; mutation KM11/3) ;
- délétion des 13 résidus N-terminaux et substitution du résidu tyrosine du premier motif YxxL (SEQ ID NO.:23 et SEQ ID NO. :24; mutation KTMY) ;
- délétion des 13 résidus N-terminaux et substitution du résidu tyrosine du deuxième motif YxxL (SEQ ID NO.:25 et SEQ ID NO. :26; mutation KM9) ;
- délétion des 13 résidus N-terminaux et substitution du résidu tyrosine du troisième motif YxxL (SEQ ID NO.:27 et SEQ ID NO.:28; mutation KM13) ;
- délétion des 13 résidus N-terminaux et substitution du résidu sérine se trouvant avant le premier motif YxxL (SEQ ID NO.:29 et SEQ ID NO. :30; mutation S);
- délétion des 13 résidus N-terminaux et substitution du résidu acide glutamique se trouvant avant le deuxième motif YxxL (SEQ ID NO.:31 et SEQ ID NO. :32 ; mutation E) ;
- délétion des 13 résidus N-terminaux et substitution du résidu acide aspartique se trouvant avant le troisième motif YxxL (SEQ ID NO.:33 et ; SEQ ID NO. :34; mutation D) ;
- séquence tronquée à 6 résidus - délétion des 13 résidus N-terminaux et des 39 résidus C-terminaux (SEQ ID NO. : 35 et SEQ ID NO. :36 ; mutation KS5) ;
- séquence tronquée à 15 résidus - délétion des 13 résidus N-terminaux et des 30 résidus C-terminaux (SEQ ID NO. :37 et SEQ ID NO. :38; mutation KS6) ;
- séquence tronquée à 21 résidus - délétion des 13 résidus N-terminaux et des 24 résidus C-terminaux (SEQ ID NO. : 39 et SEQ ID NO.:40; mutation KS8) ;
- séquence tronquée à 26 résidus - délétion des 13 résidus N-terminaux et des 19 résidus C-terminaux (SEQ ID NO.:41 et SEQ ID NO. :42; mutation KS9) ;
- séquence tronquée à 31 résidus - délétion des 13 résidus N-terminaux et des 14 résidus C-terminaux (SEQ ID NO.:43 et SEQ ID NO. :44; mutation KS10) ;
- séquence tronquée à 37 résidus - délétion des 13 résidus N-terminaux et des 8 résidus C-terminaux (SEQ ID NO. :45 et SEQ ID NO. :46; mutation KS12) ;
- séquence tronquée à 41 résidus - délétion des 13 résidus N-terminaux et des 4 résidus C-terminaux (SEQ ID NO. :47 et SEQ ID NO.48; mutation KS14).

Les mutants de substitution et de délétion ont été obtenus par mutagénèse dirigée ; les résidus substitués ont été remplacés par un résidu alanine. L'arrêt de traduction des mutants de délétion a été obtenu par addition d'un codon stop (codon TGA, TAG ou TAA).

Les séquences ADN codant pour les 18 mutants, ainsi que la séquence ADN CD™ sauvage dans laquelle seuls les 13 résidus N-terminaux ont été délétés (SEQ D NO. : 7 ; séquence appelée « séquence sauvage » ci-après) ont été sous-clonées en aval d'une séquence codant pour l'ectodomaine du CD8α murin. Les 19 gènes chimères obtenus ont ensuite été clonés dans un vecteur d'expression viral. Les vecteurs recombinants ainsi obtenus ont été transfectés dans des cellules eucaryotes (cellules HEK) afin d'analyser le ciblage vers les exosomes des protéines chimères résultantes. 48 heures après, l'expression protéique dans les cellules a été examinée par Western Blot. Parallèlement, les exosomes ont été purifiés par ultracentrifugation permettant d'évaluer ainsi le tri de la protéine chimère dans les exosomes par FACS et western blot.

Les résultats présentés ci-après mettent en évidence la nécessité de deux motifs peptidiques individuellement reconnus dans la littérature pour leurs interactions avec des protéines associées à la machinerie ESCRT et à la machinerie de transfert intermembranaire (en particulier les adaptines dont AP3). C'est la première fois que l'on apporte des évidences expérimentales que ces deux motifs peptidiques jouent, en synergie, un rôle déterminant dans le ciblage exosomal.

Ces résultats apportent des informations inédites et intéressantes en termes de signalisation inter-cellulaires utilisant les exosomes. Ils permettent ainsi d'avoir en main un outil bien défini pour cibler des protéines avec les exosomes. D'un point de vue industriel, ils faciliteront l'élaboration d'une nouvelle génération de vaccin et d'un outil unique de criblage de molécules thérapeutiques ou d'anticorps par exemple.

### 1- Obtention des constructions moléculaires

### A- Préparation des inserts par PCR:

Les trois mutants de substitutions S (Ser→Ala), D (Ac. Asp→Ala) et E (Ac.Glut→Ala) ont été obtenus par mutagenèse dirigée par une double PCR utilisant les amorces de mutations suivantes :
- Amorce S vers A, sens:
   5' CCCTAAACCCGATGCTGATTATCAGGCGTTGCTACCATCC 3'
- Amorce S vers A, anti-sens:
   5' CGCGGATGGTAGCAACGCCTGATAATCAGCATCGGGTTTA 3'
- Amorce D vers A, sens:
   5' CCACCAAGCCGGCATACATCAACCT 3'
- Amorce D vers A, anti-sens:
   5' TCGAAGGTTGATGTATGCCGGCTTGGT 3'
- Amorce E vers A, sens:
   5' GCTACCATCCGCGCCAGCGATCTAC 3'
- Amorce E vers A, anti-sens:
   5' GTAGATCGCTGGCGCGGATGGTA 3'.

Les PCR ont été réalisées à l'aide du kit Expand High Fidelity PCR system (Roche®) qui possède un mélange enzymatique contenant de l'ADN polymérase *Taq* thermostable et d'une ADN polymérase Tgo thermostable pourvue d'une activité correctrice (activité exonucléasique 3'-5') qui permet de limiter les erreurs lors de la polymérisation et d'obtenir des fragments à bouts francs. Deux mélanges de réactifs ont été préparés à 4°C :
A (25µL) :10ng d'ADN à amplifier, 1µL de dNTP 10mM (200µM final chacun), 1,5µL de chacune des deux amorces sens et anti-sens à 10µM (300nM final), eau stérile (qsp 25µL) ; et
B (25µL) :5µL de tampon « Expand High Fidelity » 10X à 15mM en MgCl₂ (1,5mM final), 0,75µL de mélange enzymatique « High Fidelity » (2,6U final), eau stérile (qsp 25µL).

A et B ont été mélangés à 4°C puis les cycles d'amplication suivants ont été réalisés :
- dénaturation de l'ADN double brin 2 minutes à 94°C ;
- 4 cycles de dénaturation (94°C, 10 secondes), hybridation (50°C, 15 secondes) et élongation (72°C, 20 secondes) ;
- 25 cycles : 10 secondes à 94°C, 15 secondes à 64°C puis 20 secondes à 72°C;
- élongation finale de 7 minutes à 72°C.

Le produit PCR obtenu a été maintenu à 4°C.

Les séquences ADN codant pour les 18 mutants, ainsi que la séquence ADN sauvage ont été modifiées par PCR (mutagénèse dirigée) afin qu'elles soient encadrées par des sites de restriction particuliers ; le protocole indiqué ci-dessus a été mis en oeuvre, en utilisant deux amorces possédant les sites de restrictions Xbal et NotI.

### B- Clonage des produits PCRs dans TOPO-bluntll et contrôles

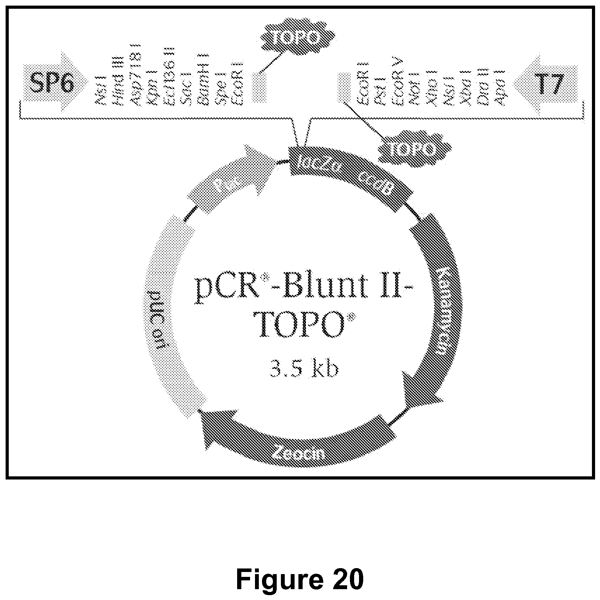

Chacun des 19 ADNs a été ligué dans un vecteur de clonage TOPO (voir figure 20) du kit Topo-blunt cloning (Invitrogen) pour être introduit dans des bactéries chimiocompétentes. Les clones transformés ont été sélectionnés et toutes les séquences d'ADNs vérifiées par séquençage.

### a) Ligation dans le plasmide et Transformation dans Top10

Chacun des différents produits de PCR a été intégré dans un plasmide TOPO-Bluntll déjà ouvert et à bouts francs portant le gène de résistance à la kanamycine. Les bactéries chimiocompétentes Top10 ont été transformées par ces plasmides et mises en culture sur boîte de LB/agar (100µg/mL de kanamycine). Le plasmide TOPO-Bluntll sans insert a servi de témoin négatif et un autre témoin (1ng du plasmide PUC19) a été utilisé comme témoin positif de transformation. Après culture, seules les bactéries transformées par un plasmide contenant l'insert ou PUC19 (témoin positif) se sont développées en présence de l'agent de sélection (antibiotique).

### b) Criblage des bons clones et séquençage:

Suivant les résultats des clonages, 2 à 10 colonies ont été amplifiées pour réaliser l'extraction de l'ADN plasmidique. Pour chaque construction et chaque clone, nous avons obtenu un volume d'ADN plasmidique de 100µL à environ 150ng/µL. Le rapport absorption à 260nm / absorption à 280nm donne pour chaque purification une valeur comprise entre 1,8 et 2 ce qui témoigne de la pureté de la préparation (une valeur inférieure à 1,8 témoignerait d'une contamination protéique).

Pour s'assurer de la présence de l'insert dans le plasmide, les ADNs plasmidiques ont été digérés par l'enzyme de restriction EcoRI qui encadre les séquences d'intérêt. La visualisation des ces produits de digestion a été faite sur gel d'agarose 2% où la présence d'un fragment (d'environ 300pb) constitue la preuve d'un ADN recombinant.

Une fois les bons clones identifiés, 2 à 4 µg d'ADN plasmidique de chaque mutant ont été séquencés afin de vérifier l'intégrité de chaque séquence d'intérêt et donc du cadre ouvert de lecture. Les mutations et les sites de restrictions rajoutés ont été contrôlés par la même occasion.

### C- Obtention des gènes chimères dans un vecteur de clonage pKSII

Chacune des 19 séquences (1 sauvage et 18 mutées) a été placée en aval d'une séquence codant pour l'ectodomaine CD8α de souris, pour donner 19 constructions.

### a) Préparation d'un vecteur de clonage pKSII-CD8

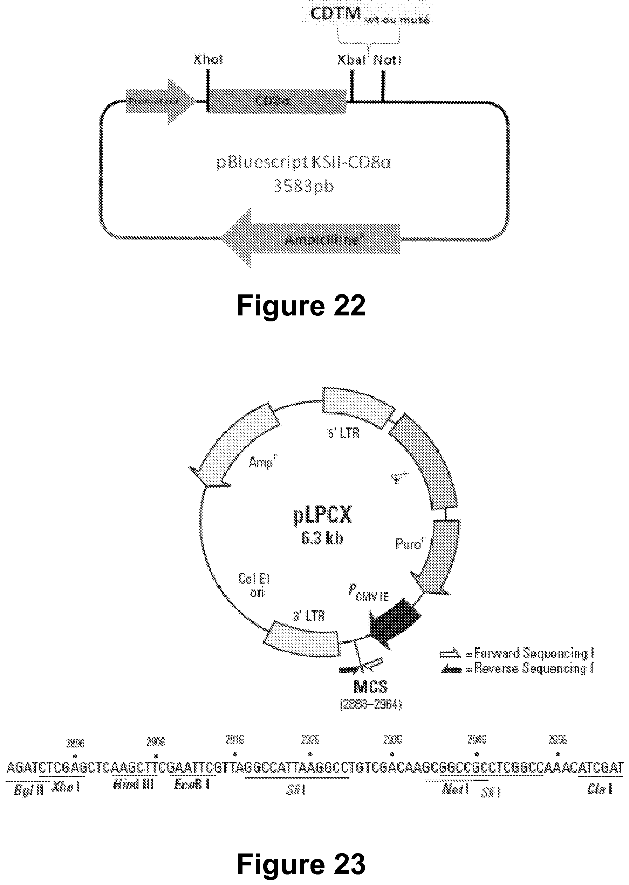

Le vecteur pKSII-CD8α (voir figure 22) a été digéré successivement avec les enzymes de restrictions XbaI et NotI pour pouvoir accueillir les inserts. Les digestions effectuées, le plasmide a été déphosphorylé, précipité à l'éthanol et purifié sur gel d'agarose 0,8%.

### b) Préparation des inserts

Les différents inserts encadrés par les sites de restrictions Xbal-Notl ont été digérés par les mêmes enzymes de restrictions que le plasmide afin que celui-ci puisse les intégrer.

### c) Obtention des plasmides chimères par clonage

Les ADNs ont été insérés dans le plasmide pKSII-CD8α linéarisé :
Les inserts digérés ont été purifiés sur gel d'agarose 2,5%. Leur réinsertion dans le vecteur pKSII-CD8α a été réalisée par la technique de ligation dans le gel. Un fragment de gel vierge a servi de témoin négatif de ligation.

Ayant utilisé les mêmes enzymes de restrictions pour le vecteur et les inserts, ils possèdent donc des sites cohésifs XbaI et NotI complémentaires : plasmide et inserts devraient pouvoir établir des liaisons entre eux. C'est une enzyme, la ligase, qui catalyse la formation d'une liaison phosphodiester entre une extrémité 3'-OH et une extrémité 5'-Phosphate de deux acides nucléiques.

Une fois l'étape de ligation achevée, des bactéries DH5α sont transformées par ces plasmides portant le gène de résistance à l'ampicilline. Après culture des différentes bactéries transformées à 37°C sur LB/agar (50 µg/mL d'ampicilline) et comparaison avec les témoins négatifs, on constate le développement de colonies uniquement chez les cellules transformées par les produits de ligation en présence d'insert CD™. Cela suggère que les colonies obtenues ont bien été transformées par un vecteur contenant un insert entre les sites Xbal et NotI.

### d) Criblage :

Pour confirmer l'obtention des plasmides chimères, différents clones de chaque mutant ont été criblés en digérant l'ADN plasmidique par les deux enzymes XhoI/ NotI et après migration des produits de digestion sur gel d'agarose 0,8%. Cette double digestion excise l'ensemble du gène chimère créé, c'est-à-dire avec le CD8α en phase avec le CD™.

Pour chaque clone et chaque construction (pKSII-CD8α-CD™ muté ou sauvage), il a été observé une première bande à 2,9 kpb correspondant à la taille du plasmide pKSII linéarisé. Une deuxième bande a été repérée à environ 950pb ; elle correspond au gène codant pour les chimères CD8α- CD™ mutés ou non.

### D- Obtention des gènes chimères dans le vecteur d'expression rétroviral pLPCX :

Le vecteur d'expression pKSII, s'il est facile à manipuler de par sa taille et ses sites de restrictions, ne permet pas l'expression protéique dans les cellules eucaryotes. Nous avons donc choisi pLPCX (Clontech Laboratories Inc., voir figure 23), qui permet d'introduire un gène autant par transfection que par transduction au moyen d'un vecteur rétroviral.

Chaque gène chimère a été excisé du plasmide pKSII entre les sites Xhol-Notl pour être purifié par extraction sur gel d'agarose 2% en utilisant le kit Nucleospin Extract II® (Macherey-Nagel).

Le vecteur d'expression pLPCX a lui aussi été préalablement digéré par le couple d'enzymes Xhol-Notl, déphosphorylé puis précipité à l'isopropanol (cette étape permet d'éliminer le court fragment d'ADN (inférieur à 100pb) situé entre Xhol et Notl libéré lors de la digestion).

La ligation des gènes chimères avec pLPCX a été réalisée en utilisant la T4 DNA ligase (Biolabs) (rapport insert/vecteur d'environ 3/1 molécule à molécule). Des bactéries chimio-compétentes StbI2 (MAX Efficiency® StbI2™ Competent Cells, Invitrogen) ont été transformées par ces produits de ligation. Un témoin positif (1 ng du plasmide pUC19) et un témoin négatif (pLPCX « ligué » sans insert) ont été préparés en parallèle. Après culture bactérienne à 30°C sur milieu gélosé contenant 50µg/mL d'ampicilline, seules les bactéries transformées par le témoin positif ou par les produits de ligation avec inserts se sont développées.

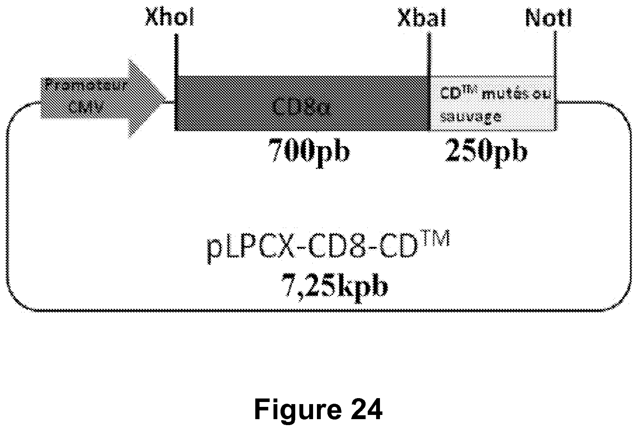

Pour pouvoir procéder au criblage et avoir une quantité conséquente d'ADN plasmidique nécessaire pour les transfections dans cellules eucaryotes, des Midipreprations ont été effectuées. La présence des inserts dans le plasmide a été vérifiée sur gel d'agarose 2% après digestion par Xhol puis NotI. Pour chaque construction (pLPCX-CD8α-CD™ mutées et sauvage ; voir figure 24) on observe une bande à 6,3kpb correspondant au pLPCX linéarisé et une bande à 950pb correspondant aux gènes chimères excisés.

### 2- Expression et analyse du ciblage vers les exosomes :

### A- Transfections dans les cellules HEH 293T :

Pour s'assurer de la transfection des cellules eucaryotes HEK 293T et estimer le pourcentage de cellules transduites, les cellules sont transfectées par le plasmide contenant le gène *LacZ* et incubées dans une solution de X-Gal.

D'abord à l'oeil et après observation au microscope optique (grossissement X40) nous avons constaté que plus de 50% des cellules étaient colorées en bleu ce qui prouve que la grande majorité ont été transduites par le plasmide *LacZ.* Les mêmes conditions ayant été respectées pour la transfection de nos gènes chimères, il est probable que plus de 50% des cellules aient été transduites par les gènes chimères.

En parallèle, vingt transfections simultanées dans des cellules HEK 293T ont été effectuées. Elles correspondent à chacun des plasmides ainsi qu'à un témoin négatif (pLPCX-CD8 sans CD™).

### B- Expression des protéines chimères dans la cellule et ciblage vers les exosomes

### a) Analyse par western blot :

Les lysats cellulaires et exosomaux issus des transfections ont été analysés par migration sur gel SDS-PAGE à 10%, suivie d'un transfert sur membrane PVDF (polyvinylidène difluoride, Immobilon-P, Millipore). Ces membranes ont ensuite été révélées à l'aide d'un sérum primaire de lapin anti-CD™ suivi d'un anticorps secondaire anti-IgG de lapin couplés à la peroxydase. Après révélation, ces anticorps sont éliminés et les membranes de transfert révélées de la même façon mais en utilisant un sérum de lapin anti-CD8α.

Les résultats sont présentés sur les figures 25 et 26.

### • Comparaison des niveaux d'expression des chimères dans les cellules et les exosomes :

On constate que les lysats cellulaires ne révèlent qu'une faible expression des protéines chimères. Par contre, la présence de certaines protéines chimères dans les exosomes est parfois très forte.

Outre les niveaux d'expression, la différence majeure que l'on remarque entre protéines cellulaires et protéines exosomales est la présence de 2 à 3 bandes pour les cellules et d'une seule pour les exosomes. Ceci vient du fait que la cellule contient des formes non-glycosylées et plus ou moins glycosylées. Seule la forme correctement glycosylée se retrouve dans les exosomes.

### • Analyse par western blot du ciblage exosomal des témoins positifs (CD8α-CD™) et négatif (CD8α seul) :

Avec le sérum anti-CD™, une bande migrant à 31 kDa est présente dans le lysat exosomal du témoin CD8α-CD™ sauvage alors qu'elle est absente dans le lysat des cellules transfectées par le témoin négatif. Cette bande est caractéristique de la protéine chimère attendue.

Avec le sérum anti-CD8α, le témoin négatif CD8α seul présente une bande vers 27kDa qui correspond à l'expression et au ciblage exosomal de CD8α dépourvu de CD™. Comme précédemment, une bande migrant à 31 kDa est présente dans le lysat exsomal du témoin CD8α-CD™ sauvage. La différence d'intensité entre les bandes 31 kDa et 27kDa indique que le CD8α est bien plus ciblé sur les exosomes quand il est fusionné au CD™.

### • Analyse par western blot des variations de ciblage des CDBα-CD™ mutés :

Seuls les résultats obtenus avec le sérum anti-CD8α sont comparables entre eux. Les résultats obtenus avec le sérum anti-CD™ sont uniquement utilisés pour confirmer les précédents. Suivant la mutation de la séquence codant pour le CD™, ces résultats montrent une variation dans l'expression et le ciblage des protéines chimères vers les exosomes. Ceci est particulièrement clair pour les mutations qui inhibent fortement le ciblage des protéines sur les exosomes. Les mutants concernés sont les mutants KM8, KM13, D et KS8.

De ces observations on peut conclure que le motif PSAP (mutant KM8) et le motif DY (au niveau du dernier motif YxxL (mutants KM13 et D)) sont importants pour le ciblage exosomal.

### b) Quantification des protéines chimères sur les exosomes par FACs :

La présence des protéines chimères a été aussi recherchée par une analyse en cytofluorométrie (FACscan) utilisant un anticorps monoclonal anti-CD8 fluorescent.

Après fixation des exosomes sur billes de latex (billes IDC (Interfacial Dynamics Corporation) ultraclean aldehyde/sulfate Latex), les protéines chimères présentes à la surface des exosomes ont été marquées à l'aide d'un anticorps monoclonal de souris anti-CD8α couplé à la fluorescéine (anticorps 53-6.7 de chez Pharmingen) et analysées au cytofluorimètre (FACScan).

Les résultats obtenus sont particulièrement clairs pour les mutants KM8, KM13 et D, qui révèlent l'importance des acides aminés mutés pour le ciblage des protéines chimères sur les exosomes (voir Figures 26 et 27). Ces résultats confirment l'impact des motifs PSAP, D et Y (du motif DYxxL)dans le ciblage des protéines vers les exosomes déjà révélés par les résultats des westerns blots.

### Conclusion :

Au cours de cette étude, nous avons construit des gènes chimères permettant d'exprimer le peptide pilote CD™ muté ou sauvage fusionné avec le CD8α de souris. Ces mutations sur des acides aminés ou des motifs remarquables du CD™ avaient pour but d'identifier les aminoacides et motifs consensus importants dans le ciblage exosomal.

Les différents gènes chimères ont été intégrés dans un vecteur d'expression rétroviral pour transfecter des cellules eucaryotes HEK 293T afin d'obtenir l'expression de ces protéines chimères. Les bandes observées en western blot suggèrent que, comme la protéine CD8α native, ces protéines sont différemment glycosylées lors de leur passage dans l'appareil de Golgi. Seules les protéines correctement glycosylées se retrouveraient dans les exosomes. Ces protéines ont subi les modifications post-traductionnelles adéquates, condition indispensable à l'expression d'épitopes conformationnels essentiels à la future élaboration d'une immunité vaccinale ou au criblage de molécules thérapeutiques. Cependant, ces glycosylations, qui sont plus ou moins présentes, conduisent à de multiples bandes diffuses qui nous ont gênés lors de la quantification comparée des protéines. Pour palier ce problème, il faudra traiter les lysats avec une endoglycosylase afin d'observer une seule bande sur gel.

D'après ces résultats, les motifs PSAP et DY (du dernier YxxL) sont indispensables à l'expression et au ciblage des protéines chimères vers les exosomes. Ces résultats sont inédits et sont intéressants aussi bien du point de vue fondamental que du point de vue application industrielle.

Il est probable que le motif PSAP soit responsable d'une interaction avec la protéine Tsg101 du complexe ESCRT. Quant au motif DYxxL, il pourrait être impliqué dans l'interaction avec la protéine ALIX du complexe ESCRT. Ainsi, pour la première fois, des données expérimentales suggèrent que le complexe ESCRT serait impliqué dans la formation des exosomes.

### EXEMPLE 3 : ciblage de récepteurs à domaines transmembranaires vers les exosomes.

Les récepteurs membranaires sont des cibles majeures pour le développement de molécules thérapeutiques. En général, le criblage à haut débit de drogues s'effectue en particulier avec des récepteurs à multiples domaines membranaires exprimés sur des cellules en culture. Outre les difficultés pour obtenir une forte expression de récepteurs à la surface des cellules, cette technique entraîne des difficultés de robotisation. C'est pourtant actuellement la seule solution, puisque l'utilisation de récepteurs recombinants purifiés est pour l'instant techniquement inenvisageable.

Dans ce contexte, des exosomes porteurs de récepteurs en particulier de récepteurs à multiples domaines seraient un matériel simple d'emploi et bien adapté au criblage en raison de leur stabilité et de leur facilité de manipulation.

La présente étude visait à produire des exosomes porteurs de récepteurs à simple ou multi- domaines transmembranaires, en particulier le récepteur CxCR4 (récepteur de la chimiokine SDS-1 (CXCL-12) et du HIV) et le récepteur CD4 (récepteur du HIV).

Trois gènes chimères ont été synthétisés. Ils comportent, à l'extrémité 3', le peptide CD™-BLV de séquence SEQ ID. NO. : 8, et à l'extrémité 5', un ADN codant pour le récepteur humain CxCR4, pour une version du récepteur CxCR4 tronquée en partie C-terminale comportant 307 acides aminés (CxCR4 (307)) ou pour une version du récepteur CD4 tronquée en partie C-terminale comportant 403 acides aminés (CD4 (403)).

Les récepteurs CD4 et CxCR4 comportent respectivement un et sept domaines transmembranaires.

Les trois gènes chimères ont été clonés dans un vecteur d'expression rétroviral pLPCX. Ces différents plasmides ont été transfectés dans des cellules eucaryotes humaines HEK293T afin d'observer l'expression des différentes protéines chimères dans ces cellules ainsi que leur tri vers les exosomes.

La stratégie de clonage et sous-clonage utilisée est similaire à celle décrite pour l'exemple 2 :
Les ADNs codant pour les récepteurs CxCR4, CxCR4 (307) et CD4 (403) ainsi que celui codant pour le peptide pilote CD™ sont amplifiés par PCR en utilisant des amorces comportant les séquences de sites de restrictions que l'on souhaite intégrer à chaque extrémité des fragments amplifiés (les fragments CxCR4, CxCR4 (307) et CD4 (403) seront flanqués en 5' par le site EcoRI et en 3' par le site Xbal et CD™/BLV sera flanqué en 5' par le site Xbal et en 3' par le site NotI).

Les inserts ainsi produits sont clonés dans des vecteurs d'amplification Topo (voir figure 20). Les plasmides obtenus sont ensuite digérés par des enzymes de restriction et analysés sur gel d'agarose 1,5%, puis séquencés afin de vérifier l'intégrité de leur séquence.

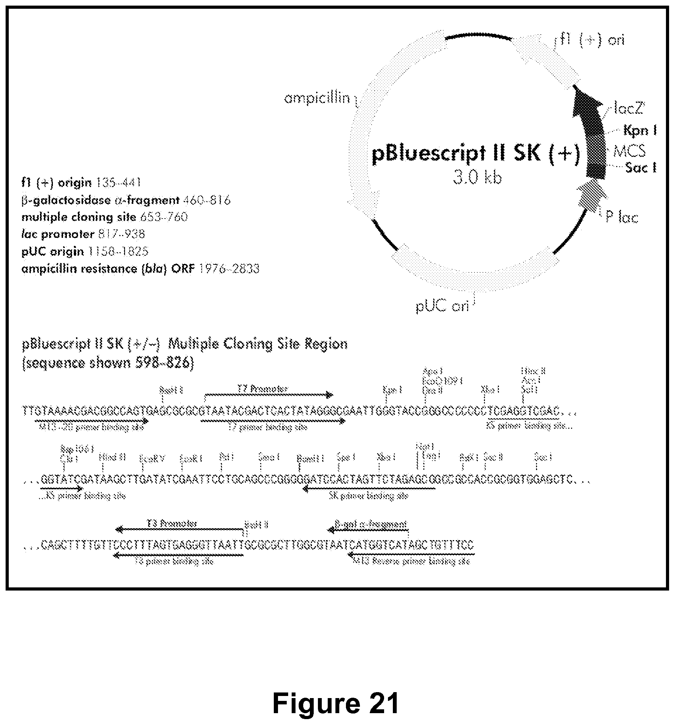

L'insert CD™/BLV est ensuite excisé du vecteur Topo par digestion enzymatique en utilisant le couple Xbal/Notl puis sous-cloné dans le vecteur d'amplification pKS2 (voir figure 21). Le vecteur pKS2 recombinant ainsi que les vecteurs Topo recombinants contenant les inserts CxCR4, CxCR4 (307) et CD4 (403) sont alors digérés avec les enzymes de restriction EcoRI et Xbal, afin de pouvoir sous-cloner les fragments CxCR4, CxCR4 (307) et CD4 (403) dans le vecteur d'amplification pKS2, lesdits fragments étant placés en 5' de la séquence codant pour le peptide CD™.

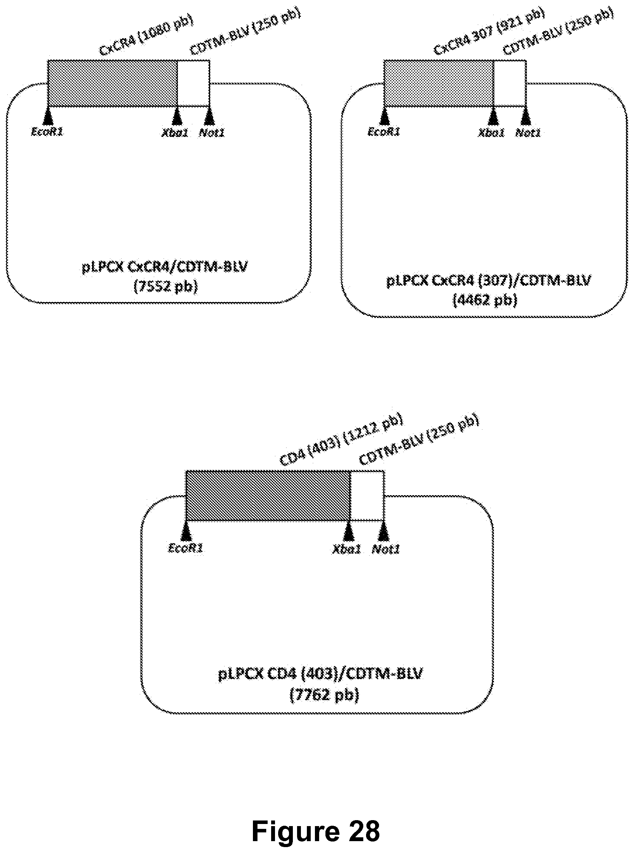

Les différentes constructions ainsi obtenues sont excisées des plasmides pKS2 recombinants par digestion avec le couple d'enzyme EcoRI/NotI puis sous-clonées dans le vecteur d'expression rétroviral pLPCX (voir figure 22). Les vecteurs obtenus (voir figure 28) sont vérifiés par digestion enzymatique en utilisant le couple d'enzymes EcoR1/Xba1.

Les différents plasmides pLPCX sont transfectés dans des cellules eucaryotes humaines HEK293T afin d'exprimer les protéines chimères CxCR4/CD™, CxCR4(307)/CD™ et CD4 (403)/CD™.

Un extrait des protéines cellulaires totales (100 µg) et les protéines d'une suspension d'exosomes produits par chacun des lots de cellules transfectées sont alors soumis à une migration en SDS-PAGE (10%) en présence ou en absence de β-mercaptoéthanol. Les protéines d'intérêt sont révélées par Western Blot grâce à l'utilisation d'un sérum primaire de lapin anti-CD™ et d'anticorps secondaires anti-IgG de lapin couplés à la péroxydase. La révélation des anticorps est réalisée en chambre noire et par utilisation d'une solution ECL. Enfin l'empreinte protéique de chaque échantillon est révélée par coloration au bleu de Coomassie.

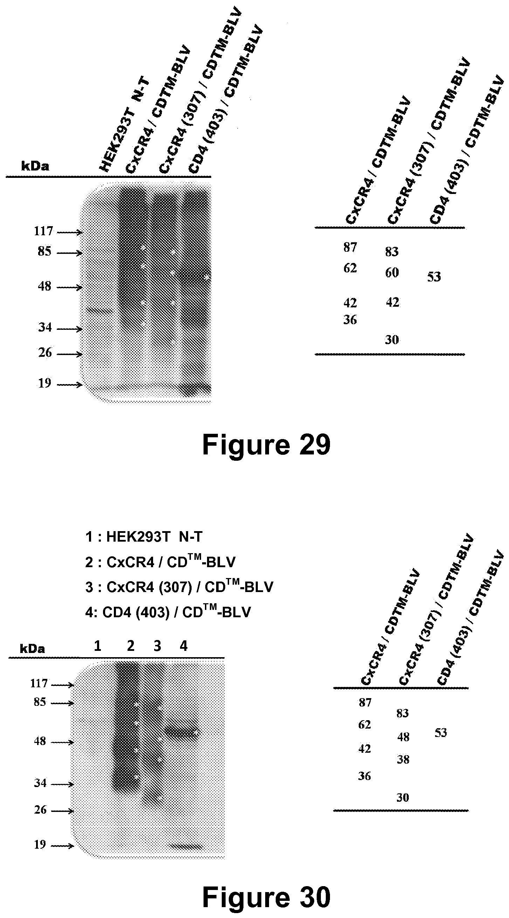

Les résultats sont présentés en figures 29-31.

On observe que les chimères CxCR4/CD™, CxCR4 (307)/CD™ et CD4 (403)/CD™ sont exprimées dans les extraits de protéines cellulaires (voir figure 29).

Plusieurs bandes caractérisent la chimère CxCR4/CD™ : 36, 42, 62 et 87 kDa. L'expression du récepteur CxCR4 sauvage se caractérise en effet par plusieurs isoformes notamment dans les cellules HEK293T ; des bandes 34, 40, 47, 62, 73 et 80 kDa peuvent être identifiées (Sloane JA, *et al*.). Les tailles supérieures des bandes de la chimère CxCR4/CD™ dans les HEK293T sont dues à la présence du peptide pilote (domaine CD™) dans la chimère CxCR4/CD™. De même, les bandes 30, 42, 60 et 83 kDa révèlent la présence de la chimère CxCR4 (307)/CD™. La variation de taille des bandes représentant les différentes isoformes de cette chimère s'explique par le fait que le récepteur CxCR4 est tronqué. La chimère CD4 (403)/CD™, elle, est caractérisée par une bande nettement visible de 53 kDa.

En outre ces chimères sont toutes triées vers les exosomes comme le montre la présence les bandes 36, 42, 62 et 87 kDa (pour CxCR4/CD™) ; 30, 38, 48, et 83 kDa (pour CxCR4 (307)/CD™) et la bande 53 kDa (pour CD4 (403)/CD™) (voir figure 30).

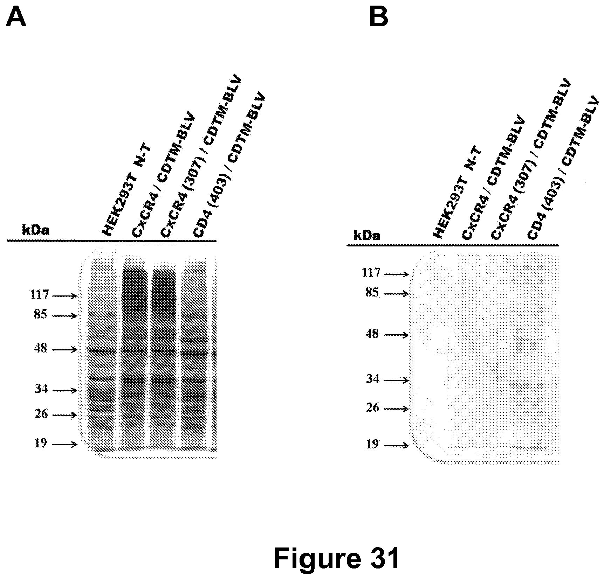

La révélation au bleu de Coomassie des différentes empreintes protéiques montre que les quantités de protéines totales d'origine exosomale (figure 31 B) utilisées au cours de ces expériences sont nettement inférieures aux quantités de protéines totales d'origine cellulaire (figure 31A) alors que le signal en Western blot est équivalent. On constate que toutes les protéines présentes dans le lysat cellulaire témoin ne sont pas triées vers les exosomes. Les chimères CxCR4/CD™ et CD4(403)/CD™ sont adressées très fortement vers les exosomes. En revanche, la chimère CD4 (403)/CD™ est triée presque totalement vers les exosomes.

### Conclusion

La transfection de cellules HEK293T avec différents plasmides pLPCX a permis l'expression des chimères CxCR4/CD™-BLV, CxCR4(403) /CD™-BLV et CD4 (403)/CD™-BLV. L'analyse par Western blot a mis en évidence leur présence dans les lysats cellulaires.

Comme attendu, plusieurs isoformes des chimères CxCR4/CD™-BLV et CxCR4 (307)/CD™-BLV sont exprimées dans les cellules HEK293T transfectées avec les plasmides pLPCX CxCR4/CD™-BLV et pLPCX CxCR4 (307)/CD™-BLV. Les chimères CxCR4/CD™-BLV et CxCR4 (307)/CD™-BLV sont efficacement triées vers les exosomes et il semble que ces protéines de fusion soient partagées à parts égales dans les cellules et les exosomes.

En outre on observe la présence de la chimère CD4 (403)/CD™-BLV dans les cellules HEK293T transfectées avec le plasmide pLPCX CD4 (403)/CD™-BLV. Il semble que le peptide pilote CD™-BLV favorise un tri très important de la chimère CD4 (403)/CD™-BLV vers les exosomes.

Les résultats décrits ci-dessus montrent que le peptide pilote CD™-BLV est capable de trier indifféremment des protéines à simple ou multiples domaines transmembranaires vers les exosomes.

On sait qu'il est très difficile de travailler sur ces récepteurs en solution car s'ils ne sont pas intégrés dans une membrane plasmique, ils ne gardent pas leur structure native. Actuellement les études réalisées sur ces protéines sont souvent faites à partir de lignées cellulaires stables et exprimant les récepteurs d'intérêt. Il est toutefois contraignant en termes de temps et d'économie d'acquérir, de cultiver et d'entretenir ces lignées qui peuvent mourir à tout moment si elles sont mal traitées. C'est pourquoi le fait d'utiliser des exosomes porteurs de récepteurs à multiples domaines transmembranaires pour leurs études représente une solution intéressante car les exosomes possèdent tous les avantages d'une cellule pour ces études sans les inconvénients puisqu'ils ne sont pas vivants.

Des exosomes dans la membrane desquels sont intégrées des protéines recombinantes et en particulier des protéines comportant de multiples domaines transmembranaires pourraient être utilisés en vaccinologie et comme outil de criblage.

### EXEMPLE 4

Nous avons découvert qu'un fragment de l'extrémité C-terminale de la protéine TM du BLV muté par une délétion d'un peptide et par une substitution d'une cystéine pour une alanine présentait des propriétés exacerbées de ciblage vers les exosomes d'un peptide ou polypeptide d'intérêt qui lui est fusionné. L'analyse des acides aminés impliqués dans cette propriété de ciblage exosomal révèle que ce peptide interagirait avec des protéines de la machinerie de bourgeonnement ESCRT. Il est connu que cette machinerie interagit avec des protéines transmembranaires (par exemple, des récepteurs) et aussi avec des protéines cytoplasmiques (Hrs, capside de virus par exemple) qui interagissent avec la membrane à laquelle elles se lient tout en restant cytosoliques. Nous avons voulu savoir si une protéine chimère cytosolique ou nucléaire à laquelle on adjoindrait deux propriétés, l'une de liaison à la face interne des membranes et l'autre de ciblage exosomal, serait effectivement triée vers les exosomes. Pour cela, nous avons utilisé une protéine interne à la cellule et impliquée dans le métabolisme de nucléotides pour démontrer cette hypothèse, la protéine SNAP (Covalys, NEB).

La protéine SNAP est une appellation commerciale de l'entreprise NEB (New England Biochemical, Ipswich, MA, USA) pour un mutant de l'enzyme 06-alkylguanine-DNA alkyltransferase (hAGT). Elle est impliquée dans la voie de synthèse des nucléotides (dans la réparation de l'AND).Nous avons utilisé l'ADN du plasmide pSNAPm de l'entreprise NEB comme ADN matrice des amplifications PCR du fragment d'ADN fusionné entre les fragment src et CDTM. La protéine SNAP peut fixer plusieurs substrats (Keppler et al., 2002; Keppler et al., 2004).

Les propriétés d'interaction aux membranes cellulaires sont le fait de nombreuses protéines cellulaires, en particulier des protéines impliquées dans la signalisation. Ces protéines interagissent avec la membrane par l'intermédiaire d'acides gras qu'elles acquièrent post-traductionnellement et aussi par la présence d'acides aminés basiques qui interagissent avec les têtes polaires de certains acides gras de la membrane (par exemple la choline). Ces acides gras sont en général des acides myristiques, des acides palmitiques ou des géranyl-géranyl.

Le prototype de ces protéines est l'oncogène c-Src qui possède une glycine myristoylable en position 2 et de nombreuses lysines et arginines en N-terminal. Il est connu que, lorsqu'il est fusionné à une protéine cytoplasmique, ce fragment N-terminal lui confère la propriété de se fixer à la face interne de membranes cellulaires. De nombreux autres fragments de protéines (en particulier de protéines cellulaires ou virales) se fixant sous les membranes pourrait jouer le même rôle que celui du fragment N-terminal de c-Src si ces fragments étaient fusionnés avec une protéine cytoplasmique. La localisation du fragment fusionné doit conserver les propriétés de fixation à la membrane. Aussi, il peut être préférable (mais pas forcément obligatoire) que le peptide de ciblage sous-membranaire soit localisé à l'extrémité N-terminal de la protéine de fusion. Par exemple, la myristylation de c-src ne se fait que sur la glycine en position 2. Pour d'autres acylations, exemple la palmitoylation, la position des acides aminés substitués est généralent plus interne à la séquence protéique.

La séquence CD™ de ciblage exosomal utilisée dans le polypeptide chimérique de l'invention peut varier (voir par exemple les nombreux mutants de la séquence CD™ dans les exemples précédents), mais elle doit contenir au moins la séquence PSAP (ou PTAP) et YxxL pour être pleinement efficace. La localisation de ce domaine CD dans le polypeptide chimérique de l'invention ne semble pas être cruciale pour la propriété de ciblage vers les exosomes. Toutefois, la localisation optimale de ce domaine CD dans le polypeptide chimérique de l'invention est la position C-terminale.

Dans le polypeptide chimérique de l'invention, le peptide ou polypeptide d'intérêt auquel on adjoint ces deux peptides (le domaine CD ou son dérivé muté et le domaine de ciblage sous-membranaire) afin qu'elle acquiert un ciblage exosomal peut être de différentes natures :
Par exemple, cela peut être la protéine SNAP, comme dans les polypeptides SNC et DSC décrits ci-après. Dans ce cas, il est possible d'utiliser les propriétés de cette protéine SNAP (comme décrit par exemple par Keppler et al., 2002 et Keppler et al., 2004), pour introduire un fluorophore ou une biotine ou toute autres molécules à un site particulier dans l'exosome, par exemple, au niveau d'une protéine dite protéine G.

Alternativement, le peptide ou polypeptide d'intérêt du polypeptide chimérique de l'invention peut être aussi une enzyme particulière. L'exosome peut ainsi être utilisé comme véhicule pouvant livrer cette enzyme (en particulier d'enzyme cytosolique) à une cellule (en particulier une cellule d'un hôte humain ou non humain) ou à un organe dépourvu de cette activité enzymatique. Cela peut permettre notamment de traiter des patients atteints d'une maladie due à un déficit métabolique ou fonctionnel.

Alternativement, le peptide ou polypeptide d'intérêt du polypeptide chimérique de l'invention peut être un antigène (de pathogène, de tumeurs ou autres). L'exosome le contenant peut ainsi susciter une réaction immune contre cet antigène, ce qui serait particulièrement efficace car les exosomes sont connus comme étant capturés par les cellules de la réponses immunes (par les cellules dendritiques en particulier).

Alternativement, le peptide ou polypeptide d'intérêt du polypeptide chimérique de l'invention peut être une protéine fixant spécifiquement une séquence nucléique (par exemple un ADN).L'exosome de l'invention peut alors fixerun acide nucléique aisément détectable par PCR, par exemple, ce qui permet de faire du diagnostic. Un exemple de technique de fixation spécifique d'une protéine à un ARN est donné dans l'article de Bertrand et al. (1998). La technique décrite par Bertrand et al. repose sur l'utilisation de la protéine de capside du phage à ARN simple brin MS2 (Fouts et al., 1997) et d'un ARN contenant plusieurs copies d'une séquence de 19 nucléotides reconnues par cette protéine.

### I. SYNTHESE D'UN GENE CHIMERE.

Nous voulons associer les propriétés de ciblage sous-membranaire du fragment N-terminal de la protéine c-Src avec des propriétés, que nous avons découvert d'interaction du peptide CDTM15 issu de la protéine TM du BLV (virus de la leucémie bovine) avec la machinerie de ciblage vers les exosomes. Nous pensons ainsi faciliter l'interaction de CDTM avec les protéines de cette machinerie. Notre prototype intègre une protéine rapporteur, la protéine SNAP (Covalys et NEB). Cette protéine pourrait être remplacée par tout autre protéine d'intérêt. Pour cela nous avons créé le gène chimère Src-SNAP-CDTM (SEQ ID NOs.:121 ; 122) qui code pour la protéine chimère SSC (pour Src-SNAP-CDTM ; SEQ ID NOs.:123 ; 124).

La synthèse du gène chimère codant pour la protéine SSC est effectuée en plusieurs étapes. Tout d'abord, obtention d'un ADN synthétique de 75 nt codant pour l'extrémité N-terminale de la protéine c-Src. Ensuite amplification séparément de 3 fragments d'ADN: a) l'ADN synthétique bordé par les sites enzymatiques EcoR1 en amont et Nhe I en aval ; b) l'ADN codant pour la protéine SNAP (NEB) bordé par les sites Nhe I en amont et Sbfl et Ascl en aval; c) l'ADN codant pour un fragment CDTM avec les sites Sbfl et Ascl en amont et Not1 en aval. Enfin, une amplification est effectuée en utilisant simultanément comme ADN matrices les trois fragments d'ADN précédent dont les séquences sont chevauchantes; cette dernière amplification permet d'obtenir un ADN reliant ensemble les trois fragments dans l'ordre Src-SNAP-CDTM.

### 1) Préparation d'un ADN synthétique

Les 5 oligonucléotides suivants sont synthétisés par l'entreprise MWG:
**S1(5'**GCCACCATGGGCAGCAGCAAGAGCAAGCCCAAGGAC 3') (SEQ ID NO.:108),
**S2** (*5'P*-CCCAGCCAGCGCCGCCGCAAGTCTAGAGGCCCGGGAGGC 3') (SEQ ID NO.:109),
**AS1(5'**GCCTCCCGGGCCTCTAGACTTG 3') (SEQ ID NO.:110),
**AS2** (*5'P*-CGGCGGCGCTGGCTGGGGTCCTTGGGCTTGCTCTT3') (SEQ ID NO.:111)
**AS3** (*5'P*-GCTGCTGCCCATGGTGGC 3') (SEQ ID NO.:112).

Synthèse de l'ADN : 20 µl de chaque oligo sont mélangés en quantités équimolaires (100µM de S1,S2, AS1, AS2 et AS3). Addition de 11 µl de tampon ligation (NEB) 10X. Dans l'appareil PCR faire un seul Cycle : 95°C, 2min., suivi d'une descente de température sur la paillasse jusqu'à 25°C. A 10 µl de mix oligos hybridés sont ajoutés 9 µl de tampon ligation 10X, 81 µl de H₂O et 1 µl de T4 DNA ligase (NEB). La ligation est effectuée à 22°C, 10 min.

L'ADN synthétique est ensuite amplifié par PCR dans un mix contenant 10 ng d'ADN matrice, les amorces S1 et AS1, les 4 dNTP, le tampon 1X et 2,5U. de Pfu Turbo DNA-polymerase (Stratagene) dans les conditions du fournisseur de l'enzyme. Après 2 min de dénaturation à 95 °C, 25 cycles d'amplification (95°C 30 sec, 67°C 30 sec, 72°C 5 sec) sont effectués.

**2) Amplifications par PCR** individuelles de chacun des 3 fragments d'ADN constitutifs du gène Src-SNAP-CD™ afin de greffer à chacunes de leurs extrémités des séquences communes et portant des sites d'endonucléase de restriction. La séquence nucléotidique du fragment Src (codant pour le domaine de ciblage sous-membranaire ou domaine (ii)) est SEQ ID NO.:120. La séquence nucléotidique du fragment CD™ (codant pour le domaine CD ou domaine (iii)) est SEQ ID NO.:120.

Les 3 amplifications sont effectuées dans les conditions et selon les recommandations de l'entreprise Finnzyme, fournisseur de la DNA polymérase Phusion utilisée.
a) L'amplification du fragment Src et l'addition de sites de restrictions sont obtenues en utilisant l'ADN synthétique obtenu ci-dessus et les amorces suivantes :
   R1Src (GAATTCGCCACCATGGGCAGCAGCAAGAGCAAG) (SEQ ID NO.:113) et SrcNhe1 (CATGCTAGCGCTGCCTCCCGGGCCTCTAGACTTTC) (SEQ ID NO.:114).
b) L'amplification du fragment SNAP et l'addition de sites de restrictions est obtenu en utilisant l'ADN du plasmide pSNAP (NEB) et les amorces suivantes :
   NheSNAP (GGAGGCAGCGCTAGCATGGACAAAGACTGCGAAATGA) (SEQ ID NO.:115) et
   SNAPSbfAsc (GCGCGCCGCTTCCTGCAGGACCCAGCCCAGGCTTG) (SEQ ID NO.:116).
c) L'amplification du fragment DCTM et l'addition de sites de restrictions est obtenu en utilisant un ADN codant pour l'extrémité C-terminale de la TM du BLV dont la cystéine C-terminale est mutée en alanine, elle s'effectue en présence des amorces suivantes :
   SbfAscDCTM15 (CCTGCAGGAAGCGGCGCGCCCCACTTCCCTGAAATC) (SEQ ID NO.:117) et
   DCTM15Not (GCGGCCGCTTCGAACTCGGTGCTGGCAGCAAGA) (SEQ ID NO.:118).

### 3) Amplification pour obtenir l'ADN Src-SNAP-DCTM.

Les 3 fragments d'ADN précédents sont purifiés sur gel. Une amplification du mélange de ces 3 ADN comme matrice est effectuée en présence des amorces R1Src et DCTM15Not (voir ci-dessus) dans les conditions et selon les recommandations de l'entreprise Finnzyme, fournisseur de la DNA polymérase Phusion utilisée. L'ADN obtenu fusionne en phase les 3 ADN précédent.

### II. CLONAGE DANS LE VECTEUR DE CLONAGE PCR^{®} BLUNTII-TOPO^{®}

### 1) Ligation des produits PCR dans le vecteur de clonage pCR® Bluntll-TOPO®

La ligation est réalisée à l'aide d'un vecteur plasmidique linéarisé possédant des enzymes topoisomérase I à ses extrémités (*cf.* *Figure 6*) permettant ainsi d'intégrer l'insert sans l'intervention d'une ligase. De plus, ce vecteur à une coupe franche de part et d'autre de ses extrémités, l'insert doit donc être aussi à bout franc pour pouvoir se liguer. Afin de sélectionner les transformants, le plasmide possède un gène de résistance à un antibiotique : la kanamycine.

Dans la glace, on mélange : 0,3µL de produit PCR, 0,25µL de « salt solution », 0,7µL d'eau stérile et 0,25µL de vecteur TOPO du kit Topo Blunt cloning (Invitrogen), soit 1,5µL au final. Incubation de 5 à 30min à température ambiante.

### 2) Transformation des bactéries TOP10 chimio-compétentes

Les bactéries TOP 10 chimio-compétentes (10µL) sont transformées par la totalité du mélange de ligation TOPO/insert. Il y a un témoin positif de transformation (1ng de PUC19: plasmide portant le gène de résistance à l'ampicilline) et un témoin négatif (plasmide Topo sans insert). Après incubation 30min dans la glace, le mélange subit un choc thermique pendant 30sec à 42°C. Les tubes sont ensuite immédiatement remis dans la glace. Il est ensuite ajouté et homogénéisé délicatement 250µL de milieu SOC (Bacto-tryptone 2%, Bacto-yeast extract 0,5%, NaCl 0,05% et glucose 0,2%) qui sont incubés 1h à 37°C au bain-marie le temps de l'expression phénotypique. Enfin, 150µL des suspensions sont ensemencées sur boîte LB (Luria Broth)/agar à 50µg/mL de kanamycine et incubées 24h à 37°C. Sur les boîtes, les colonies ayant acquis le gène de résistance à la kanamycine et donc, qui possèdent le vecteur de clonage avec ou sans le gène d'intérêt, se sont développées. Des clones sont repiqués et numérotés sur une deuxième boîte de gélose, incubés 24h à 37°C et des cultures liquides de 10mL (LB+kanamycine 100µM) de clones repiqués sont produites durant 16h à 37°C sous agitation.

### 3) Minipréparation d'ADN plasmidique

La culture bactérienne est centrifugée 15min à 3500rpm et à 4°C. Le culot est repris dans 250µL de tampon de re-suspension A1 (kit plasmid DNA Purification Nucleospin (Macherey-Nagel)). Les bactéries sont lysées pendant 5min à température ambiante avec 250µL de tampon de lyse A2. On ajoute ensuite 300µL de tampon de neutralisation A3 et il est important de bien mélanger par retournement pour ne pas obtenir un culot diffus. Les tubes sont centrifugés 10min à 11000rpm à 4°C. Le surnageant est passé sur colonne placée sur un tube de collecte, le tout centrifugé 1min à 11000rpm. Après lavage avec 500µL de tampon de lavage A4, la colonne est séchée 2min à 11000rpm. On passe 50µL de tampon d'élution et on récolte l'éluat dans un tube Eppendorf. L'ADN obtenu est dosé par spectrophotométrie à 260nm.

### 4) Digestion enzymatique (EcoRI) et séquençage

Afin de vérifier si l'ADN plasmidique obtenu est bien recombiné, les plasmides sont digérés par une enzyme de restriction spécifique (Biolabs^{®}) EcoRI qui encadre le site d'insertion. Il est digéré environ 200ng de chaque plasmide avec 4 unités d'enzyme. A cela, il est ajouté 1µL de tampon NEB1 10X et de l'eau stérile afin d'obtenir 10µL au final. La digestion se fait à 37°C pendant 1 h. Après identification des bons clones sur gel d'agarose 2%, 1,5µg d'ADN plasmidique est envoyée à séquencer chez la société Eurofins MWG Operon. Ceci permet de vérifier l'intégrité de chaque séquence en vérifiant si les sites de restrictions rajoutés sont bien présents ainsi que la mutation. Plusieurs clones sont obtenus, leurs ADN plasmidiques ontété analysés et séquencés. Deux plasmides sont retenus: le premier Src-SNAP-CDTM représente le gène chimère attendu, le second D-SNAP-CDTM correspond à une séquence présentant une délétion d'une partie du fragment Src qui a du se produire lors de la dernière PCR. La protéine codée par l'ADN D-SNAP-CDTM contient aussi une glycine dans une position myristoylable mais a perdu une partie de ses acides aminés basiques qui renforcent les propriétés d'ancrage sous-membranaire.

### III. CLONAGE DANS LE VECTEUR D'EXPRESSION PCDNA 3.1

### 1) Préparation du vecteur d'expression

Le vecteur pCDNA3.1 est digéré par les enzymes de restriction EcoR1 et NotI, puis déphosphorylé et extrait au phénol/chloroforme.

Une précipitation à l'isopropanol permet d'éliminer le court fragment d'ADN (<100pb situé entre EcoR1 et NotI) libéré lors de la digestion et qui pourrait se religuer avec le plasmide. Ajout de 0,15 fois le volume d'acétate de sodium 3M et 0,6 fois le volume en isopropanol froid. Incuber 30min à 4°C puis centrifuger à 4°C pendant 15min à 15000rpm. Laver le culot avec 500µL d'éthanol 70% et centrifuger de nouveau 10min à 15000rpm. Sécher le culot sous vide pendant 15min et le reprendre dans 50µL de TE.

### 2) Préparation des inserts

Digestions successives EcoR1-NotI: 2µg d'ADN plasmidique pTopo contenant les inserts Src-SNAP-CDTM et D-SNAP-CDTM sont digérés de façon successive par les 2 enzymes de restrictions encadrant l'insert, à savoir EcoR1 et NotI. Après inactivation des enzymes 20min à 65°C, l'ADN est précipité à l'éthanol et re-suspendu dans 20µL de TE. Il est ensuite stocké à -20°C.

Purification des inserts dans un gel d'agarose low melting (EUROBIO^{®}) : Les fragments d'insert sont extraits sur gel d'agarose low melting 2% et purifiés sur colonne à l'aide du kit Nucleospin (Kit Nucleospin Extract II^{®}, MACHEREY-NAGEL). Le volume final est de 50µL.

### 3) Ligation

Dans un tube, 35ng de vecteur déphosporylé est mélangé à l'insert avec un rapport insert/vecteur d'environ 3/1 (molécule à molécule). Il est additionné : 1µL de tampon ligase 10X, eau stérile (qsp 10µL) et 1U de T4 DNA ligase (Biolabs^{®}). Un témoin négatif est réalisé en remplaçant le volume d'insert par le même volume de TE1X. Les mélanges sont incubés toute la nuit à température ambiante.

### 4) Transformation de bactéries DH5α

Cette transformation repose sur un choc thermique. Ajout de 200µL de bactéries DH5 α dans le produit de ligation ; 30min dans la glace ; 2min à 42°C ; 5min dans la glace ; ajout de 800µL de milieu LB ; incubation 1 h à 37°C sous agitation ; ensemencement de 100µL sur boîte LB/agar/ ampicilline (50µg/mL) et incubation 16h à l'étuve à 37°C.

### 5) Vérification de la présence de l'insert et visualisation sur gel d'agarose

Des Midipréparations et une vérification par digestion EcoR1-NotI ont été réalisées afin de sélectionner les bons clones et un stock des ADN palsmidiques est préparé pour l'étape de transfection et d'expression protéique.

### IV. EXPRESSION DES PROTEINES CHIMERES ET ET CIBLAGE VERS LES EXOSOMES

### 1) Culture cellulaire

Des cellules eucaryotes HEK 293T (Human Embryonic Kidney) sont cultivées dans des flasques en milieu DMEM (Dulbecco's modified eagle's médium), contenant 10% de sérum de veau foetal (SVF) et de la gentamycine à 20 µg/mL, à 37°C sous 5% de CO₂.

### 2) Transfection

Dans des plaques 6 puits, il est ensemencé 0,5.10⁶cellules HEK 293T/ puits dans 4mL de DMEM 10%SVF sans antibiotique. Après 24h de culture à 37°C sous 5% de CO₂ les cellules sont à 90% de confluence et sont transfectées par un complexe formés entre 1µg de plasmide d'intérêt et 2µL de JetPEI (PolyPlus^{®}) dans 500µL de milieu, l'ADN est internalisé par endocytose. Après 6h d'incubation, le milieu est remplacé par du DMEM à 10% en SVF exempt d'exosomes contenus dans le sérum (éliminés par une ultracentrifugation à 42000rpm durant 18h (rotor Ti 45, Beckman). A 48h post-transfection, le surnageant est repris afin de récupérer les exosomes produits et les cellules sont lysées pour obtenir les protéines cellulaires.

### 3) Préparation des exosomes

Une fois le milieu cellulaire récupéré, il est centrifugé 10min à 1400rpm pour éliminer les cellules du milieu de culture. Le surnageant est centrifugé 10min à 10000rpm à 4°C afin d'éliminer les débris cellulaires. Le surnageant est récupéré et un coussin de TNE à 20% de sucrose est coulé au fond du tube pour être ultracentrifugé à 42000rpm dans un rotor Ti50 (Beckman) durant 2h à 4°C. Le culot d'exosomes purs est ensuite repris dans 100µL de PBS. **4) Extraction des protéines**

Les cellules sont lavées au PBS à 4°C, puis lysées avec du tampon RIPA (TNE 1X, NP40 0,5%, Aprotinine 20µg/mL, Leupeptine 20µM, H₂O stérile, PMSF 0,2mM). Le lysat est centrifugé 20 min à 14 000 rpm à 4°C et le surnageant est récupéré.

Les protéines des extraits cellulaires et des exosomes sont dosées par la méthode de Bradford grâce à un spectrophotomètre NanoDrop et une gamme étalon de BSA (0-200 µg/mL).

### 5) Electrophorèse SDS-PAGE et Western Blot:

Les échantillons (20µg pour les extraits protéiques cellulaires et 2µg pour les lysats exosomaux) sont séparés sur gel de polyacrylamide 10% durant 1h30 à 60mA puis transférés sur une membrane de PVDF (polyvinylidène difluoride, Immobilon-P, Millipore), préalablement activée dans un bain de méthanol, rincée à l'eau puis au TBST (Tris Base 20mM pH 7,4 ; NaCl 0,15M ; Tween 0,5%), durant toute la nuit à 50mA en chambre froide. La membrane est ensuite saturée avec 5% de lait en poudre dans du TBST pendant 1h. Ensuite, elle est incubée toute la nuit à 4°C avec l'anticorps primaire : sérum de lapin anti-CD™ préparé au laboratoire. La membrane est lavée 3 fois 5min au TBST puis incubée 1h avec l'anticorps secondaire anti IgG de lapin couplé à la peroxydase (Jackson ImmunoResearch). Elle est à nouveau lavée dans du TBST puis déposée sur du papier Whatmam imbibé d'une solution d'ECL (Enhanced chemiluminescence, Amersham)). La révélation s'effectue à l'aide d'une caméra Lumi-imager F1 (Roche^{®}).

Comme le montre la figure 34, les protéines DSC (pistes 2 et 5) et SSC CDTM (pistes 3 et 6) font environ 30 kDA sont exprimées dans les cellules (voir figure 34B). Les deux protéines DSC et SSC se retrouvent ciblées dans les exosomes (voir figure 34A). L'examen de ratios entre protéines sécrétées dans les exosomes / protéines produites dans les cellules montre que le ciblage de la protéine SSC dans les exosomes est plus efficace que celui de la protéine DSC.

### BIBLIOGRAPHIE

Alberts B, Bray D, Lewis J, Raff M, Roberts K, Watson JD (1995). "Molecular Biology of the Cell (3rd Ed.)" Garland, NY.
Bertrand E, Chartrand P, Schaefer M, Shenoy SM, Singer RH, Long RM. (1998). "Localization of ASH1 mRNA particles in living yeast." Mol Cell. Vol. 2 (4), 437-445.
Cann AJ, Churcher MJ, Boyd M, O'Brien W, Zhao JQ, Zack J, Chen IS (1992). "The region of the envelope gene of human immunodeficiency virus type 1 responsible for determination of cell tropism." J Virol. 1992 ; 66(1):305-9.
Chaput N, Taïeb J, Schartz N, André F, Angevin E, Zitvogel L. (2004). "Exosomes-based immunotherapy." Cancer Immunol Immunother, 53:234-239.
Colino, J. et Snapper C.M. (2006). Journal of Immunology, 177 :37576
De Gassart A, Geminard C, Hoekstra D, Vidal M. (2004). "Exosome secretion : the art of reutilizing nonrecycled proteins." Traffic. ; 5:896-903**.**
De Gassart A, Trentin B, Martin M, Hocquellet A, Bette-Bobillo P, Mamoun R, Vidal M. (2009) "Exosomal sorting of the cytoplasmic domain of bovine leukemia virus TM Env protein." Cell Biol Int. 2009 (1):36-48. Epub 2008 Oct 22**.**Delamarre L, Rosenberg AR, Pique C, Pham D, Callebaut I, Dokhelar MC. (1996). "The HTLV-I envelope glycoproteins: structure and functions." J Acquir Immune Defic Syndr Hum Retrovirol; 13 Suppl 1:S85-91.
Delcayre, A., et al. 2005, Blood Cells Mol Dis 35 :158 **;** Thery C. et al., (2002); Nature Immunology 3 :1156.
Delcayre A, Le Pecq JB. (2006) "Exosomes as a novel therapeutic nanodevices." Curr Op in Mol Ther.; 8:1464-1471.
Gould SJ, Booth A, Hildret JE. (2003) "The Trojan exosome hypothesis." Proc Natl Acad Sci USA.; 100:10592-10597.
Herbein G, Coaquette A, Perez-Bercoff D, Pancino G. (2002) "Macrophage activation and HIV infection: can the trojan horse turn into a fortress?" Curr Mol Med.; 2:723-738.
Hurley JH. (2006). "Membrane binding domains". Biochim Biophys Acta. 2006 Aug;1761 (8):805-11. Epub 2006 Mar 24. Review.
Keppler A, Gendreizig S, Gronemeyer T, Pick H, Vogel H, Johnsson K. (2002). "A general method for the covalent labeling of fusion proteins with small molecules in vivo". Nat Biotechnol. 2003 Jan;21(1):86-89. Epub 2002 Dec 9.
Keppler A, Pick H, Arrivoli C, Vogel H, Johnsson K. (2004). "Labeling of fusion proteins with synthetic fluorophores in live cells". Proc Natl Acad Sci USA. 2004 Jul 6;101(27):9955-9. Epub 2004 Jun 28.
Levine AJ. (1992). "Viruses" (Scientific American Library, No. 37). W. H. Freeman/Scientific American Library
Pornillos O, Garrus J, Sundquist WI. (2002). "Mechanism of enveloped RNA virus budding." Trends Cell Biol. 2; 12:569-579.
Raposo G, Moore M, Innes D, Leijenderkker R, Leigh-Brown A, Benaroch P, Geuze H. (2002). "Human macrophage accumulate HIV-1 particles in MHC II compartments." Traffic. ; 3:718-729.
Resh MD. (1994). "Myristylation and palmitylation of Src family members: the fats of the matter." Cell. 1994 Feb 11;76(3):411-3 .Sloane JA, and al. (2005). Marked structural and functional heterogeneity in CxCR4: Separation of HIV-1 and SDF-1 alpha responses. Immunology and Cell Biology; 83: 129-143.
Straub OC, Levy D. (1999). "Bovine immunodeficiency virus and analogies with human immunodeficiency virus." Leukemia.;13 Suppl 1:S106-9.
Zitvogel L, Regnault A, Lozier A, Wolfers J, Tenza D, Raposo G, Amigorena S. (1998). "Dendritic cell-derived exosomes elicit potent antitumour immune responses in vivo." Nat. Med. ; 4:594-600.

## Revendications

1. Polypeptide chimérique capable d'être sécrété en association avec des exosomes, **caractérisé en ce qu'**il comprend ou consiste en les domaines suivants:
(i) un peptide ou polypeptide d'intérét ;
(ii) un domaine de ciblage sous-membranaire, permettant audit polypeptide chimérique d'être ancré à la membrane d'un exosome par l'intermédiaire d'une ou plusieurs molécule(s) d'ancrage et/ou d'interactions entre ledit domaine de ciblage sous-membranaire et ladite membrane, mais sans s'insérer ni traverser la bicouche lipidique de la membrane de cet exosome, la séquence dudit domaine de ciblage sous-membranaire étant la séquence d'un fragment d'une protéine de la famille Src et comprenant ou consistant en la séquence M-G-S-S-K-S-K-P-K-D-P-S-Q-R-R-R ou en la séquence M-G-S-S-K-S-K-P-K-D-P-S-Q-R-R-R-K-S-R-G-P-G-G; et
(iii) un dérivé muté du domaine cytoplasmique (CD) d'une protéine membranaire, ce dérivé muté étant défini par la substitution, la délétion et/ou l'insertion d'un ou plusieurs résidu(s) dans la séquence dudit domaine CD, ledit domaine CD étant le domaine CD de SEQ ID NO : 6, qui est le domaine CD de la protéine transmembranaire (TM) du virus de la leucémie bovine (BLV), ledit dérivé muté ayant conservé la capacité dudit domaine CD à adresser ledit polypeptide chimérique aux exosomes et/ou à un(des) compartiment(s) cellulaire(s) impliqué(s) dans la formation des exosomes, ledit dérivé muté de domaine CD comprenant au moins un motif YxxL ou DYxxL, et un motif PxxP, dans lequel x représente un résidu quelconque et Y, L, D et P représentent respectivement un résidu tyrosine, leucine, acide aspartique et proline, la séquence dudit dérivé muté étant identique à au moins 80% à la séquence de SEQ ID NO : 8, et étant différente de la séquence de SEQ ID NO : 6 par au moins l'une des caractéristiques (a)-(b) suivantes :
(a) ledit dérivé muté est dépourvu de la séquence KCLTSRLLKLLRQ qui est contenue dans la séquence de SEQ ID NO : 6,
(b) dans ledit dérivé muté, le résidu cystéine de la séquence PCP qui est contenue dans SEQ ID NO : 6 est délété ou est remplacé par le résidu alanine ;
étant entendu que lesdits domaines (i) et (ii) et (iii) sont des domaines cytosoliques ou nucléaires et que ledit polypeptide chimérique est dépourvu de peptide signal d'importation dans le réticulum endoplasmique, et dans lequel les domaines (i) à (iii) sont positionnés successivement dans l'ordre suivant, de l'extrémité N-terminale vers l'extrémité C-terminale dans le polypeptide : (ii) - (i) - (iii), ou (ii) - (iii) - (i).

2. Polypeptide chimérique selon la revendication 1, dans lequel le motif PXXP est le motif PSAP (SEQ ID NO. 88) ou PTAP (SEQ ID NO. 89).

3. Polypeptide chimérique selon la revendication 1 ou 2, dans lequel ledit dérivé muté de domaine CD comprend :
- au moins un motif YxxL ou DYxxL choisi parmi YINL, YSHL et DYINL ; ou
- au moins un motif PxxP choisi parmi PSAP ou PTAP.

4. Polypeptide chimérique selon l'une quelconque des revendications 1 à 3, dans lequel ledit dérivé muté de domaine CD comprend :
- au moins un motif YxxL ou DYxxL choisi parmi YINL, YSHL et DYINL ; et
- au moins un motif PxxP choisi parmi PSAP ou PTAP.

5. Polypeptide chimérique selon l'une quelconque des revendications 1 à 4, dans lequel le domaine de ciblage sous-membranaire comprend plusieurs résidus d'acides aminés basiques.

6. Polypeptide chimérique selon l'une quelconque des revendications 1 à 5, dans lequel le domaine de ciblage sous-membranaire comprend plusieurs résidus choisis parmi les résidus K, R et H.

7. Polypeptide chimérique selon l'une quelconque des revendications 1 à 6, dans lequel ladite protéine de la famille Src est choisie parmi les protéines Src, Yes, Lyn, Fyn, Lck, Blk, Fgr, Hck et Yrk.

8. Polypeptide chimérique selon l'une quelconque des revendications 1 à 7, comprenant en outre au moins une molécule de liaison liant deux domaines successifs, ladite molécule de liaison étant un peptide ou un polypeptide.

9. Polypeptide chimérique selon l'une quelconque des revendications 1 à 8, dans lequel ledit dérivé muté de domaine CD diffère de la séquence de SEQ ID NO : 6 par au moins l'une des, ou les deux caractéristiques (a)-(b) suivantes :
(a) ledit dérivé muté est dépourvu de la séquence KCLTSRLLKLLRQ et/ou
(b) dans ledit dérivé muté, le résidu cystéine de la séquence PCP qui est contenue dans SEQ ID NO : 6 est remplacé par le résidu alanine.

10. Polypeptide chimérique selon l'une quelconque des revendications 1 à 9, dans lequel la séquence dudit dérivé muté de domaine CD présente au moins 90% ou 95% d'identité avec la séquence de SEQ ID NO : 8.

11. Polypeptide chimérique selon l'une quelconque des revendications 1 à 10, dans lequel la séquence dudit dérivé muté de domaine CD comprend ou consiste en une séquence choisie parmi les séquences suivantes:
PxxPxxxxxxxxxxxxYxxL
PxxPxxxxxxxxxxxDYxxL
PxxPxxYxxxxxxxxxYxxL ;
PxxPxxYxxxxxxxxDYxxL ;
PxxPExYxxLxPxxPDYxxL ;
PxxPxₙYxxL
PxxPxₙDYxxL
PxxPxₙYxₙYxxL ;
PxxPxₙYxₙDYxxL ;
PxxPExₙYxxLxₙPxxPDYxxL ;
PxxPxxxxPxxPxxxYxxLxPxxPExYxxLxPxxPDYxxL ;
PxxPxₙPxxPxnYxxLxₙPxxPEXₙYxxLxₙPxxPDYxxL ;
PxxPxxxxPxxPxxxYxxLxPxxPExYxxLxPxxPDYxxLxxxx ; et
PxxPxₙPxxPxₙYxxLxₙPxxPEXₙYxxLxₙPxxPDYxxLxxxx,
dans lesquelles x et xₙ représentent respectivement un résidu quelconque et un ou plusieurs résidu(s) quelconque(s) et dans lesquelles au moins un des motifs PxxP est le motif PSAP ou PTAP.

12. Polypeptide chimérique selon l'une quelconque des revendications 1 à 11, dans lequel le peptide ou le polypeptide d'intérêt comprend ou consiste en :
- un ou plusieurs domaine(s) d'une protéine cytosolique ou un ou des fragment(s) de ce(s) domaine(s) ; et/ou
- un ou plusieurs domaine(s) d'une protéine nucléaire ou un ou des fragment(s) de ce(s) domaine(s).

13. Polypeptide chimérique selon l'une quelconque des revendications 1 à 12, dans lequel ledit peptide ou le polypeptide d'intérêt est un fragment d'au moins 6 acides aminés d'une protéine nucléaire ou cytosolique.

14. Polypeptide chimérique selon l'une quelconque des revendications 1 à 13, dans lequel le peptide ou le polypeptide d'intérêt provient d'un organisme pathogène, d'un virus, d'une bactérie, d'un parasite, d'un agent pathogène, d'un antigène tumoral, d'un antigène cytoplasmique, d'un récepteur de ligand, d'un récepteur à multiples domaines membranaires, d'un récepteur à sept domaines transmembranaires, d'un récepteur de cytokines ou d'un fragment de ceux-ci, ou d'une toxine ou partie de toxine, ou d'une enzyme ou d'un polypeptide liant un acide nucléique.

15. Polypeptide chimérique selon l'une quelconque des revendications 1 à 14, pour une utilisation comme médicament, ou pour la prophylaxie et/ou le traitement d'une infection bactérienne, virale, parasitaire, ou d'une tumeur ou d'un déficit fonctionnel ou métabolique.

16. Polypeptide chimérique pour une utilisation selon la revendication 15, **caractérisé en ce qu'**il est utilisé en combinaison avec un polypeptide chimérique additionnel, ledit polypeptide chimérique additionnel, étant capable d'être sécrété en association avec des exosomes, et ledit polypeptide chimérique additionnel étant **caractérisé en ce qu'**il comprend ou consiste en les domaines suivants:
- un peptide ou polypeptide d'intérêt;
- un domaine transmembranaire; et
- le domaine cytoplasmique (CD) de la protéine transmembranaire (TM) du virus de la leucémie bovine (BLV), qui est de SEQ ID NO : 6, ou un dérivé muté de ce domaine CD de SEQ ID NO: 6, ledit dérivé muté étant défini par la substitution, la délétion et/ou l'insertion d'un ou plusieurs résidu(s) dans la séquence dudit domaine CD et ledit dérivé muté ayant conservé la capacité à adresser ledit polypeptide chimérique additionnel à des exosomes et/ou à un(des) compartiment(s) cellulaire(s) impliqué(s) dans la formation des exosomes, ledit dérivé muté comprenant au moins un motif YxxL ou DYxxL et un motif PxxP, dans lequel x représente un résidu quelconque et Y, L, D et P représentent respectivement un résidu tyrosine, leucine, acide aspartique et proline ;
le domaine CD ou le dérivé muté du polypeptide chimérique additionnel étant identique à ou différent du dérivé muté du domaine CD du polypeptide chimérique selon l'une quelconque des revendications 1 à 12.

17. Polypeptide chimérique pour son utilisation selon la revendication 16, dans lequel le domaine CD dudit polypeptide chimérique additionnel ou le dérivé muté de domaine CD dudit polypeptide chimérique additionnel comprend au moins un motif YxxL ou DYxxL choisi parmi YINL, YSHL et DYINL, et/ou au moins un motif PxxP choisi parmi PSAP et PTAP.

18. Polypeptide chimérique pour son utilisation selon la revendication 16 ou 17, dans lequel le domaine CD dudit polypeptide chimérique additionnel ou le dérivé muté de domaine CD dudit polypeptide chimérique additionnel est dépourvu de la séquence KCLTSRLLKLLRQ et/ou dépourvu de la séquence PCP.

19. Polypeptide chimérique pour son utilisation selon l'une quelconque des revendications 16 à 18, dans lequel la séquence du dérivé muté de domaine CD est identique à au moins 80% à la séquence de SEQ ID NO : 8.

20. Polypeptide chimérique pour son utilisation selon l'une quelconque des revendications 16 à 19, dans lequel le peptide ou polypeptide d'intérêt dudit polypeptide chimérique additionnel est un fragment d'au moins 6 acides aminés d'une protéine cytosolique ou nucléaire, ou un fragment d'au moins 6 acides aminés d'une protéine extracellulaire ou de surface.

21. Polypeptide chimérique pour une utilisation selon l'une quelconque des revendications 16 à 20, dans lequel dans le polypeptide chimérique additionnel, les domaines suivants sont positionnés successivement dans l'ordre suivant, de l'extrémité N-terminale vers l'extrémité C-terminale dans le polypeptide : peptide ou polypeptide d'intérêt - domaine membranaire - domaine cytoplasmique (CD) ou dérivé muté.

22. Polypeptide chimérique selon l'une quelconque des revendications 1 à 14, pour une utilisation en immunisation.

23. Polypeptide chimérique pour une utilisation selon la revendication 22, qui est utilisé en combinaison avec un polypeptide chimérique additionnel tel que défini à l'une quelconque des revendications 16 à 21.

24. Un exosome, comportant un polypeptide chimérique selon l'une quelconque des revendications 1 à 15 et 22 à 23.

25. L'exosome de la revendication 24, qui comprend en outre un polypeptide chimère additionnel tel que défini à l'une quelconque des revendications 16 à 21.

26. Une composition thérapeutique dont le principe actif comprend un ou plusieurs exosomes selon la revendication 24 ou 25.

27. Composition thérapeutique selon la revendication 26, qui est destinée à être utilisée pour traiter des cellules cibles chez un hôte et/ou pour détruire des cellules cibles chez un hôte et/ou pour faire du diagnostic chez un hôte et/ou pour la prophylaxie et/ou le traitement d'une infection bactérienne, virale, parasitaire, ou d'une tumeur.

28. Un polynucléotide **caractérisé en ce qu'**il code pour un polypeptide chimérique selon l'une quelconque des revendications 1 à 15 et 22 à 23, ou **en ce qu'**il code pour un polypeptide chimérique selon l'une quelconque des revendications 1 à 15 et 22 à 23 et pour un polypeptide chimérique additionnel tel que défini à l'une quelconque des revendications 16 à 21.

29. Une composition thérapeutique comprenant :
- un acide nucléique, **caractérisé en ce qu'**il comprend ou consiste en un polynucléotide selon la revendication 28 ; et
- un support, un diluant ou un véhicule pharmaceutiquement acceptable.

30. Cellule recombinante productrice d'exosomes, **caractérisée en ce que** :
- elle est recombinée avec un ou plusieurs polynucléotide(s) selon la revendication 28 ; ou
- elle a absorbé un ou plusieurs exosomes selon la revendication 24 ou 25.

31. Utilisation d'un ou plusieurs exosomes selon la revendication 24 ou 25, pour la détection *in vitro* de partenaires spécifiques capables d'interagir avec ledit peptide ou polypeptide d'intérêt ou avec un fragment dudit peptide ou polypeptide, ou avec un polypeptide chimérique additionnel tel que défini à l'une quelconque des revendications 16 à 21.

32. Procédé de production *in vitro* d'exosomes comportant un peptide ou un polypeptide d'intérêt, ledit procédé comprenant les étapes suivantes :
a) l'introduction, d'un ou plusieurs polynucléotide(s) selon la revendication 28, qui code(nt) pour un polypeptide comprenant ledit peptide ou polypeptide d'intérêt, dans une cellule productrice d'exosomes, ou la mise en contact d'une cellule productrice d'exosomes avec un ou plusieurs exosomes selon la revendication 24 ou 25, qui comporte(nt) un polypeptide comprenant ledit peptide ou polypeptide d'intérêt,
b) la culture de ladite cellule productrice d'exosomes;
c) la récupération des exosomes produits par ladite cellule productrice d'exosomes.

33. Procédé de préparation d'un sérum polyclonal dirigé contre un ou plusieurs peptide(s) ou polypeptide(s) antigénique(s) d'intérêt exprimé(s) à la surface d'exosomes, comprenant les étapes suivantes :
a) l'administration à un animal non humain, d'exosomes selon la revendication 24 ou 25, d'une composition selon l'une quelconque des revendications 26, 27 et 29, ou d'un polynucléotide selon la revendication 28, associés ou non à un adjuvant; et
b) la récupération des anticorps formés, capables de reconnaître le ou les peptide(s) ou polypeptide(s) antigénique(s) d'intérêt.

34. Procédé de préparation d'anticorps monoclonaux dirigés contre un ou plusieurs peptide(s) ou polypeptide(s) antigénique(s) exprimé(s) à la surface de ou dans des exosomes, ledit procédé comprenant les étapes suivantes :
a) la fusion, avec des cellules de myélome, de cellules spléniques préalablement obtenues chez un hôte auquel on a administré des exosomes selon la revendication 24 ou 25, une composition selon l'une quelconque des revendications 26, 27 et 29, ou un polynucléotide selon la revendication 28 ;
b) la culture et la sélection des hybridomes dans des conditions permettant la production d'anticorps;
c) la récupération des anticorps monoclonaux dirigés contre les peptide(s) ou polypeptide(s) antigénique(s) d'intérêt.

35. Procédé de criblage *in vitro* de molécules interagissant avec un peptide ou un polypeptide d'intérêt ou avec un fragment dudit peptide ou un polypeptide d'intérêt, ledit procédé comprenant :
a) la mise en contact d'exosomes selon la revendication 24 ou 25 avec une ou plusieurs molécules susceptibles d'interagir avec ledit peptide ou polypeptide d'intérêt ;
b) la détection d'une éventuelle interaction entre ledit peptide ou polypeptide d'intérêt ou un fragment dudit peptide ou polypeptide d'intérêt et ladite ou lesdites molécules.

## Patentansprüche

1. Chimäres Polypeptid, das fähig ist, in Assoziation mit Exosomen sezerniert zu werden, **dadurch gekennzeichnet, dass** es die folgenden Domänen umfasst oder aus ihnen besteht:
(i) ein Peptid oder Polypeptid von Interesse;
(ii) eine Submembran-Zieldomäne, die es dem chimären Polypeptid erlaubt, an die Membran eines Exosoms mittels einem oder mehreren Ankermolekül(en) und/oder Wechselwirkungen zwischen der Submembran-Zieldomäne und der Membran verankert zu sein, aber ohne sich in die Lipiddoppelschicht der Membran des Exosoms zu insertieren oder sie zu durchqueren, wobei die Sequenz der Submembran-Zieldomäne die Sequenz eines Proteins der Src-Familie ist und die Sequenz M-G-S-S-K-S-K-P-K-D-P-S-Q-R-R-R oder die Sequenz M-G-S-S-K-S-K-P-K-D-P-S-Q-R-R-R-K-S-R-G-P-G-G umfasst oder daraus besteht; und
(iii) ein mutiertes Derivat der cytoplasmatischen Domäne (CD) eines Membranproteins, wobei das Derivat durch die Substitution, die Deletion und/oder die Insertion von einem oder mehreren Rest(en) in der Sequenz der CD-Domäne definiert ist, wobei die CD-Domäne die CD-Domäne von SEQ ID NO: 6 ist, die die CD-Domäne des Transmembranproteins (TM) des bovinen Leukämievirus (BLV) ist, wobei das mutierte Derivat die Fähigkeit der CD-Domäne beibehalten hat, das chimäre Polypeptid an Exosomen und/oder an ein/eines der Zellkompartiment(e), das/die in die Bildung der Exosomen einbezogen ist/sind, zu adressieren, wobei das mutierte Derivat der CD-Domäne mindestens ein Motiv YxxL oder DYxxL, und ein Motiv PxxP umfasst, in dem x einen beliebigen Rest darstellt, und Y, L, D und P jeweils einen Tyrosin-, Leucin-, Aparaginsäure- und Prolinrest darstellen, wobei die Sequenz des mutierten Derivats zu mindestens 80% identisch zur Sequenz von SEQ ID NO: 8 ist, und sich zur Sequenz von SEQ ID NO: 6 durch mindestens eine der folgenden Eigenschaften (a)-(b) unterscheidet:
(a) das mutierte Derivat ist frei von der Sequenz KCLTSRLLKLLRQ, die in der Sequenz von SEQ ID NO: 6 enthalten ist,
(b) in dem mutierten Derivat ist der Cysteinrest der Sequenz PCP, die in SEQ ID NO: 6 enthalten ist, deletiert oder ist durch einen Alaninrest ersetzt;
wobei verstanden wird, dass die Domänen (i) und (ii) und (iii) cytosolische oder nukleäre Domänen sind, und dass das chimäre Polypeptid frei von einem Peptidsignal für den Import in das endoplasmatische Retikulum ist, und in dem die Domänen (i) bis (iii) aufeinanderfolgend in der folgenden Reihenfolge angeordnet sind, vom N-terminalen Ende zum C-terminalen Ende im Polypeptid: (ii) - (i) - (iii) oder (ii) - (iii) - (i).

2. Chimäres Polypeptid nach Anspruch 1, in dem das Motiv PXXP das Motiv PSAP (SEQ ID NO: 88) oder PTAP (SEQ ID NO: 89) ist.

3. Chimäres Polypeptid nach Anspruch 1 oder 2, in dem das mutierte Derivat der CD-Domäne umfasst:
- mindestens ein YxxL- oder DYxxL-Motiv ausgewählt aus YINL, YSHL und DYINL; oder
- mindestens ein PxxP-Motiv ausgewählt aus PSAP oder PTAP.

4. Chimäres Polypeptid nach einem beliebigen der Ansprüche 1 bis 3, in dem das mutierte Derivat der CD-Domäne umfasst:
- mindestens ein YxxL- oder DYxxL-Motiv ausgewählt aus YINL, YSHL und DYINL; und
- mindestens ein PxxP-Motiv ausgewählt aus PSAP oder PTAP.

5. Chimäres Polypeptid nach einem beliebigen der Ansprüche 1 bis 4, in dem die Submembran-Zieldomäne mehrere basische Aminosäurereste umfasst.

6. Chimäres Polypeptid nach einem beliebigen der Ansprüche 1 bis 5, in dem die Submembran-Zieldomäne mehrere Reste ausgewählt aus K, R und H umfasst.

7. Chimäres Polypeptid nach einem beliebigen der Ansprüche 1 bis 6, in dem das Protein der Src-Familie ausgewählt ist aus den Proteinen Src, Yes, Lyn, Fyn, Lck, Blk, Fgr, Hck und Yrk.

8. Chimäres Polypeptid nach einem beliebigen der Ansprüche 1 bis 7, weiterhin umfassend mindestens ein Verbindungsmolekül, das zwei aufeinanderfolgende Domänen verbindet, wobei das Verbindungsmolekül ein Peptid oder ein Polypeptid ist.

9. Chimäres Polypeptid nach einem beliebigen der Ansprüche 1 bis 8, in dem das mutierte Derivat der CD-Domäne sich von der Sequenz von SEQ ID NO: 6 durch mindestens eine der, oder beide, folgenden Eigenschaften (a)-(b) unterscheidet:
(a) das mutierte Derivat ist frei von der Sequenz KCLTSRLLKLLRQ und/oder
(b) im mutierten Derivat ist der Cysteinrest der Sequenz PCP, die in SEQ ID NO: 6 enthalten ist, durch einen Alaninrest ersetzt.

10. Chimäres Polypeptid nach einem beliebigen der Ansprüche 1 bis 9, in dem die Sequenz des mutierten Derivats der CD-Domäne mindestens 90% oder 95% Identität mit der Sequenz von SEQ ID NO: 8 aufweist.

11. Chimäres Polypeptid nach einem beliebigen der Ansprüche 1 bis 10, in dem die Sequenz des mutierten Derivats der CD-Domäne eine Sequenz ausgewählt aus den folgenden Sequenzen umfasst oder daraus besteht:
PxxPxxxxxxxxxxxxYxxL
PxxPxxxxxxxxxxxDYxxL
PxxPxxYxxxxxxxxxYxxL;
PxxPxxYxxxxxxxxDYxxL ;
PxxPExYxxLxPxxPDYxxL;
PxxPxₙYxxL
PxxPxₙDYxxL
PxxPxₙYxₙYxx L ;
PxxPxₙYxₙDYxxL ;
PxxPExₙYxxLxₙPxxPDYxxL ;
PxxPxxxxPxxPxxxYxxLxPxxPExYxxLxPxxPDYxxL ;
PxxPxₙPxxPxₙYxxLxₙPxxPEXₙYxxLxₙPxxPDYxxL ;
PxxPxxxxPxxPxxxYxxLxPxxPExYxxLxPxxPDYxxLxxxx ; und
PxxPxₙPxxPxₙYxxLxₙPxxPEXₙYxxLxₙPxxPDYxxLxxxx,
in denen x und xₙ jeweils einen beliebigen Rest und einen oder mehrere beliebige(n) Rest(e) darstellen, und in denen mindestens eines der Motive PxxP das Motiv PSAP oder PTAP ist.

12. Chimäres Polypeptid nach einem beliebigen der Ansprüche 1 bis 11, in dem das Peptid oder Polypeptid von Interesse umfasst oder besteht aus:
- einer oder mehreren Domäne(n) eines cytosolischen Proteins oder einem oder Fragment(en) dieser Domäne(n); und/oder
- einem oder mehreren Domäne(n) eines nuklearen Proteins oder einem oder Fragment(en) dieser Domäne(n).

13. Chimäres Polypeptid nach einem beliebigen der Ansprüche 1 bis 12, in dem das Peptid oder Polypeptid von Interesse ein Fragment von mindestens 6 Aminosäuren eines nukleären oder cytosolischen Proteins ist.

14. Chimäres Polypeptid nach einem beliebigen der Ansprüche 1 bis 13, in dem das Peptid oder Polypeptid von Interesse aus einem pathogenen Organismus, einem Virus, einer Bakterie, einem Parasiten, einem pathogenen Mittel, einem Tumorantigen, einem cytoplasmatischen Antigen, einem Ligandenrezeptor, einem Rezeptor mit mehreren Membrandomänen, einem Sieben-Transmembrandomänen-Rezeptor, einem Cytokinrezeptor oder einem Fragment dessen, oder einem Toxin oder einem Teil eines Toxins, oder einem Enzym oder einem eine Nukleinsäure verbindenden Polypeptid stammt.

15. Chimäres Polypeptid nach einem beliebigen der Ansprüche 1 bis 14, für eine Verwendung als Medikament, oder zur Vorbeugung und/oder zur Behandlung einer bakteriellen, viralen, parasitären Infektion, oder eines Tumors oder eines funktionellen oder metabolischen Defizits.

16. Chimäres Polypeptid für eine Verwendung nach Anspruch 15, **dadurch gekennzeichnet, dass** es in Kombination mit einem zusätzlichen chimären Polypeptid verwendet wird, wobei das zusätzliche chimäre Polypeptid, das fähig ist, in Assoziation mit Exosomen sezerniert zu werden, und das zusätzliche chimäre Polypeptid **dadurch gekennzeichnet sind, dass** es die folgenden Domänen umfasst oder daraus besteht:
- ein Peptid oder Polypeptid von Interesse;
- eine Transmembrandomäne; und
- die cytoplasmatische Domäne (CD) des Transmembranproteins (TM) des bovinen Leukämievirus (BLV), die aus SEQ ID NO: 6 ist, oder ein mutiertes Derivat dieser CD-Domäne von SEQ ID NO: 6, wobei das mutierte Derivat durch die Substitution, Deletion und/oder Insertion von einem oder mehreren Rest(en) in der Sequenz der CD-Domäne definiert ist, und das mutierte Derivat die Fähigkeit beibehalten hat, das zusätzliche chimäre Polypeptid an Exosomen und/oder ein/eines der Zellkompartiment(e), das/die in die Bildung der Exosomen einbezogen ist/sind, zu adressieren, wobei das mutierte Derivat mindestens ein Motiv YxxL oder DYxxL und ein Motiv PxxP umfasst, in dem x einen beliebigen Rest darstellt, und Y, L, D und P jeweils einen Tyrosin-, Leucin-, Aparaginsäure- und Prolinrest darstellen;
wobei die CD-Domäne oder das mutierte Derivat des zusätzlichen chimären Polypeptids identisch oder verschieden zum mutierten Derivat der CD-Domäne des chimären Polypeptids nach einem beliebigen der Ansprüche 1 bis 12 sind.

17. Chimäres Polypeptid für seine Verwendung nach Anspruch 16, in dem die CD-Domäne des zusätzlichen chimären Polypeptids oder das mutierte Derivat der CD-Domäne des zusätzlichen chimären Polypeptids mindestens ein Motiv YxxL oder DYxxL ausgewählt aus YINL, YSHL und DYINL, und/oder mindestens ein PxxP-Motiv ausgewählt aus PSAP und PTAP umfasst.

18. Chimäres Polypeptid für seine Verwendung nach Anspruch 16 oder 17, in dem die CD-Domäne des zusätzlichen chimären Polypeptids oder das mutierte Derivat der CD-Domäne des zusätzlichen chimären Polypeptids frei von der Sequenz KCLTSRLLKLLRQ und /oder frei von der Sequenz PCP ist.

19. Chimäres Polypeptid für seine Verwendung nach einem beliebigen der Ansprüche 16 bis 18, in dem die Sequenz des mutierten Derivats der CD-Domäne zu mindestens 80% identisch zur Sequenz von SEQ ID NO: 8 ist.

20. Chimäres Polypeptid für seine Verwendung nach einem beliebigen der Ansprüche 16 bis 19, in dem das Peptid oder Polypeptid von Interesse des zusätzlichen chimären Polypeptids ein Fragment von mindestens 6 Aminosäuren eines cytosolischen oder nukleären Proteins, oder ein Fragment von mindestens 6 Aminosäuren eines extrazellulären oder Oberflächenproteins ist.

21. Chimäres Polypeptid für eine Verwendung nach einem beliebigen der Ansprüche 16 bis 20, in dem im zusätzlichen chimären Polypeptid die folgenden Domänen aufeinanderfolgend in der folgenden Reihenfolge angeordnet sind, vom N-terminalen Ende zum C-terminalen Ende im Polypeptid: Peptid oder Polypeptid von Interesse - Membrandomäne - cytoplasmatische Domäne (CD) oder mutiertes Derivat.

22. Chimäres Polypeptid nach einem beliebigen der Ansprüche 1 bis 14, für eine Verwendung in der Immunisierung.

23. Chimäres Polypeptid für eine Verwendung nach Anspruch 22, das in Kombination mit einem zusätzlichen chimären Polypeptid wie in einem beliebigen der Ansprüche 16 bis 21 definiert verwendet wird.

24. Exosom, enthaltend ein chimäres Polypeptid gemäß einem beliebigen der Ansprüche 1 bis 15 und 22 bis 23.

25. Exosom nach Anspruch 24, das außerdem ein zusätzliches chimäres Polypeptid wie in einem beliebigen der Ansprüche 16 bis 21 definiert umfasst.

26. Therapeutische Zusammensetzung, deren Wirkstoff ein oder mehrere Exosomen gemäß Anspruch 24 oder 25 umfasst.

27. Therapeutische Zusammensetzung nach Anspruch 26, die vorgesehen ist, zum Behandeln von Zielzellen bei einem Wirt und/oder zum Zerstören von Zielzellen bei einem Wirt und/oder zum Durchführen von Diagnostik bei einem Wirt und/oder zur Vorbeugung und/oder der Behandlung einer bakteriellen, viralen, parasitären Infektion oder eines Tumors verwendet zu werden.

28. Polynukleotid, **dadurch gekennzeichnet, dass** es ein chimäres Polypeptid gemäß einem beliebigen der Ansprüche 1 bis 15 und 22 bis 23 kodiert, oder dadurch, dass es ein chimäres Polypeptid gemäß einem beliebigen der Ansprüche 1 bis 15 und 22 bis 23 und ein zusätzliches chimäres Polypeptid wie in einem beliebigen der Ansprüche 16 bis 21 definiert kodiert.

29. Therapeutische Zusammensetzung, umfassend:
- eine Nukleinsäure, **dadurch gekennzeichnet, dass** sie ein Polynukleotid gemäß Anspruch 28 umfasst oder daraus besteht; und
- einen Träger, ein Verdünnungsmittel oder pharmazeutisch verträgliches Vehikel.

30. Rekombinante, Exosomen herstellende Zelle, **dadurch gekennzeichnet, dass**:
- sie mit einem oder mehreren Polynukleotid(en) gemäß Anspruch 28 rekombiniert ist; oder
- sie ein oder mehrere Exosomen gemäß Anspruch 24 oder 25 absorbiert hat.

31. Verwendung von einem oder mehreren Exosomen gemäß Anspruch 24 oder 25, zum *in vitro*-Nachweis von spezifischen Partnern, die zum Wechselwirken mit dem Peptid oder Polypeptid von Interesse oder mit einem Fragment des Peptids oder Polypeptids, oder mit einem zusätzlichen chimären, wie gemäß einem der Ansprüche 16 bis 21 definierten Polypeptid fähig sind.

32. *In vitro*-Herstellungsverfahren von Exosomen, die ein Peptid oder ein Polypeptid von Interesse enthalten, wobei das Verfahren die folgenden Schritte umfasst:
(a) die Einführung von einem oder mehreren Polynukleotid(en) gemäß Anspruch 28, das/die ein Polypeptid kodiert/kodieren, das das Peptid oder Polypeptid von Interesse umfasst, in eine Exosomen herstellende Zelle, oder das Inkontaktbringen einer Exosomen herstellenden Zelle mit einem oder mehreren Exosomen gemäß Anspruch 24 oder 25, das/die ein Polypeptid enthält/enhalten, das das Peptid oder Polypeptid von Interesse umfasst,
(b) die Kultur der Zelle, die Exosomen herstellt,
(c) das Gewinnen der Exosomen, die durch die Exosomen herstellende Zelle hergestellt werden.

33. Herstellungsverfahren eines polyklonalen Serums, das gegen ein oder mehrere antigene(s) Peptid(e) oder Polypeptid(e) von Interesse gerichtet ist, das/die an der Oberfläche von Exosomen exprimiert wird/werden, umfassend die folgenden Schritte:
(a) die Verabreichung an ein nicht menschliches Tier von Exosomen gemäß Anspruch 24 oder 25, von einer Zusammensetzung gemäß einem beliebigen der Ansprüche 26, 27 und 29, oder von einem Polynukleotid gemäß Anspruch 28, assoziiert oder nicht an ein Adjuvans; und
(b) die Gewinnung der gebildeten Antikörper, die fähig sind, das oder die antigene(n) Peptid(e) oder Polypeptid(e) von Interesse zu erkennen.

34. Herstellungsverfahren von monoklonalen Antikörpern, die gegen ein oder mehrere antigen(e) Peptid(e) oder Polypeptid(e) gerichtet sind, die auf der Oberfläche von oder in Exosomen exprimiert werden, wobei das Verfahren die folgenden Schritte umfasst:
(a) die Fusion mit Myelomzellen von Milzzellen, die zuvor bei einem Wirt erhalten wurden, dem Exosomen gemäß Anspruch 24 oder 25, eine Zusammensetzung gemäß einem der Ansprüche 26, 27 und 29, oder ein Polynukleotid gemäß Anspruch 28 verabreicht wurden;
(b) die Kultur und die Auswahl von Hybridomen unter Bedingungen, die die Herstellung von Antikörpern erlauben;
(c) das Gewinnen von monoklonalen Antikörpern, die gegen das/die antigene(n) Peptid(e) oder Polypeptid(e) von Interesse gerichtet sind.

35. *In vitro*-Screening-Verfahren von Molekülen, die mit einem Peptid oder Polypeptid von Interesse oder mit einem Fragment des Peptids oder Polypeptids von Interesse wechselwirken, wobei das Verfahren umfasst:
(a) Inkontaktbringen von Exosomen gemäß Anspruch 24 oder 25 mit einem oder mehreren Molekülen, die geeignet sind, mit dem Peptid oder Polypeptid von Interesse zu wechselwirken;
(b) der Nachweis einer möglichen Wechselwirkung zwischen dem Peptid oder Polypeptid von Interesse oder einem Fragments des Peptids oder Polypeptids von Interesse und dem oder den Molekül(en).

## Claims

1. A chimeric polypeptide that is capable of being secreted in association with exosomes, **characterized in that** it comprises or consists of the following domains:
(i) a peptide or polypeptide of interest;
(ii) an infra-membrane targeting domain, enabling said chimeric polypeptide to be anchored to the membrane of an exosome via one or more molecule(s) for anchoring and/or for interactions between said infra-membrane targeting domain and said membrane but without either being inserted into or passing through the lipid bilayer of the membrane of that exosome, the sequence of said infra-membrane targeting domain being the sequence of a fragment of a protein of the Src family and comprising or consisting of the sequence M-G-S-S-K-S-K-P-K-D-P-S-Q-R-R-R or of the sequence M-G-S-S-K-S-K-P-K-D-P-S-Q-R-R-R-K-S-R-G-P-G-G; and
(iii) a mutated derivative of the cytoplasmic domain (CD) of a membrane protein, said mutated derivative being defined by substitution, deletion and/or insertion of one or more residue(s) in the sequence of said CD domain, said CD domain being the CD domain of SEQ ID NO. 6 which is the CD domain of the transmembrane (TM) protein of the bovine leukemia virus (BLV), said mutated derivative having conserved the capacity of said CD domain for addressing said chimeric polypeptide to exosomes and/or to one of the cellular compartment(s) involved in exosome formation, said mutated derivative of the CD domain comprising at least one motif YxxL or DYxxL, and a motif PxxP, in which x represents any residue and Y, L, D and P respectively represent a tyrosine, leucine, aspartic acid and proline residue, the sequence of said mutated derivative having at least 80% identity with the sequence of SEQ ID NO. 8 and differing from the sequence of SEQ ID NO. 6 in at least one of the following characteristics (a) - (b):
(a) said mutated derivative is deprived of the sequence KCLTSRLLKLLRQ which is contained in the sequence of SEQ ID NO. 6,
(b) in said mutated derivative, the cysteine residue of the sequence PCP which is contained in SEQ ID NO. 6 is deleted or is replaced by the alanine residue;
it being understood that said domains (i) and (ii) and (iii) are cytosolic or nuclear domains and that said chimeric polypeptide is deprived of a signal peptide for importation into the endoplasmic reticulum, and in which the domains (i) to (iii) are positioned in succession in the following order, from the N-terminal end towards the C-terminal end in the polypeptide: (ii) - (i) - (iii), or (ii) - (iii) - (i).

2. The chimeric polypeptide as claimed in claim 1, in which the motif PXXP is the motif PSAP (SEQ ID NO. 88) or PTAP (SEQ ID NO. 89).

3. The chimeric polypeptide as claimed in claim 1 or claim 2, in which said mutated derivative of the CD domain comprises:
• at least one motif YxxL or DYxxL selected from YINL, YSHL and DYINL; or
• at least one motif PxxP selected from PSAP or PTAP.

4. The chimeric polypeptide as claimed in any one of claims 1 to 3, in which said mutated derivative of the CD domain comprises:
• at least one motif YxxL or DYxxL selected from YINL, YSHL and DYINL; and
• at least one motif PxxP selected from PSAP or PTAP.

5. The chimeric polypeptide as claimed in any one of claims 1 to 4, in which the infra-membrane targeting domain comprises a plurality of basic amino acid residues.

6. The chimeric polypeptide as claimed in any one of claims 1 to 5, in which the infra-membrane targeting domain comprises a plurality of residues selected from the residues K, R and H.

7. The chimeric polypeptide as claimed in any one of claims 1 to 6, in which said protein of the Src family is selected from the proteins Src, Yes, Lyn, Fyn, Lck, Blk, Fgr, Hck and Yrk.

8. The chimeric polypeptide as claimed in any one of claims 1 to 7, further comprising at least one linker linking two successive domains, said linker being a peptide or a polypeptide.

9. The chimeric polypeptide as claimed in any one of claims 1 to 8, in which said mutated derivative of the CD domain differs from the sequence of SEQ ID NO. 6 in at least one or both of the following two characteristics (a) - (b):
(a) said mutated derivative is deprived of the sequence KCLTSRLLKLLRQ, and/or
(b) in said mutated derivative, the cysteine residue of the sequence PCP which is contained in SEQ ID NO. 6 is replaced by the alanine residue.

10. The chimeric polypeptide as claimed in any one of claims 1 to 9, in which the sequence of said mutated derivative of the CD domain has at least 90% or 95% identity with the sequence of SEQ ID NO. 8.

11. The chimeric polypeptide as claimed in any one of claims 1 to 10, in which the sequence of said mutated derivative of the CD domain comprises or consists of a sequence selected from the following sequences:
PxxPxxxxxxxxxxxxYxxL
PxxPxxxxxxxxxxxDYxxL
PxxPxxYxxxxxxxxxYxxL;
PxxPxxYxxxxxxxxDYxxL;
PxxPExYxxLxPxxPDYxxL;
PxxPxₙYxxL
PxxPxₙDYxxL
PxxPxₙYxₙYxxL;
PxxPxₙYxₙDYxxL;
PxxPExₙYxxLxₙPxxPDYxxL;
PxxPxxxxPxxPxxxYxxLxPxxPExYxxLxPxxPDYxxL;
PxxPxₙPxxPxₙYxxLxₙPxxPEXₙYxxLxₙPxxPDYxxL;
PxxPxxxxPxxPxxxYxxLxPxxPExYxxLxPxxPDYxxLxxxx; and
PxxPxₙPxxPxₙYxxLxₙPxxPEXₙYxxLxₙPxxPDYxxLxxxx,
in which x and xₙ respectively represent any residue and one or more of any residue(s) and in which at least one of the motifs PxxP is the motif PSAP or PTAP.

12. The chimeric polypeptide as claimed in any one of claims 1 to 11, in which the peptide or the polypeptide of interest comprises or consists of:
• one or more domain(s) of a cytosolic protein or one or more fragment(s) of this (these) domain(s); and/or
• one or more domain(s) of a nuclear protein or one or more fragment(s) of this (these) domain(s).

13. The chimeric polypeptide as claimed in any one of claims 1 to 12, in which said peptide or polypeptide of interest is a fragment of at least 6 amino acids of a nuclear or cytosolic protein.

14. The chimeric polypeptide as claimed in any one of claims 1 to 13, in which the peptide or the polypeptide of interest originates from a pathogenic organism, a virus, a bacterium, a parasite, a pathogenic agent, a tumoral antigen, a cytoplasmic antigen, a ligand receptor, a receptor with multiple membrane domains, a receptor with seven transmembrane domains, a cytokine receptor or a fragment thereof, or from a toxin or toxin portion, or from an enzyme or from a polypeptide binding a nucleic acid.

15. The chimeric polypeptide as claimed in any one of claims 1 to 14, for use as a drug, or for the prophylaxis and/or treatment of a bacterial, viral or parasitic infection, or of a tumor or of a functional or metabolic deficit.

16. Chimeric polypeptide for use as claimed in claim 15, **characterized in that** it is used in combination with an additional chimeric polypeptide, said additional chimeric polypeptide being capable of being secreted in combination with exosomes, and said additional chimeric polypeptide being **characterized in that** it comprises or consists of the following domains:
• a peptide or polypeptide of interest;
• a transmembrane domain; and
• the cytoplasmic domain (CD) of the transmembrane (TM) protein of the bovine leukemia virus (BLV) which is SEQ ID NO. 6, or a mutated derivative of this CD domain of SEQ ID NO. 6, said mutated derivative being defined by the substitution, deletion and/or insertion of one or more residue(s) into the sequence of said CD domain and said mutated derivative having conserved the capacity for addressing said additional chimeric polypeptide to exosomes and/or to one of the cellular compartment(s) involved in the formation of exosomes, said mutated derivative comprising at least one motif YxxL or DYxxL and a motif PxxP, in which x represents any residue and Y, L, D and P respectively represent a tyrosine, leucine, aspartic acid and proline residue;
the CD domain or the mutated derivative of the additional chimeric polypeptide being identical to or different from the mutated derivative of the CD domain of the chimeric polypeptide as claimed in any one of claims 1 to 12.

17. Chimeric polypeptide for use as claimed in claim 16, in which the CD domain of said additional chimeric polypeptide or the mutated derivative of the CD domain of said additional chimeric polypeptide comprises at least one motif YxxL or DYxxL selected from YINL, YSHL and DYINL, and/or at least one motif PxxP selected from PSAP and PTAP.

18. Chimeric polypeptide for use as claimed in claim 16 or 17, in which the CD domain of said additional chimeric polypeptide or the mutated derivative of the CD domain of said additional chimeric polypeptide is deprived of the sequence KCLTSRLLKLLRQ and/or deprived of the sequence PCP.

19. Chimeric polypeptide for use as claimed in any one of claims 16 to 18, in which the sequence of the mutated derivative of the CD domain has at least 80% identity with the sequence of SEQ ID NO. 8.

20. Chimeric polypeptide for use as claimed in any one of claims 16 to 19, in which the peptide or polypeptide of interest of said additional chimeric polypeptide is a fragment of at least 6 amino acids of a cytosolic or nuclear protein, or a fragment of at least 6 amino acids of an extracellular or surface protein.

21. Chimeric polypeptide for use as claimed in any one of claims 16 to 20, in which in the additional chimeric polypeptide, the following domains are positioned in succession in the following order, from the N-terminal end towards the C-terminal end in the polypeptide: peptide or polypeptide of interest - membrane domain - cytoplasmic domain (CD) or mutated derivative.

22. The chimeric polypeptide as claimed in any one of claims 1 to 14, for use in immunization.

23. Chimeric polypeptide for use as claimed in claim 22, which is used in combination with an additional chimeric polypeptide as defined in any one of claims 16 to 21.

24. An exosome comprising a chimeric polypeptide as claimed in any one of claims 1 to 15 and 22 to 23.

25. The exosome as claimed in claim 24, which further comprises an additional chimeric polypeptide as defined in any one of claims 16 to 21.

26. A therapeutic composition in which the active principle comprises one or more exosomes as claimed in claim 24 or claim 25.

27. The therapeutic composition as claimed in claim 26, intended for use in treating target cells in a host and/or to destroy target cells in a host and/or to carry out a diagnostic in a host and/or for the prophylaxis and/or treatment of a bacterial, viral or parasitic infection, or of a tumor.

28. A polynucleotide, **characterized in that** it codes for a chimeric polypeptide as claimed in any one of claims 1 to 15 and 22 to 23 or **in that** it codes for a chimeric polypeptide as claimed in any one of claims 1 to 15 and 22 to 23 and for an additional chimeric polypeptide as defined in any one of claims 16 to 21.

29. A therapeutic composition comprising:
• a nucleic acid, **characterized in that** it comprises or consists of a polynucleotide as claimed in claim 28; and
• a pharmaceutically acceptable support, a diluent or a vehicle.

30. A recombinant exosome-producing cell, **characterized in that**:
• it is recombined with one or more polynucleotide(s) as claimed in claim 28; or
• it has absorbed one or more exosomes as defined in claim 24 or 25.

31. Use of one or more exosome(s) as claimed in claim 24 or 25, for the *in vitro* detection of specific partners that are capable of interacting with said peptide or polypeptide of interest or with a fragment of said peptide or polypeptide, or with an additional chimeric polypeptide as defined in any one of claims 16 to 21.

32. A method for *in vitro* production of exosomes comprising a peptide or a polypeptide of interest, said method comprising the following steps:
a) introducing one or more polynucleotide(s) as claimed in claim 28, which code(s) for a polypeptide comprising said peptide or polypeptide of interest, into an exosome-producing cell, or bringing an exosome-producing cell into contact with one or more exosomes as claimed in claim 24 or claim 25, which comprise(s) a polypeptide comprising said peptide or polypeptide of interest ;
b) culturing said exosome-producing cell;
c) recovering the exosomes produced by said exosome-producing cell.

33. A method for preparing a polyclonal serum directed against one or more antigenic peptide(s) or polypeptide(s) of interest expressed at the surface of exosomes, comprising the following steps:
a) administering, to a non-human animal, exosomes as claimed in claim 24 or claim 25, a composition as claimed in any one of claims 26, 27 and 29, or a polynucleotide as claimed in claim 28, associated or not associated with an adjuvant; and
b) recovering antibodies that are formed, which are capable of recognizing the antigenic peptide(s) or polypeptide(s) of interest.

34. A method for preparing monoclonal antibodies directed against one or more antigenic peptide(s) or polypeptide(s) expressed at the surface of or in exosomes, said method comprising the following steps:
a) fusing, with myeloma cells, spleen cells that have been previously obtained from a host to which exosomes as claimed in claim 24 or claim 25, a composition as claimed in any one of claims 26, 27 and 29, or a polynucleotide as claimed in claim 28 have been administered;
b) culturing and selecting hybridomas under conditions enabling the production of antibody;
c) recovering monoclonal antibodies directed against the antigenic peptide(s) or polypeptide(s) of interest.

35. A method for *in vitro* screening of molecules interacting with a peptide or a polypeptide of interest or with a fragment of said peptide or a polypeptide of interest, said method comprising:
a) bringing exosomes as claimed in claim 24 or claim 25 into contact with one or more molecules that are susceptible of interacting with said peptide or polypeptide of interest;
b) detecting any interaction between said peptide or polypeptide of interest or a fragment of said peptide or polypeptide of interest and said molecule or molecules.


### SEQUENCE LISTING

<110> CENTRE NATIONAL DE LA RECHERCHE SCIENTIFIQUE
<120> Nouveaux polynucléotides et polypeptides chimériques permettant
   la sécrétion d'un polypeptide d'intérêt en association avec des
   exosomes et leurs utilisations.
<130> B08525A_AD/SL/CAL
<150> FR 09/04576
   <151> 2009-09-24
<160> 124
<170> PatentIn version 3.3
<210> 1
   <211> 81
   <212> DNA
   <213> Mus musculus
<220>
   <221> CDS
   <222> (1)..(81)
<220>
   <221> misc_feature
   <222> (1)..(81)
   <223> Peptide signal d'importation dans le réticulum endoplasmique de
   la protéine CD8 alpha
<400> 1
   atg gcc tca ccg ttg acc cgc ttt ctg tcg ctg aac ctg ctg ctg ctg       48
   Met Ala Ser Pro Leu Thr Arg Phe Leu Ser Leu Asn Leu Leu Leu Leu
   1               5                   10                  15
   ggt gag tcg att atc ctg ggg agt gga gaa gct                           81
   Gly Glu Ser Ile Ile Leu Gly Ser Gly Glu Ala
               20                  25
<210> 2
   <211> 27
   <212> PRT
   <213> Mus musculus
<400> 2
   Met Ala Ser Pro Leu Thr Arg Phe Leu Ser Leu Asn Leu Leu Leu Leu
   1               5                   10                  15
   Gly Glu Ser Ile Ile Leu Gly Ser Gly Glu Ala
               20                  25
<210> 3
   <211> 69
   <212> DNA
   <213> Bovine leukemia virus
<220>
   <221> misc_feature
   <222> (1)..(69)
   <223> Domaine transmembranaire de la protéine TM du BLV (séquence
   sauvage)
<220>
   <221> CDS
   <222> (1)..(69)
<400> 3
   ctc att cat tct gtt cta agc cta ttc cta tta gcc ctt ttt ttg ctc       48
   Leu Ile His Ser Val Leu Ser Leu Phe Leu Leu Ala Leu Phe Leu Leu
   1               5                   10                  15
   ttc ttg gcc ccc tgc ctg ata                                           69
   Phe Leu Ala Pro Cys Leu Ile
               20
<210> 4
   <211> 23
   <212> PRT
   <213> Bovine leukemia virus
<400> 4
   Leu Ile His Ser Val Leu Ser Leu Phe Leu Leu Ala Leu Phe Leu Leu
   1               5                   10                  15
   Phe Leu Ala Pro Cys Leu Ile
               20
<210> 5
   <211> 177
   <212> DNA
   <213> Bovine leukemia virus
<220>
   <221> misc_feature
   <222> (1)..(177)
   <223> Domaine cytoplasmique (CD) sauvage de la protéine TM
<220>
   <221> CDS
   <222> (1)..(177)
<220>
   <221> misc_feature
   <222> (42)..(43)
   <223> n = t ou a
<400> 5
   aaa tgc ttg acc tct cgc ctt tta aaa ctc ctc cgg cag gcn ccc cac       48
   Lys Cys Leu Thr Ser Arg Leu Leu Lys Leu Leu Arg Gln Ala Pro His
   1               5                   10                  15
   ttc cct gaa atc tcc ttc ccc cct aaa ccc gat tct gat tat cag gcc       96
   Phe Pro Glu Ile Ser Phe Pro Pro Lys Pro Asp Ser Asp Tyr Gln Ala
               20                  25                  30
   ttg cta cca tcc gcg cca gag atc tac tct cac ctc tcc ccc acc aaa      144
   Leu Leu Pro Ser Ala Pro Glu Ile Tyr Ser His Leu Ser Pro Thr Lys
           35                  40                  45
   ccc gat tac atc aac ctt cga ccc tgc ccc tag                         177
   Pro Asp Tyr Ile Asn Leu Arg Pro Cys Pro
       50                  55
<210> 6
   <211> 58
   <212> PRT
   <213> Bovine leukemia virus
<400> 6
   Lys Cys Leu Thr Ser Arg Leu Leu Lys Leu Leu Arg Gln Ala Pro His
   1               5                   10                  15
   Phe Pro Glu Ile Ser Phe Pro Pro Lys Pro Asp Ser Asp Tyr Gln Ala
               20                  25                  30
   Leu Leu Pro Ser Ala Pro Glu Ile Tyr Ser His Leu Ser Pro Thr Lys
           35                  40                  45
   Pro Asp Tyr Ile Asn Leu Arg Pro Cys Pro
       50                  55
<210> 7
   <211> 135
   <212> DNA
   <213> Artificial
<220>
   <223> polynucléotide
<220>
   <221> misc_feature
   <222> (1)..(135)
   <223> Dérivé muté du CD de la protéine TM du virus de la leucémie
   bovine (BLV) : délétion des 13 résidus N-terminaux
<220>
   <221> CDS
   <222> (1)..(135)
<400> 7
   gca ccc cac ttc cct gaa atc tcc ttc ccc cct aaa ccc gat tct gat        48
   Ala Pro His Phe Pro Glu Ile Ser Phe Pro Pro Lys Pro Asp Ser Asp
   1               5                   10                  15
   tat cag gcc ttg cta cca tcc gcg cca gag atc tac tct cac ctc tcc        96
   Tyr Gln Ala Leu Leu Pro Ser Ala Pro Glu Ile Tyr Ser His Leu Ser
               20                  25                  30
   ccc acc aaa ccc gat tac atc aac ctt cga ccc tgc ccc                   135
   Pro Thr Lys Pro Asp Tyr Ile Asn Leu Arg Pro Cys Pro
           35                  40                  45
<210> 8
   <211> 45
   <212> PRT
   <213> Artificial
<220>
   <223> Synthetic Construct
<400> 8
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Pro Thr Lys Pro Asp Tyr Ile Asn Leu Arg Pro Cys Pro
           35                  40                  45
<210> 9
   <211> 174
   <212> DNA
   <213> Artificial
<220>
   <223> polynucléotide
<220>
   <221> misc_feature
   <222> (1)..(174)
   <223> Dérivé muté du CD de la protéine TM du BLV : substitution du
   résidu C du motif PCT par un résidu A
<220>
   <221> CDS
   <222> (1)..(174)
<400> 9
   aaa tgc ttg acc tct cgc ctt tta aaa ctc ctc cgg cag gca ccc cac        48
   Lys Cys Leu Thr Ser Arg Leu Leu Lys Leu Leu Arg Gln Ala Pro His
   1               5                   10                  15
   ttc cct gaa atc tcc ttc ccc cct aaa ccc gat tct gat tat cag gcc        96
   Phe Pro Glu Ile Ser Phe Pro Pro Lys Pro Asp Ser Asp Tyr Gln Ala
               20                  25                  30
   ttg cta cca tcc gcg cca gag atc tac tct cac ctc tcc ccc acc aaa       144
   Leu Leu Pro Ser Ala Pro Glu Ile Tyr Ser His Leu Ser Pro Thr Lys
           35                  40                  45
   ccc gat tac atc aac ctt cga ccg gcg ccc                               174
   Pro Asp Tyr Ile Asn Leu Arg Pro Ala Pro
       50                  55
<210> 10
   <211> 58
   <212> PRT
   <213> Artificial
<220>
   <223> Synthetic Construct
<400> 10
   Lys Cys Leu Thr Ser Arg Leu Leu Lys Leu Leu Arg Gln Ala Pro His
   1               5                   10                  15
   Phe Pro Glu Ile Ser Phe Pro Pro Lys Pro Asp Ser Asp Tyr Gln Ala
               20                  25                  30
   Leu Leu Pro Ser Ala Pro Glu Ile Tyr Ser His Leu Ser Pro Thr Lys
           35                  40                  45
   Pro Asp Tyr Ile Asn Leu Arg Pro Ala Pro
       50                  55
<210> 11
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13
   résidus N-terminaux et substitution du résidu C du motif PCP par
   un résidu A
<400> 11
   gca ccc cac ttc cct gaa atc tcc ttc ccc cct aaa ccc gat tct gat       48
   Ala Pro His Phe Pro Glu Ile Ser Phe Pro Pro Lys Pro Asp Ser Asp
   1               5                   10                  15
   tat cag gcc ttg cta cca tcc gcg cca gag atc tac tct cac ctc tcc       96
   Tyr Gln Ala Leu Leu Pro Ser Ala Pro Glu Ile Tyr Ser His Leu Ser
               20                  25                  30
   ccc acc aaa ccc gat tac atc aac ctt cga ccg gcg ccc                  135
   Pro Thr Lys Pro Asp Tyr Ile Asn Leu Arg Pro Ala Pro
           35                  40                  45
<210> 12
   <211> 45
   <212> PRT
   <213> Artificial
<220>
   <223> Synthetic Construct
<400> 12
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Pro Thr Lys Pro Asp Tyr Ile Asn Leu Arg Pro Ala Pro
           35                  40                  45
<210> 13
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13
   résidus N-terminaux et substitution des 2 résidus P du 1er motif
   PxxP
<400> 13
   gca gcc cac ttc gct gag ata tcc ttc ccc cct aaa ccc gat tct gat       48
   Ala Ala His Phe Ala Glu Ile Ser Phe Pro Pro Lys Pro Asp Ser Asp
   1               5                   10                  15
   tat cag gcc ttg cta cca tcc gcg cca gag atc tac tct cac ctc tcc       96
   Tyr Gln Ala Leu Leu Pro Ser Ala Pro Glu Ile Tyr Ser His Leu Ser
               20                  25                  30
   ccc acc aaa ccc gat tac atc aac ctt cga ccc tgc ccc                  135
   Pro Thr Lys Pro Asp Tyr Ile Asn Leu Arg Pro Cys Pro
           35                  40                  45
<210> 14
   <211> 45
   <212> PRT
   <213> Artificial
<220>
   <223> Synthetic Construct
<400> 14
   Ala Ala His Phe Ala Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Pro Thr Lys Pro Asp Tyr Ile Asn Leu Arg Pro Cys Pro
           35                  40                  45
<210> 15
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13
   résidus N-terminaux et substitution des 2 résidus P du 2ème motif
   PxxP
<400> 15
   gca ccc cac ttc cct gag ata tcc ttc gcc cct aaa gcc gat tct gat        48
   Ala Pro His Phe Pro Glu Ile Ser Phe Ala Pro Lys Ala Asp Ser Asp
   1               5                   10                  15
   tat cag gcc ttg cta cca tcc gcg cca gag atc tac tct cac ctc tcc        96
   Tyr Gln Ala Leu Leu Pro Ser Ala Pro Glu Ile Tyr Ser His Leu Ser
               20                  25                  30
   ccc acc aaa ccc gat tac atc aac ctt cga ccc tgc ccc                   135
   Pro Thr Lys Pro Asp Tyr Ile Asn Leu Arg Pro Cys Pro
           35                  40                  45
<210> 16
   <211> 45
   <212> PRT
   <213> Artificial
<220>
   <223> Synthetic Construct
<400> 16
   Ala Pro His Phe Pro Glu Ile Ser Phe Ala Pro Lys Ala Asp Ser Asp
   1               5                   10                  15
   Tyr Gln Ala Leu Leu Pro Ser Ala Pro Glu Ile Tyr Ser His Leu Ser
               20                  25                  30
   Pro Thr Lys Pro Asp Tyr Ile Asn Leu Arg Pro Cys Pro
           35                  40                  45
<210> 17
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13
   résidus N-terminaux et substitution des 2 résidus proline du 3ème
   motif PxxP
<400> 17
   gca ccc cac ttc cct gaa atc tcc ttc ccc cct aaa ccc gat tct gat        48
   Ala Pro His Phe Pro Glu Ile Ser Phe Pro Pro Lys Pro Asp Ser Asp
   1               5                   10                  15
   tat cag gcc ttg cta gca tcg gcg gca gag att tac tct cac ctc tcc        96
   Tyr Gln Ala Leu Leu Ala Ser Ala Ala Glu Ile Tyr Ser His Leu Ser
               20                  25                  30
   ccc acc aaa ccc gat tac atc aac ctt cga ccc tgc ccc                   135
   Pro Thr Lys Pro Asp Tyr Ile Asn Leu Arg Pro Cys Pro
           35                  40                  45
<210> 18
   <211> 45
   <212> PRT
   <213> Artificial
<220>
   <223> Synthetic Construct
<400> 18
   Ala Pro His Phe Pro Glu Ile Ser Phe Pro Pro Lys Pro Asp Ser Asp
   1               5                   10                  15
   Tyr Gln Ala Leu Leu Ala Ser Ala Ala Glu Ile Tyr Ser His Leu Ser
               20                  25                  30
   Pro Thr Lys Pro Asp Tyr Ile Asn Leu Arg Pro Cys Pro
           35                  40                  45
<210> 19
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13
   résidus N-terminaux et substitution du 1er résidu P du 4ème motif
   PxxP
<400> 19
   gca ccc cac ttc cct gaa atc tcc ttc ccc cct aaa ccc gat tct gat       48
   Ala Pro His Phe Pro Glu Ile Ser Phe Pro Pro Lys Pro Asp Ser Asp
   1               5                   10                  15
   tat cag gcc ttg cta cca tcc gcg cca gag ata tac tct cac ctc tcc       96
   Tyr Gln Ala Leu Leu Pro Ser Ala Pro Glu Ile Tyr Ser His Leu Ser
               20                  25                  30
   gcc acc aaa ccc gat tac atc aac ctt cga ccc tgc ccc                  135
   Ala Thr Lys Pro Asp Tyr Ile Asn Leu Arg Pro Cys Pro
           35                  40                  45
<210> 20
   <211> 45
   <212> PRT
   <213> Artificial
<220>
   <223> Synthetic Construct
<400> 20
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Ala Thr Lys Pro Asp Tyr Ile Asn Leu Arg Pro Cys Pro
           35                  40                  45
<210> 21
   <211> 135
   <212> DNA
   <213> Artificial
<220>
   <223> polynucléotide
<220>
   <221> misc_feature
   <222> (1)..(135)
   <223> Dérivé muté du CD de la protéine TM du BLV : délétion des 13
   résidus N-terminaux et substitution des 2 résidus P du 4ème motif
   PxxP
<220>
   <221> misc_feature
   <222> (1)..(135)
   <223> Dérivé muté du CD de la protéine TM du BLV : délétion des 13
   résidus N-terminaux et substitution du résidu du 4ème motif C du
   motif PCP par un résidu A du 4ème motif PxxP
<220>
   <221> CDS
   <222> (1)..(135)
<400> 21
   gca ccc cac ttc cct gaa atc tcc ttc ccc cct aaa ccc gat tct gat       48
   Ala Pro His Phe Pro Glu Ile Ser Phe Pro Pro Lys Pro Asp Ser Asp
   1               5                   10                  15
   tat cag gcc ttg cta cca tcc gcg cca gag ata tac tct cac ctc tcc       96
   Tyr Gln Ala Leu Leu Pro Ser Ala Pro Glu Ile Tyr Ser His Leu Ser
               20                  25                  30
   gcc acc aaa gcc gat tac atc aac ctt cga ccc tgc ccc                  135
   Ala Thr Lys Ala Asp Tyr Ile Asn Leu Arg Pro Cys Pro
           35                  40                  45
<210> 22
   <211> 45
   <212> PRT
   <213> Artificial
<220>
   <223> Synthetic Construct
<400> 22
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Ala Thr Lys Ala Asp Tyr Ile Asn Leu Arg Pro Cys Pro
           35                  40                  45
<210> 23
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13
   résidus N-terminaux et substitution du résidu Y du 1er motif YxxL
<400> 23
   gca ccc cac ttc cct gaa atc tcc ttc ccc cct aaa ccc gat tct gat        48
   Ala Pro His Phe Pro Glu Ile Ser Phe Pro Pro Lys Pro Asp Ser Asp
   1               5                   10                  15
   gcg cag gca ttg cta cca tcg gcg cca gag atc tac tct cac ctc tcc        96
   Ala Gln Ala Leu Leu Pro Ser Ala Pro Glu Ile Tyr Ser His Leu Ser
               20                  25                  30
   ccc acc aaa ccc gat tac atc aac ctt cga ccc tgc ccc                   135
   Pro Thr Lys Pro Asp Tyr Ile Asn Leu Arg Pro Cys Pro
           35                  40                  45
<210> 24
   <211> 45
   <212> PRT
   <213> Artificial
<220>
   <223> Synthetic Construct
<400> 24
   Ala Pro His Phe Pro Glu Ile Ser Phe Pro Pro Lys Pro Asp Ser Asp
   1               5                   10                  15
   Ala Gln Ala Leu Leu Pro Ser Ala Pro Glu Ile Tyr Ser His Leu Ser
               20                  25                  30
   Pro Thr Lys Pro Asp Tyr Ile Asn Leu Arg Pro Cys Pro
           35                  40                  45
<210> 25
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13
   résidus N-terminaux et substitution du résidu Y du 2ème motif
   YxxL
<400> 25
   gca ccc cac ttc cct gaa atc tcc ttc ccc cct aaa ccc gat tct gat        48
   Ala Pro His Phe Pro Glu Ile Ser Phe Pro Pro Lys Pro Asp Ser Asp
   1               5                   10                  15
   tat cag gcc ttg cta cca tcg gcg cca gag atc gca tct cac ctc tcc        96
   Tyr Gln Ala Leu Leu Pro Ser Ala Pro Glu Ile Ala Ser His Leu Ser
               20                  25                  30
   ccc acc aaa ccc gat tac atc aac ctt cga ccc tgc ccc                   135
   Pro Thr Lys Pro Asp Tyr Ile Asn Leu Arg Pro Cys Pro
           35                  40                  45
<210> 26
   <211> 45
   <212> PRT
   <213> Artificial
<220>
   <223> Synthetic Construct
<400> 26
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Ala Ser His Leu Ser
               20                  25                  30
   Pro Thr Lys Pro Asp Tyr Ile Asn Leu Arg Pro Cys Pro
           35                  40                  45
<210> 27
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : substitution du
   résidu Y du 3ème motif YxxL
<400> 27
   gca ccc cac ttc cct gaa atc tcc ttc ccc cct aaa ccc gat tct gat       48
   Ala Pro His Phe Pro Glu Ile Ser Phe Pro Pro Lys Pro Asp Ser Asp
   1               5                   10                  15
   tat cag gcc ttg cta cca tcc gcg cca gag atc tac tct cac ctc tcc       96
   Tyr Gln Ala Leu Leu Pro Ser Ala Pro Glu Ile Tyr Ser His Leu Ser
               20                  25                  30
   ccc acc aaa ccc gat gcc atc aac ctt agg ccc tgc ccc                  135
   Pro Thr Lys Pro Asp Ala Ile Asn Leu Arg Pro Cys Pro
           35                  40                  45
<210> 28
   <211> 45
   <212> PRT
   <213> Artificial
<220>
   <223> Synthetic Construct
<400> 28
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Pro Thr Lys Pro Asp Ala Ile Asn Leu Arg Pro Cys Pro
           35                  40                  45
<210> 29
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : substitution des 13
   résidus Y N-terminaux et substitution du résidu S se trouvant
   avant le 1er motif YxxL
<220>
   <221> misc_feature
   <222> (1)..(135)
   <223> Dérivé muté du CD de la protéine TM du BLV : substitution des 13
   résidus N-terminaux et substitution du résidu S se trouvant avant
   le 1er motif YxxL
<400> 29
   gca ccc cac ttc cct gaa atc tcc ttc ccc cct aaa ccc gat gct gat       48
   Ala Pro His Phe Pro Glu Ile Ser Phe Pro Pro Lys Pro Asp Ala Asp
   1               5                   10                  15
   tat cag gcg ttg cta cca tcc gcg cca gag atc tac tct cac ctc tcc       96
   Tyr Gln Ala Leu Leu Pro Ser Ala Pro Glu Ile Tyr Ser His Leu Ser
               20                  25                  30
   ccc acc aaa ccc gat tac atc aac ctt cga ccc tgc ccc                  135
   Pro Thr Lys Pro Asp Tyr Ile Asn Leu Arg Pro Cys Pro
           35                  40                  45
<210> 30
   <211> 45
   <212> PRT
   <213> Artificial
<220>
   <223> Synthetic Construct
<400> 30
   Ala Pro His Phe Pro Glu Ile Ser Phe Pro Pro Lys Pro Asp Ala Asp
   1               5                   10                  15
   Tyr Gln Ala Leu Leu Pro Ser Ala Pro Glu Ile Tyr Ser His Leu Ser
               20                  25                  30
   Pro Thr Lys Pro Asp Tyr Ile Asn Leu Arg Pro Cys Pro
           35                  40                  45
<210> 31
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du CD de la protéine TM du BLV : délétion des 13
   résidus N-terminaux et substitution du résidu E se trouvant avant
   le 2ème motif YxxL
<400> 31
   gca ccc cac ttc cct gaa atc tcc ttc ccc cct aaa ccc gat tct gat        48
   Ala Pro His Phe Pro Glu Ile Ser Phe Pro Pro Lys Pro Asp Ser Asp
   1               5                   10                  15
   tat cag gcc ttg cta cca tcc gcg cca gcg atc tac tct cac ctc tcc        96
   Tyr Gln Ala Leu Leu Pro Ser Ala Pro Ala Ile Tyr Ser His Leu Ser
               20                  25                  30
   ccc acc aaa ccc gat tac atc aac ctt cga ccc tgc ccc                   135
   Pro Thr Lys Pro Asp Tyr Ile Asn Leu Arg Pro Cys Pro
           35                  40                  45
<210> 32
   <211> 45
   <212> PRT
   <213> Artificial
<220>
   <223> Synthetic Construct
<400> 32
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Ala Ile Tyr Ser His Leu Ser
               20                  25                  30
   Pro Thr Lys Pro Asp Tyr Ile Asn Leu Arg Pro Cys Pro
           35                  40                  45
<210> 33
   <211> 135
   <212> DNA
   <213> Artificial
<220>
   <223> polynucléotide
<220>
   <221> CDS
   <222> (1)..(135)
<220>
   <221> misc_feature
   <222> (1)..(135)
   <223> Dérivé muté du domaine CD de la protéine TM du BLV : délétion des
   13 résidus N-terminaux et substitution du résidu D se trouvant
   avant le 3ème motif YxxL
<400> 33
   gca ccc cac ttc cct gaa atc tcc ttc ccc cct aaa ccc gat tct gat       48
   Ala Pro His Phe Pro Glu Ile Ser Phe Pro Pro Lys Pro Asp Ser Asp
   1               5                   10                  15
   tat cag gcc ttg cta cca tcc gcg cca gag atc tac tct cac ctc tcc       96
   Tyr Gln Ala Leu Leu Pro Ser Ala Pro Glu Ile Tyr Ser His Leu Ser
               20                  25                  30
   ccc acc aag ccg gct tac atc aac ctt cga ccc tgc ccc                  135
   Pro Thr Lys Pro Ala Tyr Ile Asn Leu Arg Pro Cys Pro
           35                  40                  45
<210> 34
   <211> 45
   <212> PRT
   <213> Artificial
<220>
   <223> Synthetic Construct
<400> 34
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Pro Thr Lys Pro Ala Tyr Ile Asn Leu Arg Pro Cys Pro
           35                  40                  45
<210> 35
   <211> 18
   <212> DNA
   <213> Artificial
<220>
   <223> polynucléotide
<220>
   <221> CDS
   <222> (1)..(18)
<220>
   <221> misc_feature
   <222> (1)..(18)
   <223> Dérivé muté du domaine CD de la protéine TM du BLV : délétion des
   13 résidus N-terminaux et des 39 résidus C-terminaux
<400> 35
   gca ccc cac ttc cct gaa                                               18
   Ala Pro His Phe Pro Glu
   1               5
<210> 36
   <211> 6
   <212> PRT
   <213> Artificial
<220>
   <223> Synthetic Construct
<400> 36
   Ala Pro His Phe Pro Glu
   1               5
<210> 37
   <211> 45
   <212> DNA
   <213> Artificial
<220>
   <223> polynucléotide
<220>
   <221> CDS
   <222> (1)..(45)
<220>
   <221> misc_feature
   <222> (1)..(45)
   <223> Dérivé muté du domaine CD de la protéine TM du BLV : délétion des
   13 résidus N-terminaux et des 30 résidus C-terminaux
<400> 37
   gca ccc cac ttc cct gaa atc tcc ttc ccc cct aaa ccc gat tct         45
   Ala Pro His Phe Pro Glu Ile Ser Phe Pro Pro Lys Pro Asp Ser
   1               5                   10                  15
<210> 38
   <211> 15
   <212> PRT
   <213> Artificial
<220>
   <223> Synthetic Construct
<400> 38
   Ala Pro His Phe Pro Glu Ile Ser Phe Pro Pro Lys Pro Asp Ser
   1               5                   10                  15
<210> 39
   <211> 63
   <212> DNA
   <213> Artificial
<220>
   <223> polynucléotide
<220>
   <221> CDS
   <222> (1)..(63)
<220>
   <221> misc_feature
   <222> (1)..(63)
   <223> Dérivé muté du domaine CD de la protéine TM du BLV : délétion des
   13 résidus N-terminaux et des 24 résidus C-terminaux
<400> 39
   gca ccc cac ttc cct gaa atc tcc ttc ccc cct aaa ccc gat tct gat       48
   Ala Pro His Phe Pro Glu Ile Ser Phe Pro Pro Lys Pro Asp Ser Asp
   1               5                   10                  15
   tat cag gcc ttg cta                                                   63
   Tyr Gln Ala Leu Leu
               20
<210> 40
   <211> 21
   <212> PRT
   <213> Artificial
<220>
   <223> Synthetic Construct
<400> 40
   Ala Pro His Phe Pro Glu Ile Ser Phe Pro Pro Lys Pro Asp Ser Asp
   1               5                   10                  15
   Tyr Gln Ala Leu Leu
               20
<210> 41
   <211> 78
   <212> DNA
   <213> Artificial
<220>
   <223> polynucléotide
<220>
   <221> CDS
   <222> (1)..(78)
<220>
   <221> misc_feature
   <222> (1)..(78)
   <223> Dérivé muté du domaine CD de la protéine TM du BLV : délétion des
   13 résidus N-terminaux et des 19 résidus C-terminaux
<400> 41
   gca ccc cac ttc cct gaa atc tcc ttc ccc cct aaa ccc gat tct gat      48
   Ala Pro His Phe Pro Glu Ile Ser Phe Pro Pro Lys Pro Asp Ser Asp
   1               5                   10                  15
   tat cag gcc ttg cta cca tcc gcg cca gag                              78
   Tyr Gln Ala Leu Leu Pro Ser Ala Pro Glu
               20                  25
<210> 42
   <211> 26
   <212> PRT
   <213> Artificial
<220>
   <223> Synthetic Construct
<400> 42
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu
               20                  25
<210> 43
   <211> 93
   <212> DNA
   <213> Artificial
<220>
   <223> polynucléotide
<220>
   <221> CDS
   <222> (1)..(93)
<220>
   <221> misc_feature
   <222> (1)..(93)
   <223> Dérivé muté du domaine CD de la protéine TM du BLV : délétion des
   13 résidus N-terminaux et des 14 résidus C-terminaux
<400> 43
   gca ccc cac ttc cct gaa atc tcc ttc ccc cct aaa ccc gat tct gat      48
   Ala Pro His Phe Pro Glu Ile Ser Phe Pro Pro Lys Pro Asp Ser Asp
   1               5                   10                  15
   tat cag gcc ttg cta cca tcc gcg cca gag atc tac tct cac ctc          93
   Tyr Gln Ala Leu Leu Pro Ser Ala Pro Glu Ile Tyr Ser His Leu
               20                  25                  30
<210> 44
   <211> 31
   <212> PRT
   <213> Artificial
<220>
   <223> Synthetic Construct
<400> 44
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu
               20                  25                  30
<210> 45
   <211> 111
   <212> DNA
   <213> Artificial
<220>
   <223> polynucléotide
<220>
   <221> CDS
   <222> (1)..(111)
<220>
   <221> misc_feature
   <222> (1)..(111)
   <223> Dérivé muté du domaine CD de la protéine TM du BLV : délétion des
   13 résidus N-terminaux et des 8 résidus C-terminaux
<400> 45
   gca ccc cac ttc cct gaa atc tcc ttc ccc cct aaa ccc gat tct gat       48
   Ala Pro His Phe Pro Glu Ile Ser Phe Pro Pro Lys Pro Asp Ser Asp
   1               5                   10                  15
   tat cag gcc ttg cta cca tcc gcg cca gag atc tac tct cac ctc tcc       96
   Tyr Gln Ala Leu Leu Pro Ser Ala Pro Glu Ile Tyr Ser His Leu Ser
               20                  25                  30
   ccc acc aaa ccc gat                                                  111
   Pro Thr Lys Pro Asp
           35
<210> 46
   <211> 37
   <212> PRT
   <213> Artificial
<220>
   <223> Synthetic Construct
<400> 46
   Ala Pro His Phe Pro Glu Ile Ser Phe Pro Pro Lys Pro Asp Ser Asp
   1               5                   10                  15
   Tyr Gln Ala Leu Leu Pro Ser Ala Pro Glu Ile Tyr Ser His Leu Ser
               20                  25                  30
   Pro Thr Lys Pro Asp
           35
<210> 47
   <211> 123
   <212> DNA
   <213> Artificial
<220>
   <223> polynucléotide
<220>
   <221> CDS
   <222> (1)..(123)
<220>
   <221> misc_feature
   <222> (1)..(123)
   <223> Dérivé muté du domaine CD de la protéine TM du BLV : délétion des
   13 résidus N-terminaux et des 4 résidus C-terminaux
<400> 47
   gca ccc cac ttc cct gaa atc tcc ttc ccc cct aaa ccc gat tct gat      48
   Ala Pro His Phe Pro Glu Ile Ser Phe Pro Pro Lys Pro Asp Ser Asp
   1               5                   10                  15
   tat cag gcc ttg cta cca tcc gcg cca gag atc tac tct cac ctc tcc      96
   Tyr Gln Ala Leu Leu Pro Ser Ala Pro Glu Ile Tyr Ser His Leu Ser
               20                  25                  30
   ccc acc aaa ccc gat tac atc aac ctt                                  123
   Pro Thr Lys Pro Asp Tyr Ile Asn Leu
           35                  40
<210> 48
   <211> 41
   <212> PRT
   <213> Artificial
<220>
   <223> Synthetic Construct
<400> 48
   Ala Pro His Phe Pro Glu Ile Ser Phe Pro Pro Lys Pro Asp Ser Asp
   1 5 10 15
   Tyr Gln Ala Leu Leu Pro Ser Ala Pro Glu Ile Tyr Ser His Leu Ser
   20 25 30
   Pro Thr Lys Pro Asp Tyr Ile Asn Leu
   35 40
<210> 49
   <211> 25
   <212> PRT
   <213> Homo sapiens
<220>
   <221> MISC_FEATURE
   <222> (1)..(24)
   <223> CD4
<400> 49
   Met Asn Arg Gly Val Pro Phe Arg His Leu Leu Leu Val Leu Gln Leu
   1 5 10 15
   Ala Leu Leu Pro Ala Ala Thr Gln Gly
   20 25
<210> 50
   <211> 22
   <212> PRT
   <213> Mus musculus
<220>
   <221> MISC_FEATURE
   <222> (1)..(22)
   <223> CD4
<400> 50
   Met Cys Arg Ala Ile Ser Leu Arg Arg Leu Leu Leu Leu Leu Leu Gln
   1 5 10 15
   Leu Ser Gln Leu Leu Ala
   20
<210> 51
   <211> 27
   <212> PRT
   <213> Mus musculus
<220>
   <221> MISC_FEATURE
   <222> (1)..(27)
   <223> CD8 alpha
<400> 51
   Met Ala Ser Pro Leu Thr Arg Phe Leu Ser Leu Asn Leu Leu Leu Leu
   1               5                   10                  15
   Gly Glu Ser Ile Ile Leu Gly Ser Gly Glu Ala
               20                  25
<210> 52
   <211> 25
   <212> PRT
   <213> Artificial
<220>
   <223> protéine CD8 alpha bovine
<400> 52
   Met Ala Ser Leu Leu Thr Ala Leu Ile Leu Pro Leu Ala Leu Leu Leu
   1               5                   10                  15
   Leu Asp Ala Ala Lys Val Leu Gly Ser
               20                  25
<210> 53
   <211> 21
   <212> PRT
   <213> Homo sapiens
<220>
   <221> MISC_FEATURE
   <222> (1)..(21)
   <223> CD8 alpha
<400> 53
   Met Ala Leu Pro Val Thr Ala Leu Leu Leu Pro Leu Ala Leu Leu Leu
   1               5                   10                  15
   His Ala Ala Arg Pro
               20
<210> 54
   <211> 26
   <212> PRT
   <213> Rattus norvegicus
<220>
   <221> MISC_FEATURE
   <222> (1)..(26)
   <223> CD8 alpha
<400> 54
   Met Ala Leu Pro Val Thr Ala Leu Leu Leu Pro Leu Ala Leu Leu Leu
   1               5                   10                  15
   His Ala Ala Arg Pro Ser Gln Phe Arg Val
               20                  25
<210> 55
   <211> 27
   <212> PRT
   <213> Homo sapiens
<220>
   <221> MISC_FEATURE
   <222> (1)..(27)
   <223> IL1R1
<400> 55
   Met Lys Val Leu Leu Arg Leu Ile Cys Phe Ile Ala Leu Leu Ile Ser
   1               5                   10                  15
   Ser Leu Glu Ala Asp Lys Cys Lys Glu Arg Glu
               20                  25
<210> 56
   <211> 24
   <212> PRT
   <213> Homo sapiens
<220>
   <221> MISC_FEATURE
   <222> (1)..(24)
   <223> EGFR 1, HER 1
<400> 56
   Met Arg Pro Ser Gly Thr Ala Gly Ala Ala Leu Leu Ala Leu Leu Ala
   1               5                   10                  15
   Ala Leu Cys Pro Ala Ser Arg Ala
               20
<210> 57
   <211> 22
   <212> PRT
   <213> Homo sapiens
<220>
   <221> MISC_FEATURE
   <222> (1)..(22)
   <223> HER 2
<400> 57
   Met Glu Leu Ala Ala Leu Cys Arg Trp Gly Leu Leu Leu Ala Leu Leu
   1 5 10 15
   Pro Pro Gly Ala Ala Ser
   20
<210> 58
   <211> 19
   <212> PRT
   <213> Homo sapiens
<220>
   <221> MISC_FEATURE
   <222> (1)..(19)
   <223> HER 3
<400> 58
   Met Arg Ala Asn Asp Ala Leu Gln Val Leu Gly Leu Leu Phe Ser Leu
   1 5 10 15
   Ala Arg Gly
<210> 59
   <211> 25
   <212> PRT
   <213> Homo sapiens
<220>
   <221> MISC_FEATURE
   <222> (1)..(25)
   <223> HER 4
<400> 59
   Met Lys Pro Ala Thr Gly Leu Trp Val Trp Val Ser Leu Leu Val Ala
   1 5 10 15
   Ala Gly Thr Val Gln Pro Ser Asp Ser
   20 25
<210> 60
   <211> 20
   <212> PRT
   <213> Mus musculus
<220>
   <221> MISC_FEATURE
   <222> (1)..(20)
   <223> IL-2
<400> 60
   Met Tyr Ser Met Gln Leu Ala Ser Cys Val Thr Leu Thr Leu Val Leu
   1               5                   10                  15
   Leu Val Asn Ser
               20
<210> 61
   <211> 24
   <212> PRT
   <213> Mus musculus
<220>
   <221> MISC_FEATURE
   <222> (1)..(24)
   <223> IL-6
<400> 61
   Met Lys Phe Leu Ser Ala Arg Asp Phe His Pro Val Ala Phe Leu Gly
     1               5                   10                  15
   Leu Met Leu Val Thr Thr Thr Ala
               20
<210> 62
   <211> 25
   <212> PRT
   <213> Homo sapiens
<220>
   <221> MISC_FEATURE
   <222> (1)..(25)
   <223> IL-7
<400> 62
   Met Phe His Val Ser Phe Arg Tyr Ile Phe Gly Leu Pro Pro Leu Ile
   1               5                   10                  15
   Leu Val Leu Leu Pro Val Ala Ser Ser
               20                  25
<210> 63
   <211> 18
   <212> PRT
   <213> Mus musculus
<220>
   <221> MISC_FEATURE
   <222> (1)..(18)
   <223> IL-10
<400> 63
   Met Pro Gly Ser Ala Leu Leu Cys Cys Leu Leu Leu Leu Thr Gly Met
   1 5 10 15
   Arg Ile
<210> 64
   <211> 23
   <212> PRT
   <213> Homo sapiens
<220>
   <221> MISC_FEATURE
   <222> (1)..(23)
   <223> MIP-1-alpha chimiokine
<400> 64
   Met Gln Val Ser Thr Ala Ala Leu Ala Val Leu Leu Cys Thr Met Ala
   1 5 10 15
   Leu Cys Asn Gln Phe Ser Ala
   20
<210> 65
   <211> 15
   <212> PRT
   <213> Influenza B virus
<220>
   <221> MISC_FEATURE
   <222> (1)..(15)
   <223> Hemagglutinine virus Influenza B
<400> 65
   Met Lys Ala Ile Ile Val Leu Leu Met Val Val Thr Ser Asn Ala
   1 5 10 15
<210> 66
   <211> 16
   <212> PRT
   <213> Influenza A virus
<220>
   <221> MISC_FEATURE
   <222> (1)..(16)
   <223> Hemagglutinine virus Influenza A H1N1
<400> 66
   Met Lys Ala Lys Leu Leu Val Leu Leu Cys Ala Leu Ser Ala Thr Asp
   1 5 10 15
<210> 67
   <211> 15
   <212> PRT
   <213> Influenza A virus
<220>
   <221> MISC_FEATURE
   <222> (1)..(15)
   <223> Hemagglutinine virus Influenza A H2N2
<400> 67
   Met Ala Ile Ile Tyr Leu Ile Leu Leu Phe Thr Ala Val Arg Gly
   1 5 10 15
<210> 68
   <211> 16
   <212> PRT
   <213> Influenza A virus
<220>
   <221> MISC_FEATURE
   <222> (1)..(16)
   <223> Hemagglutinine virus Influenza A H3N2
<400> 68
   Met Lys Thr Ile Ile Ala Leu Ser Tyr Ile Phe Cys Leu Ala Leu Gly
   1 5 10 15
<210> 69
   <211> 16
   <212> PRT
   <213> Influenza A virus
<220>
   <221> MISC_FEATURE
   <222> (1)..(16)
   <223> Hemagglutinine virus Influenza A H4N6
<400> 69
   Met Leu Ser Ile Val Ile Leu Phe Leu Leu Ile Ala Glu Asn Ser Ser
   1 5 10 15
<210> 70
   <211> 16
   <212> PRT
   <213> Influenza A virus
<220>
   <221> MISC_FEATURE
   <222> (1)..(16)
   <223> Hemagglutinine virus Influenza A H5N1
<400> 70
   Met Glu Lys Ile Val Leu Leu Leu Ala Ile Ile Ser Leu Val Lys Ser
   1 5 10 15
<210> 71
   <211> 16
   <212> PRT
   <213> Influenza A virus
<220>
   <221> MISC_FEATURE
   <222> (1)..(16)
   <223> Hemagglutinine virus Influenza A H6N5
<400> 71
   Met Ile Ala Ile Ile Val Val Ala Ile Leu Ala Thr Ala Gly Arg Ser
   1               5                   10                  15
<210> 72
   <211> 18
   <212> PRT
   <213> Influenza A virus
<220>
   <221> MISC_FEATURE
   <222> (1)..(18)
   <223> Hemagglutinine virus Influenza A H7N7
<400> 72
   Met Asn Thr Gln Ile Leu Ile Leu Thr Leu Val Ala Ala Ile His Thr
   1               5                   10                  15
   Asn Ala
<210> 73
   <211> 16
   <212> PRT
   <213> Influenza A virus
<220>
   <221> MISC_FEATURE
   <222> (1)..(16)
   <223> Hemagglutinine virus Influenza A H8N4
<400> 73
   Met Glu Lys Phe Ile Ala Ile Ala Thr Leu Ala Ser Thr Asn Ala Tyr
   1               5                   10                  15
<210> 74
   <211> 18
   <212> PRT
   <213> Influenza A virus
<220>
   <221> MISC_FEATURE
   <222> (1)..(18)
   <223> Hemagglutinine virus Influenza A H9N2
<400> 74
   Met Glu Thr Lys Ala Ile Ile Ala Ala Leu Leu Met Val Thr Ala Ala
   1 5 10 15
   Asn Ala
<210> 75
   <211> 16
   <212> PRT
   <213> Influenza A virus
<220>
   <221> MISC_FEATURE
   <222> (1)..(16)
   <223> Hemagglutinine virus Influenza A H10N7
<400> 75
   Met Tyr Lys Val Val Val Ile Ile Ala Leu Leu Gly Ala Val Lys Gly
   1 5 10 15
<210> 76
   <211> 16
   <212> PRT
   <213> Influenza A virus
<220>
   <221> MISC_FEATURE
   <222> (1)..(16)
   <223> Hemagglutinine virus Influenza A H11N6
<400> 76
   Met Glu Lys Thr Leu Leu Phe Ala Ala Ile Phe Leu Cys Val Lys Ala
   1 5 10 15
<210> 77
   <211> 17
   <212> PRT
   <213> Influenza A virus
<220>
   <221> MISC_FEATURE
   <222> (1)..(17)
   <223> Hemagglutinine virus Influenza A H12N5
<400> 77
   Met Glu Lys Phe Ile Ile Leu Ser Thr Val Leu Ala Ala Ser Phe Ala
   1 5 10 15
   Tyr
<210> 78
   <211> 18
   <212> PRT
   <213> Influenza A virus
<220>
   <221> MISC_FEATURE
   <222> (1)..(18)
   <223> Hemagglutinine virus Influenza A H13N6
<400> 78
   Met Ala Leu Asn Val Ile Ala Thr Leu Thr Leu Ile Ser Val Cys Val
   1               5                   10                  15
   His Ala
<210> 79
   <211> 246
   <212> DNA
   <213> Artificial
<220>
   <223> polynucléotide
<220>
   <221> misc_feature
   <222> (1) .. (246)
   <223> Construction X2
<220>
   <221> CDS
   <222> (1) .. (246)
<400> 79
   ctc att cat tct gtt cta agc cta ttc cta tta gcc ctt ttt ttg ctc       48
   Leu Ile His Ser Val Leu Ser Leu Phe Leu Leu Ala Leu Phe Leu Leu
   1               5                   10                  15
   ttc ttg gcc ccc tgc ctg ata aaa tgc ttg acc tct cgc ctt tta aaa       96
   Phe Leu Ala Pro Cys Leu Ile Lys Cys Leu Thr Ser Arg Leu Leu Lys
               20                  25                  30
   ctc ctc cgg cag gct ccc cac ttc cct gaa atc tcc ttc ccc cct aaa      144
   Leu Leu Arg Gln Ala Pro His Phe Pro Glu Ile Ser Phe Pro Pro Lys
           35                  40                  45
   ccc gat tct gat tat cag gcc ttg cta cca tcc gcg cca gag atc tac      192
   Pro Asp Ser Asp Tyr Gln Ala Leu Leu Pro Ser Ala Pro Glu Ile Tyr
       50                  55                  60
   tct cac ctc tcc ccc acc aaa ccc gat tac atc aac ctt cga ccc tgc      240
   Ser His Leu Ser Pro Thr Lys Pro Asp Tyr Ile Asn Leu Arg Pro Cys
   65                  70                  75                  80
   ccc tag                                                              246
   Pro
<210> 80
   <211> 81
   <212> PRT
   <213> Artificial
<220>
   <223> Synthetic Construct
<400> 80
   Leu Ile His Ser Val Leu Ser Leu Phe Leu Leu Ala Leu Phe Leu Leu
   1               5                   10                  15
   Phe Leu Ala Pro Cys Leu Ile Lys Cys Leu Thr Ser Arg Leu Leu Lys
               20                  25                  30
   Leu Leu Arg Gln Ala Pro His Phe Pro Glu Ile Ser Phe Pro Pro Lys
           35                  40                  45
   Pro Asp Ser Asp Tyr Gln Ala Leu Leu Pro Ser Ala Pro Glu Ile Tyr
       50                  55                  60
   Ser His Leu Ser Pro Thr Lys Pro Asp Tyr Ile Asn Leu Arg Pro Cys
   65                  70                  75                  80
   Pro
<210> 81
   <211> 246
   <212> DNA
   <213> Artificial
<220>
   <223> polynucléotide
<220>
   <221> misc_feature
   <222> (1)..(246)
   <223> Construction X3
<220>
   <221> CDS
   <222> (1) .. (246)
<400> 81
   att tac atc tgg gca ccc ttg gcc gga atc tgc gtg gcc ctt ctg cta        48
   Ile Tyr Ile Trp Ala Pro Leu Ala Gly Ile Cys Val Ala Leu Leu Leu
   1               5                   10                  15
   agc ttg atc ccc tgc ctg ata aaa tgc ctg acc tct cgc ctt tta aaa        96
   Ser Leu Ile Pro Cys Leu Ile Lys Cys Leu Thr Ser Arg Leu Leu Lys
               20                  25                  30
   ctc ctc cgg cag gct ccc cac ttc cct gaa atc tcc ttc ccc cct aaa       144
   Leu Leu Arg Gln Ala Pro His Phe Pro Glu Ile Ser Phe Pro Pro Lys
           35                  40                  45
   ccc gat tct gat tat cag gcc ttg cta cca tcc gcg cca gag atc tac       192
   Pro Asp Ser Asp Tyr Gln Ala Leu Leu Pro Ser Ala Pro Glu Ile Tyr
       50                  55                  60
   tct cac ctc tcc ccc acc aaa ccc gat tac atc aac ctt cga ccc tgc       240
   Ser His Leu Ser Pro Thr Lys Pro Asp Tyr Ile Asn Leu Arg Pro Cys
   65                  70                  75                  80
   ccc tag                                                               246
   Pro
<210> 82
   <211> 81
   <212> PRT
   <213> Artificial
<220>
   <223> Synthetic Construct
<400> 82
   Ile Tyr Ile Trp Ala Pro Leu Ala Gly Ile Cys Val Ala Leu Leu Leu
   1               5                   10                  15
   Ser Leu Ile Pro Cys Leu Ile Lys Cys Leu Thr Ser Arg Leu Leu Lys
               20                  25                  30
   Leu Leu Arg Gln Ala Pro His Phe Pro Glu Ile Ser Phe Pro Pro Lys
           35                  40                  45
   Pro Asp Ser Asp Tyr Gln Ala Leu Leu Pro Ser Ala Pro Glu Ile Tyr
       50                  55                  60
   Ser His Leu Ser Pro Thr Lys Pro Asp Tyr Ile Asn Leu Arg Pro Cys
   65                  70                  75                  80
   Pro
<210> 83
   <211> 225
   <212> DNA
   <213> Artificial
<220>
   <223> polynucléotide
<220>
   <221> misc_feature
   <222> (1)..(225)
   <223> Construction X4
<220>
   <221> CDS
   <222> (1)..(225)
<400> 83
   att tac atc tgg gca ccc ttg gcc gga atc tgc gtg gcc ctt ctg ctg        48
   Ile Tyr Ile Trp Ala Pro Leu Ala Gly Ile Cys Val Ala Leu Leu Leu
   1               5                   10                  15
   tcc ttg atc atc act ctc atc tgc tac cac agg tct aga gct ccc cac        96
   Ser Leu Ile Ile Thr Leu Ile Cys Tyr His Arg Ser Arg Ala Pro His
               20                  25                  30
   ttc cct gaa atc tcc ttc ccc cct aaa ccc gat tct gat tat cag gcc       144
   Phe Pro Glu Ile Ser Phe Pro Pro Lys Pro Asp Ser Asp Tyr Gln Ala
           35                  40                  45
   ttg cta cca tcc gcg cca gag atc tac tct cac ctc tcc ccc acc aaa       192
   Leu Leu Pro Ser Ala Pro Glu Ile Tyr Ser His Leu Ser Pro Thr Lys
       50                  55                  60
   ccc gat tac atc aac ctt cga ccc tgc ccc tag                           225
   Pro Asp Tyr Ile Asn Leu Arg Pro Cys Pro
   65                  70
<210> 84
   <211> 74
   <212> PRT
   <213> Artificial
<220>
   <223> Synthetic Construct
<400> 84
   Ile Tyr Ile Trp Ala Pro Leu Ala Gly Ile Cys Val Ala Leu Leu Leu
   1               5                   10                  15
   Ser Leu Ile Ile Thr Leu Ile Cys Tyr His Arg Ser Arg Ala Pro His
               20                  25                  30
   Phe Pro Glu Ile Ser Phe Pro Pro Lys Pro Asp Ser Asp Tyr Gln Ala
           35                  40                  45
   Leu Leu Pro Ser Ala Pro Glu Ile Tyr Ser His Leu Ser Pro Thr Lys
       50                  55                  60
   Pro Asp Tyr Ile Asn Leu Arg Pro Cys Pro
   65                  70
<210> 85
   <211> 947
   <212> DNA
   <213> Artificial
<220>
   <223> polynucléotide
<220>
   <221> misc_feature
   <222> (1)..(947)
   <223> chimère CD8 alpha de souris / domaine CD de TM du BLV
<220>
   <221> CDS
   <222> (28)..(831)
<400> 85
   ggatcccttg ctggtggaga gcacacc atg gcc tca ccg ttg acc cgc ttt ctg      54
                                 Met Ala Ser Pro Leu Thr Arg Phe Leu
                                 1               5
   tcg ctg aac ctg ctg ctg ctg ggt gag tcg att atc ctg ggg agt gga       102
   Ser Leu Asn Leu Leu Leu Leu Gly Glu Ser Ile Ile Leu Gly Ser Gly
   10                  15                  20                  25
   gaa gct aag cca cag gca ccc gaa ctc cga atc ttt cca aag aaa atg       150
   Glu Ala Lys Pro Gln Ala Pro Glu Leu Arg Ile Phe Pro Lys Lys Met
                   30                  35                  40
   gac gcc gaa ctt ggt cag aag gtg gac ctg gta tgt gaa gtg ttg ggg       198
   Asp Ala Glu Leu Gly Gln Lys Val Asp Leu Val Cys Glu Val Leu Gly
               45                  50                  55
   tcc gtt tcg caa gga tgc tct tgg ctc ttc cag aac tcc agc tcc aaa      246
   Ser Val Ser Gln Gly Cys Ser Trp Leu Phe Gln Asn Ser Ser Ser Lys
           60                  65                  70
   ctc ccc cag ccc acc ttc gtt gtc tat atg gct tca tcc cac aac aag      294
   Leu Pro Gln Pro Thr Phe Val Val Tyr Met Ala Ser Ser His Asn Lys
       75                  80                  85
   ata acg tgg gac gag aag ctg aat tcg tcg aaa ctg ttt tct gcc atg      342
   Ile Thr Trp Asp Glu Lys Leu Asn Ser Ser Lys Leu Phe Ser Ala Met
   90                  95                  100                 105
   agg gac acg aat aat aag tac gtt ctc acc ctg aac aag ttc agc aag      390
   Arg Asp Thr Asn Asn Lys Tyr Val Leu Thr Leu Asn Lys Phe Ser Lys
                   110                 115                 120
   gaa aac gaa ggc tac tat ttc tgc tca gtc atc agc aac tcg gtg atg      438
   Glu Asn Glu Gly Tyr Tyr Phe Cys Ser Val Ile Ser Asn Ser Val Met
               125                 130                 135
   tac ttc agt tct gtc gtg cca gtc ctt cag aaa gtg aac tct act act      486
   Tyr Phe Ser Ser Val Val Pro Val Leu Gln Lys Val Asn Ser Thr Thr
           140                 145                 150
   acc aag cca gtg ctg cga act ccc tca cct gtg cac cct acc ggg aca      534
   Thr Lys Pro Val Leu Arg Thr Pro Ser Pro Val His Pro Thr Gly Thr
       155                 160                 165
   tct cag ccc cag aga cca gaa gat tgt cgg ccc cgt ggc tca gtg aag      582
   Ser Gln Pro Gln Arg Pro Glu Asp Cys Arg Pro Arg Gly Ser Val Lys
   170                 175                 180                 185
   ggg acc gga ttg gac ttc gcc tgt gat att tac atc tgg gca ccc ttg      630
   Gly Thr Gly Leu Asp Phe Ala Cys Asp Ile Tyr Ile Trp Ala Pro Leu
                   190                 195                 200
   gcc gga atc tgc gtg gcc ctt ctg ctg tcc ttg atc atc act ctc atc      678
   Ala Gly Ile Cys Val Ala Leu Leu Leu Ser Leu Ile Ile Thr Leu Ile
               205                 210                 215
   tgc tac cac agg tct aga gct ccc cac ttc cct gaa atc tcc ttc ccc      726
   Cys Tyr His Arg Ser Arg Ala Pro His Phe Pro Glu Ile Ser Phe Pro
           220                 225                 230
   cct aaa ccc gat tct gat tat cag gcc ttg cta cca tcc gcg cca gag      774
   Pro Lys Pro Asp Ser Asp Tyr Gln Ala Leu Leu Pro Ser Ala Pro Glu
       235                 240                 245
   atc tac tct cac ctc tcc ccc acc aaa ccc gat tac atc aac ctt cga      822
   Ile Tyr Ser His Leu Ser Pro Thr Lys Pro Asp Tyr Ile Asn Leu Arg
   250                 255                 260                 265
   ccc tgc ccc taggaccccc atgtttcacg caccctcagg ctgtggtggg              871
   Pro Cys Pro
   gcactggctt agtggaatag tcagtgtacc atcacaagcc tcttcttgct gccagcaccg    931
   agttcgaagc ggccgc                                                    947
<210> 86
   <211> 268
   <212> PRT
   <213> Artificial
<220>
   <223> Synthetic Construct
<400> 86
   Met Ala Ser Pro Leu Thr Arg Phe Leu Ser Leu Asn Leu Leu Leu Leu
   1               5                   10                  15
   Gly Glu Ser Ile Ile Leu Gly Ser Gly Glu Ala Lys Pro Gln Ala Pro
               20                  25                  30
   Glu Leu Arg Ile Phe Pro Lys Lys Met Asp Ala Glu Leu Gly Gln Lys
           35                  40                  45
   Val Asp Leu Val Cys Glu Val Leu Gly Ser Val Ser Gln Gly Cys Ser
       50                  55                  60
   Trp Leu Phe Gln Asn Ser Ser Ser Lys Leu Pro Gln Pro Thr Phe Val
   65                  70                  75                  80
   Val Tyr Met Ala Ser Ser His Asn Lys Ile Thr Trp Asp Glu Lys Leu
                   85                  90                  95
   Asn Ser Ser Lys Leu Phe Ser Ala Met Arg Asp Thr Asn Asn Lys Tyr
               100                 105                 110
   Val Leu Thr Leu Asn Lys Phe Ser Lys Glu Asn Glu Gly Tyr Tyr Phe
           115                 120                 125
   Cys Ser Val Ile Ser Asn Ser Val Met Tyr Phe Ser Ser Val Val Pro
       130                 135                 140
   Val Leu Gln Lys Val Asn Ser Thr Thr Thr Lys Pro Val Leu Arg Thr
   145                 150                 155                 160
   Pro Ser Pro Val His Pro Thr Gly Thr Ser Gln Pro Gln Arg Pro Glu
                   165                 170                 175
   Asp Cys Arg Pro Arg Gly Ser Val Lys Gly Thr Gly Leu Asp Phe Ala
               180                 185                 190
   Cys Asp Ile Tyr Ile Trp Ala Pro Leu Ala Gly Ile Cys Val Ala Leu
           195                 200                 205
   Leu Leu Ser Leu Ile Ile Thr Leu Ile Cys Tyr His Arg Ser Arg Ala
       210                 215                 220
   Pro His Phe Pro Glu Ile Ser Phe Pro Pro Lys Pro Asp Ser Asp Tyr
   225 230 235 240
   Gln Ala Leu Leu Pro Ser Ala Pro Glu Ile Tyr Ser His Leu Ser Pro
   245 250 255
   Thr Lys Pro Asp Tyr Ile Asn Leu Arg Pro Cys Pro
   260 265
<210> 87
   <211> 12
   <212> DNA
   <213> Artificial
<220>
   <223> oligonucleotide
<220>
   <221> CDS
   <222> (1)..(12)
<400> 87
   cca tcc gcg cca 12
   Pro Ser Ala Pro
   1
<210> 88
   <211> 4
   <212> PRT
   <213> Artificial
<220>
   <223> Synthetic Construct
<400> 88
   Pro Ser Ala Pro
   1
<210> 89
   <211> 4
   <212> PRT
   <213> Artificial
<220>
   <223> polypeptide
<400> 89
   Pro Thr Ala Pro
   1
<210> 90
   <211> 12
   <212> DNA
   <213> Artificial
<220>
   <223> oligonucleotide
<220>
   <221> CDS
   <222> (1)..(12)
<400> 90
   tac atc aac ctt 12
   Tyr Ile Asn Leu
   1
<210> 91
   <211> 4
   <212> PRT
   <213> Artificial
<220>
   <223> Synthetic Construct
<400> 91
   Tyr Ile Asn Leu
   1
<210> 92
   <211> 15
   <212> DNA
   <213> Artificial
<220>
   <223> oligonucleotide
<220>
   <221> CDS
   <222> (1)..(15)
<400> 92
   gat tac atc aac ctt 15
   Asp Tyr Ile Asn Leu
   1 5
<210> 93
   <211> 5
   <212> PRT
   <213> Artificial
<220>
   <223> Synthetic Construct
<400> 93
   Asp Tyr Ile Asn Leu
   1 5
<210> 94
   <211> 60
   <212> DNA
   <213> Artificial
<220>
   <223> oligonucleotide
<220>
   <221> CDS
   <222> (1)..(60)
<220>
   <221> misc_feature
   <222> (1)..(60)
   <223> Dérivé muté du CD de la protéine TM du BLV
<400> 94
   cca tcc gcg cca gag atc tac tct cac ctc tcc ccc acc aaa ccc gat       48
   Pro Ser Ala Pro Glu Ile Tyr Ser His Leu Ser Pro Thr Lys Pro Asp
   1               5                   10                  15
   tac atc aac ctt                                                       60
   Tyr Ile Asn Leu
               20
<210> 95
   <211> 20
   <212> PRT
   <213> Artificial
<220>
   <223> Synthetic Construct
<400> 95
   Pro Ser Ala Pro Glu Ile Tyr Ser His Leu Ser Pro Thr Lys Pro Asp
   1               5                   10                  15
   Tyr Ile Asn Leu
               20
<210> 96
   <211> 12
   <212> DNA
   <213> Artificial
<220>
   <223> Polynucleotide
<220>
   <221> CDS
   <222> (1)..(12)
<400> 96
   tac tct cac ctc 12
   Tyr Ser His Leu
   1
<210> 97
   <211> 4
   <212> PRT
   <213> Artificial
<220>
   <223> Synthetic Construct
<400> 97
   Tyr Ser His Leu
   1
<210> 98
   <211> 40
   <212> DNA
   <213> Artificial
<220>
   <223> Amorce S vers A, sens
<400> 98
   ccctaaaccc gatgctgatt atcaggcgtt gctaccatcc 40
<210> 99
   <211> 40
   <212> DNA
   <213> Artificial
<220>
   <223> Amorce S vers A, anti-sens
<400> 99
   cgcggatggt agcaacgcct gataatcagc atcgggttta 40
<210> 100
   <211> 25
   <212> DNA
   <213> Artificial
<220>
   <223> Amorce D vers A, sens
<400> 100
   ccaccaagcc ggcatacatc aacct 25
<210> 101
   <211> 27
   <212> DNA
   <213> Artificial
<220>
   <223> Amorce D vers A, anti-sens
<400> 101
   tcgaaggttg atgtatgccg gcttggt 27
<210> 102
   <211> 25
   <212> DNA
   <213> Artificial
<220>
   <223> amorce E vers A, sens
<400> 102
   gctaccatcc gcgccagcga tctac 25
<210> 103
   <211> 23
   <212> DNA
   <213> Artificial
<220>
   <223> Amorce E vers A, anti-sens
<400> 103
   gtagatcgct ggcgcggatg gta 23
<210> 104
   <211> 16
   <212> PRT
   <213> Artificial
<220>
   <223> polypeptide - domaine de ciblage sous-membranaire issue d'une protéine Src
<400> 104
   Met Gly Ser Ser Lys Ser Lys Pro Lys Asp Pro Ser Gln Arg Arg Arg
   1               5                   10                  15
<210> 105
   <211> 23
   <212> PRT
   <213> Artificial
<220>
   <223> polypeptide - domaine de ciblage sous-membranaire issu d'une protéine Src
<400> 105
   Met Gly Ser Ser Lys Ser Lys Pro Lys Asp Pro Ser Gln Arg Arg Arg
   1               5                   10                  15
   Lys Ser Arg Gly Pro Gly Gly
               20
<210> 106
   <211> 7
   <212> PRT
   <213> Artificial
<220>
   <223> polypeptide - linker
<400> 106
   Pro Ala Gly Ser Gly Ala Pro
   1               5
<210> 107
   <211> 21
   <212> DNA
   <213> Artificial
<220>
   <223> oligonucléotide - linker
<400> 107
   cctgcaggaa gcggcgcgcc c 21
<210> 108
   <211> 36
   <212> DNA
   <213> Artificial
<220>
   <223> oligonucléotide
<400> 108
   gccaccatgg gcagcagcaa gagcaagccc aaggac 36
<210> 109
   <211> 39
   <212> DNA
   <213> Artificial
<220>
   <223> oligonucléotide
<400> 109
   cccagccagc gccgccgcaa gtctagaggc ccgggaggc 39
<210> 110
   <211> 22
   <212> DNA
   <213> Artificial
<220>
   <223> oligonucléotide
<400> 110
   gcctcccggg cctctagact tg 22
<210> 111
   <211> 35
   <212> DNA
   <213> Artificial
<220>
   <223> oligonucléotide
<400> 111
   cggcggcgct ggctggggtc cttgggcttg ctctt 35
<210> 112
   <211> 18
   <212> DNA
   <213> Artificial
<220>
   <223> oligonucléotide
<400> 112
   gctgctgccc atggtggc 18
<210> 113
   <211> 33
   <212> DNA
   <213> Artificial
<220>
   <223> oligonucléotide - R1Src
<400> 113
   gaattcgcca ccatgggcag cagcaagagc aag 33
<210> 114
   <211> 35
   <212> DNA
   <213> Artificial
<220>
   <223> oligonucléotide - SrcNhe 1
<400> 114
   catgctagcg ctgcctcccg ggcctctaga ctttc 35
<210> 115
   <211> 37
   <212> DNA
   <213> Artificial
<220>
   <223> oligonucléotide - NheSNAP
<400> 115
   ggaggcagcg ctagcatgga caaagactgc gaaatga 37
<210> 116
   <211> 35
   <212> DNA
   <213> Artificial
<220>
   <223> oligonucléotide - SNAPSbfAsc
<400> 116
   gcgcgccgct tcctgcagga cccagcccag gcttg 35
<210> 117
   <211> 36
   <212> DNA
   <213> Artificial
<220>
   <223> oligonucléotide - SbfscDCTM15
<400> 117
   cctgcaggaa gcggcgcgcc ccacttccct gaaatc 36
<210> 118
   <211> 33
   <212> DNA
   <213> Artificial
<220>
   <223> oligonucléotide - DCTM15Not
<400> 118
   gcggccgctt cgaactcggt gctggcagca aga 33
<210> 119
   <211> 268
   <212> DNA
   <213> Artificial
<220>
   <223> Polynucléotide - Domaine CD : SbfAscDCTM_M15 Not
<400> 119
   cctgcaggaa ggctggcgcg ccccacttcc ctgaaatctc cttcccccct aaacccgatt 60
   ctgattatca ggccttgcta ccatccgcgc cagagatcta ctctcacctc tcccccacca 120
   aacccgatta catcaacctt cgaccggcgc cctaggaccc ccatgtttca cgcaccctca 180
   ggctgtggtg gggcactggc ttagtggaat agtcagtgta ccatcacaag cctcttcttg 240
   ctgccagcac cgagttcgaa gcggccgc 268
<210> 120
   <211> 75
   <212> DNA
   <213> Artificial
<220>
   <223> Polynucléotide - Fragment Src
<400> 120
   gccaccatgg gcagcagcaa gagcaagccc aaggacccca gccagcgccg ccgcaagtct      60
   agaggcccgg gaggc                                                        75
<210> 121
   <211> 902
   <212> DNA
   <213> Artificial
<220>
   <223> Polynucléotide - Gène Src-SNAP-DCTM (SSC)
<220>
   <221> CDS
   <222> (13)..(789)
<400> 121
   gaattcgcca cc atg ggc agc agc aag agc aag ccc aag gac ccc agc cag      51
   cgc cgc cgc aag tct aga ggc ccg gga ggc agc gct agc atg gac aaa        99
   gac tgc gaa atg aag cgc acc acc ctg gat agc cct ctg ggc aag ctg       147
   gaa ctg tct ggg tgc gaa cag ggc ctg cac gag atc aag ctg ctg ggc       195
   aaa gga aca tct gcc gcc gac gcc gtg gaa gtg cct gcc cca gcc gcc       243
   gtg ctg ggc gga cca gag cca ctg atg cag gcc acc gcc tgg ctc aac       291
   gcc tac ttt cac cag cct gag gcc atc gag gag ttc cct gtg cca gcc       339
   ctg cac cac cca gtg ttc cag cag gag agc ttt acc cgc cag gtg ctg       387
   tgg aaa ctg ctg aaa gtg gtg aag ttc gga gag gtc atc agc tac cag       435
   cag ctg gcc gcc ctg gcc ggc aat ccc gcc gcc acc gcc gcc gtg aaa       483
   acc gcc ctg agc gga aat ccc gtg ccc att ctg atc ccc tgc cac cgg       531
   gtg gtg tct agc tct ggc gcc gtg ggg ggc tac gag ggc ggg ctc gcc       579
   gtg aaa gag tgg ctg ctg gcc cac gag ggc cac aga ctg ggc aag cct       627
   ggg ctg ggt cct gca gga agc ggc gcg ccc cac ttc cct gaa atc tcc       675
   ttc ccc cct aaa ccc gat tct gat tat cag gcc ttg cta cca tcc gcg       723
   cca gag atc tac tct cac ctc tcc ccc acc aaa ccc gat tac atc aac       771
   ctt cga ccg gcg ccc tag gacccccatg tttcacgcac cctcaggctg          819
   tggtggggca ctggcttagt ggaatagtca gtgtaccatc acaagcctct tcttgctgcc    879
   agcaccgagt tcgaagcggc cgc                                          902
<210> 122
   <211> 258
   <212> PRT
   <213> Artificial
<220>
   <223> Synthetic Construct
<400> 122
<210> 123
   <211> 872
   <212> DNA
   <213> Artificial
<220>
   <223> Polynucléotide - Gène D-SNAP-DCTM (DSC)
<220>
   <221> CDS
   <222> (13)..(759)
<400> 123
   gaattcgcca cc atg ggc agc agc aag agc aag tct aga ggc ccg gga ggc      51
                 Met Gly Ser Ser Lys Ser Lys Ser Arg Gly Pro Gly Gly
                 1               5                   10
   agc gct agc atg gac aaa gac tgc gaa atg aag cgc acc acc ctg gat        99
   Ser Ala Ser Met Asp Lys Asp Cys Glu Met Lys Arg Thr Thr Leu Asp
       15                  20                  25
   agc cct ctg ggc aag ctg gaa ctg tct ggg tgc gaa cag ggc ctg cac       147
   Ser Pro Leu Gly Lys Leu Glu Leu Ser Gly Cys Glu Gln Gly Leu His
   30                  35                  40                  45
   gag atc aag ctg ctg ggc aaa gga aca tct gcc gcc gac gcc gtg gaa       195
   Glu Ile Lys Leu Leu Gly Lys Gly Thr Ser Ala Ala Asp Ala Val Glu
                   50                  55                  60
   gtg cct gcc cca gcc gcc gtg ctg ggc gga cca gag cca ctg atg cag       243
   Val Pro Ala Pro Ala Ala Val Leu Gly Gly Pro Glu Pro Leu Met Gln
               65                  70                  75
   gcc acc gcc tgg ctc aac gcc tac ttt cac cag cct gag gcc atc gag       291
   Ala Thr Ala Trp Leu Asn Ala Tyr Phe His Gln Pro Glu Ala Ile Glu
           80                  85                  90
   gag ttc cct gtg cca gcc ctg cac cac cca gtg ttc cag cag gag agc       339
   Glu Phe Pro Val Pro Ala Leu His His Pro Val Phe Gln Gln Glu Ser
       95                  100                 105
   ttt acc cgc cag gtg ctg tgg aaa ctg ctg aaa gtg gtg aag ttc gga      387
   Phe Thr Arg Gln Val Leu Trp Lys Leu Leu Lys Val Val Lys Phe Gly
   110                 115                 120                 125
   gag gtc atc agc tac cag cag ctg gcc gcc ctg gcc ggc aat ccc gcc      435
   Glu Val Ile Ser Tyr Gln Gln Leu Ala Ala Leu Ala Gly Asn Pro Ala
                   130                 135                 140
   gcc acc gcc gcc gtg aaa acc gcc ctg agc gga aat ccc gtg ccc att      483
   Ala Thr Ala Ala Val Lys Thr Ala Leu Ser Gly Asn Pro Val Pro Ile
               145                 150                 155
   ctg atc ccc tgc cac cgg gtg gtg tct agc tct ggc gcc gtg ggg ggc      531
   Leu Ile Pro Cys His Arg Val Val Ser Ser Ser Gly Ala Val Gly Gly
           160                 165                 170
   tac gag ggc ggg ctc gcc gtg aaa gag tgg ctg ctg gcc cac gag ggc      579
   Tyr Glu Gly Gly Leu Ala Val Lys Glu Trp Leu Leu Ala His Glu Gly
       175                 180                 185
   cac aga ctg ggc aag cct ggg ctg ggt cct gca gga agc ggc gcg ccc      627
   His Arg Leu Gly Lys Pro Gly Leu Gly Pro Ala Gly Ser Gly Ala Pro
   190                 195                 200                 205
   cac ttc cct gaa atc tcc ttc ccc cct aaa ccc gat tct gat tat cag      675
   His Phe Pro Glu Ile Ser Phe Pro Pro Lys Pro Asp Ser Asp Tyr Gln
                   210                 215                 220
   gcc ttg cta cca tcc gcg cca gag atc tac tct cac ctc tcc ccc acc      723
   Ala Leu Leu Pro Ser Ala Pro Glu Ile Tyr Ser His Leu Ser Pro Thr
               225                 230                 235
   aaa ccc gat tac atc aac ctt cga ccg gcg ccc tag gacccccatg           769
   Lys Pro Asp Tyr Ile Asn Leu Arg Pro Ala Pro
           240                 245
   tttcacgcac cctcaggctg tggtggggca ctggcttagt ggaatagtca gtgtaccatc    829
   acaagcctct tcttgctgcc agcaccgagt tcgaagcggc cgc                      872
<210> 124
   <211> 248
   <212> PRT
   <213> Artificial
<220>
   <223> Synthetic Construct
<400> 124